# EUROPEAN PATENT APPLICATION

(11) **EP 4 491 624 A1**
(43) Date of publication of application: **15.01.2025**
(21) Application number: 23766957.7
(22) Date of filing: 10.03.2023
(51) Int. Cl.: C07D 403/14, A61K 31/4995, A61K 31/506, A61K 31/517, A61P 1/18, A61P 35/00, C07D 407/14, C07D 413/14, C07D 417/14, C07D 487/08

(54) **HETEROCYCLIC COMPOUND FOR INDUCING DEGRADATION OF G12D MUTANT KRAS PROTEIN**

(30) Priority: 11.03.2022 JP 2022038252; 09.08.2022 JP 2022126816
(71) Applicant: Astellas Pharma Inc., Chuo-ku Tokyo 103-8411 (JP)
(72) Inventor: YOSHINARI, Tomohiro, Tokyo 103-8411 (JP); KAWAGUCHI, Kenichi, Tokyo 103-8411 (JP); KAWAMINAMI, Eiji, Tokyo 103-8411 (JP); ISHIOKA, Hiroki, Tokyo 103-8411 (JP); WATANABE, Hideyuki, Tokyo 103-8411 (JP); IMAIZUMI, Tomoyoshi, Tokyo 103-8411 (JP); HAMAGUCHI, Hisao, Tokyo 103-8411 (JP); TAKAHASHI, Fumie, Tokyo 103-8411 (JP); MORIKAWA, Takahiro, Tokyo 103-8411 (JP); KURAMOTO, Kazuyuki, Tokyo 103-8411 (JP); NAGASHIMA, Takeyuki, Tokyo 103-8411 (JP); INAMURA, Kohei, Tokyo 103-8411 (JP)
(74) Representative: Potter Clarkson
(86) International application number: PCT/JP2023/009205
(87) International publication number: WO 2023/171781

(57) **Abstract**

To provide a compound useful as an active ingredient of a pharmaceutical composition for treating pancreatic cancer.

The present inventors have studied about a compound that is useful as an active ingredient of a pharmaceutical composition for treating pancreatic cancer and have found that heterocyclic compounds represented by the formula (I) and the formula (IA) have an excellent degradation-inducing action on a G12D mutant KRAS protein and a G12D mutant KRAS inhibition activity and can be used as a therapeutic agent for pancreatic cancer, thus completing the present invention. The heterocyclic compound or a salt thereof of the present invention can be used as a therapeutic agent for pancreatic cancer.

## Description

### TECHNICAL FIELD

The present invention relates to pharmaceutical compositions and, in particular, to a heterocyclic compound that is excellent in a degradation-inducing action on a G12D mutant KRAS protein and that is expected to be useful as a G12D mutant KRAS inhibitor and to be useful as an active ingredient of, for example, a pharmaceutical composition for treating pancreatic cancer.

### BACKGROUND ART

Pancreatic cancer mainly including pancreatic ductal adenocarcinoma is a cancer with a very poor prognosis having a five-years survival rate of 10% or less (CA Cancer J. Clin., 2016, 66, p.7-30), and about 460,000 new cases are reported per year in the world (CA Cancer J. Clin., 2018, 68, p.394-424). The most effective therapy for treating pancreatic cancer is a surgery. However, the cancer has often metastasized since early detection is difficult, and the therapeutic effect of a surgery cannot be expected in many cases. When the cancer is not treated by operation, chemotherapy or radiotherapy is adopted, but the survival rate is not so good. Today, the FOLFIRINOX therapy (multidrug treatment of three chemotherapy agents of 5-FU, irinotecan and oxaliplatin, plus levofolinate) is used as a standard therapy of pancreatic cancer. However, due to the strong toxicity, the subject patient has to be cautiously selected, for example, the therapy is to be applied only to patients of an ECOG performance status of 1 or less (J. Clin. Oncol., 2018, 36, p.2545-2556). As a molecular target drug, an epidermal growth factor receptor (EGFR) inhibitor, Erlotinib, has been approved in a combination therapy with Gemcitabine. However, the extension of the overall survival is only about two weeks as compared with Gemcitabine alone, and no satisfying therapeutic effect has been achieved. A highly effective therapeutic agent remains needed (J. Clin. Oncol., 2007, 25, p.1960-1966).

RAS proteins are low molecular weight guanosine triphosphate (GTP)-binding proteins of about 21 kDa constituted of 188-189 amino acids and include four main types of proteins (KRAS (KRAS 4A and KRAS 4B), NRAS and HRAS) produced by three genes of a KRAS gene, an NRAS gene and an HRAS gene. RAS proteins are divided into an active GTP-binding type and an inactive GDP-binding type. A RAS protein is activated by replacement of guanosine diphosphate (GDP) with GTP due to, for example, ligand stimulation to a membrane receptor, such as EGFR. The active RAS binds to effector proteins as much as twenty, such as RAF, PI3K and RALGDS, to activate the downstream signal cascade. On the other hand, the active RAS is converted to the inactive type by replacement of GTP with GDP due to the intrinsic GTP hydrolysis (GTPase) activity. The GTPase activity is enhanced by a GTPase-activating protein (GAP). As can be seen from the above statement, RAS bears an important function of "molecular switch" in an intracellular signal transduction pathway for EGFR or the like and plays a critical role in the processes of cell growth, proliferation, angiogenesis and the like (Nature Rev. Cancer, 2011, 11, p.761-774, Nature Rev. Drug Discov., 2014, 13, p.828-851, Nature Rev. Drug Discov., 2016, 15, p.771-785).

Substitution of an amino acid by spontaneous mutation of the RAS gene results in a constant activated state due to hypofunction of RAS as GTPase or hyporeactivity to GAP, and then, signals are continuously sent downstream. The excessive signaling causes carcinogenesis or cancer growth acceleration. It is said that pancreatic ductal adenocarcinoma occurs through a weakly heteromorphic stage and a subsequent highly heteromorphic stage in the pancreatic intraepithelial neoplasia (PanIN), and mutation of the KRAS gene has already been recognized in an initial stage of PanIN. Subsequently, abnormality occurs in INK4A, p53 and SMAD4, which are tumor suppression genes, leading to malignancy (Nature Rev. Cancer, 2010, 10, p.683-695). Furthermore, in 90% or more of the cases of pancreatic ductal adenocarcinoma, mutation is seen in the KRAS gene, and a majority of them are a spontaneous point mutation in the codon 12 located in the KRAS exon 2 (Cancer Cell 2017, 32, p.185-203). As can be seen from the above statement, KRAS plays a critical role in the processes of carcinogenesis and development of pancreatic cancer.

As a mutation of a KRAS gene, KRAS G12C mutation, KRAS G12D mutation and the like are known. G12C mutant KRAS frequently occurs in non-small-cell lung cancer but occurs few percent in pancreatic cancer (Cancer Cell 2014, 25, p.272-281), and a therapeutic agent against another KRAS mutation is desired. G12D mutant KRAS is seen in about 34% of the cases of pancreatic cancer, and this rate is reported to be the highest in KRAS mutations (Nat. Rev. Cancer, 2018, 18, p.767-777).

Patent Documents 1, 2 and 3 disclose RAS inhibitors, and Patent Documents 2 and 3 disclose compounds represented by the following formula (A) and formula (B), respectively (the meanings of the symbols in the formulae can be found in the patent documents). Patent Documents 1, 2 and 3 state that the compounds are useful for a cancer with a mutation in the codon 12 of KRAS. The G12D mutation is one of such mutations, but any effect on the G12D mutant KRAS cancer is not described.

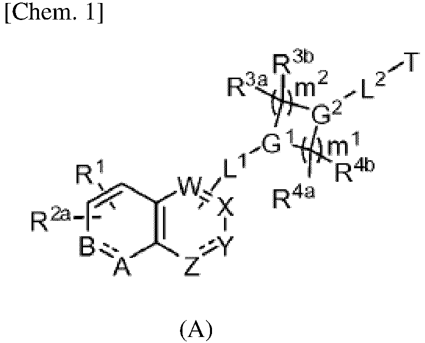

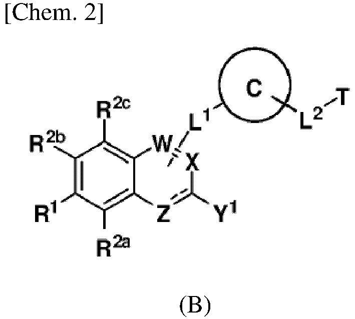

Moreover, Patent Documents 9, 10 and 11 disclose a KRAS G12D inhibitor.

In recent years, as a technique for inducing degradation of a target protein, bifunctional compounds collectively called as PROTAC (PROteolysis-TArgeting Chimera) or SNIPER (Specific and Nongenetic IAP-dependent Protein Eraser) are found and are expected as one novel technique of drug development modality (Drug. Discov. Today Technol., 2019, 31, p15-27). Such a bifunctional compound promotes formation of a composite of the target protein and an E3 ligase in a cell, and degradation of the target protein is induced using the ubiquitin-proteasome system. The ubiquitin-proteasome system is one of intracellular protein degradation mechanisms. A protein called E3 ligase recognizes a protein to be degraded to convert the protein into ubiquitin, whereby degradation by proteasome is promoted.

600 or more E3 ligases are present in an organism and are roughly divided into four types of HECT-domain E3s, U-box E3s, monomeric RING E3s and multi-subunit E3s. E3 ligases used as a bifunctional degradation inducer which are called PROTAC, SNIPER or the like are currently limited, and typical examples thereof include Von Hippel-Lindau (VHL), celebron (CRBN), inhibitor of apoptosis protein (IAP) and mouse double minute 2 homolog (MDM2). In particular, VHL is reported in Patent Document 4, and CRBN is reported in Patent Document 5.

The bifunctional compounds are compounds in which a ligand of a target protein and a ligand of an E3 ligase are bound via a linker, and some bifunctional compounds for degrading a KRAS protein have ever been reported (Non-patent Document 1, Non-patent Document 2, Patent Document 6, Patent Document 7, Patent Document 8, Patent Document 12 and Patent Document 13). Furthermore, bifunctional compounds for reducing G12D mutant KRAS protein levels have been reported in Patent Document 14 and Patent Document 15, and quinazoline compounds for inducing degradation of G12D mutant KRAS protein have been reported in Patent Document 16. However, there is no report suggesting that a bifunctional compound degrades the G12D mutant KRAS protein other than Patent Documents 14 to 16 as of now.

### CITATION LIST

### PATENT DOCUMENT

[Patent Document 1] WO 2016/049565
[Patent Document 2] WO 2016/049568
[Patent Document 3] WO 2017/172979
[Patent Document 4] WO 2013/106643
[Patent Document 5] WO 2015/160845
[Patent Document 6] US Patent Application Publication No. 2018/0015087
[Patent Document 7] WO 2019/195609
[Patent Document 8] WO 2020/018788
[Patent Document 9] WO 2021/041671
[Patent Document 10] WO 2021/106231
[Patent Document 11] WO 2021/107160
[Patent Document 12] WO 2021/051034
[Patent Document 13] WO 2021/207172
[Patent Document 14] WO 2022/148421
[Patent Document 15] WO 2022/148422
[Patent Document 16] WO 2022/173032

### NON-PATENT DOCUMENT

[Non-patent Document 1] Cell. Chem. Biol., 2020, 27, p.19-31
[Non-patent Document 2] ACS Cent. Sci., 2020, 6, p.1367-1375

### SUMMARY OF INVENTION

### TECHNICAL PROBLEM

A pharmaceutical composition, for example, a heterocyclic compound that is excellent in a degradation-inducing action on a G12D mutant KRAS protein and that is expected to be useful as a G12D mutant KRAS inhibitor and to be useful as an active ingredient of a pharmaceutical composition for treating pancreatic cancer, in particular, G12D mutant KRAS-positive pancreatic cancer, is provided.

### SOLUTION TO PROBLEM

The present inventors have intensively and extensively studied about a compound that is useful as an active ingredient of a pharmaceutical composition for treating pancreatic cancer. As a result, the present inventors have found that heterocyclic compounds of a formula (I) and a formula (IA), in particular, bifunctional compounds of the formula (I) and the formula (IA) characterized in that a substituent on the 8-position of a heterocyclic compound selected from the group consisting of quinazoline and quinoline is bound to a ligand of an E3 ligase or that a substituent on the 8-position of a heterocyclic compound selected from the group consisting of quinazoline and quinoline is bound to a ligand of an E3 ligase via a linker, have an excellent degradation-inducing action on a G12D mutant KRAS protein and a G12D mutant KRAS inhibition activity, thus completing the present invention.

Specifically, the present invention relates to a compound of the formula (I) or a salt thereof and a pharmaceutical composition that contains a compound of the formula (I) or a salt thereof and one or more pharmaceutically acceptable excipients. 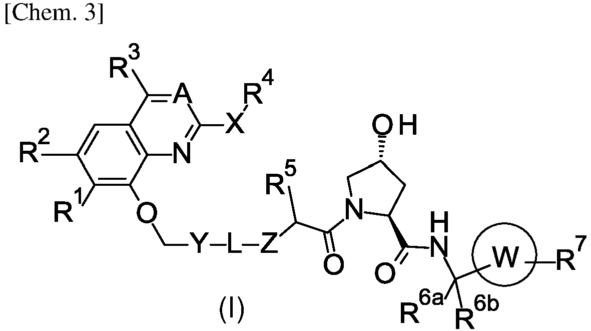 (In the formula,
A is CR^{A} or N,
R^{A} is H or C₁₋₃ alkyl, and
R¹ is naphthyl optionally substituted with OH or a group selected from the group consisting of the formula (II), the formula (III) and the formula (IV) below, 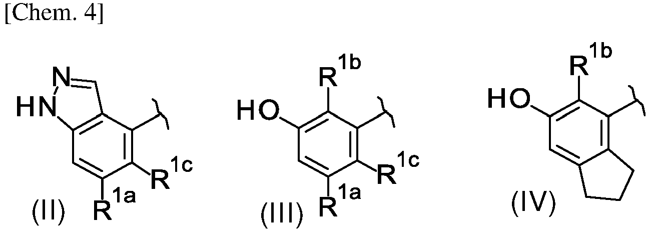
R^{1a} and R^{1b}, which are the same as or different from each other, are H, methyl, F or Cl,
R^{1c} is F, Cl, methyl or ethyl,
R² is H, halogen, optionally substituted C₁₋₃ alkyl, cyclopropyl or vinyl,
R³ is -P-Q or V,
P is -CH₂-, -O- or -N(R^{P})-,
R^{P} is H or optionally substituted C₁₋₃ alkyl, and
Q is the formula (V) or the formula (VI) below, 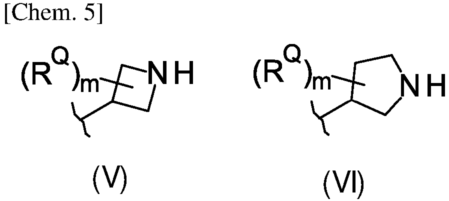
V is the formula (VII) below, 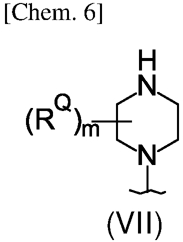
each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
m is 0 to 2,
R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms,
R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen,
R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen,
R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms,
W is optionally substituted phenylene or 6-membered optionally substituted heteroarenediyl containing one to three nitrogen atoms,
X is a bond, -CH₂-, -O-, -S- or -NR^{4x}-,
R^{4x} is H or C₁₋₃ alkyl,
Y is phenylene or pyridinediyl, wherein the phenylene is optionally substituted with F,
L is -(L¹-L²-L³-L⁴)-,
L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
R^{L1} is H or C₁₋₃ alkyl,
Z is NH or 5-membered heteroarenediyl containing one to four hetero atoms selected from the group consisting of oxygen, sulfur and nitrogen,
or Y-L-Z is the formula (VIII) below.)

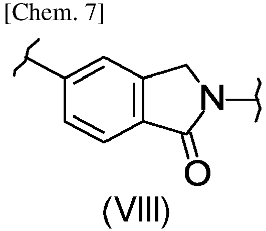

The present invention also relates to the compound of the formula (IA) or a salt thereof and a pharmaceutical composition that contains the compound of the formula (IA) or a salt thereof and one or more pharmaceutically acceptable excipients. 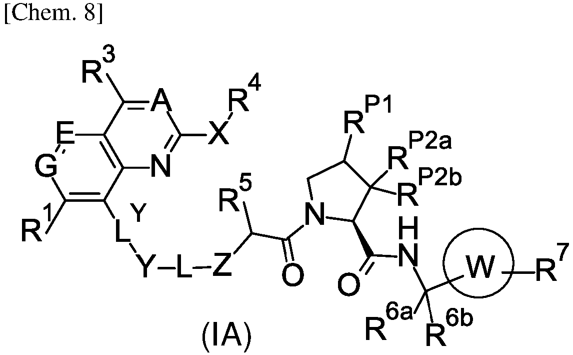 (In the formula,
A is CR^{A} or N,
R^{A} is H or C₁₋₃ alkyl,
E is CH or N,
G is CR² or N, and
R¹ is naphthyl optionally substituted with OH or a group selected from the group consisting of the formula (II), the formula (III) and the formula (IV) below, 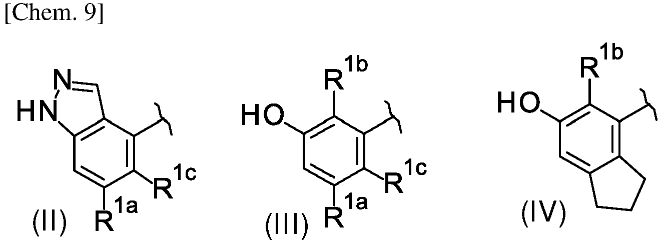
R^{1a} and R^{1b}, which are the same as or different from each other, are H, methyl, For Cl,
R^{1c} is F, Cl, methyl or ethyl,
R² is H, halogen, optionally substituted C₁₋₃ alkyl, cyclopropyl or vinyl,
R³ is -P-Q or V,
P is -CH₂-, -O- or -N(R^{P})-,
R^{P} is H or optionally substituted C₁₋₃ alkyl, and
Q is the formula (V) or the formula (VI) below, 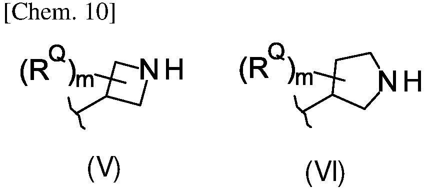
V is the formula (VII) below, 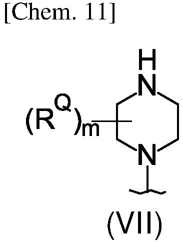
each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
(i) when A is CR^{A} or (ii) when A is N, and E or G is N,
   V may be a 7-membered or 8-membered saturated or unsaturated bridged heterocyclic group in addition to the formula (VII),
m is 0 to 2,
R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms,
R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen,
R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen,
R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms,
L^{Y} is -O-(optionally substituted C₁₋₃ alkylene)-, -S-(optionally substituted C₁₋₃ alkylene)-, -SO₂-(optionally substituted C₁₋₃ alkylene)-, -NR^{Y}-(optionally substituted C₁₋₃ alkylene)-, -(optionally substituted C₁₋₃ alkylene)-O-, -(optionally substituted C₁₋₃ alkylene)-S-, -(optionally substituted C₁₋₃ alkylene)-SO₂- or -(optionally substituted C₁₋₃ alkylene)-NR^{Y}-,
R^{Y} is H or C₁₋₃ alkyl,
R^{P1} is OH or F,
R^{P2a} is H or F,
R^{P2b} is H,
W is optionally substituted phenylene or 6-membered optionally substituted heteroarenediyl containing one to three nitrogen atoms,
X is a bond, -CH₂-, -O-, -S- or -NR^{4x}-,
R^{4x} is H or C₁₋₃ alkyl,
Y is phenylene or pyridinediyl, wherein the phenylene is optionally substituted with F,
L is -(L¹-L²-L³-L⁴)-,
L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
R^{L1} is H or C₁₋₃ alkyl,
Z is NH or 5-membered heteroarenediyl containing one to four hetero atoms selected from the group consisting of oxygen, sulfur and nitrogen,
or Y-L-Z is the formula (VIII) below.)

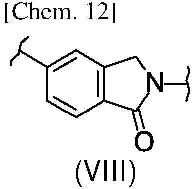

The compound of the formula (IA) includes the compound of the formula (I).

Note that, when a symbol in a chemical formula herein is used in another chemical formula, the same symbol represents the same meaning unless otherwise specified.

The present invention also relates to a pharmaceutical composition containing the compound of the formula (I) or a salt thereof and one or more pharmaceutically acceptable excipients, in one embodiment, a pharmaceutical composition for treating pancreatic cancer, in one embodiment, a pharmaceutical composition for treating G12D mutant KRAS-positive pancreatic cancer, in one embodiment, a pharmaceutical composition for treating metastatic pancreatic cancer, in one embodiment, a pharmaceutical composition for treating locally advanced pancreatic cancer, in one embodiment, a pharmaceutical composition for treating recurrent or refractory pancreatic cancer, in one embodiment, a pharmaceutical composition for treating pancreatic cancer of a patient who is untreated and/or has a treatment history, in one embodiment, a pharmaceutical composition for treating metastatic G12D mutant KRAS-positive pancreatic cancer, in one embodiment, a pharmaceutical composition for treating locally advanced G12D mutant KRAS-positive pancreatic cancer, in one embodiment, a pharmaceutical composition for treating recurrent or refractory G12D mutant KRAS-positive pancreatic cancer, in one embodiment, a pharmaceutical composition for treating G12D mutant KRAS-positive pancreatic cancer of a patient who is untreated and/or has a treatment history. Note that the pharmaceutical composition includes a therapeutic agent containing the compound of the formula (I) or a salt thereof for pancreatic cancer, G12D mutant KRAS-positive pancreatic cancer in one embodiment.

The present invention also relates to use of the compound of the formula (I) or a salt thereof for the manufacture of a pharmaceutical composition for treating pancreatic cancer, in one embodiment, G12D mutant KRAS-positive pancreatic cancer, in one embodiment, metastatic pancreatic cancer, in one embodiment, locally advanced pancreatic cancer, in one embodiment, recurrent or refractory pancreatic cancer, in one embodiment, pancreatic cancer of a patient who is untreated and/or has a treatment history, in one embodiment, metastatic G12D mutant KRAS-positive pancreatic cancer, in one embodiment, locally advanced G12D mutant KRAS-positive pancreatic cancer, in one embodiment, recurrent or refractory G12D mutant KRAS-positive pancreatic cancer, in one embodiment, G12D mutant KRAS-positive pancreatic cancer of a patient who is untreated and/or has a treatment history, to use of the compound of the formula (I) or a salt thereof for treating pancreatic cancer, in one embodiment, G12D mutant KRAS-positive pancreatic cancer, to the compound of the formula (I) or a salt thereof for use in treatment of pancreatic cancer, in one embodiment, G12D mutant KRAS-positive pancreatic cancer and to a method for treating pancreatic cancer, in one embodiment, G12D mutant KRAS-positive pancreatic cancer, the method comprising administering an effective amount of the compound of the formula (I) or a salt thereof to a subject.

The present invention also relates to the compound of the formula (I) or a salt thereof that is a G12D mutant KRAS protein degradation inducer and/or a G12D mutant KRAS inhibitor, to the compound of the formula (I) or a salt thereof for use as a G12D mutant KRAS protein degradation inducer and/or a G12D mutant KRAS inhibitor and to a G12D mutant KRAS protein degradation inducer and/or a G12D mutant KRAS inhibitor comprising the compound of the formula (I) or a salt thereof.

Note that the "subject" is a human or another animal that needs the treatment, and in one embodiment, the "subject" is a human who needs the prevention or treatment.

The present invention also relates to a pharmaceutical composition containing the compound of the formula (IA) or a salt thereof and one or more pharmaceutically acceptable excipients, in one embodiment, a pharmaceutical composition for treating pancreatic cancer, in one embodiment, a pharmaceutical composition for treating G12D mutant KRAS-positive pancreatic cancer, in one embodiment, a pharmaceutical composition for treating metastatic pancreatic cancer, in one embodiment, a pharmaceutical composition for treating locally advanced pancreatic cancer, in one embodiment, a pharmaceutical composition for treating recurrent or refractory pancreatic cancer, in one embodiment, a pharmaceutical composition for treating pancreatic cancer of a patient who is untreated and/or has a treatment history, in one embodiment, a pharmaceutical composition for treating metastatic G12D mutant KRAS-positive pancreatic cancer, in one embodiment, a pharmaceutical composition for treating locally advanced G12D mutant KRAS-positive pancreatic cancer, in one embodiment, a pharmaceutical composition for treating recurrent or refractory G12D mutant KRAS-positive pancreatic cancer, in one embodiment, a pharmaceutical composition for treating G12D mutant KRAS-positive pancreatic cancer of a patient who is untreated and/or has a treatment history. Note that the pharmaceutical composition includes a therapeutic agent containing the compound of the formula (IA) or a salt thereof for pancreatic cancer, G12D mutant KRAS-positive pancreatic cancer in one embodiment.

The present invention also relates to use of the compound of the formula (IA) or a salt thereof for the manufacture of a pharmaceutical composition for treating pancreatic cancer, in one embodiment, G12D mutant KRAS-positive pancreatic cancer, in one embodiment, metastatic pancreatic cancer, in one embodiment, locally advanced pancreatic cancer, in one embodiment, recurrent or refractory pancreatic cancer, in one embodiment, pancreatic cancer of a patient who is untreated and/or has a treatment history, in one embodiment, metastatic G12D mutant KRAS-positive pancreatic cancer, in one embodiment, locally advanced G12D mutant KRAS-positive pancreatic cancer, in one embodiment, recurrent or refractory G12D mutant KRAS-positive pancreatic cancer, in one embodiment, G12D mutant KRAS-positive pancreatic cancer of a patient who is untreated and/or has a treatment history, to use of the compound of the formula (IA) or a salt thereof for treating pancreatic cancer, in one embodiment, G12D mutant KRAS-positive pancreatic cancer, to the compound of the formula (IA) or a salt thereof for use in treatment of pancreatic cancer, in one embodiment, G12D mutant KRAS-positive pancreatic cancer and to a method for treating pancreatic cancer, in one embodiment, G12D mutant KRAS-positive pancreatic cancer, the method comprising administering an effective amount of the compound of the formula (IA) or a salt thereof to a subject.

The present invention also relates to the compound of the formula (IA) or a salt thereof that is a G12D mutant KRAS protein degradation inducer and/or a G12D mutant KRAS inhibitor, to the compound of the formula (IA) or a salt thereof for use as a G12D mutant KRAS protein degradation inducer and/or a G12D mutant KRAS inhibitor and to a G12D mutant KRAS protein degradation inducer and/or a G12D mutant KRAS inhibitor comprising the compound of the formula (IA) or a salt thereof.

Note that the "subject" is a human or another animal that needs the treatment, and in one embodiment, the "subject" is a human who needs the prevention or treatment.

### ADVANTAGEOUS EFFECTS OF INVENTION

The compound of the formula (I) or the formula (IA) or a salt thereof has a degradation-inducing action on a G12D mutant KRAS protein and a G12D mutant KRAS inhibition activity and can be used as a therapeutic agent for pancreatic cancer, in particular, G12D mutant KRAS-positive pancreatic cancer.

### DESCRIPTION OF EMBODIMENTS

The present invention will be described in detail below.

As used herein, "optionally substituted" means being unsubstituted or having one to five substituents. In one embodiment, the "optionally substituted" means being unsubstituted or having one to three substituents. Note that when there are multiple substituents, the substituents may be the same as or different from each other.

"C₁₋₁₂ Alkyl" is linear or branched alkyl having 1 to 12 carbon atoms, and examples thereof include methyl, ethyl, n-propyl, isopropyl, n-butyl, isobutyl, sec-butyl, tert-butyl, n-pentyl, n-hexyl, dodecyl and the like (the carbon atom numbers are described similarly hereinafter). The "C₁₋₁₂ alkyl" is ethyl or dodecyl in one embodiment.

Similarly, "C₁₋₆ alkyl" is linear or branched alkyl having 1 to 6 carbon atoms, and examples thereof include methyl, ethyl, n-propyl, isopropyl, n-butyl, isobutyl, sec-butyl, tert-butyl, n-pentyl and n-hexyl. The "C₁₋₆ alkyl" is methyl, ethyl, n-propyl, isopropyl or sec-butyl in one embodiment, methyl, ethyl, isopropyl or tert-butyl in one embodiment or methyl, ethyl, n-propyl, isopropyl or n-butyl in one embodiment.

Similarly, "C₁₋₃ alkyl" is linear or branched alkyl having 1 to 3 carbon atoms, and examples thereof include methyl, ethyl, n-propyl, isopropyl. The "C₁₋₃ alkyl" is methyl or ethyl in one embodiment, n-propyl or isopropyl in one embodiment, methyl or isopropyl in one embodiment, ethyl or isopropyl in one embodiment, methyl in one embodiment, ethyl in one embodiment, isopropyl in one embodiment or n-propyl in one embodiment.

"C₃₋₆ Cycloalkyl" is cycloalkyl having 3 to 6 carbon atoms, and examples thereof include cyclopropyl, cyclobutyl, cyclopentyl and cyclohexyl. The "C₃₋₆ cycloalkyl" is cyclobutyl, cyclopentyl or cyclohexyl in one embodiment, cyclobutyl or cyclopentyl in one embodiment, cyclopentyl or cyclohexyl in one embodiment, cyclopropyl or cyclobutyl in one embodiment, cyclopropyl in one embodiment, cyclobutyl in one embodiment, cyclopentyl in one embodiment or cyclohexyl in one embodiment.

"C₁₋₃ Alkylene" is a divalent group formed by removal of a hydrogen atom from C₁₋₃ alkyl and is linear or branched C₁₋₃ alkylene, and examples thereof include methylene, ethylene, trimethylene, methylmethylene, 1,1-dimethylmethylene and the like. The "C₁₋₃ alkylene" is linear or branched C₁₋₃ alkylene in one embodiment, methylene, ethylene or trimethylene in one embodiment, methylene or ethylene in one embodiment, methylene in one embodiment or ethylene in one embodiment.

"Saturated heterocyclic group" is a saturated hydrocarbon ring group containing a hetero atom selected from the group consisting of oxygen, sulfur and nitrogen as ring-forming atom. Further, the sulfur atom as a ring-forming atom of the saturated heterocyclic group is optionally oxidized.

Therefore, "4-membered to 6-membered saturated heterocyclic group" is a 4-membered to 6-membered saturated heterocyclic group containing a hetero atom selected from the group consisting of oxygen, sulfur and nitrogen as a ring-forming atom. The "4-membered to 6-membered saturated heterocyclic group" in one embodiment is a 4-membered to 6-membered saturated heterocyclic group containing one or two hetero atoms selected from the group consisting of oxygen, sulfur and nitrogen as ring-forming atoms. The 4-membered to 6-membered saturated heterocyclic group containing one or two hetero atoms selected from the group consisting of oxygen, sulfur and nitrogen as ring-forming atoms in one embodiment is a 4-membered to 6-membered saturated heterocyclic group containing one hetero atom selected from the group consisting of oxygen, sulfur and nitrogen as a ring-forming atom, a 5-membered or 6-membered saturated heterocyclic group containing one or two hetero atoms selected from the group consisting of oxygen, sulfur and nitrogen as ring-forming atoms in one embodiment, a 4-membered saturated heterocyclic group containing one hetero atom selected from the group consisting of oxygen, sulfur and nitrogen as a ring-forming atom in one embodiment, a 5-membered saturated heterocyclic group containing one or two hetero atoms selected from the group consisting of oxygen, sulfur and nitrogen as ring-forming atoms in one embodiment, a 6-membered saturated heterocyclic group containing one or two hetero atoms selected from the group consisting of oxygen, sulfur and nitrogen as ring-forming atoms in one embodiment, oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl or dioxothiomorpholinyl in one embodiment, oxetanyl, tetrahydrofuranyl, tetrahydropyranyl, azetidinyl, pyrrolidinyl, piperidinyl, piperazinyl, morpholinyl, thiomorpholinyl or dioxothiomorpholinyl in one embodiment, oxetanyl, tetrahydrofuranyl, tetrahydropyranyl, pyrrolidinyl, piperidinyl or morpholinyl in one embodiment, oxetanyl, tetrahydrofuranyl, tetrahydropyranyl, pyrrolidinyl or piperidinyl in one embodiment, oxetanyl, tetrahydrofuranyl or tetrahydropyranyl in one embodiment, pyrrolidinyl or piperidinyl in one embodiment, oxetanyl in one embodiment, tetrahydrofuranyl in one embodiment, tetrahydropyranyl in one embodiment, pyrrolidinyl in one embodiment, piperidinyl in one embodiment, morpholinyl in one embodiment or oxazolidinyl in one embodiment.

"Heteroaryl" is an unsaturated heterocyclic group containing a hetero atom selected from the group consisting of oxygen, sulfur and nitrogen as a ring-forming atom.

Therefore, "5-membered heteroaryl" is a 5-membered unsaturated heterocyclic group containing one to four hetero atoms selected from the group consisting of oxygen, sulfur and nitrogen as ring-forming atoms.

The "5-membered heteroaryl" is a 5-membered unsaturated heterocyclic group containing one to three hetero atoms selected from the group consisting of oxygen, sulfur and nitrogen as ring-forming atoms in one embodiment, pyrazolyl, imidazolyl, triazolyl, tetrazolyl, oxazolyl, isoxazolyl, thiazolyl, isothiazolyl, oxadiazolyl or thiadiazolyl in one embodiment, pyrazolyl, imidazolyl, triazolyl, oxazolyl or thiazolyl in one embodiment, pyrazolyl, imidazolyl, oxazolyl or thiazolyl in one embodiment, pyrazolyl, imidazolyl, triazolyl or isoxazolyl in one embodiment, pyrazolyl, oxazolyl or thiazolyl in one embodiment, pyrazolyl, triazolyl or isoxazolyl in one embodiment, pyrazolyl or thiazolyl in one embodiment, pyrazolyl or triazolyl in one embodiment, pyrazolyl in one embodiment, imidazolyl in one embodiment, oxazolyl in one embodiment, thiazolyl in one embodiment or triazolyl in one embodiment. "5-Membered heteroarenediyl" is a divalent group formed by removal of any one hydrogen atom from the "5-membered heteroaryl".

"6-Membered heteroaryl" is a 6-membered unsaturated heterocyclic group containing one to three nitrogen atoms as ring-forming atoms. The "6-membered heteroaryl" is pyridyl, pyrimidinyl, pyrazinyl, pyridazinyl or triazinyl in one embodiment, pyridyl or pyridazinyl in one embodiment, pyridyl or pyrimidinyl in one embodiment, pyridyl in one embodiment or pyrimidinyl in one embodiment. "6-Membered heteroarenediyl" is a divalent group formed by removal of any one hydrogen atom from the "6-membered heteroaryl".

"Halogen" means F, Cl, Br and I. The "halogen" is F, Cl or Br in one embodiment, F or Cl in one embodiment, F or Br in one embodiment, F in one embodiment, Cl in one embodiment or Br in one embodiment.

"7-Membered or 8-membered saturated or unsaturated bridged heterocyclic group" is a saturated 7-membered or 8-membered bridged heterocyclic group containing one or two nitrogen atoms as ring-forming atoms or a 7-membered or 8-membered bridged heterocyclic group containing one or two nitrogen atoms and having an unsaturated bond. The "7-membered or 8-membered saturated or unsaturated bridged heterocyclic group" is a saturated 7-membered or 8-membered bridged heterocyclic group containing two nitrogen atoms in one embodiment or a saturated 7-membered or 8-membered bridged heterocyclic group containing two nitrogen atoms in which one of the two nitrogen atoms bonds to one hydrogen atom. Examples thereof include diazabicyclo[2.2.2]octanyl, diazabicyclo[3.2.1]octanyl, diazabicyclo[3.2.1]octenyl, diazabicyclo[3.1.1]heptanyl, diazabicyclo[2.2.1]heptanyl and diazabicyclo[2.2.1]heptenyl. The "7-membered or 8-membered saturated or unsaturated bridged heterocyclic group" is diazabicyclo[2.2.2]octanyl, diazabicyclo[3.2.1]octanyl, diazabicyclo[3.2.1]oct-6-enyl, diazabicyclo[3.2.1]oct-2-enyl, diazabicyclo[3.1.1]heptanyl, diazabicyclo[2.2.1]heptanyl or diazabicyclo[2.2.1]hept-5-enyl in one embodiment, diazabicyclo[2.2.2]octanyl, diazabicyclo[3.2.1]octanyl, diazabicyclo[3.1.1]heptanyl or diazabicyclo[2.2.1]heptanyl in one embodiment, 2,5-diazabicyclo[2.2.2]octanyl, 3,8-diazabicyclo[3.2.1]octanyl, 3,6-diazabicyclo[3.1.1]heptanyl or 2,5-diazabicyclo[2.2.1]heptanyl in one embodiment, diazabicyclo[2.2.1]heptanyl in one embodiment, 2,5-diazabicyclo[2.2.1]heptanyl in one embodiment or 2,5-diazabicyclo[2.2.1]heptan-2-yl in one embodiment.

Substituents acceptable in "optionally substituted C₁₋₆ alkyl" and "optionally substituted C₁₋₃ alkyl" in one embodiment are F, OH, OCH₃, N(CH₃)₃, optionally substituted C₃₋₆ cycloalkyl, azabicyclo[3.3.0]octanyl or a 4-membered to 6-membered optionally substituted saturated heterocyclic group containing one or two hetero atoms selected from the group consisting of oxygen, sulfur and nitrogen. The substituents are F, OH, OCH₃, N(CH₃)₂, hydroxymethyl, methoxymethyl, difluoroethyl, optionally substituted cyclopropyl, tetrahydrofuranyl, optionally substituted tetrahydropyranyl, morpholinyl, optionally substituted pyrrolidinyl, optionally substituted piperidinyl or azabicyclo[3.3.0]octanyl in one embodiment, F, OH, OCH₃, N(CH₃)₂, hydroxymethyl, methoxymethyl, optionally substituted cyclopropyl, tetrahydrofuranyl, optionally substituted tetrahydropyranyl or optionally substituted pyrrolidinyl in one embodiment, F, OH, OCH₃, N(CH₃)₂, hydroxymethyl, methoxymethyl, cyclopropyl, (hydroxymethyl)cyclopropyl, (methoxymethyl)cyclopropyl, tetrahydrofuranyl, tetrahydropyranyl, (hydroxymethyl)tetrahydropyranyl, (methoxymethyl)tetrahydropyranyl, pyrrolidinyl or methylpyrrolidinyl in one embodiment, F, OH, OCH₃, (methoxymethyl)cyclopropyl, tetrahydrofuranyl or methylpyrrolidinyl in one embodiment, F, OH, or cyclopropyl in one embodiment, F, OH, or OCH₃ in one embodiment, OH or OCH₃ in one embodiment, F or OCH₃ in one embodiment, OH in one embodiment, F in one embodiment or OCH₃ in one embodiment.

Substituents acceptable in "5-membered optionally substituted heteroaryl", "6-membered optionally substituted heteroaryl", "6-membered optionally substituted heteroarenediyl", "optionally substituted C₃₋₆ cycloalkyl", "optionally substituted pyrazolyl", "optionally substituted pyridyl", "optionally substituted pyrimidinyl", "optionally substituted phenyl" and "optionally substituted cyclopropyl" in one embodiment are C₁₋₃ alkyl optionally substituted with a group selected from the group consisting of OH and OCH₃, -SO₂CH₃, halogen, OH, OCH₃ or C₃₋₆ cycloalkyl. The substituents are C₁₋₃ alkyl optionally substituted with a group selected from the group consisting of OH and OCH₃ in one embodiment, C₁₋₃ alkyl optionally substituted with OH in one embodiment, C₁₋₃ alkyl optionally substituted with OCH₃ in one embodiment, C₁₋₃ alkyl or halogen in one embodiment, methyl, ethyl, methoxymethyl or F in one embodiment or methyl, ethyl or F in one embodiment.

Substituents acceptable in "a 4-membered to 6-membered optionally substituted saturated heterocyclic group", "optionally substituted pyrrolidinyl", "optionally substituted piperidinyl", "optionally substituted oxetanyl", "optionally substituted tetrahydrofuranyl" and "optionally substituted tetrahydropyranyl" in one embodiment are C₁₋₃ alkyl optionally substituted with a group selected from the group consisting of F, OH and OCH₃, F, OH, OCH₃, oxo or oxetanyl. The substituents are F, OH or OCH₃ in one embodiment, OH or methyl in one embodiment, C₁₋₃ alkyl optionally substituted with a group selected from the group consisting of F, OH and OCH₃, F, oxo or oxetanyl in one embodiment, C₁₋₃ alkyl optionally substituted with a group selected from the group consisting of F, OH and OCH₃ or oxo in one embodiment, C₁₋₃ alkyl optionally substituted with a group selected from the group consisting of F, OH and OCH₃ in one embodiment, C₁₋₃ alkyl optionally substituted with F in one embodiment, C₁₋₃ alkyl optionally substituted with OH in one embodiment, C₁₋₃ alkyl optionally substituted with OCH₃ in one embodiment or C₁₋₃ alkyl in one embodiment.

Substituents acceptable in "optionally substituted pyrrolidinediyl", "optionally substituted piperidinediyl", "optionally substituted piperazinediyl" and "optionally substituted C₁₋₃ alkylene" in one embodiment are F, OH, OCH₃ or optionally substituted C₁₋₃ alkyl. The substituents are F, OH, OCH₃, methyl, ethyl, hydroxymethyl or methoxymethyl in one embodiment or F, OH, OCH₃ or methyl in one embodiment.

"C₁₋₃ Alkyl optionally substituted with F" in one embodiment is methyl optionally substituted with F or ethyl optionally substituted with F. Examples thereof include methyl, ethyl, monofluoromethyl, difluoromethyl, trifluoromethyl, monofluoroethyl, difluoroethyl and trifluoroethyl. The "C₁₋₃ alkyl optionally substituted with F" is methyl, ethyl, monofluoromethyl, difluoromethyl or difluoroethyl in one embodiment, monofluoromethyl or difluoromethyl in one embodiment, monofluoromethyl or difluoroethyl in one embodiment, difluoromethyl or difluoroethyl in one embodiment, monofluoromethyl in one embodiment, difluoromethyl in one embodiment, difluoroethyl in one embodiment or 2,2-difluoroethyl in one embodiment.

"C₁₋₃ Alkyl optionally substituted with OH" in one embodiment is methyl optionally substituted with one OH group or ethyl optionally substituted with one or two OH groups. Examples thereof include methyl, ethyl, hydroxymethyl, 1-hydroxyethyl, 2-hydroxyethyl and 1,2-dihydroxyethyl. The "C₁₋₃ alkyl optionally substituted with OH" is methyl, ethyl or hydroxymethyl in one embodiment, methyl or hydroxymethyl in one embodiment, hydroxymethyl or hydroxyethyl in one embodiment, hydroxymethyl in one embodiment or hydroxyethyl in one embodiment.

"C₁₋₃ Alkyl optionally substituted with OCH₃" in one embodiment is methyl optionally substituted with one OCH₃ group or ethyl optionally substituted with one or two OCH₃ groups. Examples thereof include methyl, ethyl, methoxymethyl, 1-methoxyethyl, 2-methoxyethyl and 1,2-dimethoxyethyl. The "C₁₋₃ alkyl optionally substituted with OCH₃" is methoxymethyl or methoxyethyl in one embodiment, methoxymethyl in one embodiment or methoxyethyl in one embodiment.

"C₁₋₃ Alkyl optionally substituted with N(CH₃)₂" in one embodiment is methyl optionally substituted with one N(CH₃)₂ group or ethyl optionally substituted with one N(CH₃)₂ group. The "C₁₋₃ alkyl optionally substituted with N(CH₃)₂" is methyl, ethyl, dimethylaminomethyl or dimethylaminoethyl in one embodiment, methyl or dimethylaminomethyl in one embodiment, dimethylaminomethyl in one embodiment or dimethylaminoethyl in one embodiment.

"Phenylene optionally substituted with F" in one embodiment is phenylene optionally substituted with one or two F atoms. The "phenylene optionally substituted with F" is phenylene optionally substituted with one F atom in one embodiment, phenylene or fluorophenylene in one embodiment, phenylene in one embodiment, 2-fluoro-1,4-phenylene in one embodiment or 3-fluoro-1,4-phenylene in one embodiment.

"G12D Mutation" represents a mutation in which the amino acid residue corresponding to the codon 12 in a wildtype protein is converted from glycine to aspartic acid.

"G12D Mutant KRAS" represents KRAS having the "G12D mutation".

"Pancreatic cancer" is a malignant tumor occurring in the pancreas. Examples thereof include pancreatic ductal carcinoma and pancreatic ductal adenocarcinoma, and the "pancreatic cancer" is pancreatic ductal carcinoma in one embodiment or pancreatic ductal adenocarcinoma in one embodiment. Moreover, the "pancreatic cancer" is metastatic pancreatic cancer in one embodiment, locally advanced pancreatic cancer in one embodiment, recurrent or refractory pancreatic cancer in one embodiment or pancreatic cancer of a patient who is untreated and/or has a treatment history in one embodiment.

"G12D Mutant KRAS-positive pancreatic cancer" is pancreatic cancer that is positive for G12D mutant KRAS. Examples thereof include a pancreatic cancer in which the KRAS G12D mutation occurs and a pancreatic cancer which has a high positive rate for G12D mutant KRAS. The "G12D mutant KRAS-positive pancreatic cancer" is G12D mutant KRAS-positive pancreatic ductal carcinoma in one embodiment or G12D mutant KRAS-positive pancreatic ductal adenocarcinoma in one embodiment.

Embodiments of the compound of the formula (I) or a salt thereof of the present invention are shown below.
(1-1) The compound or a salt thereof in which A is CR^{A} or N, and R^{A} is H or C₁₋₃ alkyl.
(1-2) The compound or a salt thereof in which A is CR^{A}, and R^{A} is H or C₁₋₃ alkyl.
(1-3) The compound or a salt thereof in which A is N.
(1-4) The compound or a salt thereof in which A is CH or N.
(1-5) The compound or a salt thereof in which A is CR^{A} or N, and R^{A} is C₁₋₃ alkyl.
(1-6) The compound or a salt thereof in which A is CH.
(2-1) The compound or a salt thereof in which R¹ is naphthyl optionally substituted with OH or a group selected from the group consisting of the formula (II), the formula (III) and the formula (IV) below, 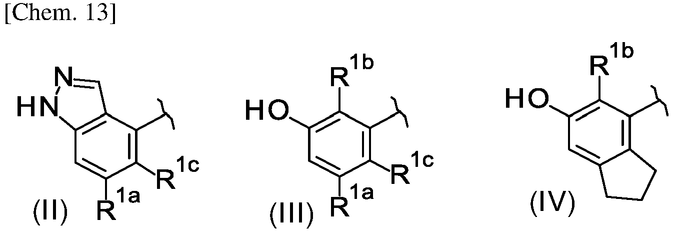
   R^{1a} and R^{1b}, which are the same as or different from each other, are H, methyl, For Cl, and
   R^{1c} is F, Cl, methyl or ethyl.
(2-2) The compound or a salt thereof in which R¹ is naphthyl optionally substituted with OH or a group selected from the group consisting of the formula (II) and the formula (III) below, 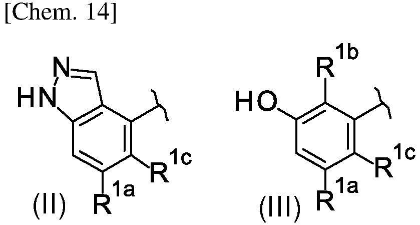
   R^{1a} and R^{1b}, which are the same as or different from each other, are H, methyl, F or Cl, and
   R^{1c} is F, Cl, methyl or ethyl.
(2-3) The compound or a salt thereof in which R¹ is the formula (IIa) or the formula (IIIa) below, 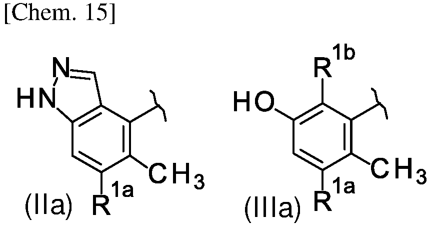 and
   R^{1a} and R^{1b}, which are the same as or different from each other, are H, methyl, For Cl.
(2-4) The compound or a salt thereof in which R¹ is the formula (IIa) or the formula (Illa), and
   R^{1a} and R^{1b}, which are the same as or different from each other, are H or F.
(2-5) The compound or a salt thereof in which R¹ is the formula (IIa) or the formula (Illa), R^{1a} is F, and R^{1b} is H.
(2-6) The compound or a salt thereof in which R¹ is the following formula (IIb). 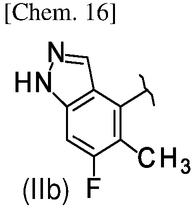
(3-1) The compound or a salt thereof in which R² is H, halogen, optionally substituted C₁₋₃ alkyl, cyclopropyl or vinyl.
(3-2) The compound or a salt thereof in which R² is halogen, C₁₋₃ alkyl, cyclopropyl or vinyl, where the C₁₋₃ alkyl is optionally substituted with a group selected from the group consisting of OH and OCH₃.
(3-3) The compound or a salt thereof in which R² is halogen, C₁₋₃ alkyl, cyclopropyl or vinyl.
(3-4) The compound or a salt thereof in which R² is cyclopropyl.
(4-1) The compound or a salt thereof in which
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 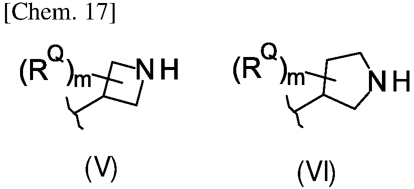
   V is the formula (VII) below, 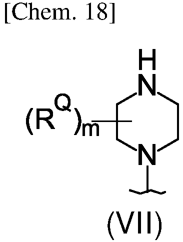
   each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
   m is 0 to 2.
(4-2) The compound or a salt thereof in which R³ is as follows,
   (i) when A is CR^{A}, R³ is -P-Q, and
      P is -O-, or
   (ii) when A is N, R³ is -P-Q or V,
      P is -O- or -N(R^{P})-,
      R^{p} is H or C₁₋₃ alkyl,
      in either case (i) or (ii), Q is the formula (V) or the formula (VI) below, 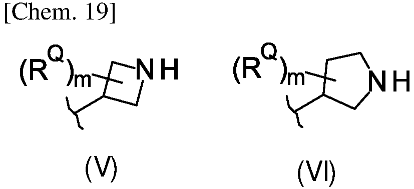
      V is the formula (VII) below, 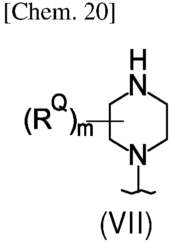
      each R^{Q}, which is the same as or different from each other, are OH or methyl,
         wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
      m is 0 to 2.
(4-3) The compound or a salt thereof in which R³ is as follows,
   (i) when A is CH, R³ is -P-Q, and
      P is -O-, or
   (ii) when A is N, R³ is -P-Q or V,
      P is -O- or -N(R^{P})-, and
      R^{p} is H or C₁₋₃ alkyl,
      in either case (i) or (ii), Q is the formula (V) or the formula (VI) below, 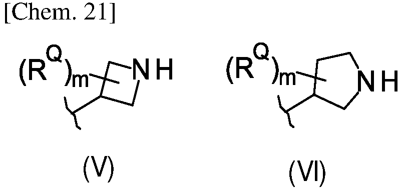
      V is the formula (VII) below, 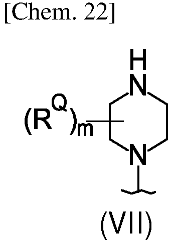
      each R^{Q}, which is the same as or different from each other, are OH or methyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
      m is 0 to 2.
(4-4) The compound or a salt thereof in which
   R³ is -P-Q,
   P is -O- or -N(R^{P})-,
   R^{p} is H or C₁₋₃ alkyl, and
   Q is the formula (V) or the formula (VI) below, 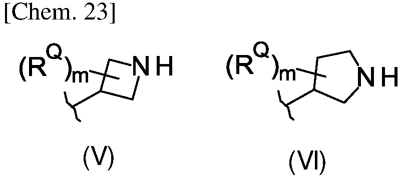
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI), and
   m is 0 to 2.
(4-5) The compound or a salt thereof in which
   R³ is -P-Q,
   P is -O-, and
   Q is the formula (V) or the formula (VI) below, 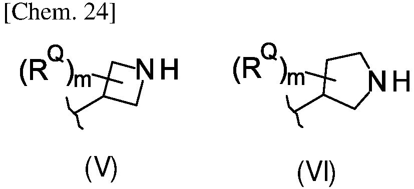
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI), and
   m is 0 to 2.
(4-6) The compound or a salt thereof in which
   R³ is -P-Q,
   P is -O-, and
   Q is the formula (V) or the formula (VI) below, 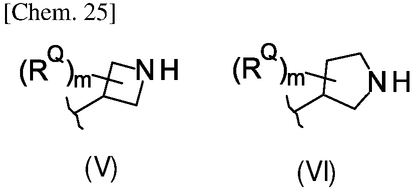
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI), and
   m is 0 or 1.
(4-7) The compound or a salt thereof in which
   R³ is V, and
   V is the formula (VII) below, 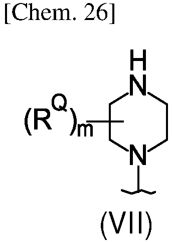
   each R^{Q}, which is the same as or different from each other, are OH or methyl, wherein R^{Q} is attached only to a carbon atom that is a ring-forming atom of a piperazine ring represented by the formula (VII), and
   m is 0 to 2.
(4-8) The compound or a salt thereof in which
   R³ is V, and
   V is the formula (VII) below, 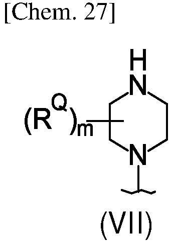
   each R^{Q}, which is the same as or different from each other, are OH or methyl, wherein R^{Q} is attached only to a carbon atom that is a ring-forming atom of a piperazine ring represented by the formula (VII), and
   m is 0 or 1.
(4-9) The compound or a salt thereof in which
   R³ is -P-Q,
   P is -O- or -N(R^{P})-,
   R^{p} is H or C₁₋₃ alkyl, and
   Q is the formula (V) or the formula (VI) below, 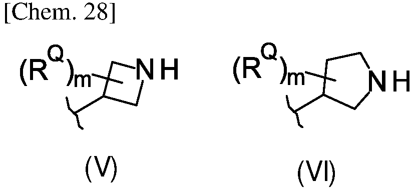
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI),
   m is 0 or 1.
(4-10) R³ is -P-Q,
   P is -N(R^{P})-,
   R^{p} is H or C₁₋₃ alkyl, and
   Q is the formula (V) or the formula (VI) below, 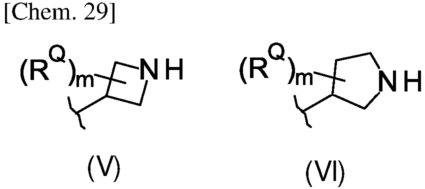
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI), and
   m is 0 or 1.
(4-11) The compound or a salt thereof in which
   R³ is -P-Q,
   P is -N(R^{P})-,
   R^{p} is H or C₁₋₃ alkyl, and
   Q is the formula (VI) below, 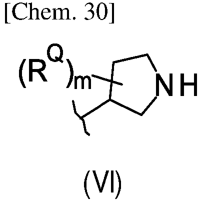 , and
   m is 0.
(4-12) The compound or a salt thereof in which
   R³ is -P-Q,
   P is -N(R^{P})-,
   R^{P} is methyl or ethyl,
   Q is the formula (VI), 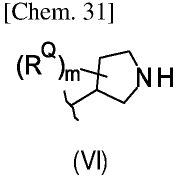 and
   m is 0.
(5-1) The compound or a salt thereof in which R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl optionally substituted containing one to three nitrogen atoms.
(5-2) The compound or a salt thereof in which R⁴ is optionally substituted C₁₋₆ alkyl, optionally substituted oxetanyl, optionally substituted tetrahydrofuranyl, optionally substituted tetrahydropyranyl, optionally substituted pyrazolyl, optionally substituted pyridyl, optionally substituted pyrimidinyl, optionally substituted pyrrolidinyl or optionally substituted piperidinyl .
(5-3) The compound or a salt thereof in which R⁴ is optionally substituted C₁₋₆ alkyl, a 4-membered to 6-membered optionally substituted saturated heterocyclic group containing one or two hetero atoms selected from the group consisting of oxygen, sulfur and nitrogen, optionally substituted pyrazolyl, optionally substituted pyridyl or optionally substituted pyrimidinyl.
(5-4) The compound or a salt thereof in which R⁴ is C₁₋₆ alkyl optionally substituted with a group selected from the group consisting of F, OH, OCH₃, CF₃, CHF₂, optionally substituted cyclopropyl, optionally substituted pyrrolidinyl and optionally substituted tetrahydrofuranyl, optionally substituted tetrahydropyranyl, optionally substituted pyrazolyl, optionally substituted pyridyl or optionally substituted pyrimidinyl.
(5-5) The compound or a salt thereof in which R⁴ is C₁₋₆ alkyl optionally substituted with a group selected from the group consisting of F, OH, OCH₃, CF₃, CHF₂, optionally substituted cyclopropyl, optionally substituted pyrrolidinyl and optionally substituted tetrahydrofuranyl or tetrahydropyranyl.
(5-6) The compound or a salt thereof in which R⁴ is C₁₋₆ alkyl optionally substituted with a group selected from the group consisting of F, OH, OCH₃, CF₃, CHF₂, cyclopropyl substituted with one methoxymethyl, pyrrolidinyl optionally substituted with C₁₋₆ alkyl and tetrahydrofuranyl optionally substituted with C₁₋₆ alkyl, tetrahydropyranyl optionally substituted with C₁₋₆ alkyl, pyrazolyl optionally substituted with C₁₋₆ alkyl, pyridyl optionally substituted with C₁₋₆ alkyl or pyrimidinyl optionally substituted with C₁₋₆ alkyl.
(5-7) The compound or a salt thereof in which R⁴ is C₁₋₆ alkyl optionally substituted with a group selected from the group consisting of F, OH, OCH₃, CF₃, CHF₂, cyclopropyl substituted with one methoxymethyl, pyrrolidinyl optionally substituted with C₁₋₆ alkyl and tetrahydrofuranyl optionally substituted with C₁₋₆ alkyl or tetrahydropyranyl.
(5-8) The compound or a salt thereof in which R⁴ is C₁₋₆ alkyl optionally substituted with a group selected from the group consisting of OCH₃ and tetrahydrofuranyl or tetrahydropyranyl.
(6-1) The compound or a salt thereof in which R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen.
(6-2) The compound or a salt thereof in which R⁵ is methyl, ethyl, isopropyl, tert-butyl or C₃₋₆ cycloalkyl.
(6-3) The compound or a salt thereof in which R⁵ is ethyl, isopropyl, tert-butyl or C₃₋₆ cycloalkyl.
(6-4) The compound or a salt thereof in which R⁵ is isopropyl or C₃₋₆ cycloalkyl.
(6-5) The compound or a salt thereof in which R⁵ is isopropyl.
(7-1) The compound or a salt thereof in which R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen.
(7-2) The compound or a salt thereof in which R^{6a} and R^{6b}, which are the same as or different from each other, are H or C₁₋₃ alkyl, where the C₁₋₃ alkyl is optionally substituted with a group selected from the group consisting of F, OH, OCH₃ and N(CH₃)₂, or R^{6a} and R^{6b} may form C₃₋₆ cycloalkyl together with the carbon to which they are attached.
(7-3) The compound or a salt thereof in which R^{6a} and R^{6b}, which are the same as or different from each other, are H or C₁₋₃ alkyl, where the C₁₋₃ alkyl is optionally substituted with a group selected from the group consisting of F, OH and N(CH₃)₂, or R^{6a} and R^{6b} may form cyclopropyl together with the carbon to which they are attached.
(7-4) The compound or a salt thereof in which R^{6a} is H, and R^{6b} is C₁₋₃ alkyl optionally substituted with OH.
(7-5) The compound or a salt thereof in which R^{6a} is H, and R^{6b} is hydroxymethyl.
(8-1) The compound or a salt thereof in which R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms.
(8-2) The compound or a salt thereof in which R⁷ is H, halogen, C₁₋₃ alkyl, -SO₂CH₃, C₃₋₆ cycloalkyl or a group selected from the group consisting of the formula (IX), the formula (X), the formula (XI), the formula (XII), the formula (XIII), the formula (XIV), the formula (XV), the formula (XVI), the formula (XVII) and the formula (XVIII) below, 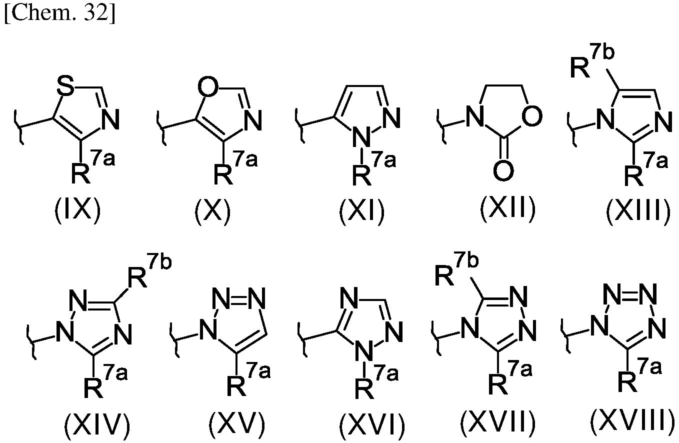 , and
   R^{7a} and R^{7b}, which are the same as or different from each other, are H or C₁₋₃ alkyl optionally substituted with OH.
(8-3) The compound or a salt thereof in which R⁷ is halogen or a group selected from the group consisting of the formula (IX), the formula (X), the formula (XI), the formula (XII), the formula (XIII) and the formula (XIV) below, 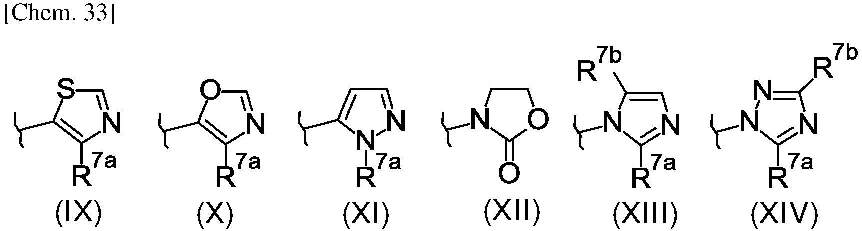 , and
   R^{7a} and R^{7b}, which are the same as or different from each other, are H or C₁₋₃ alkyl optionally substituted with OH.
(8-4) The compound or a salt thereof in which R⁷ is the formula (IX), the formula (X), the formula (XI) or the formula (XII) below, 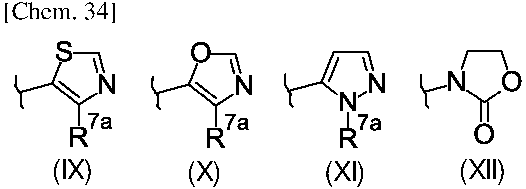 , and
   R^{7a} is C₁₋₃ alkyl optionally substituted with OH.
(8-5) The compound or a salt thereof in which R⁷ is the formula (IX) or the formula (XI) below, 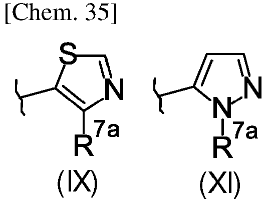 , and
   R^{7a} is C₁₋₃ alkyl.
(8-6) The compound or a salt thereof in which R⁷ is the formula (IX) below, 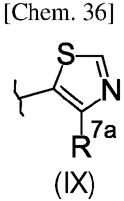 , and
   R^{7a} is C₁₋₃ alkyl.
(8-7) The compound or a salt thereof in which R⁷ is H.
(9-1) The compound or a salt thereof in which W is optionally substituted phenylene or 6-membered optionally substituted heteroarenediyl containing one to three nitrogen atoms.
(9-2) The compound or a salt thereof in which W is the following formula (XIX), 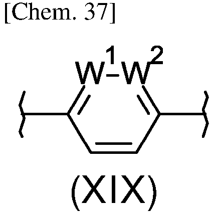 , and
   W¹ and W² are as follows,
   (i) W¹ is CH, and W² is C-SO₂CH₃, or
   (ii) W¹ and W², which are the same as or different from each other, are CH, CF, CCl, CCH₃ or N, where in the case of (i) above, R⁷ is H.
(9-3) The compound or a salt thereof W is the following formula (XIX), 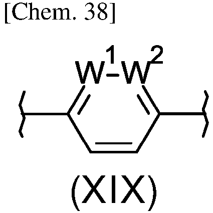 , and
   W¹ and W², which are the same as or different from each other, are CH or N.
(9-4) The compound or a salt thereof in which W is the following formula (XIX), 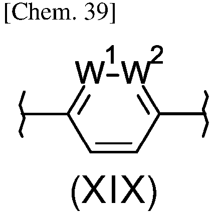 , and
   W¹ and W², which are the same as or different from each other, are CH, CF, CCl, CCH₃ or N.
(9-5) The compound or a salt thereof in which W is the following formula (XIX), 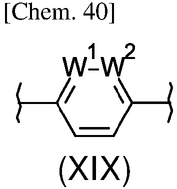 and
   W¹ and W² are each CH.
(10-1) The compound or a salt thereof in which X is a bond, -CH₂-, -O-, -S- or - NR^{4x}-, and R^{4x} is H or C₁₋₃ alkyl.
(10-2) The compound or a salt thereof in which X is -O or -NR^{4x}-, and R^{4x} is H or C₁₋₃ alkyl.
(10-3) The compound or a salt thereof in which X is -O or -NH.
(10-4) The compound or a salt thereof in which X is -O-.
(11-1) The compound or a salt thereof in which Y is phenylene or pyridinediyl, where the phenylene is optionally substituted with F.
(11-2) The compound or a salt thereof in which Y is phenylene or pyridinediyl.
(11-3) The compound or a salt thereof in which Y is phenylene optionally substituted with F.
(12-1) The compound or a salt thereof in which L is -(L¹-L²-L³-L⁴)-,
   L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O, and
   R^{L1} is H or C₁₋₃ alkyl.
(12-2) The compound or a salt thereof in which L is a bond, C₁₋₃ alkylene, C=O or a group selected from the group consisting of the formula (XX), the formula (XXI), the formula (XXII), the formula (XXIII), the formula (XXIV) and the formula (XXV) below, 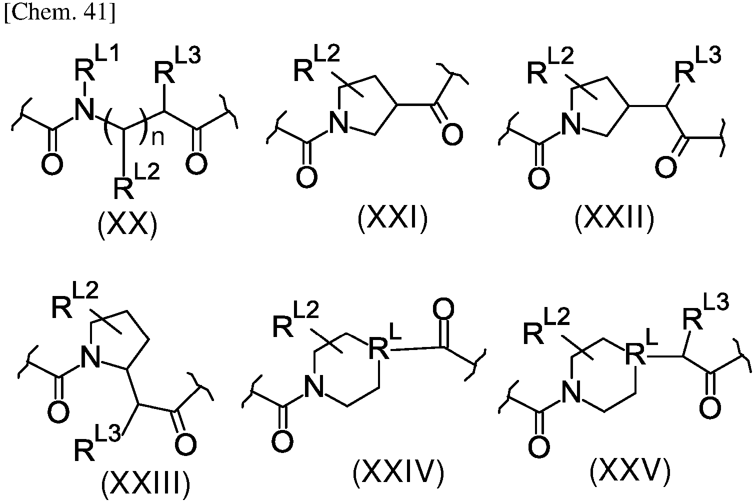
   R^{L1} is H or C₁₋₃ alkyl,
   R^{L2} and R^{L3}, which are the same as or different from each other, are H, F, OH, OCH₃ or optionally substituted C₁₋₃ alkyl,
   R^{L} is CH or N, and
   n is an integer of one or two.
(12-3) The compound or a salt thereof in which L is a bond, C=O or a group selected from the group consisting of the formula (XX) and the formula (XXII) below, 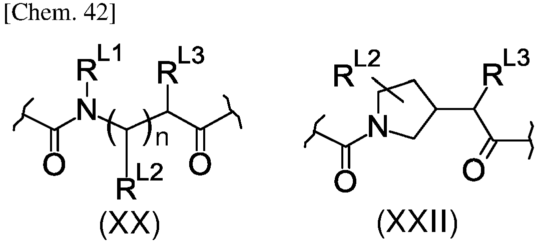
   R^{L1} is H or C₁₋₃ alkyl,
   R^{L2} and R^{L3}, which are the same as or different from each other, are H, F, OH, OCH₃ or optionally substituted C₁₋₃ alkyl, and
   n is an integer of one or two.
(12-4) The compound or a salt thereof in which L is a bond, C=O or a group selected from the group consisting of the formula (XX)-1 and the formula (XXII)-1 below, 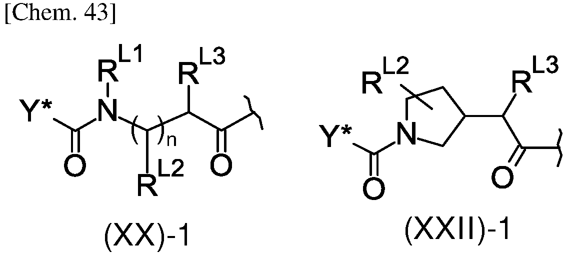 (in the formulae, Y* represents linking to Y)
   R^{L1} is C₁₋₃ alkyl,
   R^{L2} and R^{L3} are H, and
   n is 1.
(12-5) The compound or a salt thereof in which L is a bond, C=O or a group selected from the group consisting of the formula (XX) and the formula (XXII), R^{L1} is C₁₋₃ alkyl, R^{L2} and R^{L3} are H, and n is 1.
(12-6) The compound or a salt thereof in which L is a bond, C=O or a group selected from the group consisting of the formula (XX)-1 and the formula (XXII)-1, R^{L1} is C₁₋₃ alkyl, R^{L2} and R^{L3} are H, and n is 1.
(12-7) The compound or a salt thereof in which L is C=O or a group selected from the group consisting of the formula (XX) and the formula (XXII), R^{L1} is C₁₋₃ alkyl, R^{L2} and R^{L3} are H, and n is 1.
(12-8) The compound or a salt thereof in which L is C=O or a group selected from the group consisting of the formula (XX)-1 and the formula (XXII)-1, R^{L1} is C₁₋₃ alkyl, R^{L2} and R^{L3} and H, and n is 1.
(12-9) The compound or a salt thereof in which L is a bond or C=O.
(12-10) The compound or a salt thereof in which L is a bond.
(12-11) The compound or a salt thereof in which L is C=O.
(13-1) The compound or a salt thereof in which Z is NH or 5-membered heteroarenediyl containing one to four hetero atoms selected from the group consisting of oxygen, sulfur and nitrogen.
(13-2) The compound or a salt thereof in which Z is NH or a group selected from the group consisting of the formula (XXVI), the formula (XXVII), the formula (XXVIII) and the formula (XXIX) below. 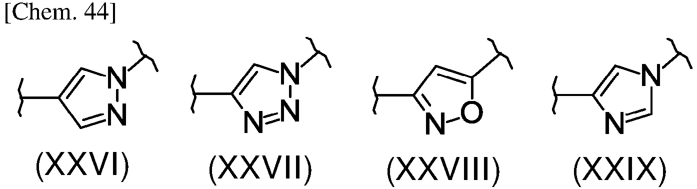
(13-3) The compound or a salt thereof in which Z is NH or a group selected from the group consisting of the formula (XXVI)-1, the formula (XXVII)-1, the formula (XXVIII)-1 and the formula (XXIX)-1 below. 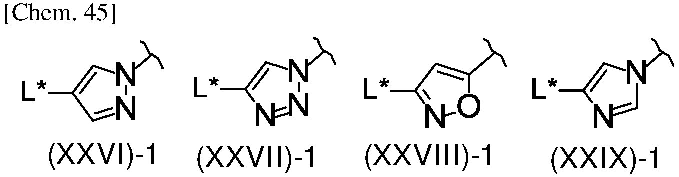 (In the formulae, L* represents linking to L.)
(13-4) The compound or a salt thereof in which Z is NH or a group selected from the group consisting of the formula (XXVI), the formula (XXVII) and the formula (XXVIII) below. 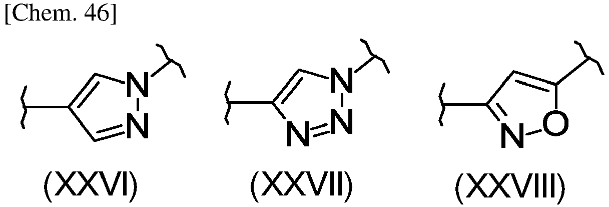
(13-5) The compound or a salt thereof in which Z is NH or a group selected from the group consisting of the formula (XXVI)-1, the formula (XXVII)-1 and the formula (XXVIII)-1 below. 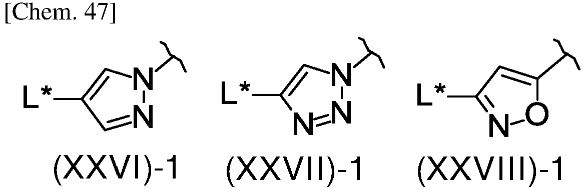 (In the formulae, L* represents linking to L.)
(13-6) The compound or a salt thereof in which Z is NH or a group selected from the group consisting of the formula (XXVI) and the formula (XXVII) below. 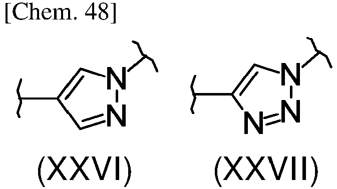
(13-7) The compound or a salt thereof in which Z is NH or a group selected from the group consisting of the formula (XXVI)-1 and the formula (XXVII)-1 below. 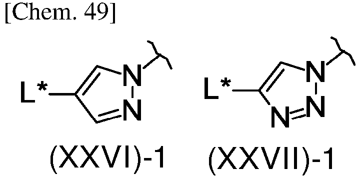 (In the formulae, L* represents linking to L.)
(13-8) The compound or a salt thereof in which Z is a group selected from the group consisting of the formula (XXVI) and the formula (XXVII).
(13-9) The compound or a salt thereof in which Z is a group selected from the group consisting of the formula (XXVI)-1 and the formula (XXVII)-1.
(13-10) The compound or a salt thereof in which Z is NH.
(13-11) The compound or a salt thereof in which Z is the formula (XXVII) below. 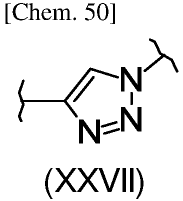
(13-12) The compound or a salt thereof in which Z is the formula (XXVII)-1 below. 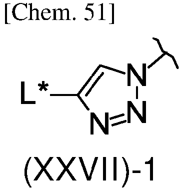 (In the formula, L* represents linking to L.)
(14-1) The compound or a salt thereof in which Y-L-Z is the following formula (VIII). 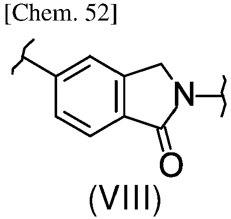
(14-2) The compound or a salt thereof in which Y-L-Z is the following formula (VIII)-1. 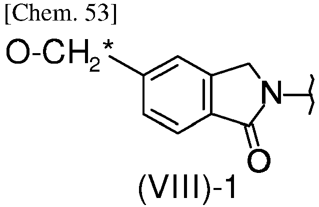 (In the formula, O-CH₂^{*} represents linking to the carbon atom in O-CH₂ that is attached to Y-L-Z.)
(15) The compound or a salt thereof which is a combination of any compatible two or more of the embodiments described in (1-1) to (14-2) above.

Specific examples of the combination described in (15) above include the following embodiments.
(15-1) A compound of the formula (I) or a salt thereof, 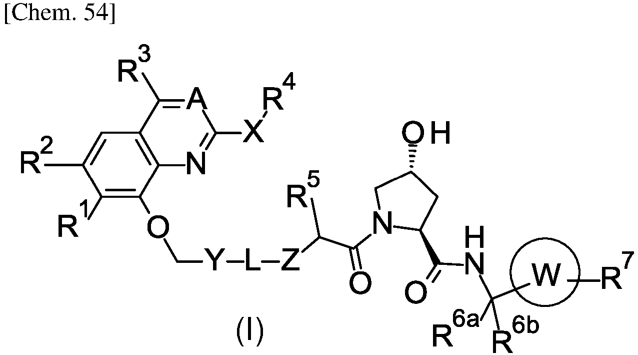 (wherein in the formula,
   A is CR^{A} or N,
   R^{A} is H or C₁₋₃ alkyl, and
   R¹ is naphthyl optionally substituted with OH or a group selected from the group consisting of the formula (II), the formula (III) and the formula (IV) below, 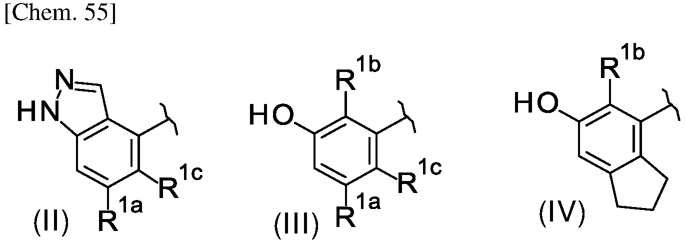
   R^{1a} and R^{1b}, which are the same as or different from each other, are H, methyl, For Cl,
   R^{1c} is F, Cl, methyl or ethyl,
   R² is H, halogen, optionally substituted C₁₋₃ alkyl, cyclopropyl or vinyl,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 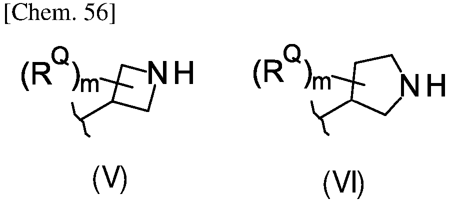
   V is the formula (VII) below, 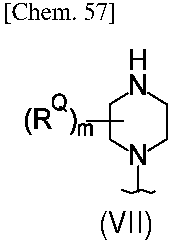
   each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
   m is 0 to 2,
   R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms,
   R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen,
   R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen,
   R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms,
   W is optionally substituted phenylene or 6-membered optionally substituted heteroarenediyl containing one to three nitrogen atoms,
   X is a bond, -CH₂-, -O-, -S- or -NR^{4x}-,
   R^{4x} is H or C₁₋₃ alkyl,
   Y is phenylene or pyridinediyl, wherein the phenylene may be substituted with F,
   L is -(L¹-L²-L³-L⁴)-,
   L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
   R^{L1} is H or C₁₋₃ alkyl, and
   Z is NH or 5-membered heteroarenediyl containing one to four hetero atoms selected from the group consisting of oxygen, sulfur and nitrogen,
   or Y-L-Z is the formula (VIII) below.) 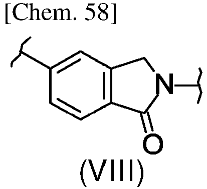
(15-2) The compound or a salt thereof described in (15-1) above,
   Y is phenylene or pyridinediyl, wherein the phenylene is optionally substituted with F,
   L is a bond, C₁₋₃ alkylene, C=O or a group selected from the group consisting of the formula (XX), the formula (XXI), the formula (XXII), the formula (XXIII), the formula (XXIV) and the formula (XXV) below, 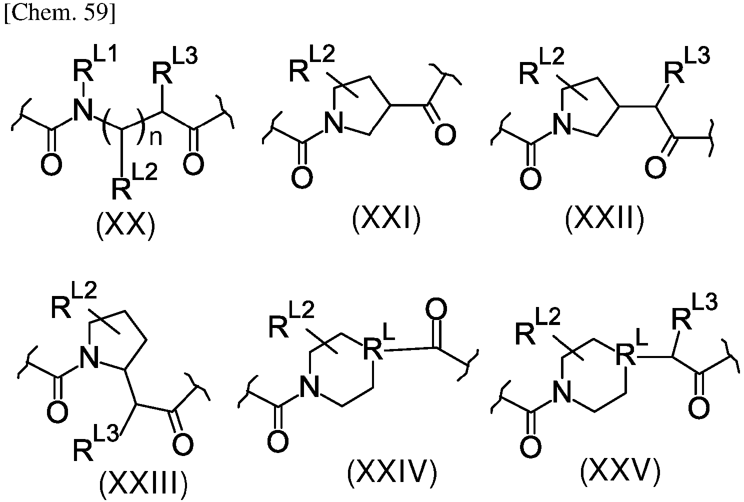
   R^{L1} is H or C₁₋₃ alkyl,
   R^{L2} and R^{L3}, which are the same as or different from each other, are H, F, OH, OCH₃ or optionally substituted C₁₋₃ alkyl,
   R^{L} is CH or N,
   n is an integer of one or two, and
   Z is NH or a group selected from the group consisting of the formula (XXVI), the formula (XXVII), the formula (XXVIII) and the formula (XXIX) below, 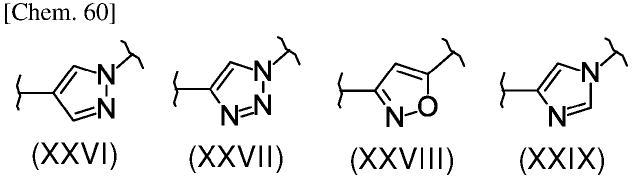
   or Y-L-Z is the formula (VIII) below. 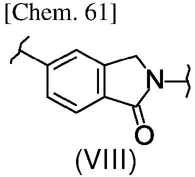
(15-3) The compound or a salt thereof described in (15-2) above, wherein R¹ is the formula (IIa) or the formula (Illa) below, 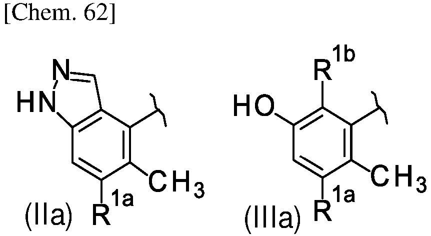
   R^{1a} and R^{1b}, which are the same as or different from each other, are H, methyl, For Cl,
   R³ is as follows,
      (i) when A is CR^{A}, R³ is -P-Q, and
         P is -O-, or
      (ii) when A is N, R³ is -P-Q or V, P is -O- or -N(R^{P})-, and
         R^{P} is H or C₁₋₃ alkyl,
            in either case (i) or (ii), Q is the formula (V) or the formula (VI) below, 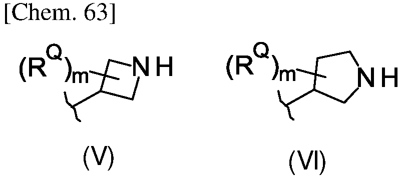
         V is the formula (VII) below, 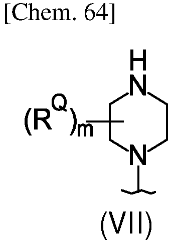
         each R^{Q}, which is the same as or different from each other, are OH or methyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
         m is 0 to 2,
         R⁴ is optionally substituted C₁₋₆ alkyl, a 4-membered to 6-membered optionally substituted saturated heterocyclic group containing one or two hetero atoms selected from the group consisting of oxygen, sulfur and nitrogen, optionally substituted pyrazolyl, optionally substituted pyridyl or optionally substituted pyrimidinyl,
         R⁵ is methyl, ethyl, isopropyl, tert-butyl or C₃₋₆ cycloalkyl,
         R^{6a} and R^{6b}, which are the same as or different from each other, are H or C₁₋₃ alkyl, wherein the C₁₋₃ alkyl may be substituted with a group selected from the group consisting of F, OH, OCH₃ and N(CH₃)₂, or R^{6a} and R^{6b} may form C₃₋₆ cycloalkyl together with the carbon to which they are attached,
         R⁷ is H, halogen, C₁₋₃ alkyl, -SO₂CH₃, C₃₋₆ cycloalkyl or a group selected from the group consisting of the formula (IX), the formula (X), the formula (XI), the formula (XII), the formula (XIII), the formula (XIV), the formula (XV), the formula (XVI), the formula (XVII) and the formula (XVIII) below, 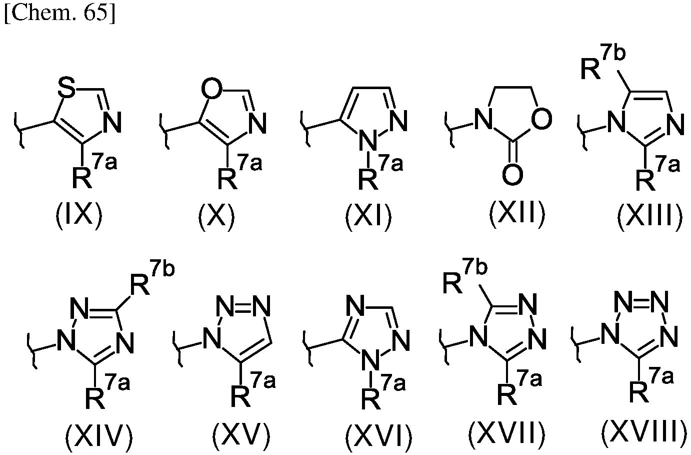
         R^{7a} and R^{7b}, which are the same as or different from each other, are H or C₁₋₃ alkyl optionally substituted with OH, and
         W is the following formula (XIX), 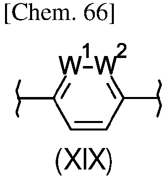
         W¹ and W² are as follows,
            (i) W¹ is CH, and W² is C-SO₂CH₃, or
            (ii) W¹ and W², which are the same as or different from each other, are CH, CF, CCl, CCH₃ or N,
         wherein when W¹ is CH and W² is C-SO₂CH₃, R⁷is H,
         X is - O or - NR^{4x}-,
         R^{4x} is H or C₁₋₃ alkyl,
         Y is phenylene or pyridinediyl, wherein the phenylene may be substituted with F,
         L is a bond, C=O or a group selected from the group consisting of the formula (XX) and the formula (XXII) below, 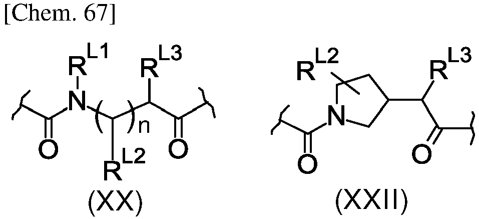
         R^{L1} is H or C₁₋₃ alkyl,
         R^{L2} and R^{L3}, which are the same as or different from each other, are H, F, OH, OCH₃ or optionally substituted C₁₋₃ alkyl,
         n is an integer of one or two, and
         Z is NH or a group selected from the group consisting of the formula (XXVI), the formula (XXVII), the formula (XXVIII) and the formula (XXIX) below, 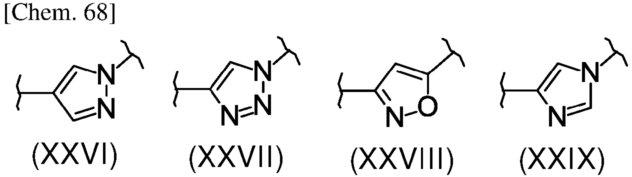
         or Y-L-Z is the formula (VIII) below, 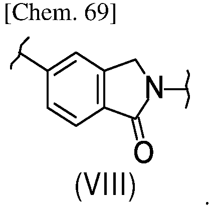
(15-4) The compound or a salt thereof described in (15-3) above, wherein A is CH or N,
   R² is halogen, C₁₋₃ alkyl, cyclopropyl or vinyl, wherein the C₁₋₃ alkyl is optionally substituted with a group selected from the group consisting of OH and OCH₃,
   R³ is as follows,
      (i) when A is CH, R³ is -P-Q, and
         P is -O-, or
      (ii) when A is N, R³ is -P-Q or V,
         P is -O- or -N(R^{P})-,
         R^{P} is H or C₁₋₃ alkyl,
         in either case (i) or (ii), Q is the formula (V) or the formula (VI) below, 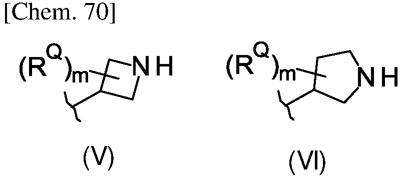
         V is the formula (VII) below, 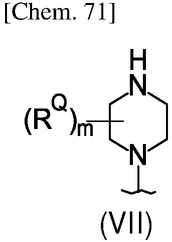
         each R^{Q}, which is the same as or different from each other, are OH or methyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
         m is 0 to 2,
         R⁴ is C₁₋₆ alkyl optionally substituted with a group selected from the group consisting of F, OH, OCH₃, CF₃, CHF₂, optionally substituted cyclopropyl, optionally substituted pyrrolidinyl and optionally substituted tetrahydrofuranyl or tetrahydropyranyl, and
         R⁷ is halogen or a group selected from the group consisting of the formula (IX), the formula (X), the formula (XI), the formula (XII), the formula (XIII) and the formula (XIV) below, 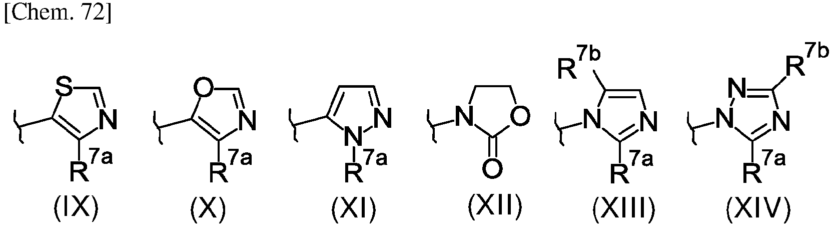
         R^{7a} and R^{7b}, which are the same as or different from each other, are H or C₁₋₃ alkyl optionally substituted with OH,
            The compound or a salt thereof in which W is the following formula (XIX), 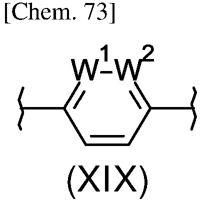
         W¹ and W², which are the same as or different from each other, are CH or N,
         X is -O- or -NH-,
         Y is phenylene or pyridinediyl, wherein the phenylene may be substituted with F,
         L is a bond, and
         Z is the formula (XXVII) below, 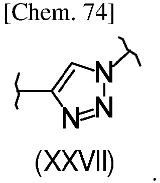
(15-5) The compound or a salt thereof described in (15-3) above,
   wherein A is N,
   R³ is -P-Q,
   P is -O- or -N(R^{P})-,
   R^{P} is H or C₁₋₃ alkyl, and
   Q is the formula (V) or the formula (VI) below, 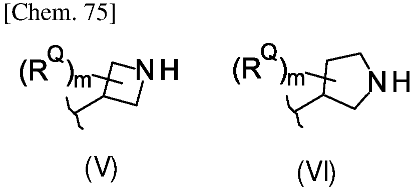
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI),
   m is 0 to 2, and
   W is the following formula (XIX), 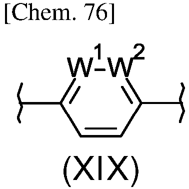
   W¹ and W², which are the same as or different from each other, are CH, CF, CCl, CCH₃ or N,
      Y is phenylene or pyridinediyl, wherein the phenylene may be substituted with F,
   L is a bond, C=O or a group selected from the group consisting of the formula (XX) and the formula (XXII) below, 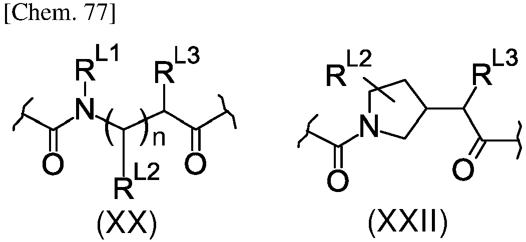
   R^{L1} is H or C₁₋₃ alkyl,
   R^{L2} and R^{L3}, which are the same as or different from each other, are H, F, OH, OCH₃ or optionally substituted C₁₋₃ alkyl,
   n is an integer of one or two, and
   Z is NH or a group selected from the group consisting of the formula (XXVI), the formula (XXVII), the formula (XXVIII) and the formula (XXIX) below, 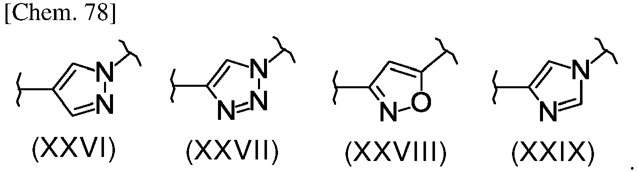
(15-6) The compound or a salt thereof described in (15-3) above,
   in which A is CH,
   R³ is -P-Q,
   P is -O-, and
   Q is the formula (V) or the formula (VI) below, 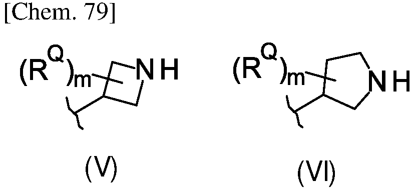
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI),
   m is 0 to 2, and
   W is the following formula (XIX), 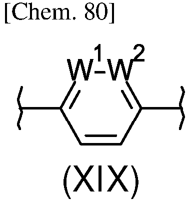
   W¹ and W², which are the same as or different from each other, are CH, CF, CCl, CCH₃ or N,
   Y is phenylene or pyridinediyl, wherein the phenylene is optionally substituted with F, and
   L is a bond, C=O or a group selected from the group consisting of the formula (XX) and the formula (XXII) below, 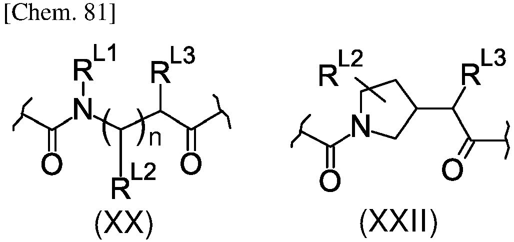
   R^{L1} is H or C₁₋₃ alkyl,
      R^{L2} and R^{L3}, which are the same as or different from each other, are H, F, OH, OCH₃ or optionally substituted C₁₋₃ alkyl,
   n is an integer of one or two, and
   Z is NH or a group selected from the group consisting of the formula (XXVI), the formula (XXVII), the formula (XXVIII) and the formula (XXIX) below, 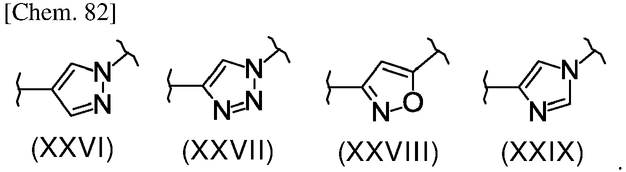
(15-7) The compound or a salt thereof described in (15-3) above,
   wherein A is N,
   R³ is V, and
   V is the formula (VII) below, 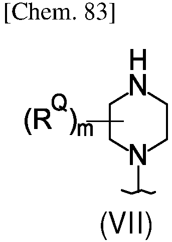
   each R^{Q}, which is the same as or different from each other, are OH or methyl, wherein R^{Q} is attached only to a carbon atom that is a ring-forming atom of a piperazine ring represented by the formula (VII),
   m is 0 to 2, and
   W is the following formula (XIX), 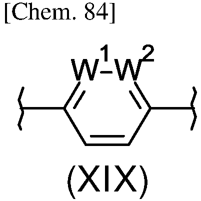
   W¹ and W², which are the same as or different from each other, are CH, CF, CCl, CCH₃ or N,
   Y is phenylene or pyridinediyl, wherein the phenylene may be substituted with F, and
   L is a bond, C=O or a group selected from the group consisting of the formula (XX) and the formula (XXII) below, 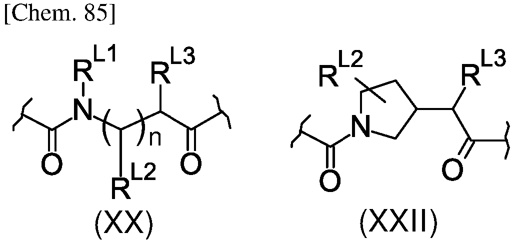
   R^{L1} is H or C₁₋₃ alkyl,
   R^{L2} and R^{L3}, which are the same as or different from each other, are H, F, OH, OCH₃ or optionally substituted C₁₋₃ alkyl,
   n is an integer of one or two, and
   Z is NH or a group selected from the group consisting of the formula (XXVI), the formula (XXVII), the formula (XXVIII) and the formula (XXIX) below, 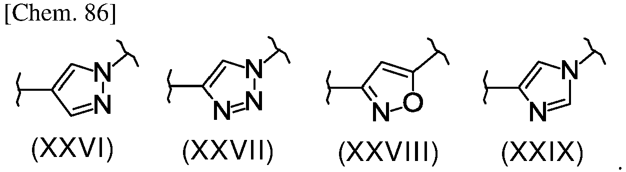
(15-8) The compound or a salt thereof described in (15-4) above, wherein A is N, and
   R¹ is the formula (IIb) below, 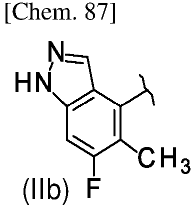
   R³ is -P-Q,
   P is -O- or -N(R^{P})-,
   R^{P} is H or C₁₋₃ alkyl, and
   Q is the formula (V) or the formula (VI) below, 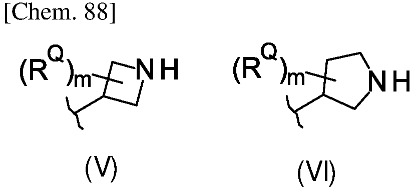
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI),
   m is 0 or 1, and
   X is -O-.
(15-9) The compound or a salt thereof described in (15-4) above, in which A is CH, and
   R¹ is the formula (IIb) below, 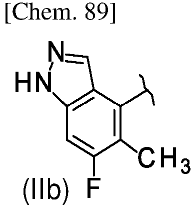
   R³ is -P-Q,
   P is -O-, and
   Q is the formula (V) or the formula (VI) below, 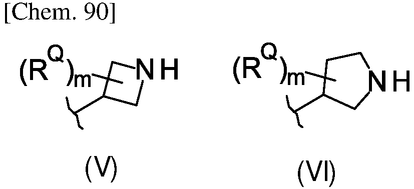
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI),
   m is 0 or 1, and
   X is -O-.
(15-10) The compound or a salt thereof described in (15-4) above, in which A is N, R¹ is the formula (IIb) below, 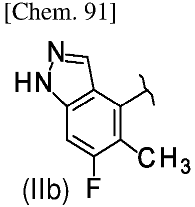
   R³ is V, and
   V is the formula (VII) below, 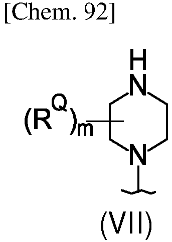
   each R^{Q}, which is the same as or different from each other, are OH or methyl, wherein R^{Q} is attached only to a carbon atom that is a ring-forming atom of a piperazine ring represented by the formula (VII),
   m is 0 or 1, and
   X is -O-.
(15-11) The compound or a salt thereof described in (15-8) above,
   wherein R² is cyclopropyl,
   R³ is -P-Q,
   P is -N(R^{P})-,
   R^{P} is H or C₁₋₃ alkyl, and
   Q is the formula (V) or the formula (VI) below, 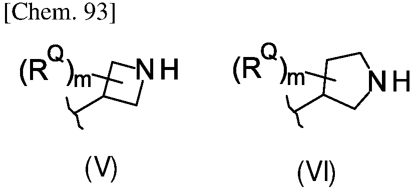
   each R^{Q}, which is the same as or different from each other, are OH or methyl, wherein R^{Q} is attached only to a carbon atom that is an atom forming a ring selected from the group consisting of an azetidine ring represented by the formula (V) and a pyrrolidine ring represented by the formula (VI),
   m is 0 or 1,
   R⁴ is C₁₋₆ alkyl optionally substituted with a group selected from the group consisting of OCH₃ and a tetrahydrofuranyl group or tetrahydropyranyl,
   R⁵ is isopropyl,
   R^{6a} is H, and R^{6b} is C₁₋₃ alkyl optionally substituted with OH, and
   R⁷ is the formula (IX), the formula (X), the formula (XI) or the formula (XII) below, 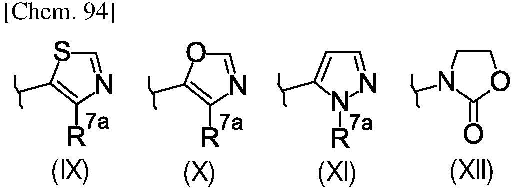
   R^{7a} is C₁₋₃ alkyl optionally substituted with OH, and
   W is the following formula (XIX), 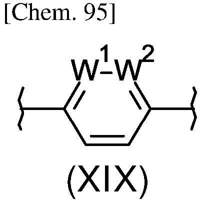
   W¹ and W² are each CH, and
   Y is phenylene optionally substituted with F.
(15-12) The compound or a salt thereof described in (15-8) above, in which R² is cyclopropyl,
   R³ is -P-Q,
   P is -N(R^{P})-,
   R^{P} is methyl or ethyl, and
   Q is the formula (VI), 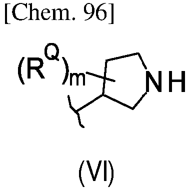
   m is 0,
   R⁵ is isopropyl,
   R^{6a} is H, R^{6b} is hydroxymethyl, and
   R⁷ is the formula (IX) or the formula (XI) below, 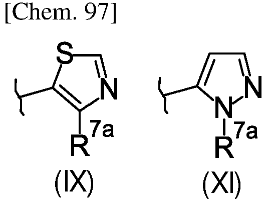
   R^{7a} is C₁₋₃ alkyl, and
   W is the following formula (XIX), 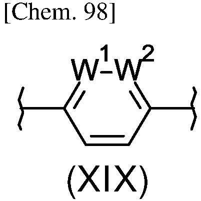
   W¹ and W² are each CH, and
   Y is phenylene optionally substituted with F.
(15-13) The compound or a salt thereof described in (15-12) above, in which R⁷ is the formula (IX) below, 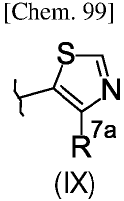 , and
   R^{7a} is C₁₋₃ alkyl.

Embodiments of the compound of the formula (IA) or a salt thereof of the present invention are shown below.
(16-1) A compound or a salt thereof in which R¹ is naphthyl optionally substituted with OH or a group selected from the group consisting of the formula (II), the formula (III) and the formula (IV) below, 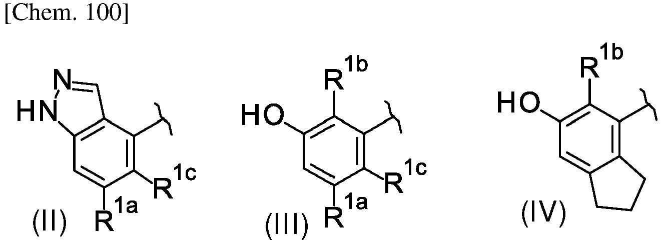
   R^{1a} and R^{1b}, which are the same as or different from each other, are H, methyl, For Cl, and
   R^{1c} is F, Cl, methyl or ethyl.
(16-2) The compound or a salt thereof in which R¹ is the formula (IIa) or the formula (IIIa) below, 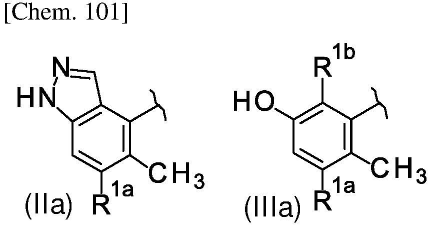 , and
   R^{1a} and R^{1b}, which are the same as or different from each other, are H, methyl, F or Cl.
(16-3) The compound or a salt thereof in which R¹ is the following formula (IIb). 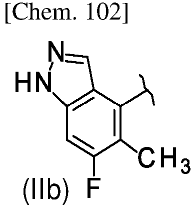
(17-1) The compound or a salt thereof in which E is CH or N,
   G is CR² or N, R² is H, halogen, optionally substituted C₁₋₃ alkyl, cyclopropyl or vinyl,
   A is CR^{A} or N, R^{A} is H or C₁₋₃ alkyl,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 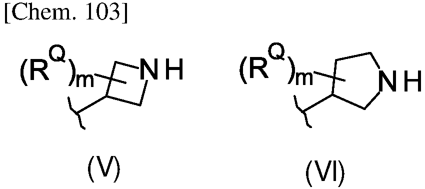
   V is the formula (VII) below, 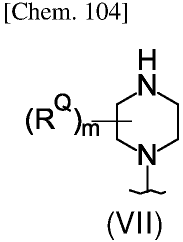
   each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
   (i) when A is CR^{A} or (ii) when A is N, and E or G is N,
   V may be a 7-membered or 8-membered saturated or unsaturated bridged heterocyclic group in addition to the formula (VII), and
   m is 0 to 2.
(17-2) The compound or a salt thereof in which E is CH,
   Gis CR², R² is H, halogen, optionally substituted C₁₋₃ alkyl, cyclopropyl or vinyl,
   A is CR^{A}, R^{A} is H or C₁₋₃ alkyl,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 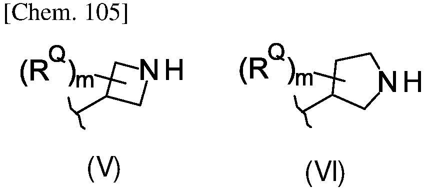
   V is a 7-membered or 8-membered saturated or unsaturated bridged heterocyclic group, and
   m is 0 to 2.
(17-3) The compound or a salt thereof in which E is CH,
   G is CR², R² is cyclopropyl,
   A is CR^{A}, R^{A} is H or C₁₋₃ alkyl,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 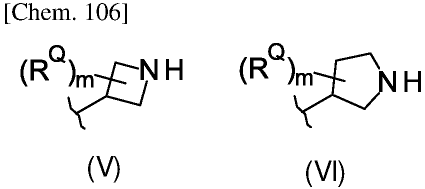
   V is a 7-membered or 8-membered saturated or unsaturated bridged heterocyclic group, and
   m is 0 to 2.
(17-4) The compound or a salt thereof in which E is CH,
   Gis CR², R² is H, halogen, optionally substituted C₁₋₃ alkyl, cyclopropyl or vinyl,
   A is CR^{A}, R^{A} is H,
   R³ is V and
   V is a 7-membered or 8-membered saturated or unsaturated bridged heterocyclic group.
(17-5) The compound or a salt thereof in which E is CH,
   G is CR², R² is cyclopropyl,
   A is CR^{A}, R^{A} is H,
   R³ is V, and
   V is a 7-membered or 8-membered saturated or unsaturated bridged heterocyclic group.
(17-6) The compound or a salt thereof in which E is N,
   Gis CR², R² is H, halogen, optionally substituted C₁₋₃ alkyl, cyclopropyl or vinyl,
   A is N,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 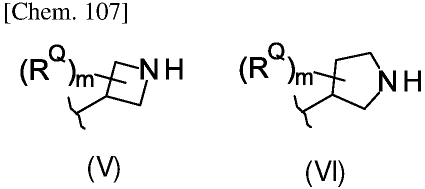
   V is the formula (VII) below or a 7-membered or 8-membered saturated or unsaturated bridged heterocyclic group, 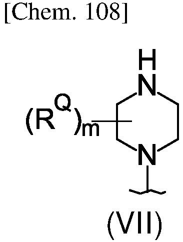
   each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
   m is 0 to 2.
(17-7) The compound or a salt thereof in which E is N,
   G is CR², R² is cyclopropyl,
   AisN,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 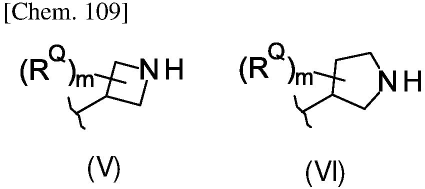
   V is the formula (VII) below or a 7-membered or 8-membered saturated or unsaturated bridged heterocyclic group, 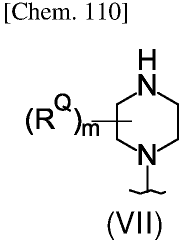
   each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
   m is 0 to 2.
(18-1) The compound or a salt thereof in which R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms.
(18-2) The compound or a salt thereof in which R⁴ is C₁₋₆ alkyl optionally substituted with a group selected from the group consisting of OCH₃ and a tetrahydrofuranyl group or tetrahydropyranyl.
(19-1)The compound or a salt thereof in which R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen.
(19-2) The compound or a salt thereof in which R⁵ is isopropyl or C₃₋₆ cycloalkyl.
(20-1) The compound or a salt thereof in which R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen.
(20-2) The compound or a salt thereof in which R^{6a} is H, and R^{6b} is C₁₋₃ alkyl optionally substituted with OH.
(21-1) The compound or a salt thereof in which R⁷ is H, halogen, C₁₋₃ alkyl, - SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms.
(21-2) The compound or a salt thereof in which R⁷ is the formula (IX), the formula (X), the formula (XI) or the formula (XII) below, 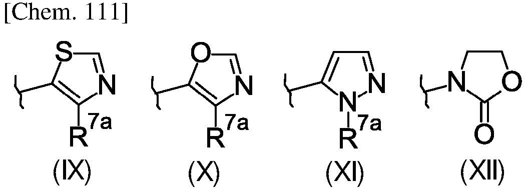 , and
   R^{7a} is C₁₋₃ alkyl optionally substituted with OH.
(22-1) The compound or a salt thereof in which L^{Y} is -O-(optionally substituted C₁₋₃ alkylene)-, -S-(optionally substituted C₁₋₃ alkylene)-, -SO₂-(optionally substituted C₁₋₃ alkylene)-, -NR^{Y}-(optionally substituted C₁₋₃ alkylene)-, -(optionally substituted C₁₋₃ alkylene)-O-, -(optionally substituted C₁₋₃ alkylene)-S-, -(optionally substituted C₁₋₃ alkylene)-SO₂- or -(optionally substituted C₁₋₃ alkylene)-NR^{Y}-, and
   R^{Y} is H or C₁₋₃ alkyl.
(22-2) The compound or a salt thereof in which L^{Y} is -S-(optionally substituted C₁₋₃ alkylene)-, -SO₂-(optionally substituted C₁₋₃ alkylene)-, -NR^{Y}-(optionally substituted C₁₋₃ alkylene)-, -(optionally substituted C₁₋₃ alkylene)-O-, -(optionally substituted C₁₋₃ alkylene)-S-, -(optionally substituted C₁₋₃ alkylene)-SO₂- or -(optionally substituted C₁₋₃ alkylene)-NR^{Y}-, and
   R^{Y} is H or C₁₋₃ alkyl.
(22-3) The compound or a salt thereof in which L^{Y} is -S-(optionally substituted C₁₋₃ alkylene)-, -SO₂-(optionally substituted C₁₋₃ alkylene)-, -NR^{Y}-(optionally substituted C₁₋₃ alkylene)-, -(optionally substituted C₁₋₃ alkylene)-S-, -(optionally substituted C₁₋₃ alkylene)-SO₂- or -(optionally substituted C₁₋₃ alkylene)-NR^{Y}-, and
   R^{Y} is H or C₁₋₃ alkyl.
(22-4) The compound or a salt thereof in which L^{Y} is -O-(optionally substituted C₁₋₃ alkylene)-.
(23-1) The compound or a salt thereof in which
   R^{P1} is OH or F,
   R^{P2a} is H or F, and
   R^{P2b} is H.
(23-2) The compound or a salt thereof in which
   R^{P1} is F,
   R^{P2a} is H or F, and
   R^{P2b} is H.
(23-3) The compound or a salt thereof in which
   R^{P1} is OH,
   R^{P2a} is H or F,
   R^{P2b} is H.
(23-4) The compound or a salt thereof in which
   R^{P1} is OH, and
   R^{P2a} and R^{P2b} are each H.
(24-1) The compound of the formula or a salt thereof in which W is optionally substituted phenylene or 6-membered optionally substituted heteroarenediyl containing one to three nitrogen atoms.
(24-2) The compound or a salt thereof in which W is the following formula (XIX), 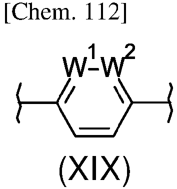 and
   W¹ and W² are each CH.
(25-1) The compound or a salt thereof in which X is a bond, -CH₂-, -O-, -S- or - Nr^{4x}-, and
   R^{4x} is H or C₁₋₃ alkyl.
(25-2) The compound or a salt thereof in which X is -O-.
(26-1) The compound or a salt thereof in which Y is phenylene or pyridinediyl, where the phenylene is optionally substituted with F.
(26-2) The compound or a salt thereof in which Y is phenylene optionally substituted with F.
(27-1) The compound or a salt thereof in which L is -(L¹-L²-L³-L⁴)-,
   L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O, and
   R^{L1} is H or C₁₋₃ alkyl.
(27-2) The compound or a salt thereof in which L is a bond.
(28-1) The compound or a salt thereof in which Z is NH or 5-membered heteroarenediyl containing one to four hetero atoms selected from the group consisting of oxygen, sulfur and nitrogen.
(28-2) The compound or a salt thereof in which Z is the following formula (XXVII)-1. 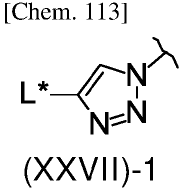 (In the formulae, L* represents linking to L.)
(29-1) The compound or a salt thereof in which Y-L-Z is the formula (VIII) below. 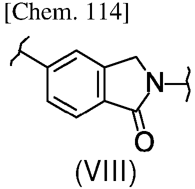
(30) The compound or a salt thereof which is a combination of any compatible two or more of the embodiments described in (16-1) to (29-1) above.

Specific examples of the combination described in (30) above include the following embodiments.
(30-1) A compound of the formula (IA) or a salt thereof, 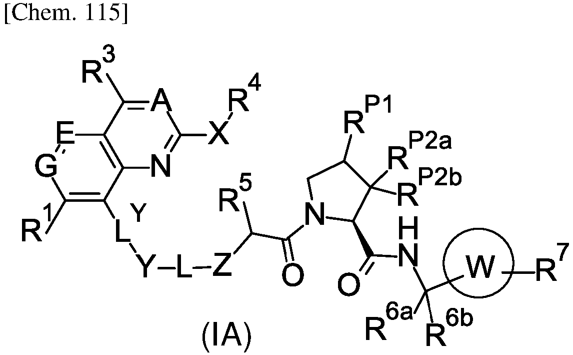 (wherein in the formula,
   A is CR^{A} or N,
   R^{A} is H or C₁₋₃ alkyl,
   E is CH or N,
   G is CR² or N, and
   R¹ is naphthyl optionally substituted with OH or a group selected from the group consisting of the formula (II), the formula (III) and the formula (IV) below, 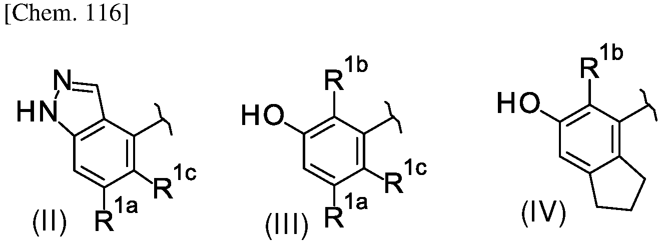
   R^{1a} and R^{1b}, which are the same as or different from each other, are H, methyl, For Cl,
   R^{1c} is F, Cl, methyl or ethyl,
   R² is H, halogen, optionally substituted C₁₋₃ alkyl, cyclopropyl or vinyl,
   R³ is -P-Q or V,
   P is -CH₂-, -O- or -N(R^{P})-,
   R^{P} is H or optionally substituted C₁₋₃ alkyl, and
   Q is the formula (V) or the formula (VI) below, 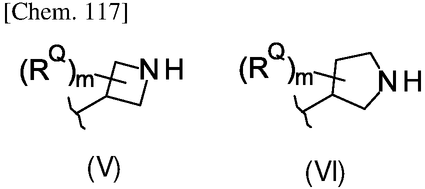
   V is the formula (VII) below, 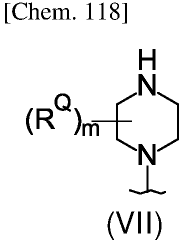
   each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
   (i) when A is CR^{A} or (ii) when A is N, and E or G is N,
   V may be a 7-membered or 8-membered saturated or unsaturated bridged heterocyclic group in addition to the formula (VII),
   m is 0 to 2,
   R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms,
   R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen,
   R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen,
   R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms,
   L^{Y} is -O-(optionally substituted C₁₋₃ alkylene)-, -S-(optionally substituted C₁₋₃ alkylene)-, -SO₂-(optionally substituted C₁₋₃ alkylene)-, -NR^{Y}-(optionally substituted C₁₋₃ alkylene)-, -(optionally substituted C₁₋₃ alkylene)-O-, -(optionally substituted C₁₋₃ alkylene)-S-, -(optionally substituted C₁₋₃ alkylene)-SO₂- or -(optionally substituted C₁₋₃ alkylene)-NR^{Y}-,
   R^{Y} is H or C₁₋₃ alkyl,
   R^{P1} is OH or F,
   R^{P2a} is H or F,
   R^{P2b} is H,
   W is optionally substituted phenylene or 6-membered optionally substituted heteroarenediyl containing one to three nitrogen atoms,
   X is a bond, -CH₂-, -O-, -S- or -NR^{4x}-,
   R^{4x} is H or C₁₋₃ alkyl,
   Y is phenylene or pyridinediyl, wherein the phenylene may be substituted with F,
   L is -(L¹-L²-L³-L⁴)-,
   L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
   R^{L1} is H or C₁₋₃ alkyl, and
   Z is NH or 5-membered heteroarenediyl containing one to four hetero atoms selected from the group consisting of oxygen, sulfur and nitrogen, or Y-L-Z is the formula (VIII) below.)

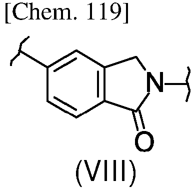

Examples of specific compounds included in the present invention include the following compounds in one embodiment.

A compound or a salt thereof selected from the group consisting of
(4R)-1-[(2S)-2-{4-[4-({[6-cyclopropyl-7-(6-fluoro-5-methyl-1H-indazol-4-yl)-4-{ methyl[(3S)-pyrrolidin-3-yl] amino }-2- { [(2R)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({[6-cyclopropyl-7-(6-fluoro-5-methyl-1H-indazol-4-yl)-4-{ methyl[(3S)-pyrrolidin-3-yl] amino }-2- { [(2S)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({[6-cyclopropyl-7-(6-fluoro-5-methyl-1H-indazol-4-yl)-2-[(2S)-2-methoxypropoxy]-4-{ methyl[(3S)-pyrrolidin-3-yl] amino } quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({[6-cyclopropyl-7-(6-fluoro-5-methyl-1H-indazol-4-yl)-2-[(2S)-2-methoxypropoxy]-4-{ methyl[(3S)-pyrrolidin-3-yl] amino } quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl } -4-hydroxy-L-prolinamide,
(4R)-1-[(2S)-2-{4-[4-({[6-cyclopropyl-7-(6-fluoro-5-methyl-1H-indazol-4-yl)-2-[(2S)-2-methoxypropoxy]-4-{ methyl[(3S)-pyrrolidin-3-yl] amino } quinazolin-8-yl]oxy}methyl}methyl)-2-fluorophenyl]-1H-1,2,3-triazol-1-yl}-3-methylbutanoyl]-4-hydroxy-N-{ (1R)-2-hydroxy-1-[4-(4-methyl-1,3-thiazol-5-yl)phenyl]ethyl}-L-prolinamide,
(4R)-1-{(2S)-2-[4-(4-{[(6-cyclopropyl-4-{ethyl[(3S)-pyrrolidin-3-yl]amino}-7-(6-fluoro-5-methyl-1H-indazol-4-yl)-2-[(2S)-2-methoxypropoxy]quinazolin-8-yl)oxy]methyl}phenyl)-1H-1,2,3-triazol-1-yl]-3-methylbutanoyl}-4-hydroxy-N-{(1R)-2-hydroxy-1-[4-(4-methyl-1,3-thiazol-5-yl)phenyl]ethyl}-L-prolinamide, and
(4R)-1-[(2S)-2-{4-[4-({ [6-cyclopropyl-7-(6-fluoro-5-methyl-1H-indazol-4-yl)-2-[(2S)-2-methoxypropoxy]-4-{ methyl[(3R)-pyrrolidin-3-yl] amino } quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide.

Examples of specific compounds included in the present invention include the following compounds in one embodiment.

A compound or a salt thereof selected from the group consisting of
(4R)-1-[(2S)-2-{4-[4-({ [(7M)-6-cyclopropyl-7-(6-fluoro-5-methyl-1H-indazol-4-yl)-4-{ methyl[(3S)-pyrrolidin-3-yl] amino }-2-{ [(2R)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({ [(7M)-6-cyclopropyl-7-(6-fluoro-5-methyl-1H-indazol-4-yl)-4-{ methyl[(3S)-pyrrolidin-3-yl] amino }-2-{ [(2S)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({ [(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({ [(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl } -4-hydroxy-L-prolinamide,
(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl}methyl)-2-fluorophenyl]-1H-1,2,3-triazol-1-yl}-3-methylbutanoyl]-4-hydroxy-N-{ (1R)-2-hydroxy-1-[4-(4-methyl-1,3-thiazol-5-yl)phenyl]ethyl }-L-prolinamide,
(4R)-1-[(2S)-2-(4-{4-[({(7M)-6-cyclopropyl-4-{ethyl[(3S)-pyrrolidin-3-yl]amino}-7-(6-fluoro-5-methyl-1H-indazol-4-yl)-2-[(2S)-2-methoxypropoxy]quinazolin-8-yl}oxy)methyl)phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, and
(4R)-1-[(2S)-2-{4-[4-({[(7M)-6-cyclopropyl-7-(6-fluoro-5-methyl-1H-indazol-4-yl)-2-[(2S)-2-methoxypropoxy] -4- { methyl[(3R)-pyrrolidin-3-yl] amino } quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide.

Examples of specific compounds included in the present invention include the following compounds in one embodiment.

A compound or a salt thereof selected from the group consisting of
(4R)-1-[(2S)-2-{4-[4-({[(7P)-6-cyclopropyl-7-(6-fluoro-5-methyl-1H-indazol-4-yl)-4-{ methyl[(3S)-pyrrolidin-3-yl] amino }-2-{ [(2R)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({ [(7P)-6-cyclopropyl-7-(6-fluoro-5-methyl-1H-indazol-4-yl)-4-{ methyl[(3S)-pyrrolidin-3-yl] amino }-2-{ [(2S)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({ [(7P)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({ [(7P)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl] -2-hydroxyethyl} -4-hydroxy-L-prolinamide,
(4R)-1-[(2S)-2-{4-[4-({[(7P)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl}methyl)-2-fluorophenyl]-1H-1,2,3-triazol-1-yl}-3-methylbutanoyl]-4-hydroxy-N-{ (1R)-2-hydroxy-1-[4-(4-methyl-1,3-thiazol-5-yl)phenyl]ethyl }-L-prolinamide,
(4R)-1-[(2S)-2-(4-{4-[({(7P)-6-cyclopropyl-4-{ethyl[(3S)-pyrrolidin-3-yl]amino}-7-(6-fluoro-5-methyl-1H-indazol-4-yl)-2-[(2S)-2-methoxypropoxy]quinazolin-8-yl}oxy)methyl)phenyl}-1H-1,2,3-triazol-1-yl)-3-methylbutanoyl] -4-hydroxy-N-{(1R)-2-hydroxy-1-[4-(4-methyl-1,3-thiazol-5-yl)phenyl]ethyl}-L-prolinamide, and
(4R)-1-[(2S)-2-{4-[4-({[(7P)-6-cyclopropyl-7-(6-fluoro-5-methyl-1H-indazol-4-yl)-2-[(2S)-2-methoxypropoxy]-4-{methyl[(3R)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide.

The compounds of the formula (I) and the formula (IA) may have tautomers or geometrical isomers depending on the type of the substituent. In this specification, the compounds of the formula (I) and the formula (IA) are sometimes described only as one of isomers, but the present invention includes isomers other than the above one and includes separated isomers or mixtures thereof.

In addition, the compounds of the formula (I) and the formula (IA) may have an asymmetric carbon atom or an axial chirality and may have diastereomers based on them. The present invention includes separated diastereomers of the compounds of the formula (I) and the formula (IA) or mixtures thereof.

Furthermore, the present invention includes pharmaceutically acceptable prodrugs of the compounds represented by the formula (I) and the formula (IA). A pharmaceutically acceptable prodrug is a compound having a group that can be converted into an amino group, a hydroxy group, a carboxyl group or the like by solvolysis or under physiological conditions. Examples of groups to form a prodrug include groups described in Prog. Med., 1985, 5, p.2157-2161 or in "Iyakuhin no Kaihatsu (development of pharmaceuticals)", Vol.7, Bunshi-sekkei (molecular design), Hirokawa Shoten, 1990, p.163-198.

In addition, the salts of the compounds of the formula (I) and the formula (IA) are pharmaceutically acceptable salts of the compound of the formula (I) and the formula (IA) and may be an acid addition salt or a salt formed with a base depending on the type of the substituent. Examples thereof include salts shown in P. Heinrich Stahl, Handbook of Pharmaceutical Salts Properties, Selection, and Use, Wiley-VCH, 2008. Specific examples include an acid addition salt with an inorganic acid, such as hydrochloric acid, hydrobromic acid, hydroiodic acid, sulfuric acid, nitric acid or phosphoric acid, or with an organic acid, such as formic acid, acetic acid, propionic acid, oxalic acid, malonic acid, succinic acid, fumaric acid, maleic acid, lactic acid, malic acid, mandelic acid, tartaric acid, dibenzoiltartaric acid, ditoluoyltartaric acid, citric acid, methanesulfonic acid, ethanesulfonic acid, benzenesulfonic acid, p-toluenesulfonic acid, aspartic acid or glutamic acid, a salt with an inorganic metal, such as sodium, potassium, magnesium, calcium or aluminum, a salt with an organic base, such as methylamine, ethylamine or ethanolamine, a salt with various amino acids and amino acid derivatives, such as acetylleucine, lysine or ornithine, an ammonium salt and the like.

Furthermore, the present invention also includes various hydrates, solvates and crystal polymorphism substances of the compounds of the formula (I) and the formula (IA) and salts thereof.

The present invention also includes all the compounds of the formula (I) and the formula (IA) or salts thereof which are labeled with one or more pharmaceutically acceptable radioactive or non-radioactive isotopes. Examples of suitable isotopes used for isotopic labeling of the compound of the present invention include isotopes of hydrogen (²H, ³H and the like), carbon (¹¹C, ¹³C, ¹⁴C and the like), nitrogen (¹³N, ¹⁵N and the like), oxygen (¹⁵O, ¹⁷O, ¹⁸O and the like), fluorine (¹⁸F and the like), chlorine (³⁶Cl and the like), iodine (¹²³I, ¹²⁵I and the like) and sulfur (³⁵S and the like).

The isotope-labeled compound of the invention of the present application can be used for research and the like such as research on tissue distribution of drugs and/or substrates. For example, radioactive isotopes such as tritium (³H) and carbon 14 (¹⁴C) can be used for this purpose due to the easiness of labeling and the convenience of detection.

Substitution by a heavier isotope, for example, substitution of hydrogen by deuterium (²H), is therapeutically advantageous through the improvement of metabolic stability in some cases (for example, increase in the *in vivo* half-life, decrease in the required dose or decrease in the interaction between drugs).

Substitution by a positron-emitting isotope (¹¹C, ¹⁸F, ¹⁵O, ¹³N or the like) can be used in a positron emission tomography (PET) test for testing occupancy of a substrate receptor.

The isotope-labeled compound of the present invention can be generally produced by a conventional method known to a person skilled in the art or by the same production methods as in the Examples or the Production Examples and the like using suitable reagents which are labeled with an isotope in place of unlabeled reagents.

### (Production method)

The compounds of the formula (I) and the formula (IA) and salts thereof can be produced by applying various known synthetic methods using characteristics based on the basic structure or the type of substituent thereof. Here, depending on the type of functional group, it is sometimes effective as a production technique to substitute the functional group with an appropriate protective group (a group that can be easily converted to the functional group) in the process from a raw material to an intermediate. Examples of the protective group include protective groups described in P. G. M. Wuts and T. W. Greene, "Greene's Protective Groups in Organic Synthesis", 5th edition, John Wiley & Sons Inc., 2014 and the like, and a group appropriately selected from the protective groups is used depending on the reaction conditions. In such a method, a reaction is carried out with the protective group introduced, and then the protective group is removed, as required, whereby a desired compound can be obtained.

In addition, prodrugs of the compounds of the formula (I) and the formula (IA) can be produced by introducing a special group in a process from a raw material to an intermediate as for the above protective group or by further carrying out a reaction using the compounds of the formula (I) and the formula (IA) obtained. This reaction can be carried out by applying a method known to a parson skilled in the art, such as common esterification, amidation or dehydration.

Typical methods for producing the compounds of the formula (I) and the formula (IA) will be described below. The production methods can also be carried out with reference to a reference attached to the description. Note that the production method of the present invention is not limited to the examples described below. The compound of the formula (IA) can be obtained by the same method as the typical production method for the compound of the formula (I), using a raw material compound corresponding to the compound of the formula (IA).

In this specification, the following abbreviations are sometimes used.

DMF: N,N-dimethylformamide, DMAc: N,N-dimethylacetamide, THF: tetrahydrofuran, MeCN: acetonitrile, MeOH: methanol, EtOH: ethanol, iPrOH: isopropyl alcohol, tBuOH: tert-butanol, DOX: 1,4-dioxane, DMSO: dimethyl sulfoxide, TEA: triethylamine, DIPEA: N,N-diisopropylethylamine, tBuOK: potassium tert-butoxide, PdCl₂(dppf) · CH₂Cl₂: [1,1'-bis(diphenylphosphino)ferrocene]palladium(II) dichloride · dichloromethane adduct, Pd/C: palladium on carbon, PyBOP: (benzotriazol-1-yloxy)tripyrrolidinophosphonium hexafluorophosphate, NMP: N-methyl-2-pyrrolidone.

### (Production Method 1)

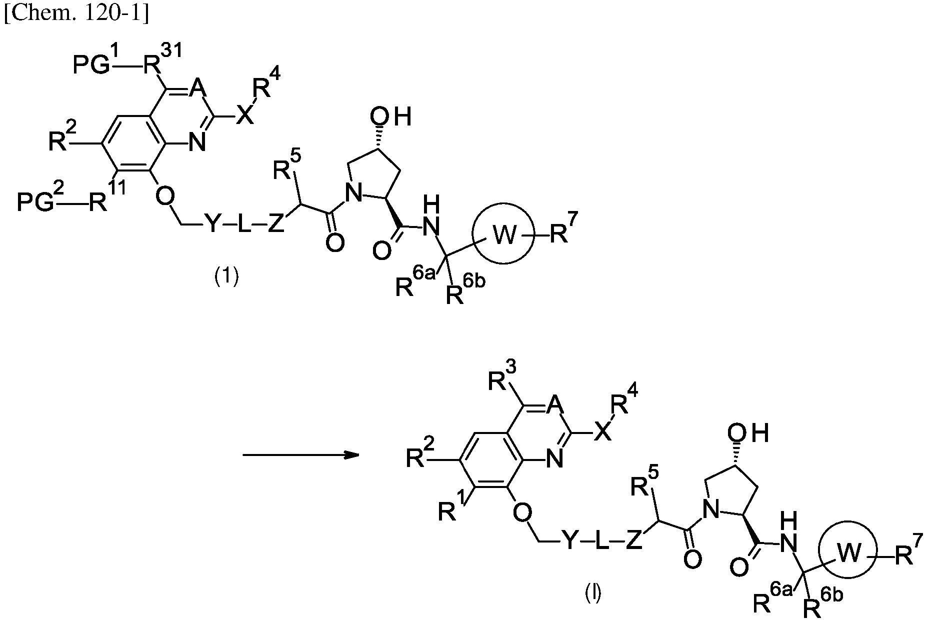

(In the formula, PG¹ represents a protective group of NH contained in R³, R³¹ represents a divalent group obtained by eliminating H from NH contained in R³, PG² represents a protective group of NH or OH contained in R¹ or a hydrogen atom, and R¹¹ represents a divalent group obtained by eliminating H from NH contained in R¹. The same shall apply hereinafter.)

The compound of the formula (I) can be obtained by subjecting a compound (1) to a deprotection reaction. Here, examples of the protective group which can be removed under acidic conditions include a tert-butoxycarbonyl group, a triphenylmethyl group, a tetrahydro-2H-pyran-2-yl group, a methoxymethyl group, a dimethylmethanediyl group, a tert-butylsulfinyl group and the like.

This reaction is performed by stirring, from under cooling to reflux with heat, generally for 0.1 hours to 5 days. Examples of the solvent used here include, but are not particularly limited to, an alcohol, such as MeOH or EtOH, a halogenated hydrocarbon, such as dichloromethane, 1,2-dichloroethane or chloroform, an ether, such as diethyl ether, THF, DOX or dimethoxyethane, DMF, DMSO, MeCN or water and a mixture thereof. Examples of the deprotection reagent include, but are not particularly limited to, acids such as hydrogen chloride (DOX solution), trifluoroacetic acid, methanesulfonic acid, phosphoric acid and p-toluenesulfonic acid.

By selecting a protective group, deprotection can also be performed by a catalytic hydrogenation reaction. Examples of the protective group include a benzyl group, a p-methoxybenzyl group, a benzyloxycarbonyl group and the like. Moreover, deprotection can also be performed with a fluoride ion source such as tetra-n-butylammonium fluoride. Examples of the protective group include a tert-butyl(dimethyl)silyl group, a (trimethylsilyl)ethoxymethyl group and the like. Examples of the protective group which can be removed under basic conditions include an acetyl group, a trifluoroacetyl group, a benzoyl group and the like. Moreover, the deprotection can also be performed in stages by selecting protective groups which can be removed under different deprotection conditions as PG¹ and PG².

For example, the following can be referred as a reference about this reaction.

P. G. M. Wuts and T. W. Greene, "Greene's Protective Groups in Organic Synthesis", 5th edition, John Wiley & Sons Inc., 2014

Note that, when the compound (1) as a raw material has an axial chirality, a stereoisomer which is obtained by once separating the compound (1) may be used for this reaction.

By subjecting the compound of the formula (I) to the following operation as a salt formation reaction, the hydrochloride salt of the compound of the formula (I) can be obtained.

The compound of the formula (I), which is believed to form a salt with hydrogen chloride due to the characteristics of the chemical structure thereof, is dissolved in CH₂Cl₂ and MeOH, and after adding hydrogen chloride (4M DOX solution, 10 equivalents) under ice-bath cooling, and the mixture is stirred under ice-bath cooling for 30 minutes. The reaction mixture is concentrated under reduced pressure, and after adding diethyl ether to the resulting residue, the produced solid is collected by filtration and is dried under reduced pressure, thus obtaining the hydrochloride salt of the compound of the formula (I).

By subjecting the hydrochloride salt of the compound of the formula (I) to the following operation as a desalting reaction, the free form of the compound of the formula (I) can be obtained.

The hydrochloride salt of the compound of the formula (I) is purified by ODS column chromatography (MeCN/0.1% aqueous formic acid solution), and after collecting fractions containing the target substance and basifying with saturated aqueous sodium hydrogen carbonate solution, the solution is extracted with CHCl₃/MeOH (5/1). The combined organic layer is dried over anhydrous sodium sulfate, and the solution is concentrated under reduced pressure. The resulting solid is washed with diethyl ether and is dried under reduced pressure, thus obtaining the compound of the formula (I).

### (Production Method 2)

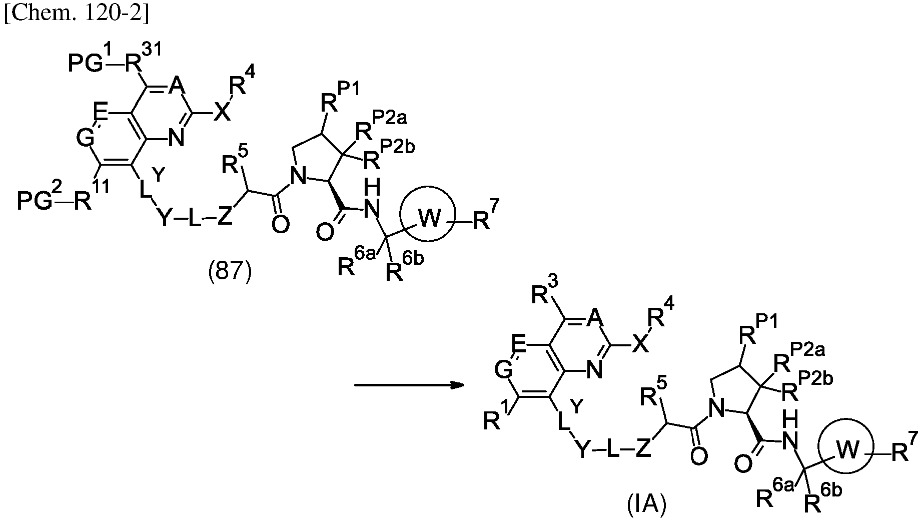

The compound of the formula (IA) can be obtained by subjecting a compound (87) to a deprotection reaction.

The reaction conditions are the same as in the Production Method 1.

### (Raw Material Synthesis 1)

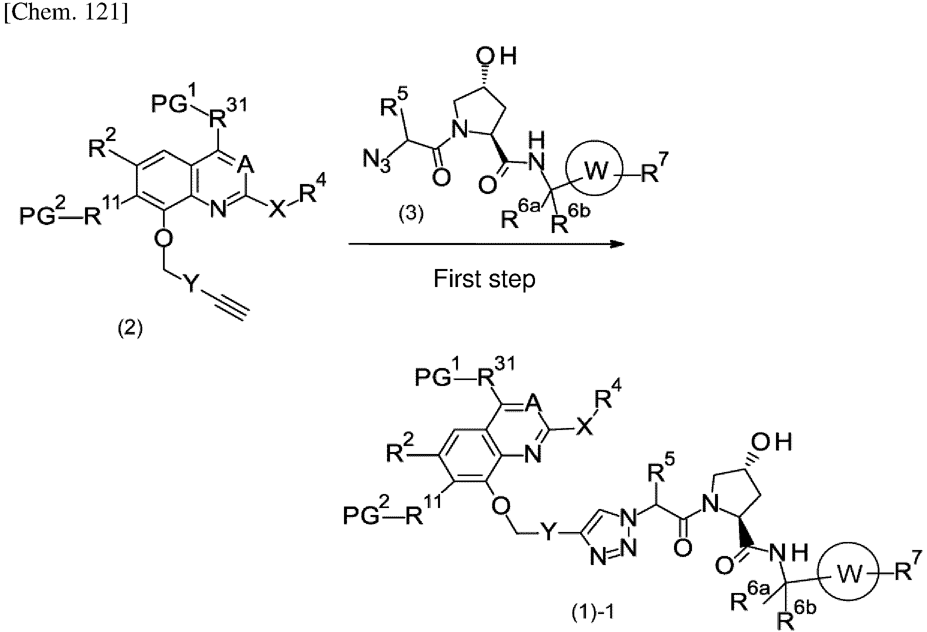

The production method is a first method for producing a compound (1)-1 included in the raw material compound (1).

### (First step)

This step is a method for producing the compound (1)-1 by a cycloaddition reaction of a compound (2) and a compound (3).

In this reaction, the compound (2) and the compound (3) are used in an equal amount or with one compound thereof in a larger amount, and the mixture of the compounds is stirred preferably in the presence of a copper salt, further preferably in the presence of a copper salt and a reductant, in a solvent inactive for the reaction or with no solvent, from under cooling to under reflux with heat, preferably at 0°C to 100°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, DMF, DMSO, ethyl acetate, MeCN, tBuOH, water and a mixture thereof. Examples of the copper salt include CuI, CuSO₄, copper(II) trifluoromethanesulfonate (CuOTf) and the like. Examples of the reductant include sodium ascorbate and the like. Performing the reaction in the presence of TEA, DIPEA, N-methylmorpholine (NMM), 2,6-lutidine, tris[(1-benzyl-1H-1,2,3-triazol-4-yl)methyl]amine (TBTA) or the like is sometimes advantageous for smoothly promoting the reaction.

### [Reference]

Angew. Chem. Int. Ed. 2002, 41, p.2596-2599.

Note that this reaction may be performed using a compound obtained by subjecting PG² of the compound (2) first to a deprotection reaction.

### (Raw Material Synthesis 2)

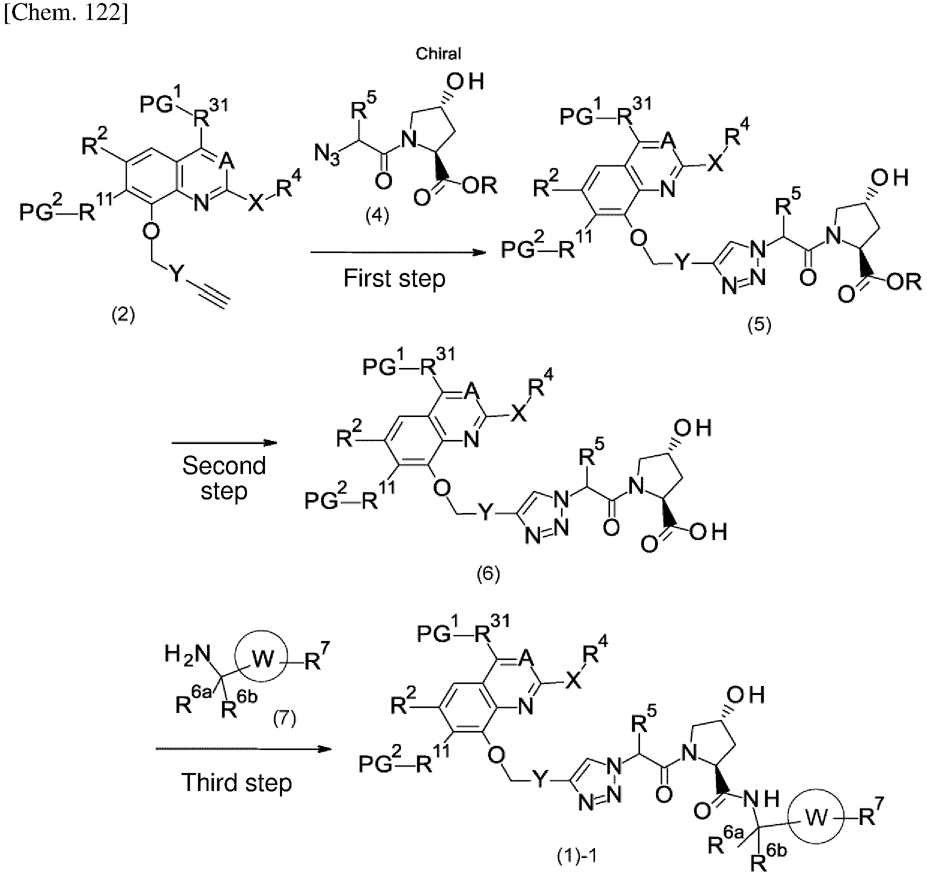

(In the formulae, R represents a C₁₋₃ alkyl group. The same shall apply hereinafter.)

This production method is a second method for producing the compound (1)-1 included in the raw material compound (1).

### (First step)

This step is a method for producing a compound (5) by a cycloaddition reaction of the compound (2) and a compound (4).

The reaction conditions are the same as in the first step of the Raw Material Synthesis 1.

### (Second step)

This step is a method for producing a compound (6) by hydrolysis of the compound (5).

This reaction is performed by stirring the compound (5) from under cooling to under reflux with heat generally for 0.1 hours to 5 days. Examples of the solvent used here include, but are not particularly limited to, an alcohol, acetone, DMF, THF and the like. In addition, a mixed solvent of the above solvent and water is sometimes suitable for the reaction. Examples of the hydrolysis reagent include, but are not particularly limited to, an aqueous sodium hydroxide solution, an aqueous potassium hydroxide solution, trimethyltin hydroxide and the like.

For example, the following can be referred as a reference about this reaction.

The Chemical Society of Japan, "Jikken Kagaku Koza (Courses in Experimental Chemistry) (5th edition)", Vol. 16 (2005) (Maruzen)

Angew. Chem. Int. Ed. 2005, 44, p.1378-1382.

### (Third step)

This step is a method for producing the compound (1)-1 by an amidation reaction of the compound (6) and a compound (7).

In this reaction, the compound (6) and the compound (7) are used in an equal amount or with one compound thereof in a larger amount, and the mixture of the compounds is stirred in the presence of a condensing agent, in a solvent inactive for the reaction, from under cooling to under heating, preferably at -20°C to 60°C, generally for 0.1 hours to 5 days. Examples of the solvent include, but are not particularly limited to, an aromatic hydrocarbon, such as toluene, an ether, such as THF or DOX, a halogenated hydrocarbon, such as dichloromethane, an alcohol, DMF, DMSO, ethyl acetate, MeCN and a mixture thereof. Examples of the condensing agent include (benzotriazol-1-yloxy)tripyrrolidinophosphonium hexafluorophosphate (PyBOP), O-(7-azabenzotriazol-1-yl)-N,N,N',N'-tetramethyluronium hexafluorophosphate (HATU), 1-(3-dimethylaminopropyl)-3-ethylcarbodiimide or the hydrochloride thereof, N,N'-dicyclohexylcarbodiimide (DCC), 1,1'-carbonyldiimidazole (CDI), diphenylphosphoryl azide (DPPA) and the like. Use of an additive (for example, 1-hydroxybenzotriazole) is sometimes preferred for the reaction. Performing the reaction in the presence of an organic base, such as TEA, DIPEA or NMM, or an inorganic base, such as potassium carbonate, sodium carbonate or potassium hydroxide, is sometimes advantageous for smoothly promoting the reaction.

Alternatively, a method in which the compound (6) is converted into a reactive derivative, which is then subjected to an acylation reaction, can be used. Examples of the reactive derivative of a carboxylic acid include an acid halogenation product obtained by a reaction with a halogenating agent, such as phosphorus oxychloride or thionyl chloride, a mixed acid anhydride obtained by a reaction with isobutyl chloroformate or the like, an active ester obtained by condensation with 1-hydroxybenzotriazole or the like and the like. The reaction of such a reactive derivative and the compound (7) can be performed in a solvent inactive for the reaction, such as a halogenated hydrocarbon, an aromatic hydrocarbon or an ether, from under cooling to under heating, preferably at -20°C to 120°C.

### [Reference]

S. R. Sandler and W. Karo, "Organic Functional Group Preparations", 2nd edition, Vol. 1, Academic Press Inc., 1991

The Chemical Society of Japan, "Jikken Kagaku Koza (Courses in Experimental Chemistry) (5th edition)", Vol. 16 (2005) (Maruzen)

### (Raw Material Synthesis 3)

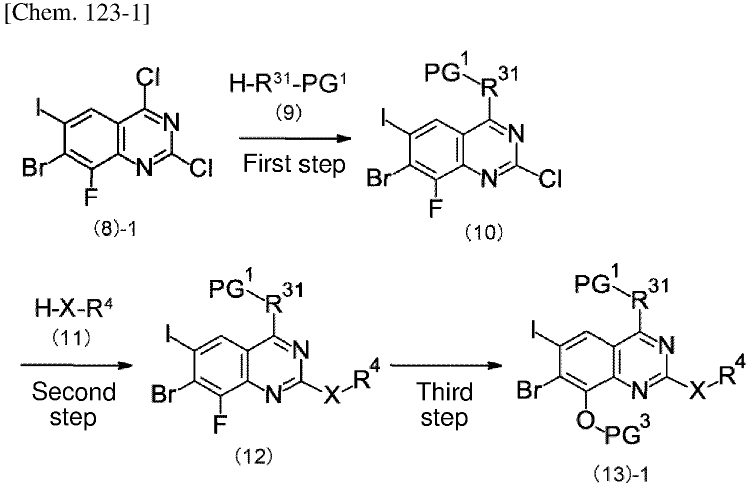

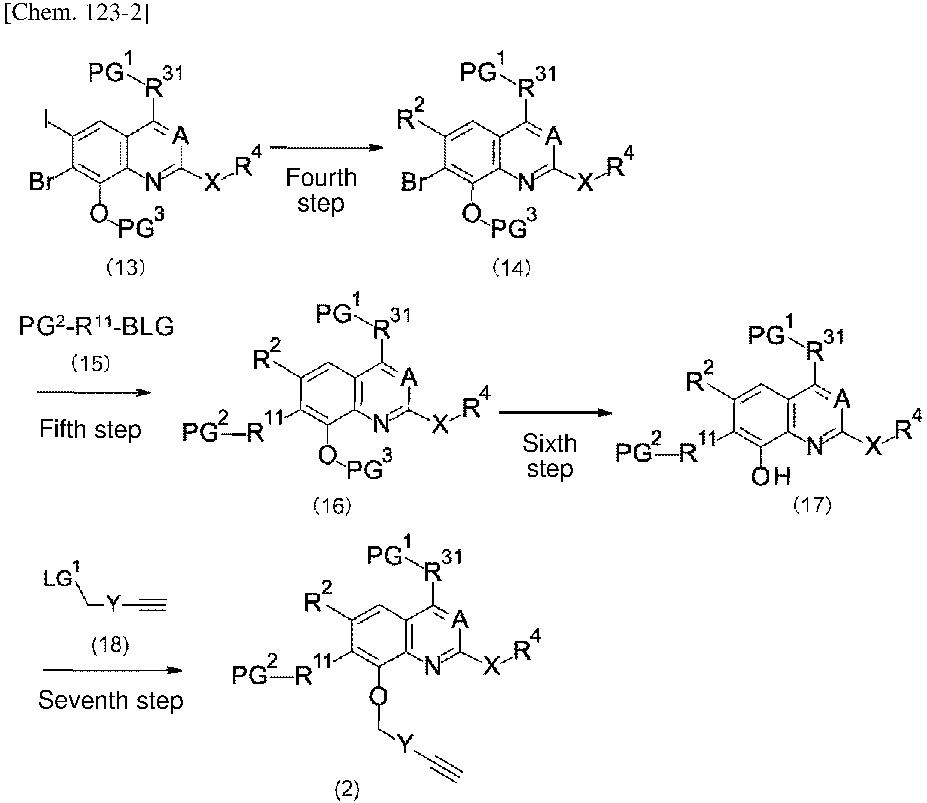

(In the formulae, PG³ represents a protective group of OH, LG¹ represents a leaving group, and BLG represents a boronic acid group, a boronic acid group protected with a protective group of boronic acid such as a boronic acid pinacol ester group or a trifluoroboric acid salt group (hereinafter sometimes described as a boronic acid group or the like). Examples of the leaving group shown here include Cl, Br, a methanesulfonyloxy group, p-toluenesulfonyloxy group and the like.)

This production method is a first method for producing a raw material compound (2).

### (First step)

This step is a method for producing a compound (10) by an ipso substitution reaction of a compound (8)-1 and a compound (9).

In this reaction, the compound (8)-1 and the compound (9) are used in an equal amount or with one compound thereof in a larger amount, and the mixture of the compounds is stirred in a solvent inactive for the reaction or with no solvent, from under cooling to under reflux with heat, preferably at 0°C to 80°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, DMF, DMAc, DMSO, ethyl acetate, MeCN and a mixture thereof. Performing the reaction in the presence of an organic base, such as TEA, DIPEA, N-methylmorpholine (NMM) or1,4-diazabicyclo[2.2.2]octane (DABCO), or an inorganic base, such as sodium hydride, potassium carbonate, sodium carbonate, cesium carbonate or tBuOK, is sometimes advantageous for smoothly promoting the reaction.

Moreover, the compound (10) can be produced by a catalytic hydrogenation reaction of the compound obtained by a Mizoroki-Heck reaction of the compound (8)-1 and the compound (9).

### (Second step)

This step is a method for producing a compound (12) by an ipso substitution reaction of the compound (10) and a compound (11).

The reaction conditions are the same as in the first step of the Raw Material Synthesis 3.

Moreover, the compound (12) can be produced by the Negishi coupling of a compound in which a hydrogen atom of the compound (11) is converted to halogen and the compound (10).

### (Third step)

This step is a method for producing a compound (13)-1 by an ipso substitution reaction of the compound (12) and PG³-OH.

Examples of the PG³-OH used here include benzyl alcohol, p-methoxybenzyl alcohol and 1-phenylethanol.

The reaction conditions are the same as in the first step of the Raw Material Synthesis 3.

### (Fourth step)

This step is a method for producing a compound (14) by a Suzuki-Miyaura coupling reaction of the compound (13) including both of the compound (13)-1 obtained in the third step of this synthesis method and the compound (13)-2 obtained in (Raw Material Synthesis 14) described below and a boronic acid derivative composed of a R²-boronic acid group or the like. Examples of the boronic acid group or the like used here include, but are not particularly limited to, a boronic acid group, a boronic acid ester group, a boronic acid pinacol ester group, a triol borate salt group and a trifluoroboric acid salt group.

In this reaction, the compound (13) and the boronic acid derivative composed of the R²-boronic acid group or the like are used in an equal amount or with one compound thereof in a larger amount, and the mixture of the compounds is stirred in a solvent inactive for the reaction, in the presence of a base and a palladium catalyst, from at room temperature to under reflux with heat, preferably at 20°C to 140°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include inorganic bases, such as tripotassium phosphate, sodium carbonate, potassium carbonate, sodium hydroxide and barium hydroxide. Examples of the palladium catalyst include tetrakis(triphenylphosphine)palladium, bis(triphenylphosphine)palladium(II) dichloride, [1,1'-bis(diphenylphosphino)ferrocene]palladium(II) dichloride · dichloromethane additive, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palladium(II) methanesulfonate, palladium(II) acetate and the like. Performing the reaction in the presence of a ligand, such as dicyclohexyl(2',6'-dimethoxybiphenyl-2-yl)phosphine, dicyclohexyl(2',6'-diisopropoxy-[1,1'-biphenyl]-2-yl)phosphine or 1,1'-bis(diphenylphosphino)ferrocene, is sometimes advantageous for smoothly promoting the reaction. In addition, heating the mixture by microwave irradiation is sometimes advantageous for smoothly promoting the reaction.

### [Reference]

J. Am. Chem. Soc., 2005, 127, p.4685-4696
Org. Lett. 2011, 13, p.3948-3951
Org. Lett. 2012, 14, p.1278-1281
The compound (14) (where R² is hydrogen) can be produced by a dehydrogenation reaction of the compound (13) with a Pd catalyst and a reducing agent.

### [Reference]

J. Org. Chem., 1977, 42, p.3491-3494
Tetrahedron Letters 2013, 54, 5207-5210

### (Fifth step)

This step is a method for producing a compound (16) by a Suzuki-Miyaura coupling reaction of the compound (14) and a compound (15).

The reaction conditions are the same as in the fourth step of the Raw Material Synthesis 3.

When the compound (16) has an axial chirality, the compound (16) is obtained as a mixture of stereoisomers, and each stereoisomer can be isolated by separation using a common separation operation, for example, ODS column chromatography or silica gel column chromatography.

### (Sixth step)

This step is a method for producing a compound (17) by deprotection by a catalytic hydrogenation reaction of the compound (16).

This reaction can be performed by stirring the compound (16) under hydrogen atmosphere, from under normal pressure to under increased pressure, in a solvent inactive for the reaction, such as MeOH, EtOH or ethyl acetate, in the presence of a metal catalyst, from under cooling to under heating, preferably at room temperature, for 1 hour to 5 days. As the metal catalyst, a palladium catalyst, such as Pd/C or palladium black, a platinum catalyst, such as a platinum plate or platinum oxide, a nickel catalyst, such as reduced nickel or Raney nickel, or the like is used.

### (Seventh step)

This step is a method for producing the compound (2) by a reaction of the compound (17) and a compound (18).

This reaction is performed by reacting a mixture of the compound (17) and the compound (18) in an equal amount or with one compound thereof in a larger amount in the presence of a base, in a solvent inactive for the reaction, from under cooling to under reflux with heat, preferably at 0°C to 80°C, generally for 0.1 hours to 5 days. The solvent used here is not particularly limited, and examples thereof include an aromatic hydrocarbon, such as benzene, toluene or xylene, an alcohol, such as MeOH or EtOH, an ether, such as diethyl ether, THF, DOX or 1,2-dimethoxyethane, a halogenated hydrocarbon, such as dichloromethane, 1,2-dichloroethane or chloroform, DMF, DMSO, ethyl acetate, MeCN and a mixture thereof. Examples of the base include, but are not particularly limited to, an organic base, for example, such as TEA, DIPEA, 1,8-diazabicyclo[5.4.0]-7-undecene, n-butyllithium or tBuOK, and an inorganic base, such as sodium hydroxide, sodium carbonate, potassium carbonate, cesium carbonate or sodium hydride. Performing the reaction in the presence of a phase transfer catalyst, such as tetra-n-butylammonium chloride, is sometimes advantageous.

For example, the following can be referred as a reference about this reaction.

The Chemical Society of Japan, "Jikken Kagaku Koza (Courses in Experimental Chemistry)", 5th edition, Vol. 14, Maruzen, 2005

The compound (2) sometimes has an axial chirality and is obtained as a mixture of stereoisomers, and each stereoisomer can be isolated by subjecting the compound (2) in which PG² is a protective group or a compound obtained by subjecting the compound (2) to a deprotection reaction to separation using a common separation operation, for example, ODS column chromatography or silica gel column chromatography.

The reaction conditions for the deprotection reaction used here are the same as in the step described in the Production Method 1.

The compound (18) in which LG¹ is halogen can be produced by halogenating a compound in which the moiety corresponding to LG¹ is a hydroxy group. Examples of the halogenating agent used here include, but are not particularly limited to, for example, thionyl chloride, phosphorus oxychloride, hydrobromic acid, phosphorus tribromide and the like.

For example, the following can be referred as a reference about this reaction.

The Chemical Society of Japan, "Jikken Kagaku Koza (Courses in Experimental Chemistry)" (5th edition), Vol. 13, Maruzen, 2004

The compound (18) in which LG¹ is a sulfonyloxy group can be produced by sulfonylation of a compound in which the moiety corresponding to LG¹ is a hydroxy group in the presence of a base. Examples of the sulfonylating reagent used here include, but are not particularly limited to, for example, methanesulfonylchloride, p-toluenesulfonylchloride, methanesulfonic anhydride and the like. Examples of the base include, but are not particularly limited to, for example, TEA, DIPEA, pyridine, tetramethylethylenediamine and the like.

For example, the following can be referred as a reference about this reaction.

Synthesis 1999, 9, p.1633-1636

### (Raw Material Synthesis 4)

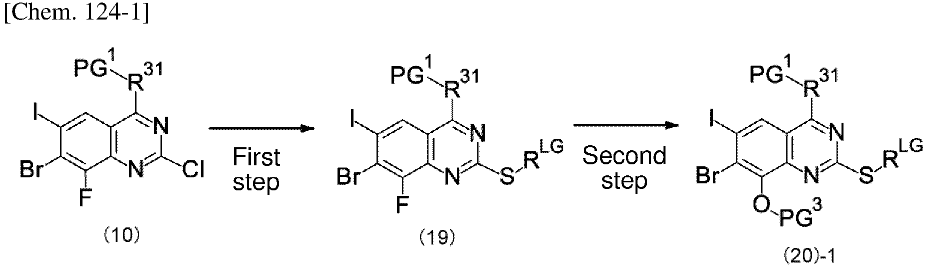

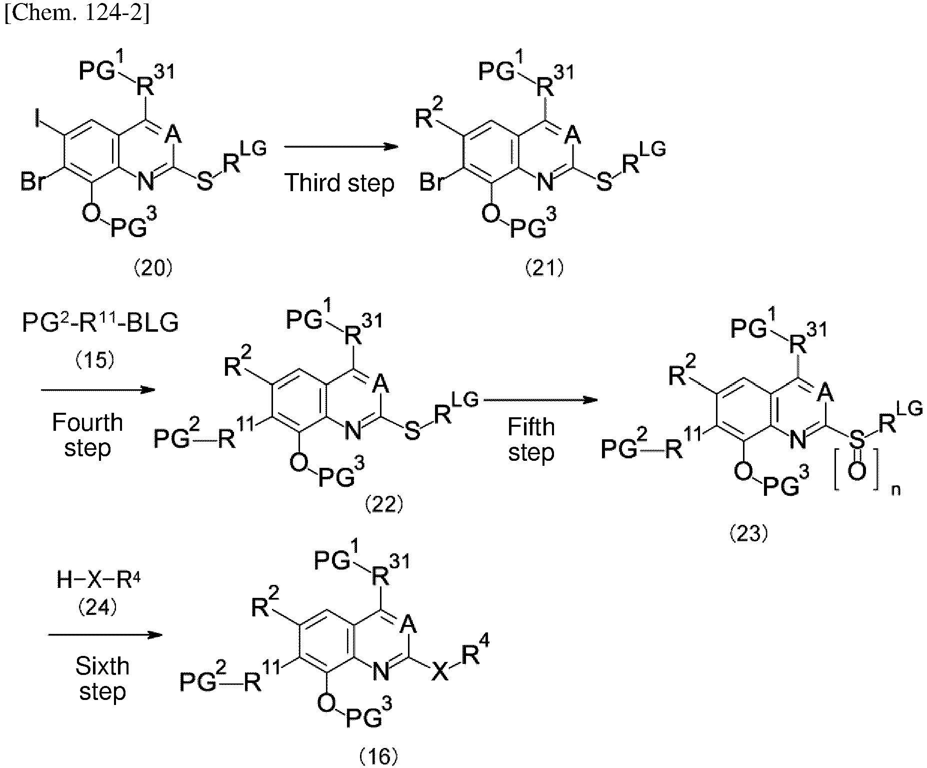

(In the formulae, R^{LG} represents a C₁₋₁₂ alkyl group, and n represents 1 or 2.)

This production method is a second method for producing the raw material compound (16).

### (First step)

This step is a method for producing a compound (19) by an ipso substitution reaction of the compound (10) and R^{LG}-SH. Examples of the R^{LG}-SH used here include C₁₋₁₂ alkylthiols, for example, ethanethiol and dodecanethiol.

The reaction conditions are the same as in the first step of the Raw Material Synthesis 3.

### (Second step)

This step is a method for producing a compound (20)-1 by an ipso substitution reaction of the compound (19) and PG³-OH. Examples of the PG³-OH used here include benzyl alcohol, p-methoxybenzyl alcohol and 1-phenylethanol.

The reaction conditions are the same as in the first step of the Raw Material Synthesis 3.

### (Third step)

This step is a method for producing a compound (21) by a Suzuki-Miyaura coupling reaction of the compound (20) including both of the compound (20)-1 obtained in the second step of this synthesis method and the compound (20)-2 obtained in (Raw Material Synthesis 13) described below and a boronic acid derivative composed of a R²-boronic acid group or the like.

The reaction conditions are the same as in the fourth step of the Raw Material Synthesis 3.

The compound (21) (where R² is hydrogen) can be produced by a dehydrogenation reaction of the compound (20) with a Pd catalyst and a reducing agent.

### [Reference]

J. Org. Chem., 1977, 42, p.3491-3494
Tetrahedron Letters 2013, 54, 5207-5210

### (Fourth step)

This step is a method for producing a compound (22) by a Suzuki-Miyaura coupling reaction of the compound (21) and the compound (15).

The reaction conditions are the same as in the fourth step of the Raw Material Synthesis 3.

### (Fifth step)

This step is a method for producing a compound (23) by an oxidation reaction of the compound (22).

In this reaction, the compound (22) is treated with an oxidant in an equal amount or an excess amount in a solvent inactive for the reaction, from under cooling to under heating, preferably at -20°C to 80°C, generally for 0.1 hours to 3 days. In this reaction, oxidation with m-chloroperbenzoic acid, perbenzoic acid, peracetic acid, sodium hypochlorite or hydrogen peroxide is suitably used. Examples of the solvent include an aromatic hydrocarbon, an ether, a halogenated hydrocarbon such as dichloromethane, DMF, DMSO, ethyl acetate, MeCN and a mixture thereof. Other examples of the oxidant include cumene hydroperoxide, Oxone, active manganese dioxide, chromic acid, potassium permanganate, sodium periodate and the like.

### [Reference]

The Chemical Society of Japan, "Jikken Kagaku Koza (Courses in Experimental Chemistry)", 5th edition, Vol. 17, Maruzen, 2004

### (Sixth step)

This step is a method for producing the compound (16) by an ipso substitution reaction of the compound (23) and a compound (24).

The reaction conditions are the same as in the first step of the Raw Material Synthesis 3.

When the compound (16) has an axial chirality, the compound (16) is obtained as a mixture of stereoisomers, and each stereoisomer can be isolated by separation using a common separation operation, for example, ODS column chromatography or silica gel column chromatography.

### (Raw Material Synthesis 5)

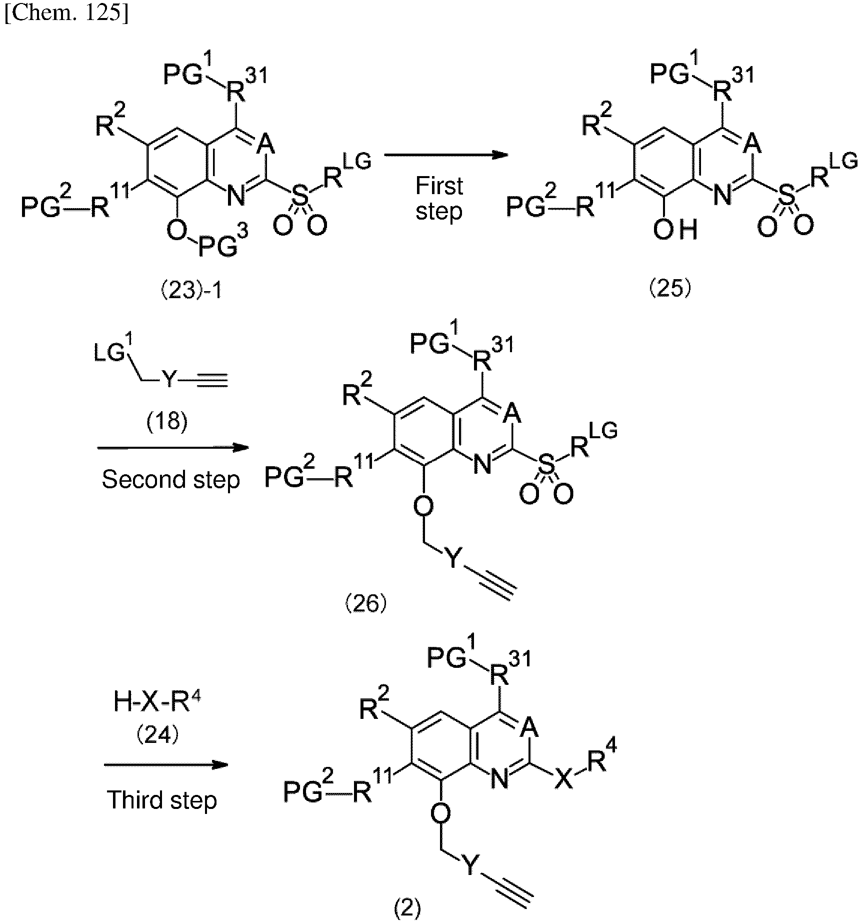

This production method is a second method for producing the raw material compound (2).

### (First step)

This step is a method for producing a compound (25) by deprotection by a catalytic hydrogenation reaction of the compound (23)-1.

The reaction conditions are the same as in the sixth step of the Raw Material Synthesis 3.

### (Second step)

This step is a method for producing a compound (26) by a reaction of the compound (25) and the compound (18).

The reaction conditions are the same as in the seventh step of the Raw Material Synthesis 3.

### (Third step)

This step is a method for producing the compound (2) by an ipso substitution reaction of the compound (26) and the compound (24).

The reaction conditions are the same as in the first step of the Raw Material Synthesis 3.

The compound (2) sometimes has an axial chirality and is obtained as a mixture of stereoisomers, and each stereoisomer can be isolated by subjecting the compound (2) in which PG² is a protective group or a compound obtained by subjecting the compound (2) to a deprotection reaction to separation using a common separation operation, for example, ODS column chromatography or silica gel column chromatography.

The reaction conditions for the deprotection reaction used here are the same as in the step described in the Production Method 1.

### (Raw Material Synthesis 6)

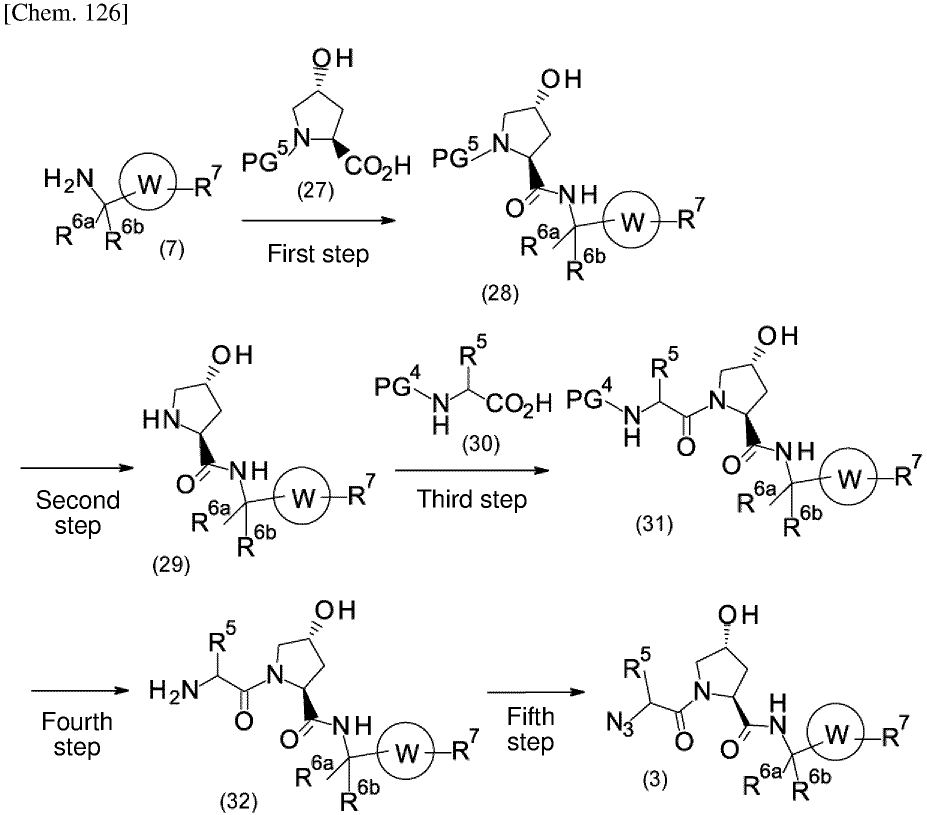

(In the formulae, PG⁴ and PG⁵ represent a protective group.)

This production method is a method for producing the raw material compound (3).

### (First step)

This step is a method for producing a compound (28) by an amidation reaction of the compound (7) and a compound (27).

The reaction conditions are the same as in the third step of the Raw Material Synthesis 2.

### (Second step)

This step is a method for producing a compound (29) by subjecting the compound (28) to a deprotection reaction.

The reaction conditions are the same as in the step described in the Production Method 1.

### (Third step)

This step is a method for producing a compound (31) by an amidation reaction of the compound (29) and a compound (30).

The reaction conditions are the same as in the third step of the Raw Material Synthesis 2.

### (Fourth step)

This step is a method for producing a compound (32) by subjecting the compound (31) to a deprotection reaction.

The reaction conditions are the same as in the step described in the Production Method 1.

### (Fifth step)

This step is a method for producing the compound (3) by a reaction of the compound (32) and a diazo-transfer reagent.

In this reaction, the compound (32) is treated with the diazo-transfer reagent in an equal amount or an excess amount in a solvent inactive for the reaction, from under cooling to under heating, preferably at 0°C to 50°C, generally for 0.1 hours to 3 days. Examples of the diazo-transfer reagent include, but are not particularly limited to, for example trifluoromethanesulfonyl azide, imidazole-1-sulfonyl azide or a salt thereof, 2-azido-1,3-dimethylimidazolinium hexafluorophosphate (ADMP) and the like. Performing the reaction in the presence of an organic base, such as TEA, 4-dimethylaminopyridine (DMAP) or 2,6-lutidine, and a catalytic amount of a copper salt, such as CuSO₄, is sometimes advantageous. Examples of the solvent include THF or a halogenated hydrocarbon, such as dichloromethane, MeCN, an alcohol, water and a mixture thereof.

### [Reference]

J. Org. Chem. 2012, 77, p.1760-1764
Nature 2019, 574, p.86-89
Org. Biomol. Chem. 2014, 12, p.4397-4406

### (Raw Material Synthesis 7)

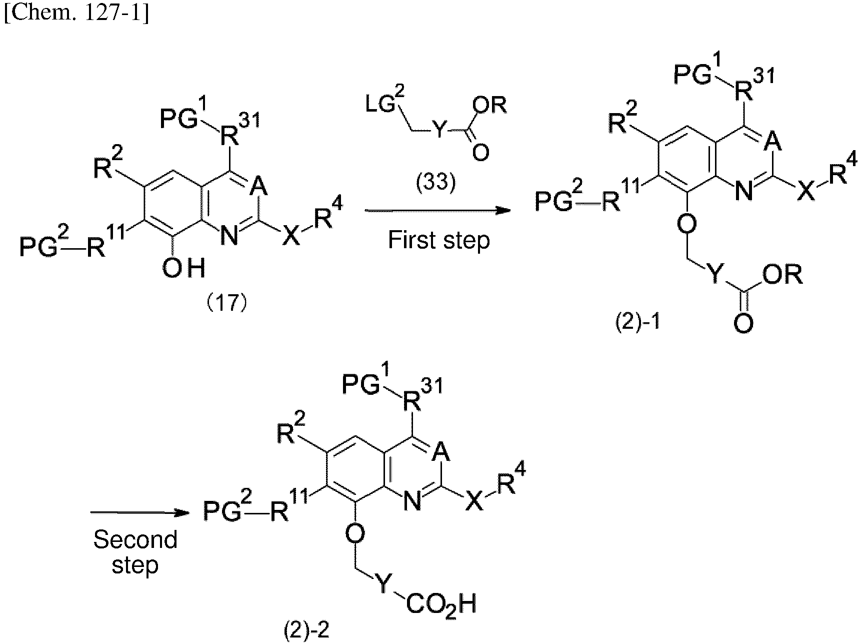

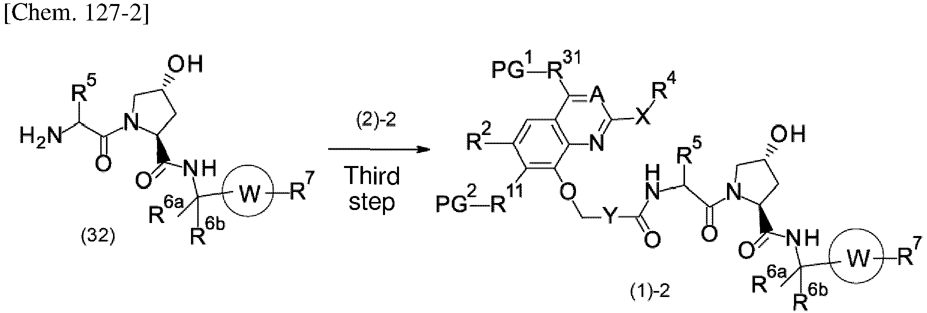

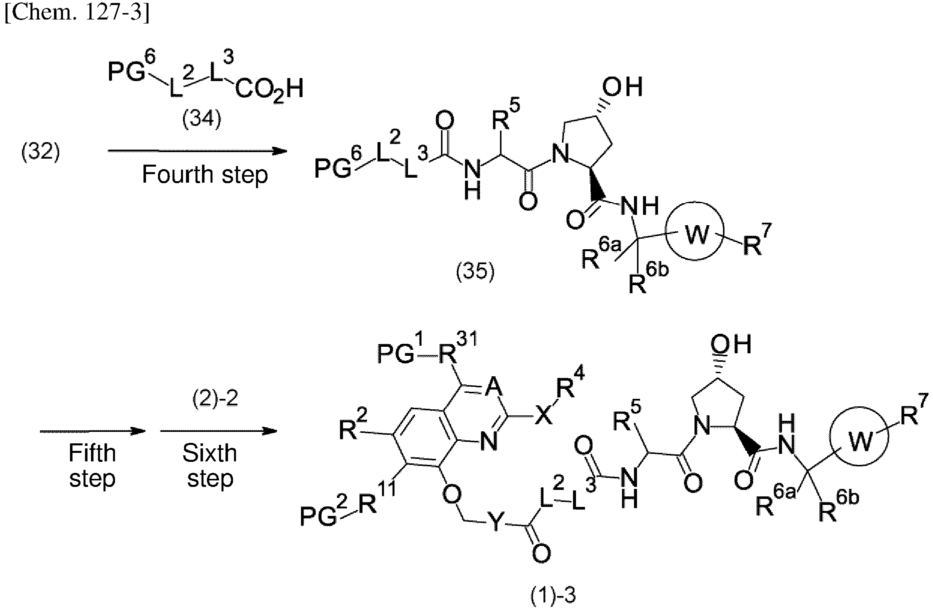

(In the formulae, LG² represents a leaving group, and PG⁶ represents a protective group of NH.)

This production method is a method for producing a raw material compound (1)-2 or a raw material compound (1)-3 included in the raw material compound (1). Here, a production method in which L² in the raw material compound (1)-3 is NR^{L1}, pyrrolidinediyl, piperidinediyl or piperazinediyl is shown.

### (First step)

This step is a method for producing a compound (2)-1 by a reaction of the compound (17) and a compound (33).

The reaction conditions are the same as in the seventh step of the Raw Material Synthesis 3.

### (Second step)

This step is a method for producing a compound (2)-2 by hydrolysis of the compound (2)-1.

The reaction conditions are the same as in the second step of the Raw Material Synthesis 2.

### (Third step)

This step is a method for producing the compound (1)-2 by an amidation reaction of the compound (32) and the compound (2)-2.

The reaction conditions are the same as in the third step of the Raw Material Synthesis 2.

### (Fourth step)

This step is a method for producing a compound (35) by an amidation reaction of the compound (32) and a compound (34).

The reaction conditions are the same as in the third step of the Raw Material Synthesis 2.

### (Fifth step and Sixth step)

The steps are a method for producing the compound (1)-3 by an amidation reaction of a compound obtained by a deprotection reaction of the compound (35) and the compound (2)-2.

The reaction conditions for the deprotection reaction are the same as in the step described in Production Method 1. Here, examples of the protective group which can be removed under acidic conditions include a tert-butoxycarbonyl group, a triphenylmethyl group, a tetrahydro-2H-pyran-2-yl group, a methoxymethyl group, a dimethylmethanediyl group, a tert-butylsulfinyl group and the like.

The reaction conditions for the amidation reaction are the same as in the third step of the Raw Material Synthesis 2.

### (Raw Material Synthesis 8)

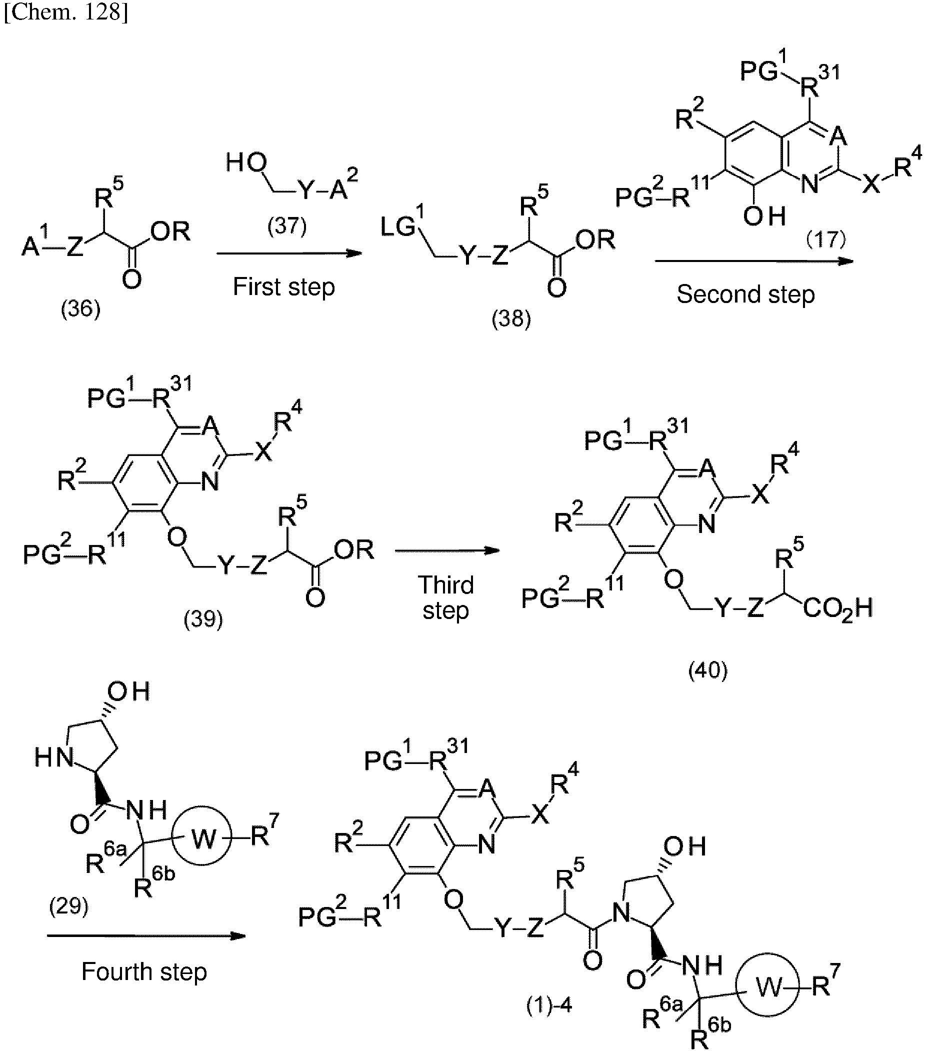

(In the formulae, when Z is NH, A¹ represents hydrogen atom, and A² represents halogen. When Z is 5-membered heteroarenediyl containing one to four hetero atoms selected from the group consisting of oxygen, sulfur and nitrogen, A² represents a boronic acid group or the like when A¹ is a group selected from the group consisting of Cl, Br and I, and A² represents a group selected from the group consisting of Cl, Br and I when A¹ is a boronic acid group or the like.)

This production method is a method for producing a raw material compound (1)-4 included in the raw material compound (1).

### (First step)

This step is a method for producing a compound (38) by an ipso reaction or a Buchwald-Hartwig amination reaction of a compound (36) and a compound (37) when Z is NH.

The reaction conditions for the ipso reaction are the same as in the first step of the Raw Material Synthesis 3.

For example, the following can be referred as a reference about the Buchwald-Hartwig amination reaction.
J. Am. Chem. Soc., 2020, 142, p.15027-15037

This step is a method for producing the compound (38) by a Suzuki-Miyaura coupling reaction of the compound (36) and the compound (37) when Z is 5-membered heteroaryl containing one to four hetero atoms selected from the group consisting of oxygen, sulfur and nitrogen.

The reaction conditions are the same as in the fourth step of the Raw Material Synthesis 3.

For example, the following can be referred as a reference about the reaction when Z is the formula (XXIX).
J. Org. Chem., 2000, 65, p.1516-1524
Chemical Communications 2014, 50, p.1867-1870
Bioorg. Med. Chem. Lett., 2001, 11, p.2061-2065

### (Second step)

This step is a method for producing a compound (39) by a reaction of the compound (38) and the compound (17).

The reaction conditions are the same as in the seventh step of the Raw Material Synthesis 3.

In this step, the compound (39) can also be produced by a Mitsunobu reaction of a compound in which the moiety corresponding to LG¹ of the compound (38) is a hydroxy group and the compound (17).

For example, the following can be referred as a reference about the Mitsunobu reaction.
Chem. Asian J. 2007, 2, p.1340-1355

### (Third step)

This step is a method for producing a compound (40) by hydrolysis of the compound (39).

The reaction conditions are the same as in the second step of the Raw Material Synthesis 2.

### (Fourth step)

This step is a method for producing the compound (1)-4 by an amidation reaction of the compound (40) and the compound (29).

The reaction conditions are the same as in the third step of the Raw Material Synthesis 2.

### (Raw Material Synthesis 9)

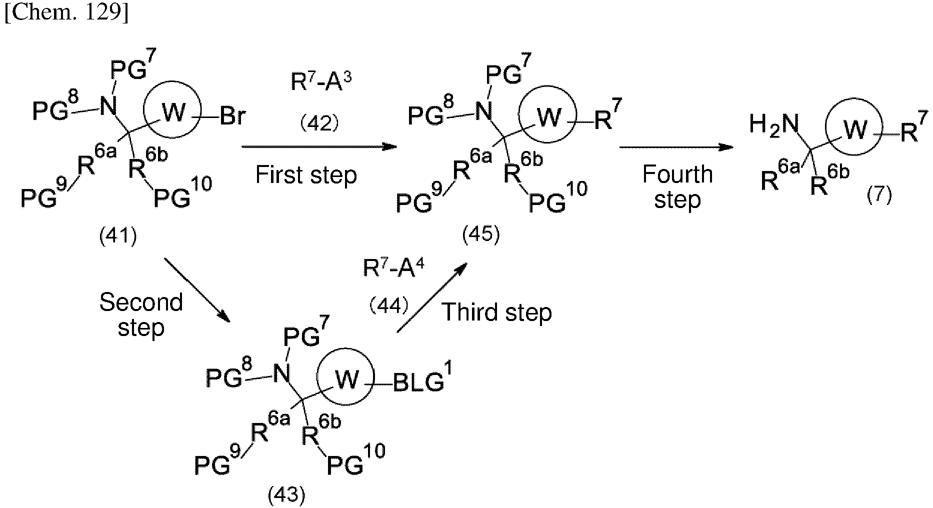

(In the formulae, R⁷ represents a group selected from the group consisting of the formula (IX), the formula (X), the formula (XI), the formula (XII), the formula (XIII), the formula (XIV), the formula (XV), the formula (XVI), the formula (XVII) and the formula (XVIII), PG⁷ represents a protective group, PG⁸, PG⁹ and PG¹⁰, which are the same as or different, represent hydrogen atom or a protective group, A³ represents hydrogen atom, a carboxyl group, a boronic acid group or the like, A⁴ represents hydrogen atom or a group selected from the group consisting of Cl, Br and I, and BLG¹ represents a boronic acid group or the like.)

This production method is a method for producing the raw material compound (7) when R⁷ is a group selected from the group consisting of the formula (IX), the formula (X), the formula (XI), the formula (XII), the formula (XIII), the formula (XIV), the formula (XV), the formula (XVI), the formula (XVII) and the formula (XVIII).

### (First step)

This step is a method for producing a compound (45) by a Mizoroki-Heck reaction of a compound (42) in which R⁷ is the formula (IX) when A³ is hydrogen atom and a compound (41).

In this reaction, the compound (42) and the compound (41) are used in an equal amount or with one compound thereof in a larger amount, and the mixture of the compounds is stirred in a solvent inactive for the reaction, in the presence of a base and a palladium catalyst, from at room temperature to under reflux with heat, preferably at 20°C to 140°C, generally for 0.1 hours to 5 days. Examples of the solvent used here include, but are not particularly limited to, an ether, such as diethyl ether, THF, DOX or 1,2-dimethoxyethane, DMF, DMAc, DMSO, MeCN, 1,3-dimethylimidazolidin-2-one, ethyl acetate, water and a mixture thereof. Examples of the base include a base, such as tripotassium phosphate, sodium carbonate, potassium carbonate and potassium acetate. Examples of the palladium catalyst include tetrakis(triphenylphosphine)palladium, bis(triphenylphosphine)palladium(II) dichloride, [1,1'-bis(diphenylphosphino)ferrocene]palladium(II) dichloride · dichloromethane additive, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palladium(II) methanesulfonate, palladium(II) acetate and the like. In addition, heating the mixture by microwave irradiation is sometimes advantageous for smoothly promoting the reaction.

For example, the following can be referred as a reference about the reaction.
Synthesis 2020, 52, p.2521-2527
PNAS 2016, 113, p.7124-7129

Alternatively, this step is, for example, a method for producing the compound (45) by an Ullmann reaction of the compound (42) in which R⁷ is a group selected from the group consisting of the formula (XII), the formula (XIII), the formula (XIV), the formula (XV) and the formula (XVII) and the compound (41).

For example, the following can be referred as a reference about the reaction.
Angew. Chem. Int. Ed., 2003, 42, p.5400-5449

This step is a method for producing the compound (45) by a decarbonation coupling reaction of the compound (42) in which R⁷ is, for example, the formula (X) when A³ is a carboxyl group and the compound (41).

For example, the following can be referred as a reference about the reaction.
Science, 2006, 313, p.662-664

This step is a method for producing the compound (45) by a Suzuki-Miyaura coupling reaction of the compound (42) in which R⁷ is, for example, a group selected from the group consisting of the formula (IX), the formula (XI) and the formula (XVI) when A³ is a boronic acid group or the like and the compound (41).

The reaction conditions are the same as in the fourth step of the Raw Material Synthesis 3.

### (Second step)

This step is a method for producing a compound (43) by a reaction for substituting the bromo group of the compound (41) by a boronic acid group or the like.

For example, the following can be referred as a reference about the reaction.
Eur. J. Med. Chem., 2019, 162, p.407-422
J. Org. Chem. 2020, 85, 16, p.10966-10972
J. Am. Chem. Soc., 2010, 132, p.17701-17703

### (Third step)

This step is a method for producing the compound (45) by a Suzuki-Miyaura coupling reaction of a compound (44) and the compound (43) when A⁴ is a group selected from the group consisting of Cl, Br and I.

The reaction conditions are the same as in the fourth step of the Raw Material Synthesis 3.

This step is a method for producing the compound (45) by a Chan-Lam-Evans Coupling reaction of the compound (44) which is, for example, a group selected from the group consisting of the formula (XII), the formula (XIII), the formula (XIV), the formula (XV) and the formula (XVII) when A⁴ is hydrogen atom and the compound (43).

For example, the following can be referred as a reference about the reaction.
Adv. Synth. Catal. 2020, 362, p.3311-3331.

### (Fourth step)

This step is a method for producing the compound (7) by subjecting the compound (45) to a deprotection reaction. Here, examples of the protective group which can be removed under acidic conditions include a tert-butoxycarbonyl group, a triphenylmethyl group, a tetrahydro-2H-pyran-2-yl group, a methoxymethyl group, a dimethylmethanediyl group, a tert-butylsulfinyl group and the like.

The reaction conditions are the same as in the step described in the Production Method 1.

### (Raw Material Synthesis 10)

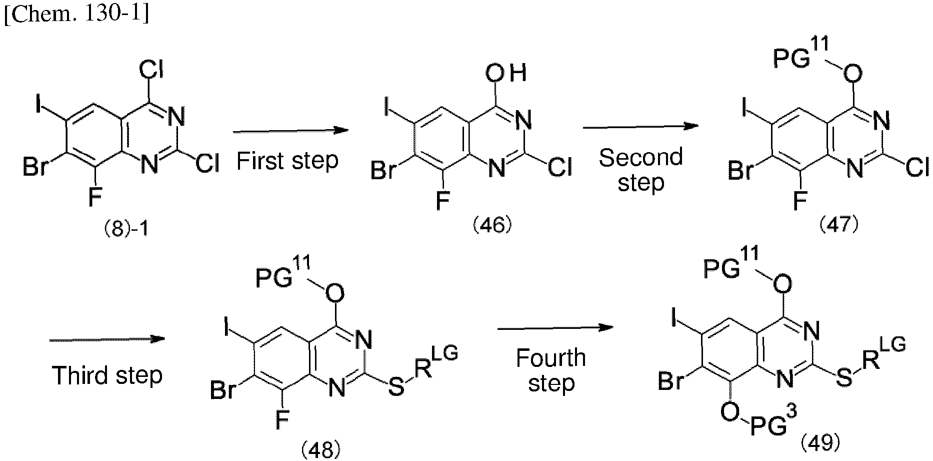

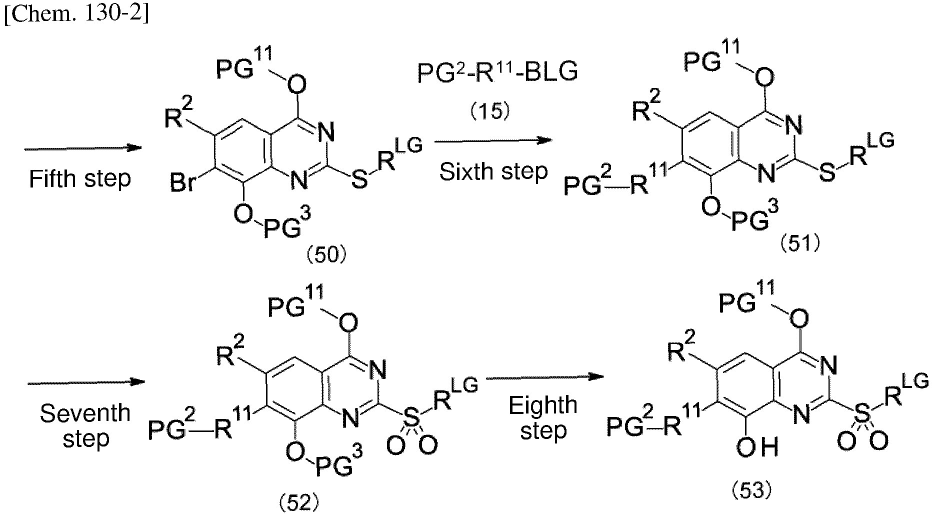

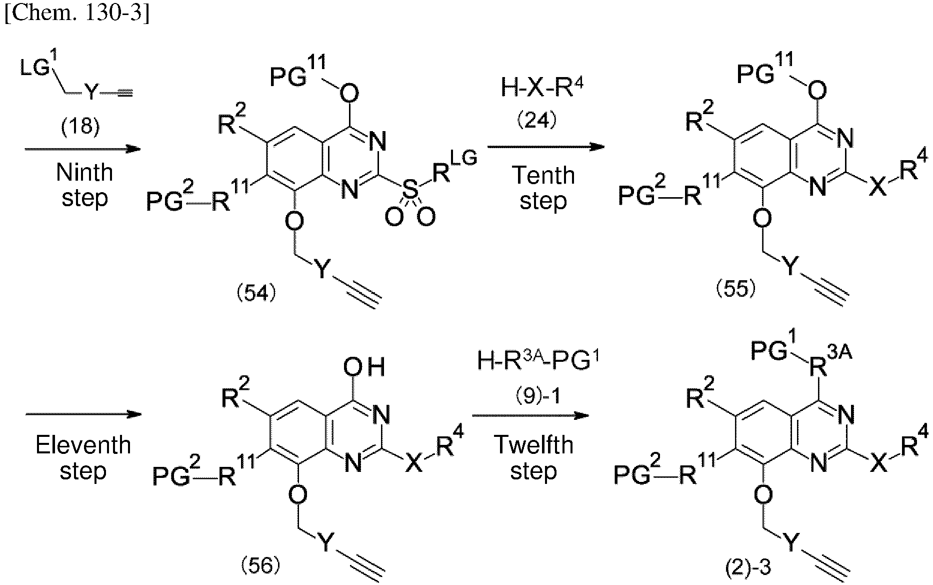

(In the formulae, PG¹¹ represents a tert-butyl group, and R^{3A} represents -O- or - N(R^{P})-.)

This production method is a method for producing a raw material compound (2)-3.

### (First step)

This step is a method for producing a compound (46) by hydrolysis of the compound (8)-1.

This reaction is performed by stirring the compound (8)-1 from under cooling to under reflux with heat generally for 0.1 hours to 5 days. Examples of the solvent used here include, but are not particularly limited to, an alcohol, acetone, DMF, THF and the like. In addition, a mixed solvent of the above solvent and water is sometimes suitable for the reaction. Examples of the hydrolysis reagent include, but are not particularly limited to, an aqueous sodium hydroxide solution, an aqueous potassium hydroxide solution and the like.

For example, the following can be referred as a reference about this reaction.

The Chemical Society of Japan, "Jikken Kagaku Koza (Courses in Experimental Chemistry) (5th edition)", Vol. 16 (2005) (Maruzen)
Angew. Chem. Int. Ed. 2005, 44, p.1378-1382.

### (Second step)

This step is a method for producing a compound (47) by the protection of a hydroxy group of the compound (46) with the tert-butyl group (PG¹¹).

This reaction is performed by stirring the compound (46) from under cooling to under reflux with heat generally for 0.1 hours to 5 days. Examples of the solvent used here include, but are not particularly limited to, an ether, such as THF or DOX, a halogenated hydrocarbon, such as dichloromethane, tBuOH, DMF and the like. Examples of the tert-butyl protection reagent include, but are not particularly limited to, isobutene, 2-tert-butyl-1,3-diisopropylisourea and the like.

Moreover, the compound (47) can be produced by a dehydration condensation reaction of the compound (46) and tBuOH.

For example, the following can be referred as a reference about this reaction.
P. G. M. Wuts and T. W. Greene, "Greene's Protective Groups in Organic Synthesis", 5th edition, John Wiley & Sons Inc., 2014
Org. Lett., 2012, 14, 17, p.4678-4681

### (Third step)

This step is a method for producing a compound (48) by an ipso substitution reaction of the compound (47) and R^{LG}-SH.

The reaction conditions are the same as in the first step of the Raw Material Synthesis 4.

### (Fourth step)

This step is a method for producing a compound (49) by an ipso substitution reaction of the compound (48) and PG³-OH.

The reaction conditions are the same as in the third step of the Raw Material Synthesis 3.

### (Fifth step)

This step is a method for producing a compound (50) by a Suzuki-Miyaura coupling reaction of the compound (49) and a boronic acid derivative composed of a R²-boronic acid group or the like.

The reaction conditions are the same as in the fourth step of the Raw Material Synthesis 3.

The compound (50) (where R² is hydrogen) can be produced by a dehydrogenation reaction of the compound (49) with a Pd catalyst and a reducing agent.

### [Reference]

J. Org. Chem., 1977, 42, p.3491-3494
Tetrahedron Letters 2013, 54, 5207-5210

### (Sixth step)

This step is a method for producing a compound (51) by a Suzuki-Miyaura coupling reaction of the compound (50) and the compound (15).

The reaction conditions are the same as in the fifth step of the Raw Material Synthesis 3.

### (Seventh step)

This step is a method for producing a compound (52) by an oxidation reaction of the compound (51).

The reaction conditions are the same as in the fifth step of the Raw Material Synthesis 4.

When the compound (52) has an axial chirality, the compound (83) may be obtained as a mixture of stereoisomers, and each stereoisomer can be isolated by separation using a common separation operation, for example, ODS column chromatography or silica gel column chromatography.

Moreover, PG² may be converted to another protective group after the compound (52) is subjected to a deprotection reaction so that the deprotection can be performed under conditions different from those for the protective group PG¹, which is introduced later.

The reaction conditions for the deprotection reaction used here are the same as in the step described in the Production Method 1.

Examples of the protective group of PG² which is subsequently converted include a tetrahydro-2H-pyran-2-yl group and the like.

For example, the following can be referred as a reference about this reaction.
P. G. M. Wuts and T. W. Greene, "Greene's Protective Groups in Organic Synthesis", 5th edition, John Wiley & Sons Inc., 2014

### (Eighth step)

This step is a method for producing a compound (53) by deprotection by a catalytic hydrogenation reaction of the compound (52).

The reaction conditions are the same as in the sixth step of the Raw Material Synthesis 3.

### (Ninth step)

This step is a method for producing the compound (54) by a reaction of the compound (53) and the compound (18).

The reaction conditions are the same as in the seventh step of the Raw Material Synthesis 3.

### (Tenth step)

This step is a method for producing a compound (55) by an ipso substitution reaction of the compound (54) and the compound (24).

The reaction conditions are the same as in the sixth step of the Raw Material Synthesis 4.

### (Eleventh step)

This step is a method for producing a compound (56) by subjecting the compound (55) to a deprotection reaction.

The reaction conditions are the same as in the step described in the Production Method 1.

### (Twelfth step)

This step is a method for producing a compound (2)-3 by a reaction of the compound (56) and a compound (9)-1.

In this reaction, the compound (56) and the compound (9)-1 are used in an equal amount or with one compound thereof in a larger amount, and the mixture of the compounds is stirred in the presence of a condensing agent, in a solvent inactive for the reaction, from under cooling to under heating, preferably at -20°C to 60°C, generally for 0.1 hours to 5 days. Examples of the solvent include an aromatic hydrocarbon, such as toluene, an ether, such as THF or DOX, a halogenated hydrocarbon such as dichloromethane, an alcohol, DMF, DMSO, ethyl acetate, MeCN and a mixture thereof. Examples of the condensing agent include PyBOP, HATU, CDI and the like. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

### (Raw Material Synthesis 11)

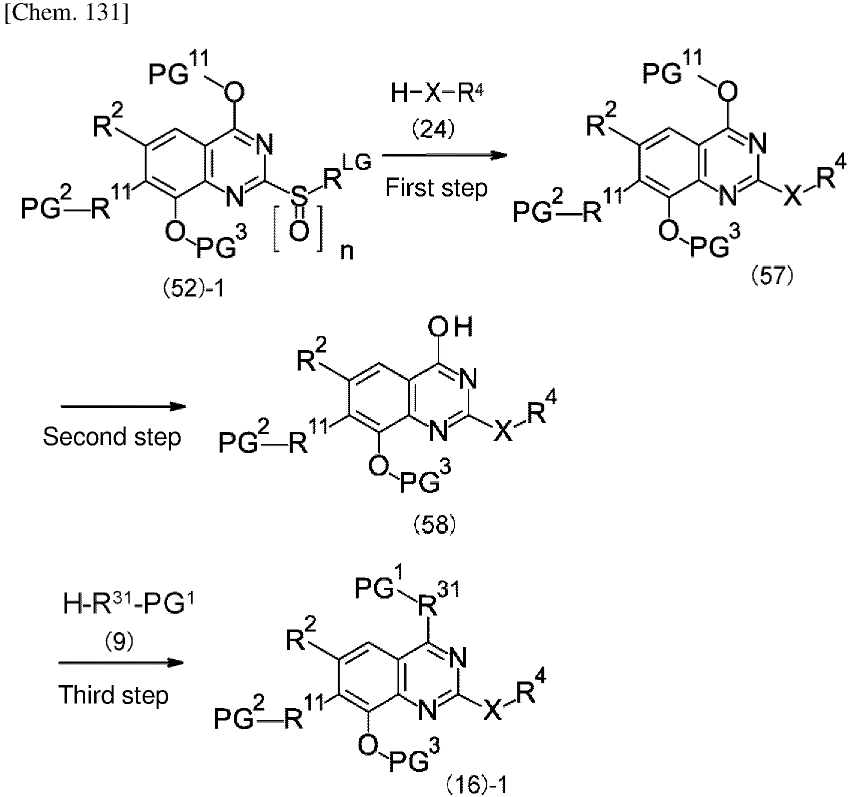

This production method is a method for producing (16)-1 in which A is N among the raw material compound (16).

### (First step)

This step is a method for producing a compound (57) by an ipso substitution reaction of a compound (52)-1 and the compound (24).

The reaction conditions are the same as in the sixth step of the Raw Material Synthesis 4.

### (Second step)

This step is a method for producing a compound (58) by subjecting the compound (57) to a deprotection reaction.

The reaction conditions are the same as in the step described in the Production Method 1.

### (Third step)

This step is a method for producing a compound (16)-1 by a reaction of the compound (58) and the compound (9).

The reaction conditions are the same as in the twelfth step of the Raw Material Synthesis 10.

### (Raw Material Synthesis 12)

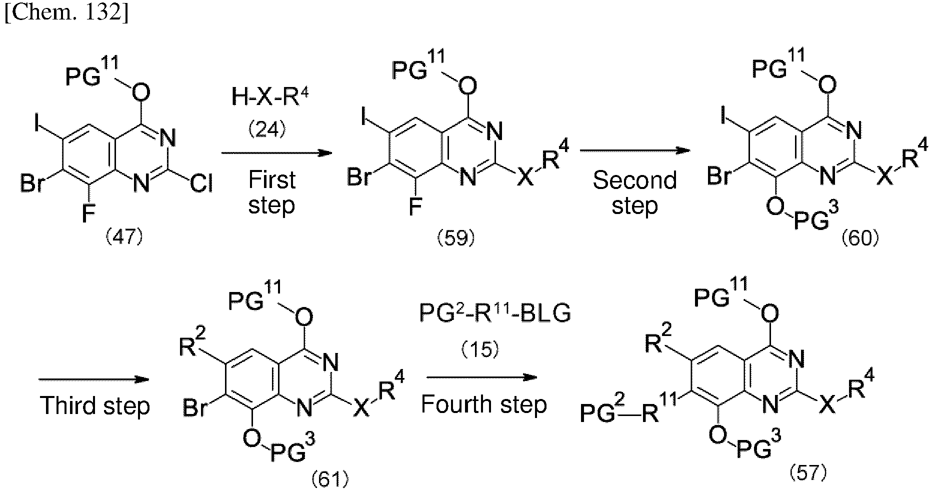

This production method is a method for producing the raw material compound (57).

### (First step)

This step is a method for producing a compound (59) by an ipso substitution reaction of the compound (47) and the compound (24).

The reaction conditions are the same as in the second step of the Raw Material Synthesis 3.

### (Second step)

This step is a method for producing a compound (60) by an ipso substitution reaction of the compound (59) and PG³-OH.

The reaction conditions are the same as in the third step of the Raw Material Synthesis 3.

### (Third step)

This step is a method for producing a compound (61) by a Suzuki-Miyaura coupling reaction of the compound (60) and a boronic acid derivative composed of a R²-boronic acid group or the like.

The reaction conditions are the same as in the fourth step of the Raw Material Synthesis 3.

The compound (61) (where R² is hydrogen) can be produced by a dehydrogenation reaction of the compound (60) with a Pd catalyst and a reducing agent.

### [Reference]

J. Org. Chem., 1977, 42, p.3491-3494
Tetrahedron Letters 2013, 54, 5207-5210

### (Fourth step)

This step is a method for producing the compound (57) by a Suzuki-Miyaura coupling reaction of the compound (61) and the compound (15).

The reaction conditions are the same as in the fifth step of the Raw Material Synthesis 3.

### (Raw Material Synthesis 13)

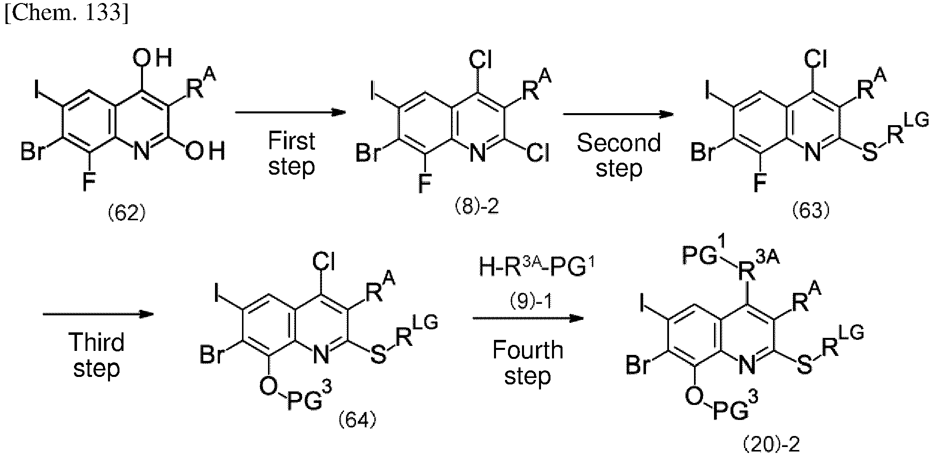

This production method is a method for producing a raw material compound (20)-2.

### (First step)

This step is a method for producing a compound (8)-2 by a chlorination reaction of the compound (62).

This reaction is performed by stirring a mixture of the compound (62) and a chlorinating agent in an equal amount or with one in a larger amount in a solvent inactive for the reaction or with no solvent, from under cooling to under reflux with heat, preferably at 60°C to under reflux with heat, generally for 0.1 hours to 5 days. Examples of the solvent used here include, but are not particularly limited to, an aromatic hydrocarbon, such as toluene, an ether, such as THF or DOX, a halogenated hydrocarbon, such as dichloromethane, and the like. Examples of the chlorinating agent include phosphorus oxychloride, thionyl chloride the like. Performing the reaction in the presence of an organic base, such as TEA, DIPEA or NMM, is sometimes advantageous for smoothly promoting the reaction.

### (Second step)

This step is a method for producing a compound (63) by an ipso substitution reaction of the compound (8)-2 and R^{LG}-SH.

The reaction conditions are the same as in the first step of the Raw Material Synthesis 4.

### (Third step)

This step is a method for producing a compound (64) by an ipso substitution reaction of the compound (63) and PG³-OH.

The reaction conditions are the same as in the third step of the Raw Material Synthesis 3.

### (Fourth step)

This step is a method for producing the compound (20)-2 by an ipso substitution reaction of the compound (64) and the compound (9)-1.

The reaction conditions are the same as in the first step of the Raw Material Synthesis 3.

### (Raw Material Synthesis 14)

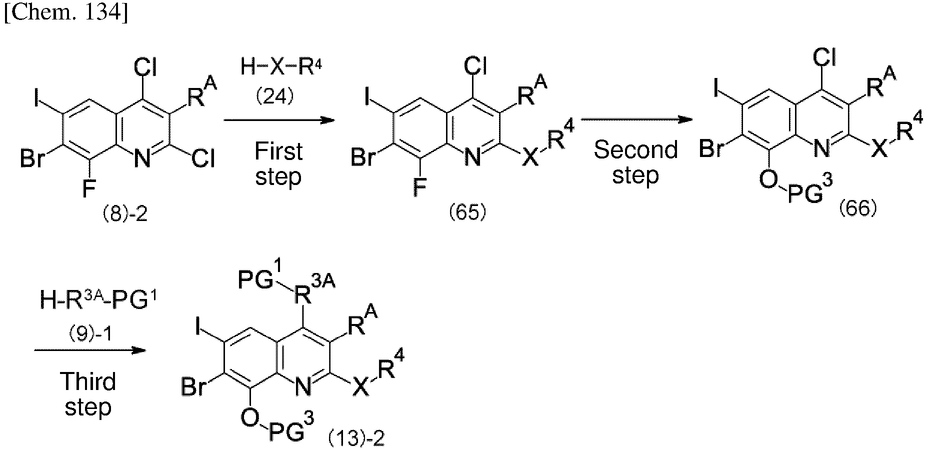

This production method is a method for producing a raw material compound (13)-2.

### (First step)

This step is a method for producing a compound (65) by an ipso substitution reaction of a compound (8)-2 and a compound (24).

The reaction conditions are the same as in the second step of the Raw Material Synthesis 3.

Moreover, the compound (65) can be produced by the Negishi coupling of a compound in which a hydrogen atom of the compound (24) is converted to halogen and the compound (8)-2.

### (Second step)

This step is a method for producing a compound (66) by an ipso substitution reaction of the compound (65) and PG³-OH.

The reaction conditions are the same as in the third step of the Raw Material Synthesis 3.

### (Third step)

This step is a method for producing the compound (13)-2 by an ipso substitution reaction of a compound (66) and the compound (9)-1.

The reaction conditions are the same as in the first step of the Raw Material Synthesis 3. Performing the reaction using DMAc or the like as a solvent at a reaction temperature of 0°C to 120°C while using potassium carbonate or the like as an inorganic base is sometimes advantageous for smoothly promoting the reaction.

### (Raw Material Synthesis 15)

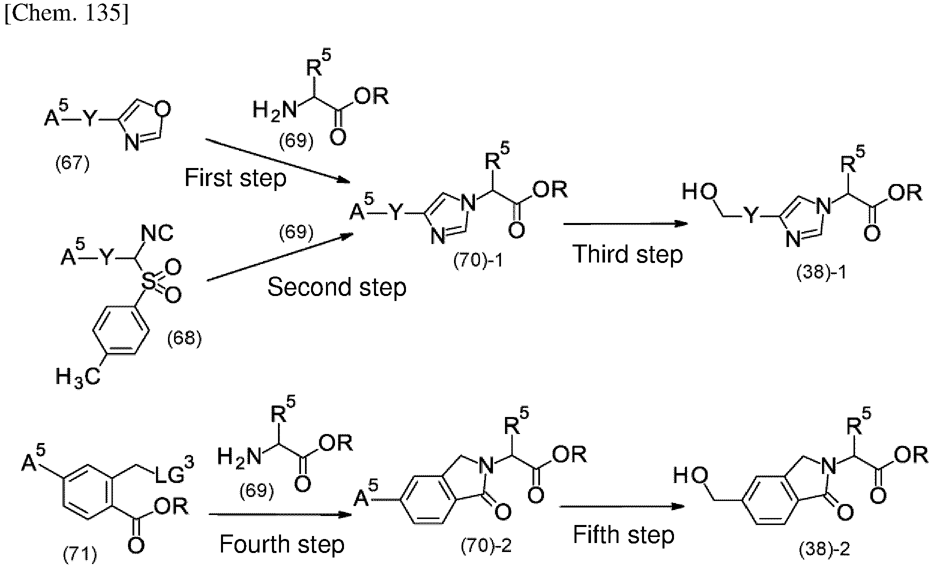

(In the formula, A⁵ represents a group selected from the group consisting of Cl, Br, mesylate and triflate, and LG³ represents a leaving group.)

This production method is a method for producing a raw material compound (38)-1 and a raw material compound (38)-2 included in the raw material compound (38).

### (First step)

This step is a method for producing a compound (70)-1 by a reaction of the compound (67) and a compound (69).

For example, the following can be referred as a reference about the reaction.
Chem. Commun., 2014, 50, 15, p.1867-1870

### (Second step)

This step is a method for producing the compound (70)-1 by a reaction of the compound (69) and glyoxylic acid using an aryl-substituted toluenesulfonylmethyl isocyanide (TosMIC) reagent.

For example, the following can be referred as a reference about the reaction.
J. Org. Chem., 2000, 65, 5, p.1516-1524
J. Am. Chem. Soc., 2007, 129, 3, p.490-491

### (Third step)

This step is a method for producing a compound (38)-1 by a Suzuki-Miyaura coupling reaction of a compound (70)-1 and an alkoxymethylboronic acid derivative followed by a deprotection reaction under acidic conditions.

The reaction conditions for the Suzuki-Miyaura coupling reaction are the same as in the fourth step of the Raw Material Synthesis 3.

Examples of the alkoxymethylboronic acid derivative used here include potassium (2-trimethylsilyl)-ethoxymethyltrifluoroborate.

For example, the following can be referred as a reference about the reaction.
Org. Lett., 2008, 10, 11, p.2135-2128
Org. Lett., 2011, 13, 15, p.3948-3951

The reaction conditions for the subsequent deprotection reaction under acidic conditions are the same as in the step described in Production Method 1. Examples of the acid used herein include trifluoroacetic acid and the like.

For example, the following can be referred as a reference about the deprotection reaction.

P. G. M. Wuts and T. W. Greene, "Greene's Protective Groups in Organic Synthesis", 5th edition, John Wiley & Sons Inc., 2014

In this step, the compound (38)-1 can also be produced by a Suzuki-Miyaura coupling reaction of the compound (70)-1 and an acetoxymethylboronic acid derivative.

For example, the following can be referred as a reference about the reaction.
Org. Lett., 2012, 14, 5, p.1278-1281

### (Fourth step)

This step is a method for producing a compound (70)-2 by a reaction of the compound (71) and a compound (69).

This reaction is performed by reacting the compound (71) and the compound (69) are used in an equal amount or with one compound thereof in a larger amount and stirring the mixture thereof in a solvent inactive for the reaction or with no solvent, from under cooling to under reflux with heat, preferably at room temperature to under reflux with heat, generally for 0.1 hours to 5 days. Examples of the solvent used here include, but are not particularly limited to, an aromatic hydrocarbon, such as toluene, an ether, such as THF or DOX, a halogenated hydrocarbon, such as dichloromethane, DMF, DMSO, ethyl acetate, MeCN and the like. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

### (Fifth step)

This step is a method for producing a compound (38)-2 by a Suzuki-Miyaura coupling reaction of a compound (70)-2 and an alkoxymethylboronic acid derivative followed by a deprotection reaction under acidic conditions.

The reaction conditions are the same as in the third step of the Raw Material Synthesis 15.

In this step, the compound (38)-2 can also be produced by a Suzuki-Miyaura coupling reaction of the compound (70)-2 and an acetoxymethylboronic acid derivative.

### (Raw Material Synthesis 16)

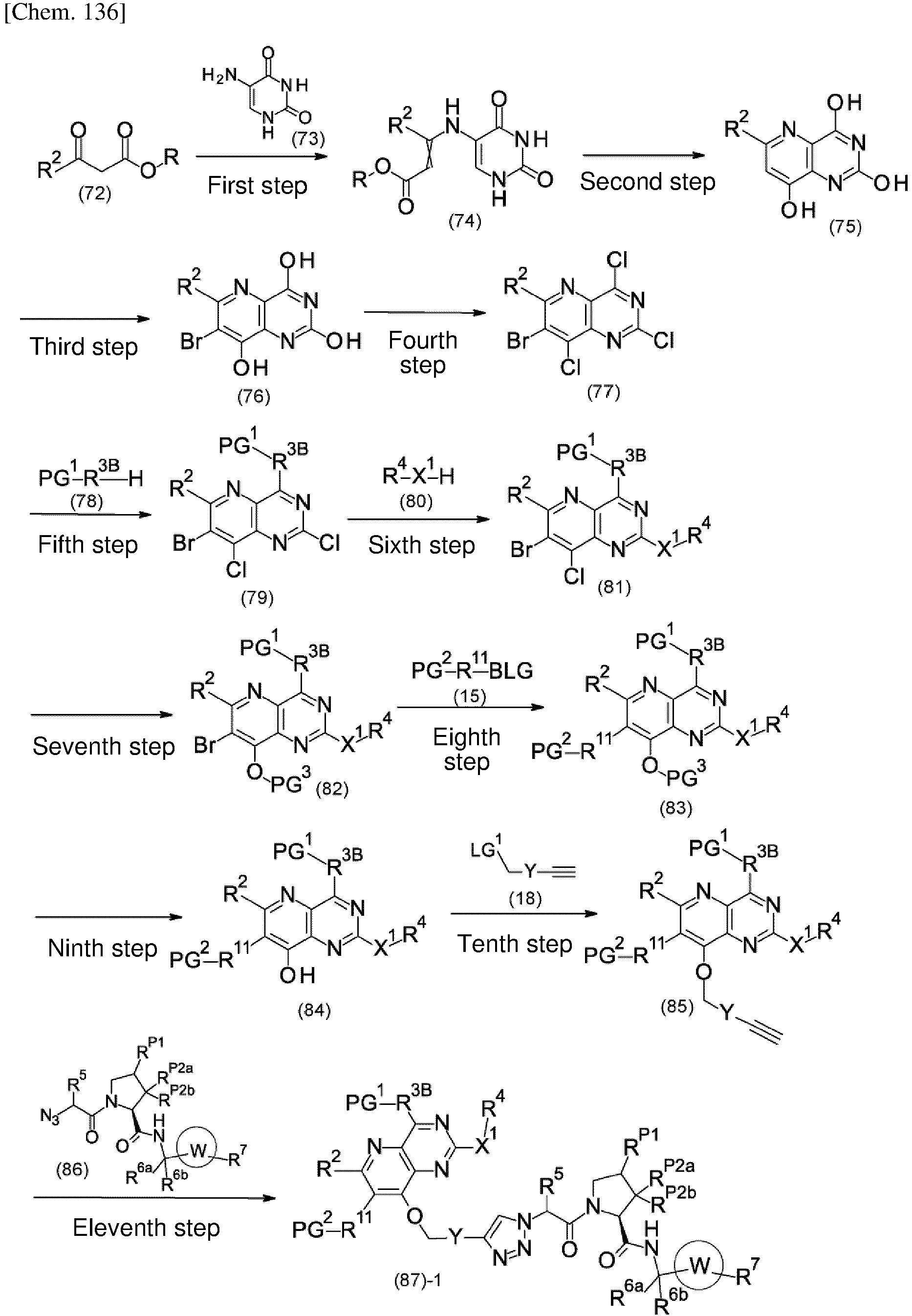

(In the formulae, R^{3B} represents a divalent group formed by the removal of one bond from a group selected from the group consisting of -P-Q, V and a 7-membered or 8-membered saturated or unsaturated bridged heterocyclic group, and X¹ represents O or NR^{4x}. The same shall apply hereinafter.)

This production method is a method for producing the compound (87)-1 in which A and E are N, G is CR², X is O or NR^{4x}, L^{Y} is -O-CH₂-, L is a bond, and Z is the formula (XXVII) below among the raw material compound (87) in the Production Method 2.

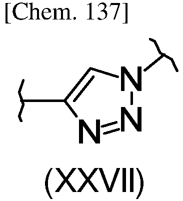

### (First step)

This step is a method for producing the compound (74) by a reaction of the compound (72) and a compound (73).

This reaction is performed by converting the compound (72) into the corresponding enolate using an orthoester, such as trimethyl orthoformate, under acidic conditions, followed by the addition of the compound (73) in an equal amount or with one compound thereof in a larger amount, and stirring the mixture thereof in a solvent inactive for the reaction under reflux with heat, preferably at 60°C to under reflux with heat, generally for 0.1 hours to 5 days. Examples of the solvent used here include, but are not particularly limited to, an aromatic hydrocarbon, such as toluene, an ether, such as THF or DOX, DMF and DMAc.

### (Second step)

This step is a method for producing a compound (75) from the compound (74).

This reaction is performed by stirring the compound (74) in a solvent inactive for the reaction under reflux with heat, preferably at 150°C to under reflux with heat, generally for 0.1 hours to 5 days. Examples of the solvent used here include, but are not particularly limited to, NMP.

### (Third step)

This step is a method for producing a compound (76) from the compound (75).

This reaction is performed by stirring a mixture of the compound (75) and a brominating agent in an equal amount or with one compound thereof in a larger amount in a solvent inactive for the reaction or with no solvent, from under cooling to under reflux with heat, preferably at room temperature, generally for 0.1 hours to 5 days. Examples of the solvent used here include, but are not particularly limited to, an aromatic hydrocarbon, such as toluene, an ether, such as THF or DOX, a halogenated hydrocarbon, such as dichloromethane, and DMF. Examples of the brominating agent include N-bromosuccinimide, N-bromosaccharin, 1,3-dibromo-5,5-dimethylhydantoin, dibromoisocyanuric acid and the like.

### (Fourth step)

This step is a method for producing a compound (77) from the compound (76).

The reaction conditions are the same as in the first step of the Raw Material Synthesis 13.

### (Fifth step)

This step is a method for producing a compound (79) by an ipso substitution reaction of a compound (77) and a compound (78).

The reaction conditions are the same as in the first step of the Raw Material Synthesis 3.

### (Sixth step)

This step is a method for producing a compound (81) by an ipso substitution reaction of a compound (79) and a compound (80).

The reaction conditions are the same as in the first step of the Raw Material Synthesis 3.

### (Seventh step)

This step is a method for producing a compound (82) by an ipso substitution reaction of the compound (81) and PG³-OH.

Examples of the PG³-OH used here include benzyl alcohol, p-methoxybenzyl alcohol and 1-phenylethanol.

The reaction conditions are the same as in the first step of the Raw Material Synthesis 3.

### (Eighth step)

This step is a method for producing a compound (83) by a Suzuki-Miyaura coupling reaction of the compound (82) and a compound (15) which is a boronic acid derivative.

The reaction conditions are the same as in the fourth step of the Raw Material Synthesis 3.

When the compound (83) has an axial chirality, the compound (83) is obtained as a mixture of stereoisomers, and each stereoisomer can be isolated by separation using a common separation operation, for example, ODS column chromatography or silica gel column chromatography.

Moreover, PG² may be converted to another protective group after the compound (83) is subjected to a deprotection reaction so that the deprotection can be performed under conditions different from those for the protective group PG¹, which is introduced later.

The reaction conditions for the deprotection reaction used here are the same as in the step described in the Production Method 1.

Examples of the protective group of PG² which is subsequently converted include a tetrahydro-2H-pyran-2-yl group and the like.

For example, the following can be referred as a reference about this reaction.
P. G. M. Wuts and T. W. Greene, "Greene's Protective Groups in Organic Synthesis", 5th edition, John Wiley & Sons Inc., 2014

### (Ninth step)

This step is a method for producing a compound (84) by deprotection by a catalytic hydrogenation reaction of the compound (83).

The reaction conditions are the same as in the sixth step of the Raw Material Synthesis 3.

### (Tenth step)

This step is a method for producing the compound (85) by a reaction of the compound (84) and the compound (18).

The reaction conditions are the same as in the seventh step of the Raw Material Synthesis 3.

### (Eleventh step)

This step is a method for producing a compound (87)-1 by a reaction of the compound (85) and a compound (86).

The reaction conditions are the same as in the first step of the Raw Material Synthesis 1.

The compounds of the formula (I) and the formula (IA) are isolated and purified as a free compound, a salt, hydrate, solvate or crystal polymorphous substance thereof or a substance in the amorphous solid form. Salts of the compounds of the formula (I) and the formula (IA) can also be produced by subjecting the compound to a salt formation reaction which is an ordinary method.

The isolation and purification are performed by applying a common chemical operation, such as extraction, fractional crystallization or various types of fraction chromatography.

Various types of isomers can be produced by selecting an appropriate raw material compound or can be separated using a difference in physiochemical properties between the isomers. For example, an optical isomer can be obtained by a general optical resolution method of a racemate (for example, fractional crystallization for inducing a racemate to a diastereomer salt with an optically active base or acid, chromatography using a chiral column or the like or the like) and can also be produced from an appropriate optically active raw material compound.

In addition, the compounds of the formula (I) and the formula (IA) or intermediates thereof sometimes have an axial chirality and are obtained as a mixture of stereoisomers, and each stereoisomer can be isolated by separation using a common separation operation, for example, octadecylsilyl (ODS) column chromatography or silica gel column chromatography.

The pharmacological activities of the compounds of the formula (I) and the formula (IA) were confirmed by the following tests.

### Test Example 1: Evaluation of KRAS degradation activity on human G12D mutant KRAS-positive pancreatic cancer line AsPC-1 (CRL-1682; ATCC)

The KRAS degradation activity of test compounds was evaluated by measuring the expression levels of KRAS G12D protein by Cell ELISA.

AsPC-1 cells were seeded at 20 µL per well on 384-well plates (from Greiner bio-one) to give 2.0 × 10⁴ cells per well. As for the cell culture conditions, RPMI 1640 (from Sigma-Aldrich) medium containing 10% fetal bovine serum (from Cytiva) was used in the presence of 5% CO₂ at 37°C.

The next day, the test compounds (10 points having final concentrations in the range of 10 µM to 0.3 nM) and DMSO (from FUJIFILM Wako Pure Chemical Corporation), which was the solvent for the test compounds, as a negative control were diluted 500-fold with a fresh medium and were added at 20 µL per well. The cells were cultured overnight.

The next day, the culture supernatant was removed, and 4% paraformaldehyde phosphate buffer (from FUJIFILM Wako Pure Chemical Corporation) was added at 20 µL per well. The plates were allowed to stand for 30 minutes at room temperature to thus immobilize the cells. Then, the supernatant was removed, and 0.1% Triton X-100 (from Amersham Biosciences)-containing Phosphate buffered saline (PBS; from FUJIFILM Wako Pure Chemical Corporation) was added at 20 µL per well. After allowing to stand for 10 minutes at room temperature, the supernatant was removed. PBS was added at 25 µL per well, and the supernatant was removed to thus wash each well. The washing was performed twice in total. Next, the supernatant was removed, and 0.5% sodium dodecyl sulfate (SDS; from Invitrogen)-containing PBS was added at 20 µL per well. After allowing to stand at room temperature for 10 minutes, the supernatant was removed by a centrifugal operation (using a centrifugal dehydrator machine, the supernatant was removed by the same method hereinafter). PBS was added at 25 µL per well, and the supernatant was removed to thus wash each well. The washing was performed twice in total. The supernatant was removed, and blocking solution (Intercept Blocking Buffer; from Li-COR Biosciences) was added at 20 µL per well. After allowing to stand for 30 minutes at room temperature, the supernatant was removed, and a solution obtained by diluting anti-β-actin antibody (Anti-beta Actin antibody [mAbcam 8226]-Loading Control; from abcam) 1,000-fold with blocking solution and a solution obtained by diluting anti-Ras (G12D Mutant Specific) antibody (Ras (G12D Mutant Specific) (D8H7) Rabbit mAb #14429; from Cell Signaling Technology) and anti-β-actin antibody (from abcam) 1,000-fold with blocking solution were respectively added to positive control wells and the other wells at 20 µL per well as a primary antibody. The plate was allowed to stand overnight at 4°C.

The next day, the supernatant was removed. PBS was added at 25 µL per well, and the supernatant was removed to thus wash each well. The washing was performed twice in total. The supernatant was removed, and a solution obtained by diluting Donkey antiMouse IgG H&L (IRDye 680RD) (from Li-COR Biosciences) and Goat anti-Rabbit IgG H&L (IRDye 800CW) (from Li-COR Biosciences) 1,000-fold with blocking solution was added at 20 µL per well as a secondary antibody. After allowing to stand for 1 hour at room temperature, the supernatant was removed. PBS was added at 25 µL per well, and the supernatant was removed to thus wash each well. The washing was performed twice in total. After removing the supernatant, the plate was dried with air at room temperature for 2 hours or more, and the 700 nm and 800 nm fluorescent signals were measured with Aerius (from Li-COR Biosciences).

After the signaling value of RAS measured at a fluorescence wavelength of 800 nm was corrected with the signaling value of β-actin measured at a fluorescence wavelength of 700 nm, with the signaling value at the time of addition of DMSO taken as 0% and with the signaling value when stained only with the anti-β-actin antibody taken as 100%, the 50% degradation concentration values (DC₅₀) of the amounts of KRAS were calculated by Sigmoid-Emax nonlinear regression analysis. The results for some test compounds of the formula (I) and the formula (IA) are shown in the following tables.

**[Table 1-1]**

| Ex | DC₅₀ (nM) | Ex | DC₅₀ (nM) | Ex | DC₅₀ (nM) |
|---|---|---|---|---|---|
| 1 | 13 | 19 | 91 | 37 | 58 |
| 2 | 20 | 20 | 41 | 38 | 25 |
| 3 | 47 | 21 | 33 | 39 | 37 |
| 4 | 35 | 22 | 59 | 40 | 47 |
| 5 | 9 | 23 | 31 | 41 | 29 |
| 6 | 24 | 24 | 48 | 42 | 91 |
| 7 | 40 | 25 | 75 | 43 | 41 |
| 8 | 18 | 26 | 22 | 44 | 43 |
| 9 | 15 | 27 | 68 | 45 | 27 |
| 10 | 18 | 28 | 47 | 46 | 25 |
| 11 | 10 | 29 | 20 | 47 | 26 |
| 12 | 61 | 30 | 121 | 48 | 38 |
| 13 | 10 | 31 | 59 | 49 | 25 |
| 14 | 20 | 32 | 51 | 50 | 18 |
| 15 | 99 | 33 | 33 | 51 | 14 |
| 16 | 26 | 34 | 53 | 52 | 11 |
| 17 | 74 | 35 | 63 | 53 | 7 |
| 18 | 508 | 36 | 82 | 54 | 34 |

**[Table 1-2]**

| Ex | DC₅₀ (nM) \| |
|---|---|
| 55 | 10 |
| 56 | 28 |

### Test Example 2: Evaluation of ERK phosphorylation inhibitory effect on human G12D mutant KRAS-positive pancreatic cancer line AsPC-1

The ERK phosphorylation inhibitory effect of test compounds was evaluated by measuring phosphorylation of threonine 202 (Thr202) and tyrosine 204 (Tyr204) of ERK located downstream of the KRAS signal by Cell ELISA.

AsPC-1 cells were seeded on 384-well plates at 36 µL/well to give 2.0 × 10⁴ cells per well. As for the cell culture conditions, RPMI 1640 medium containing 10% fetal bovine serum was used in the presence of 5% CO₂ at 37°C.

The next day, the test compounds (10 points having final concentrations in the range of 10 µM to 0.3 nM), trametinib (MEK inhibitor) of a final concentration of 1 µM as positive control and DMSO, which was the solvent for the test compounds, as negative control were diluted 100-fold with a fresh medium and were added at 4 µL per well. The cells were cultured overnight. After culturing, 30% glyoxal solution (40% glyoxal [from Nacalai Tesque] was diluted with PBS) was quickly added at 30 µL per well, and the plate was allowed to stand for 60 minutes at room temperature to thus immobilize the cells. Then, the plate was centrifuged to remove the supernatant (using a centrifugal dehydrator machine, the supernatant was removed by the same method hereinafter), and 0.1% Triton X-100-containing PBS was added at 20 µL per well. After allowing to stand for 10 minutes at room temperature, the supernatant was removed, and the same operation was further repeated. Next, 0.5% SDS-containing PBS was added at 20 µL per well, and after allowing to stand at room temperature for 30 minutes, the supernatant was removed. Subsequently, blocking solution (Intercept Blocking Buffer) was added at 20 µL per well, and the plate was allowed to stand for 1 hour at room temperature. The supernatant was removed, and an ERK (Thr202/Tyr204) phosphorylation antibody (Phospho-p44/42 MAPK (Erk 1/2) (Thr202/Tyr204) (D13.14.4E) XP Rabbit mAb; from Cell Signaling Technology) diluted 2,500-fold with blocking solution was added at 15 µL per well as a primary antibody. The plates were allowed to stand at 4°C overnight.

The next day, the supernatant was removed. 0.05% Tween-20 (from Thermo Fisher Scientific)-containing PBS was added at 50 µL per well, and the supernatant was removed to thus wash each well. The washing was performed three times in total. After washing, Goat anti-Rabbit IgG H&L (IRDye 800CW) diluted 1,000-fold with blocking solution was added at 15 µL per well as a secondary antibody, and the plates were allowed to stand for 1 hour at room temperature. The supernatant was removed, and each well was washed three times with 0.05% Tween-20-containing PBS in the same manner as after the primary antibody reaction. After removing the supernatant, the plates were dried with air at room temperature for 3 hours or more, and the 800-nm fluorescent signals were measured with Aerius.

With the signaling value at the time of addition of DMSO taken as 100% and with the signaling value at the time of addition of 1 µM trametinib taken as 0%, the 50% inhibitory concentration values (IC₅₀) were calculated by Sigmoid-Emax nonlinear regression analysis. The results for some test compounds of the formula (I) and the formula (IA) are shown in the following tables.

**[Table 2-1]**

| Ex | IC₅₀ (nM) | Ex | IC₅₀ (nM) | Ex | IC₅₀ (nM) |
|---|---|---|---|---|---|
| 1 | 7 | 19 | 95 | 37 | 32 |
| 2 | 9 | 20 | 38 | 38 | 36 |
| 3 | 16 | 21 | 47 | 39 | 57 |
| 4 | 8 | 22 | 5 | 40 | 7 |
| 5 | 12 | 23 | 13 | 41 | 9 |
| 6 | 5 | 24 | 15 | 42 | 6 |
| 7 | 9 | 25 | 6 | 43 | 7 |
| 8 | 20 | 26 | 2 | 44 | 31 |
| 9 | 11 | 27 | 7 | 45 | 16 |
| 10 | 8 | 28 | 5 | 46 | 6 |
| 11 | 24 | 29 | 6 | 47 | 22 |
| 12 | 42 | 30 | 10 | 48 | 15 |
| 13 | 8 | 31 | 20 | 49 | 32 |
| 14 | 4 | 32 | 37 | 50 | 8 |
| 15 | 7 | 33 | 45 | 51 | 59 |
| 16 | 16 | 34 | 21 | 52 | 4 |
| 17 | 7 | 35 | 12 | 53 | 9 |
| 18 | 100 | 36 | 21 | 54 | 5 |

**[Table 2-2]**

| Ex | IC₅₀ (nM) |
|---|---|
| 55 | 4 |
| 56 | 17 |

### Test Example 3: Evaluation of non-anchorage-dependent cell growth inhibitory effect on human G12D mutant KRAS-positive pancreatic cancer line AsPC-1

The non-anchorage-dependent cell growth inhibitory effect of test compounds was evaluated by spheroid 3D cell culture.

AsPC-1 cells were seeded on low-cell-adhesive round bottom 384-well plates (PrimeSurface: from Sumitomo Bakelite) at 36 µL/well to give 5 x 10² cells per well. As for the cell culture conditions, RPMI 1640 medium containing 10% fetal bovine serum was used in the presence of 5% CO₂ at 37°C.

The next day, the test compounds (10 or 6 points having final concentrations in the range of 10 µM to 0.3 nM) and DMSO, which was the solvent for the test compounds, as a negative control were diluted 100-fold with a fresh medium and were added at 4 µL per well. After culturing in the presence of 5% CO₂ at 37°C for 6 days, CellTiter Glo 2.0 (from Promega) was added at 20 µL per well. After stirring with a plate mixer (from FINEPCR) at room temperature for 1 hour, the luminescent signals were measured with ARVO X3 (from PerkinElmer).

With the signaling value in treatment with DMSO taken as 100% and with the signaling value in the medium alone without cells taken as 0%, the 50% inhibitory concentration values (IC₅₀) were calculated by Sigmoid-Emax nonlinear regression analysis. The results for some test compounds of the formula (I) and the formula (IA) are shown in the following tables.

**[Table 3-1]**

| Ex | IC₅₀ (nM) | Ex | IC₅₀ (nM) | Ex | IC₅₀ (nM) |
|---|---|---|---|---|---|
| 1 | 9 | 19 | 50 | 37 | 32 |
| 2 | 92 | 20 | 21 | 38 | 36 |
| 3 | 7 | 21 | 53 | 39 | 39 |
| 4 | 7 | 22 | 5 | 40 | 6 |
| 5 | 10 | 23 | 12 | 41 | 8 |
| 6 | 8 | 24 | 26 | 42 | 4 |
| 7 | 23 | 25 | 6 | 43 | 6 |
| 8 | 16 | 26 | 3 | 44 | 17 |
| 9 | 9 | 27 | 7 | 45 | 15 |
| 10 | 6 | 28 | 5 | 46 | 5 |
| 11 | 19 | 29 | 7 | 47 | 15 |
| 12 | 41 | 30 | 12 | 48 | 16 |
| 13 | 10 | 31 | 18 | 49 | 10 |
| 14 | 4 | 32 | 24 | 50 | 8 |
| 15 | 7 | 33 | 24 | 51 | 44 |
| 16 | 14 | 34 | 13 | 52 | 4 |
| 17 | 8 | 35 | 9 | 53 | 10 |
| 18 | 73 | 36 | 19 | 54 | 7 |

**[Table 3-2]**

| Ex | IC₅₀ (nM) |
|---|---|
| 55 | 15 |
| 56 | 73 |

**[Table 3-3]**

| Ex | IC₅₀ (nM) |
|---|---|
| 57 | 95 |

### Test Example 4: Evaluation of anti-tumor activity in human KRAS G12D mutant - positive PK-59 pancreatic cancer cell line -derived xenograft mice

PK-59 cells (RIKEN BRC, RCB1901) were cultured using a RPMI 1640 medium containing 10% fetal bovine serum in the presence of 5% CO₂ at 37°C. The PK-59 cells were collected and suspended in PBS, and after adding an equal amount of Matrigel (from Becton, Dickinson and Company), 4-5-week-old male nude mice (CAnN.Cg-Foxn1^{nu}/CrlCrlj (nu/nu), from Charles River Laboratories Japan) were subcutaneously inoculated with the cell suspension of 1.0-2.0 × 10⁷ cells/mL in a volume of 100 µL per mouse. About two weeks after the inoculation, the mice were divided into groups so that all the groups had approximately the same tumor volume and body weight, and administration of test compounds was started on the next day. The study was conducted for 5 mice each of a vehicle group and test compound administration groups. The compounds were dissolved in a solvent containing ethanol (from FUJIFILM Wako Pure Chemical Corporation), a 5% glucose solution (from Otsuka Pharmaceutical), 1M hydrochloric acid (from Kanto Chemical Co., Inc.), 50% (2-hydroxypropyl)-β-cyclodextrin (HP-βCD) solution (from ROQUETTE), HCO-40 (from Nikko Chemicals Co., Ltd.) and 1M sodium hydroxide solution (from Kanto Chemical Co., Inc.) at a liquid amount ratio of 4:84.4:1.1:1:9:0.5. The test compounds dissolved in the respective solvent for the test compound administration groups and the solvent for the vehicle group were administered into tail vein. The administration was performed once a week and twice in total. The tumor sizes and the body weights were measured twice a week. The tumor volumes were calculated using the following equation. [Tumor volume (mm3)] = [Long diameter of the tumor (mm)] × [Short diameter of the tumor (mm)]2 × 0.5

The tumor growth inhibition rate (%) by the test compounds were calculated with the tumor volumes of the test compound administration groups on the previous day of the start of the administration taken as 100% inhibition and the tumor volumes of the vehicle groups two weeks after the initial administration taken as 0% inhibition. In addition, when the tumor volume of a test compound administration group was smaller than the tumor volume on the previous day of the start of the administration, the tumor regression rate (%) by the test compound was calculated with the tumor volume on the previous day of the start of the administration taken as 0% regression and with the tumor volume 0 taken as 100% regression. The results for some test compounds of the formula (I) and the formula (IA) are shown in Table below.

**[Table 4]**

| Ex | Dose (mg/kg) | Anti-Tumor Activity Two Weeks After Initial Administration |
|---|---|---|
| 9 | 10 | 81% inhibition |
| 11 | 10 | 6% regression |
| 13 | 10 | 26% regression |
| 50 | 10 | 20% regression |
| 53 | 10 | 53% regression |

### Test Example 5: Evaluation of inhibitory effect on KRAS G12D/SOS/c-Raf complex formation

Using human recombinant KRAS G12D, SOS and c-Raf proteins, the inhibitory effect of test compounds on the formation of the complex of the proteins was examined by the time-resolved fluorescence resonance energy transfer (TR-FRET) method.

Biotinylated AviTag-KRAS G12D (amino acid region of 1-185, GDP) (2.5 µL; 400 nM) and test compounds dissolved in an assay buffer (50 mM HEPES [from Jena], 150 mM NaCl [from Nacalai Tesque], 5 mM MgCl₂ [from Thermo Fisher Scientific], 0.05% Tween 20 [from Sigma-Aldrich], pH 7.0) were added to 384-well plates (from Corning) in a liquid volume of 2.5 µL at 40,000 nM to 40 nM. Son of Sevenless (SOS) (amino acid region of 564-1049, 2.5 µL; 1.3 µM) and c-Raf (amino acid region of 51-131) GST (2.5 µL; 130 nM) containing GTP (from Sigma-Aldrich; 2 µM) were added to the plates, and the plates were allowed to stand for 1 hour at room temperature. Then, a mixture liquid (10 µL) of LANCE Ulight-anti-GST (from PerkinElmer; 120 nM) and LANCE Eu-W1024 labeled Streptoavidin (from PerkinElmer; 100 ng/mL) was added, and the 620-nm and 665-nm fluorescence intensities were measured using EnVision 2104 (from PerkinElmer) under the conditions of an excitation wavelength of 337 nm. After standardizing the values with the fluorescence intensity at a reference wavelength of 620 nm, the 50% inhibitory concentrations (IC₅₀) were calculated by Sigmoid-Emax nonlinear regression analysis with the signaling value of the solvent treatment taken as 0% inhibition and with the signaling value without the addition of GTP taken as 100% inhibition.

As a result of the above tests, G12D mutant KRAS degradation activity was observed for some compounds of the formula (I) and the formula (IA) (Test Example 1). Moreover, G12D mutant KRAS inhibitory effect was observed (Test Example 5). Furthermore, inhibitory effect on phosphorylation of ERK located downstream of the KRAS signal was observed for some compounds of the formula (I) and the formula (IA) (Test Example 2). For some compounds of the formula (I) and the formula (IA), cell growth inhibitory effect on a human G12D mutant KRAS-positive pancreatic cancer line was observed (Test Example 3), and anti-tumor activity in human G12D mutant KRAS-positive pancreatic cancer line tumor-bearing mice was observed (Test Example 4). Therefore, the compounds of the formula (I) and the formula (IA) can be used for the treatment of pancreatic cancer, in particular, G12D mutant KRAS-positive pancreatic cancer, and the like.

A pharmaceutical composition that contains one or two or more compounds of the formula (I) and the formula (IA) or salts thereof as active ingredients can be prepared by a usually used method using an excipient usually used in the art, namely, a pharmaceutical excipient, a pharmaceutical carrier or the like.

The administration may be either oral administration with a tablet, pill, capsule, granule, powder, liquid or other agent or parenteral administration with an intraarticular, intravenous, intramuscular or other injection, a transmucosal agent, an inhalant or the like.

As a solid composition for oral administration, a tablet, powder, granular or other agent is used. In such a solid composition, one or two or more active ingredients are mixed with at least one inactive excipient. The composition may contain an inactive additive, for example, a lubricant, a disintegrator, a stabilizer or a dissolution aid, according to an ordinary method. A tablet or pill may be coated with a sugar coating or a film soluble in the stomach or intestine, as needed.

Liquid compositions for oral administration include a pharmaceutically acceptable emulsion, solution, suspension, syrup, elixir agent and the like and contain a generally used inactive diluent, for example, purified water or EtOH. The liquid composition may contain, in addition to the inactive diluent, an adjuvant, such as a solubilizer, a wetting agent or a suspending agent, a sweetening agent, a flavor, an aromatic or a preservative.

The injection agents for parenteral administration include a sterile aqueous or nonaqueous solution, suspension or emulsion agent. Examples of the aqueous solvent include distilled water for injection or physiological saline. An example of the nonaqueous solvent is an alcohol, such as EtOH. Such a composition may further contain an isotonizing agent, a preservative, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition, which is dissolved or suspended in sterile water or a sterile solvent for injection before use.

The transmucosal agent, such as an inhalant or a transnasal agent, is used in a solid, liquid or semi-solid form and can be produced according to a conventionally known method. For example, a known excipient and in addition, a pH modifier, a preservative, a surfactant, a lubricant, a stabilizer, a thickener or the like may be appropriately added. The administration can be performed using an appropriate device for inhalation or insufflation. For example, the agent can be administered using a known device, such as a metering and administering inhalation device, or an atomizer, as a compound alone or a powder of a mixture formulated, or as a solution or a suspension in combination with a medically acceptable carrier. A dry powder inhaler or the like may be for a single administration or multiple administrations, and dry powder or powder-containing capsule can be used. Alternatively, the agent may be used in a form of a pressurized aerosol spray or the like using an appropriate ejection agent, for example, a suitable gas, such as a chlorofluoroalkane or carbon dioxide.

In the case of a common oral administration, the daily dose is appropriately about 0.001 to 100 mg/kg body weight, preferably 0.1 to 30 mg/kg body weight, further preferably 0.1 to 10 mg/kg body weight, and the dose is given once or is divided into two to four times in a day. In the case of intravenous administration, the daily dose is appropriately about 0.0001 to 10 mg/kg body weight and is given once or is divided into multiple times in a day. In addition, the daily dose of a transmucosal agent is about 0.001 to 100 mg/kg body weight and is given once or is divided into multiple times in a day. The dose is appropriately decided depending on the individual case taking the symptom, age, sex and the like into account.

Depending on the route of administration, dosage form, site of administration and types of excipient and additive, the pharmaceutical composition of the present invention contains 0.01 to 100% by weight, in one embodiment, 0.01 to 50% by weight, of one or more compounds of the formula (I) and the formula (IA) or salts thereof which are active ingredients.

The compounds of the formula (I) and the formula (IA) can be used in combination with various therapeutic agents or preventive agents for a disease to which the compounds of the formula (I) and the formula (IA) are considered to have an effectiveness. The combination use may be simultaneous administration or separate administration either sequential or with a desired interval. A simultaneous administration preparation may be a formulated agent or may be separately formulated.

### EXAMPLES

The production method of the compounds of the formula (I) and the formula (IA) will be described in further detail below based on Examples. Note that the present invention is not to be limited to the compounds described in the following Examples. The production methods of raw material compounds are also shown in the Production Examples. The production method of the compounds of the formula (I) and the formula (IA) is not limited only to the production methods of specific Examples described below, and the compounds of the formula (I) and the formula (IA) can also be produced by a combination of the production methods or a method that is obvious to a person skilled in the art.

Note that, in this specification, a compound is sometimes named using naming software, such as ACD/Name (registered trademark, Advanced Chemistry Development, Inc.).

For the purpose of convenience, the concentration mol/L is shown as M. For example, 1 M aqueous sodium hydroxide solution means an aqueous sodium hydroxide solution of 1 mol/L.

The "amorphous solid form" described in this specification includes both a form showing no peak in the powder X-ray diffraction (XRD) pattern and a form having a low crystallinity.

XRD is measured using Empyrean under the conditions of a vacuum tube of Cu, a tube current of 40 mA, a tube voltage of 45 kV, a step width of 0.013°, a wavelength of 1.5418 Å and a measurement diffraction angle range (2 θ) of 2.5 to 40°.

### Production Example 1

In THF (400 mL), 7-bromo-2,4-dichloro-8-fluoro-6-iodoquinazoline (40 g) was suspended, aqueous sodium hydroxide solution (1M, 190 mL) was added dropwise under ice-bath cooling so that the internal temperature was 10°C or lower, and the mixture was stirred as it was for 2 hours. The reaction solution was poured at once into an Erlenmeyer flask containing hydrochloric acid (1M, 190 mL) and ice water (about 900 g), and the mixture was stirred for about 30 minutes at room temperature (until the ice melted). The insoluble materials were filtered while washing with water and were dried under reduced pressure, thus obtaining 7-bromo-2-chloro-8-fluoro-6-iodoquinazolin-4-ol (33.56 g) as a solid.

### Production Example 2

Under nitrogen flow, to a mixture of 7-bromo-2-chloro-8-fluoro-6-iodoquinazolin-4-ol (24.6 g) and THF (260 mL), which was heated to 60°C, was added 2-tert-butyl-1,3-diisopropylisourea (73.4 g) dropwise over 15 minutes. The mixture was stirred at the same temperature for 2.5 hours. After allowing to cool to room temperature, the colorless solid was separated by filtration while washing with THF (about 500 mL). The filtrate was concentrated, MeOH (210 mL) was added to the resulting solid, and the mixture was suspended and washed by stirring at room temperature for 1 hour. The solid was filtered with MeOH (100 mL) to give 7-bromo-4-tert-butoxy-2-chloro-8-fluoro-6-iodoquinazoline (23.2 g) as a solid.

### Production Example 3

Ethanethiol (3.6 mL) and DABCO (7.7 g) were added to a CH₂Cl₂ (200 mL) suspension of 7-bromo-4-tert-butoxy-2-chloro-8-fluoro-6-iodoquinazoline (21 g) at room temperature, and under an argon atmosphere, the mixture was stirred at room temperature overnight. The reaction was quenched with water under ice-bath cooling. CHCl₃ was added, the organic layer and the aqueous layer were separated, and the aqueous layer was extracted with CHCl₃ three times.

The collected organic layer was dried over anhydrous sodium sulfate, was filtered and was concentrated under reduced pressure, thus obtaining 7-bromo-4-tert-butoxy-2-(ethylsulfanyl)-8-fluoro-6-iodoquinazoline (23.4 g) as a solid.

### Production Example 5

Under nitrogen atmosphere, to a DMF (75 mL)/THF (75 mL) suspension of 7-bromo-4-tert-butoxy-2-chloro-8-fluoro-6-iodoquinazoline (15 g) and tetrahydro-2H-pyran-4-ol (4.67 mL) were added cesium carbonate (21.3 g) and DABCO (360 mg) under ice-bath cooling, and the mixture was stirred at the same temperature for 10 minutes and at room temperature overnight. The reaction mixture was filtered through celite (registered trademark) pad and was washed with ethyl acetate. Water was added to the filtrate, and the mixture was extracted with ethyl acetate. The combined organic layer was sequentially washed with semi-saturated aqueous sodium chloride solution and saturated aqueous sodium chloride solution, was dried over anhydrous magnesium sulfate and was filtered. The filtrate was concentrated under reduced pressure. MeOH (50 ml) was added to the resulting solid, and the mixture was stirred at room temperature for 30 minutes. The resulting solid was filtered, was washed with MeOH (40 ml) and was dried under reduced pressure, thus obtaining 7-bromo-4-tert-butoxy-8-fluoro-6-iodo-2-[(oxan-4-yl)oxy]quinazoline (13.83 g) as a solid.

### Production Example 6

To a THF (400 mL) solution of 7-bromo-4-tert-butoxy-2-(ethylsulfanyl)-8-fluoro-6-iodoquinazoline (32 g) and (1S)-1-phenylethan-1-ol (11 mL) was added tBuOK (10 g) under ice-bath cooling, and under an argon atmosphere, the mixture was stirred under ice-bath cooling for 1 hour. The reaction was quenched with saturated aqueous ammonium chloride solution under ice-bath cooling. Water and ethyl acetate were added, and the organic layer and the aqueous layer were separated. The organic layer was washed with saturated aqueous sodium chloride solution, was dried over anhydrous sodium sulfate and was filtered. The filtrate was concentrated under reduced pressure, and the residue was purified by silica gel column chromatography (hexane/ethyl acetate), thus obtaining 7-bromo-4-tert-butoxy-2-(ethylsulfanyl)-6-iode-8-[(1S)-1-phenylethoxy]quinoline (36.6 g) as an oil.

### Production Example 9

At room temperature, 7-bromo-4-tert-butoxy-2-(ethylsulfanyl)-6-iodo-8-[(1S)-1-phenylethoxy]quinazoline (36.6 g), cyclopropyl boronic acid (7.5 g),
PdCl₂(dppf)·CH₂Cl₂ (7.6 g), tripotassium phosphate (53 g), MeCN (440 mL) and water (80 mL) were mixed, and under an argon atmosphere, the mixture was stirred at 90°C for 4 hours. The reaction solution was cooled to room temperature and was then diluted with ethyl acetate and water. The organic layer and the aqueous layer were separated, and the organic layer was washed with saturated aqueous sodium chloride solution, was dried over anhydrous sodium sulfate and was filtered. The filtrate was concentrated under reduced pressure, and the residue was purified by silica gel column chromatography (hexane/ethyl acetate), thus obtaining 7-bromo-4-tert-butoxy-6-cyclopropyl-2-(ethylsulfanyl)-8-[(1S)-1-phenylethoxy]quinoline (22.9 g) as an oil.

### Production Example 13

To 7-bromo-4-tert-butoxy-6-cyclopropyl-2-(ethylsulfanyl)-8-[(1S)-1-phenylethoxy]quinazoline (14.21 g) were added 6-fluoro-5-methyl-4-(4,4,5,5-tetramethyl-1,3,2-dioxaborolan-2-yl)-2-(triphenylmethyl)-2H-indazole (19.3 g), palladium(II) acetate (0.67 g), dicyclohexyl(2',6'-diisopropoxy-[1,1'-biphenyl]-2-yl)phosphine (2.67 g), anhydrous barium hydroxide (14.6 g) and DOX (500 mL)/water (100 mL), degassing and argon gas substitution operations were performed several times. Then, the mixture was heated and stirred at 50°C overnight under an argon atmosphere. The reaction suspension which was allowed to cool was filtered through celite (registered trademark) pad while washing with ethyl acetate, and the gray insoluble materials were removed by filtration. After the filtrate was concentrated to about 1/4 under reduced pressure, water was added, and the mixture was extracted twice with ethyl acetate. The collected organic layer was washed with saturated aqueous sodium chloride solution, was dried over anhydrous magnesium sulfate and was concentrated under reduced pressure. The resulting residue was purified by silica gel column chromatography (hexane/ethyl acetate), thus obtaining 4-tert-butoxy-6-cyclopropyl-2-(ethylsulfanyl)-7-[6-fluoro-5-methyl-2-(triphenylmethyl)-2H-indazol-4-yl]-8-[(1S)-1-phenylethoxy]quinazoline (about 3.3:1 diastereomeric mixture derived from axial chirality, 16.44 g) as a solid.

### Production Example 14

In DOX (10 mL), 7-bromo-4-tert-butoxy-6-cyclopropyl-2-[(oxan-4-yl)oxy]-8-[(1S)-1-phenylethoxy]quinazoline (500 mg) and 6-fluoro-5-methyl-4-(4,4,5,5-tetramethyl-1,3,2-dioxaborolan-2-yl)-2-(triphenylmethyl)-2H-indazole (670 mg) were dissolved at room temperature, and palladium(II) acetate (22 mg), dicyclohexyl(2',6'-diisopropoxy-[1,1'-biphenyl]-2-yl)phosphine (87 mg), anhydrous barium hydroxide (870 mg) and water (1 mL) were added at room temperature, and the mixture was stirred at 50°C for 45 minutes. DOX (5 mL) and 6-fluoro-5-methyl-4-(4,4,5,5-tetramethyl-1,3,2-dioxaborolan-2-yl)-2-(triphenylmethyl)-2H-indazole (90 mg) were added at the same temperature, and the mixture was stirred for 30 minutes at the same temperature. The reaction mixture was allowed to cool, ethyl acetate and celite (registered trademark) were added, and the mixture was stirred at room temperature for 30 minutes. Then, the reaction solution was filtered through celite (registered trademark) pad, and the filtrate was concentrated. The resulting residue was purified by silica gel chromatography (basic silica gel, hexane/ethyl acetate). To the resulting solid was added iPrOH (40 mL), and the mixture was stirred at 50°C for 1 hour and at room temperature for 2 hours. The resulting solid was filtered to give 4-tert-butoxy-6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazoline (about 1:1 diastereomeric mixture derived from axial chirality, 296 mg) as a solid. The filtrate was concentrated to give the desired compound (7M)-4-tert-butoxy-6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazoline (single diastereomer, 409 mg) as a solid.

### Production Example 15

Under an argon atmosphere, a mixture of tert-butyl (3S)-3-({7-bromo-6-cyclopropyl-2-(ethylsulfanyl)-8-[(1S)-1-phenylethoxy]quinolin-4-yl}oxy)pyrrolidine-1-carboxylate (2.71 g), 6-fluoro-5-methyl-4-(4,4,5,5-tetramethyl-1,3,2-dioxaborolan-2-yl)-2-(triphenylmethyl)-2H-indazole (3.44 g), dicyclohexyl(2',6'-diisopropoxy-[1,1'-biphenyl]-2-yl)phosphine (531 mg), palladium(II) acetate (106 mg), and anhydrous barium hydroxide (2.29 g) were suspended in DOX (110 mL) and water (22 mL), the suspension was stirred at 50°C for 15 hours. 6-fluoro-5-methyl-4-(4,4,5,5-tetramethyl-1,3,2-dioxaborolan-2-yl)-2-(triphenylmethyl)-2H-indazole (1.6 g) was added, and the mixture was further stirred at 50°C for 15 minutes. After the mixture was cooled to room temperature, ethyl acetate and water were added, and the insoluble materials were removed by filtration through celite (registered trademark) pad. The two layers of filtrate were separated, and the aqueous layer was extracted with ethyl acetate. The combined organic layer was washed with saturated aqueous sodium chloride solution and was dried over anhydrous magnesium sulfate. After separating the drying agent by filtration, the filtrate was concentrated under reduced pressure, and the resulting residue was purified by silica gel column chromatography (basic silica gel, hexane/ethyl acetate), thus obtaining tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 5:1 diastereomeric mixture derived from axial chirality, 3.88 g) as an oil.

Similarly, to tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 5:1 diastereomeric mixture derived from axial chirality, 100 mg) was added MeOH (2 mL), and the mixture was heated to 70°C to dissolve. The mixture was stirred at room temperature overnight and was further stirred for 3 days. Solid precipitation (pale yellow suspension) was observed. The solid was filtered and was washed with a small amount of MeOH, thus obtaining tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 1:1 diastereomeric mixture derived from axial chirality, 21 mg) as a powder. The filtrate was concentrated, thus obtaining the desired (3S)-3-[{6-cyclopropyl-2-(ethylsulfanyl)-7-[6-fluoro-5-methyl-2-(triphenylmethyl)-2H-indazol-4-yl] -8- [(1S)-1 -phenylethoxy] quinolin-4-yl} oxy)pyrrolidine-1 - carboxylate (single diastereomer with unknown axial chirality configuration, 70 mg) as a solid.

Similarly, to tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 5:1 diastereomeric mixture derived from axial chirality, 2.95 g) was added EtOH (6 mL), and the mixture was heated to 70°C to dissolve. The powder of the axially chiral mixture (obtained above) was added in a trace amount at room temperature, and the mixture was stirred at room temperature overnight. The precipitated solid was filtered and was washed with a small amount of EtOH, thus obtaining tert-butyl (3S)-3-({6-cyclopropyl-2-(ethanesulfonyl)-7-(6-fluoro-5-methyl-2-(triphenylmethyl)-2H-indazol-4-yl] -8- [(1S)-1 -phenylethoxy] quinolin-4-yl} oxy)pyrrolidine-1 - carboxylate (about 1:1 diastereomeric mixture derived from axial chirality, 699 mg) as a colorless powder. The filtrate was concentrated, thus obtaining the desired (3S)-3-[{6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (single diastereomer with unknown axial chirality configuration, 2.33 g) as a foam-like solid.

### Production Example 17

Under nitrogen flow, 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (about 3.3:1 diastereomeric mixture derived from axial chirality, 33.71 g) was dissolved in CH₂Cl₂ (500 mL), and m-chloroperbenzoic acid (about 30% water content, 22.4 g) was added under ice-bath cooling (internal temperature: 5 to 10°C). The mixture was stirred for 2 hours at room temperature. Under ice-bath cooling, an aqueous solution (300 mL) of sodium thiosulfate pentahydrate (11 g) and saturated aqueous sodium hydrogen carbonate solution (300 mL) were poured into the reaction solution, and the mixture was stirred for 30 minutes at room temperature and was then extracted with ethyl acetate twice. The combined organic layer was washed with saturated aqueous sodium hydrogen carbonate solution and saturated aqueous sodium chloride solution, was dried over anhydrous magnesium sulfate and was filtered. The filtrate was concentrated under reduced pressure, thus obtaining 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (diastereomeric mixture derived from axial chirality). iPrOH (1000 mL) was added to the resulting residue, and the mixture was stirred at room temperature overnight. The resulting solid was filtered, was washed with iPrOH and was dried under reduced pressure, thus obtaining 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (about 1:1 diastereomeric mixture derived from axial chirality, 11.52 g) as a solid. The filtrate was concentrated under reduced pressure, thus obtaining the desired (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (single diastereomer, 23.29 g) as a solid.

### Production Example 19

Under nitrogen atmosphere, to a THF (50 mL) solution of (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (2.00 g) was added 4-methylbenzene-1-sulfonic acid monohydrate (230 mg) at room temperature, and the mixture was stirred at 50°C for 1 hour. After the reaction solution was allowed to cool to room temperature, TEA (350 µL) was added, and the mixture was concentrated under reduced pressure. The resulting residue was purified by silica gel column chromatography (basic silica gel, hexane/ethyl acetate), thus obtaining (7M)-4-tert-butoxy-6-cyclopropyl-2-(ethanesulfonyl)-7-(6-fluoro-5-methyl-1H-indazol-4-yl)-8-[(1S)-1-phenylethoxy]quinazoline (740 mg) as a foam-like solid.

### Production Example 20

THF (20 mL) was added to (7M)-4-tert-butoxy-6-cyclopropyl-8-[(4-ethynylphenyl)methoxy]-7-[6-fluoro-5-methyl-2-(triphenylmethyl)-2H-indazol-4-yl]-2-[(oxan-4-yl)oxy]quinazoline (2.11 g), and methanesulfonic acid (480 µL) was added under ice-bath cooling. The mixture was stirred at room temperature for 4 hours and was poured into saturated aqueous sodium hydrogen carbonate solution to quench the reaction, and the mixture was extracted with ethyl acetate three times. The combined organic layer was dried over anhydrous magnesium sulfate, was filtered and was concentrated under reduced pressure. The residue was purified by silica gel column chromatography (CHCl₃/MeOH) and was again purified by silica gel column chromatography (basic silica gel, CHCl₃/MeOH), thus obtaining (7M)-6-cyclopropyl-8-[(4-ethynylphenyl)methoxy]-7-(6-fluoro-5-methyl-1H-indazol-4-yl)-2-[(oxan-4-yl)oxy]quinazolin-4-ol (1.02 g) as a solid.

### Production Example 21

Under nitrogen atmosphere, to a THF (10 mL) solution of (7M)-4-tert-butoxy-6-cyclopropyl-2-(ethanesulfonyl)-7-(6-fluoro-5-methyl-1H-indazol-4-yl)-8-[(1S)-1-phenylethoxy]quinazoline (735 mg) were added 3,4-dihydro-2H-pyran (900 µL) and 4-methylbenzene-1-sulfonic acid monohydrate (35 mg) at room temperature. The mixture was stirred at room temperature overnight, and the reaction solution was concentrated under reduced pressure. The resulting residue was purified by silica gel column chromatography (basic silica gel, hexane/ethyl acetate), thus obtaining (7M)-4-tert-butoxy-6-cyclopropyl-2-(ethanesulfonyl)-7-[6-fluoro-5-methyl-1-(oxan-2-yl)-1H-indazol-4-yl]-8-[(1S)-1-phenylethoxy]quinazoline (735 mg) as a foam-like solid.

### Production Example 23

Under an argon atmosphere, to a MeOH (15 mL)/THF (15 mL) solution of (7M)-4-tert-butoxy-6-cyclopropyl-2-(ethanesulfonyl)-7-[6-fluoro-5-methyl-1-(oxan-2-yl)-1H-indazol-4-yl]-8-[(1S)-1-phenylethoxy]quinazoline (730 mg) were added sodium hydrogen carbonate (450 mg) and 10% Pd/C ( about 50% water content, 250 mg) at room temperature. Under a hydrogen atmosphere, the mixture was stirred at room temperature overnight and was substituted with argon. Then, the reaction solution was filtered through celite (registered trademark) pad and was washed with ethyl acetate. The filtrate was concentrated under reduced pressure, thus obtaining (7M)-4-tert-butoxy-6-cyclopropyl-2-(ethanesulfonyl)-7-[6-fluoro-5-methyl-1-(oxan-2-yl)-1H-indazol-4-yl]quinazolin-8-ol (630 mg) as a foam-like solid.

### Production Example 25

To tert-butyl (3S)-3-[{(7M)-6-cyclopropyl-7-[6-fluoro-5-methyl-1-(oxan-2-yl)-1H-indazol-4-yl]-2-[(2S)-2-methoxypropoxy]-8-[(1S)-1-pheny1ethoxy]quinazolin-4-yl}(methyl)amino]pyrrolidine-1-carboxylate (1.90 g) was added MeOH (30 mL), and 10% Pd/C ( about 50% water content, 1.2 g) was added. Then, the mixture was stirred under a hydrogen atmosphere at room temperature overnight. The reaction mixture was filtered through celite (registered trademark) pad. The filtrate was concentrated under reduced pressure and was azeotroped with EtOH. The resulting residue was purified by silica gel column chromatography (hexane/ethyl acetate), thus obtaining tert-butyl (3S)-3-[{(7M)-6-cyclopropyl-7-[6-fluoro-5-methyl-1-(oxan-2-yl)-1H-indazol-4-yl]-8-hydroxy-2-[(2S)-2-methoxypropoxy]quinazolin-4-yl}(methyl)amino]pyrrolidine-1-carboxylate (1.5 g) as a foam-like solid.

### Production Example 29

Under nitrogen flow, (7M)-4-tert-butoxy-6-cyclopropyl-2-(ethanesulfonyl)-7-[6-fluoro-5-methyl-1-(oxan-2-yl)-1H-indazol-4-yl]quinazolin-8-ol (1.4 g) and cesium carbonate (2.4 g) were suspended in DMF (17 mL), 1-(chloromethyl)-4-ethynylbenzene (500 mg) was added at room temperature, and the mixture was stirred at 60°C for 1.5 hours. Water (100 ml) was added to the reaction mixture, and the mixture was extracted with ethyl acetate twice. The organic layer was dried over anhydrous magnesium sulfate, was filtered and was concentrated under reduced pressure. The residue was purified by silica gel column chromatography (hexane/ethyl acetate), thus obtaining (7M)-4-tert-butoxy-6-cyclopropyl-2-(ethanesulfonyl)-8-[(4-ethynylphenyl)methoxy]-7-[6-fluoro-5-methyl-1-(oxan-2-yl)-1H-indazol-4-yl]quinazoline (1.47 g) as a foam-like solid.

### Production Example 30

Under nitrogen atmosphere, to a mixture of tert-butyl (3S)-3-[{(7M)-6-cyclopropyl-7-[6-fluoro-5-methyl-1-(oxan-2-yl)-1H-indazol-4-yl]-8-hydroxy-2-[(2S)-2-methoxypropoxy]quinazolin-4-yl}(methyl)amino]pyrrolidine-1-carboxylate (150 mg), 4-(chloromethyl)-1-ethynyl-2-fluorobenzene (52 mg) and DMF (2 mL) was added cesium carbonate (130 mg) at room temperature, and the mixture was stirred at 60°C for 1 hour. After the reaction mixture was allowed to cool to room temperature, ethyl acetate was added, and the organic layer was washed with water and saturated aqueous sodium chloride solution and was dried over anhydrous magnesium sulfate. Then, the insoluble materials were removed by filtration, and the filtrate was concentrated under reduced pressure. The resulting residue was purified by silica gel column chromatography (basic silica gel, hexane/ethyl acetate), thus obtaining tert-butyl (3S)-3-[{(7M)-6-cyclopropyl-8-[(4-ethynyl-fluorophenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (117 mg) as a solid.

### Production Example 36

Under nitrogen atmosphere, to a THF (60 mL) solution of (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (5.01 g) and (2S)-2-methoxypropan-1-ol (0.94 mL) was added tBuOK (1.00 g) under ice-bath cooling, and the mixture was stirred at the same temperature for 30 minutes. Ice water and aqueous ammonium chloride solution were added to the reaction solution to quench the reaction. The mixture was extracted with ethyl acetate twice, and the organic layer was washed with saturated aqueous sodium chloride solution and was then dried over anhydrous magnesium sulfate. The residue was purified by silica gel column chromatography (hexane/ethyl acetate), thus obtaining (7M)-4-tert-butoxy-6-cyclopropyl-7-[6-fluoro-5-methyl-2-(triphenylmethyl)-2H-indazol-4-yl]-2-[(2S)-2-methoxypropoxy]-8-[(1S)-1-phenylethoxy]quinazoline (4.07 g) as a foam-like solid.

### Production Example 54

Under nitrogen atmosphere, to a THF (11 mL) solution of (7M)-4-tert-butoxy-6-cyclopropyl-2-(ethanesulfonyl)-8-[(4-ethynylphenyl)methoxy]-7-[6-fluoro-5-methyl-1-(oxan-2-yl)-1H-indazol-4-yl]quinazoline (1.47 g) and (2S)-2-methoxypropan-1-ol (0.25 mL) was added tBuOK (275 mg) under cooling in an ice/MeOH bath (-20°C to -15°C), and the mixture was stirred at the same temperature for 30 minutes. Saturated aqueous ammonium chloride solution was added to the reaction mixture at the same temperature, and the mixture was extracted with ethyl acetate twice. The combined organic layer was washed with water and brine, was dried over anhydrous magnesium sulfate and was filtered. The filtrate was concentrated under reduced pressure, thus obtaining (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazoline (1.43 g) as a foam-like solid.

### Production Example 57

Under an argon atmosphere, to a THF (8 mL) solution of (7M)-4-tert-butoxy-6-cyclopropyl-2-(ethanesulfonyl)-8-[(4-ethynylphenyl)methoxy]-7-[6-fluoro-5-methyl-1-(oxan-2-yl)-1H-indazol-4-yl]quinazoline (400 mg) and [(2R)-oxolan-2-yl]methanol (82 mg) was added tBuOK (85 mg) under ice-bath cooling, and the mixture was stirred at the same temperature for 30 minutes. Saturated aqueous ammonium chloride solution was added to the reaction mixture, and the mixture was extracted with ethyl acetate twice. The combined organic layer was washed with saturated aqueous sodium chloride solution, was dried over anhydrous magnesium sulfate and was filtered. The filtrate was concentrated under reduced pressure, and the resulting residue was purified by silica gel column chromatography (hexane/ethyl acetate, thus obtaining (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-{ [(2R)-oxolan-2-yl]methoxy}quinazoline (261 mg) as a foam-like solid.

### Production Example 58

Under an argon atmosphere, to a THF (8 mL) solution of (7M)-4-tert-butoxy-6-cyclopropyl-2-(ethanesulfonyl)-8-[(4-ethynylphenyl)methoxy]-7-[6-fluoro-5-methyl-1-(oxan-2-yl)-1H-indazol-4-yl]quinazoline (400 mg) and [(2S)-oxolan-2-yl]methanol (82 mg) was added tBuOK (85 mg) under ice-bath cooling, and the mixture was stirred at the same temperature for 30 minutes. Saturated aqueous ammonium chloride solution was added to the reaction mixture, and the mixture was extracted with ethyl acetate twice. The combined organic layer was washed with saturated aqueous sodium chloride solution, was dried over anhydrous magnesium sulfate and was filtered. The filtrate was concentrated under reduced pressure, and the resulting residue was purified by silica gel column chromatography (hexane/ethyl acetate, thus obtaining (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-{ [(2S)-oxolan-2-yl]methoxy}quinazoline (330 mg) as a foam-like solid.

### Production Example 59

Under nitrogen atmosphere, to a THF (15 mL) solution of (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazoline (1.43 g) was added 4-methylbenzene-1-sulfonic acid monohydrate (120 mg) at room temperature, and the mixture was stirred at 50°C for 3 hours. The reaction solution was concentrated under reduced pressure, and the resulting residue was purified by silica gel column chromatography (hexane/ethyl acetate), thus obtaining (7M)-6-cyclopropyl-8-[(4-ethynylphenyl)methoxy]-7-[6-fluoro-5-methyl-1-(oxan-2-yl)-1H-indazol-4-yl]-2-[(2S)-methoxypropoxy]quinazolin-4-ol (1.05 g) as a foam-like solid.

### Production Example 60

To a THF (5 mL) solution of (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-{[(2R)-oxolan-2-yl]methoxy}quinazoline (261 mg) were added 4-methylbenzene-1-sulfonic acid monohydrate (10 mg) and 3,4-dihydro-2H-pyran (1 mL) at room temperature, and the mixture was stirred at the same temperature overnight. The reaction solution was concentrated under reduced pressure, and the resulting residue was purified by silica gel column chromatography (basic silica gel, CHCl₃/MeOH), thus obtaining (7M)-6-cyclopropyl-8-[(4-ethynylphenyl)methoxy]-7-[6-fluoro-5-methyl-1-(oxan-2-yl)-1H-indazol-4-yl]-2-{[(2R)-oxolan-2-yl] methoxy }quinazolin-4-ol (215 mg) as a foam-like solid.

### Production Example 61

To a THF (5 mL) solution of (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-{ [(2S)-oxolan-2-yl]methoxy}quinazoline (330 mg) were added 4-methylbenzene-1-sulfonic acid monohydrate (11 mg) and 3,4-dihydro-2H-pyran (1 mL) at room temperature, and the mixture was stirred at the same temperature overnight. The reaction solution was concentrated under reduced pressure, and the resulting residue was purified by silica gel column chromatography (basic silica gel, CHCl₃/MeOH), thus obtaining (7M)-6-cyclopropyl-8-[(4-ethynylphenyl)methoxy]-7-[6-fluoro-5-methyl-1-(oxan-2-yl)-1H-indazol-4-yl]-2-{[(2S)-oxolan-2-yl]methoxy}quinazolin-4-ol (282 mg) as a foam-like solid.

### Production Example 62

Under nitrogen atmosphere, to a THF (20 mL) solution of (7M)-6-cyclopropyl-7-[6-fluoro-5-methyl-1-(oxan-2-yl)-1H-indazol-4-yl]-2-[(2S)-2-methoxypropoxy]-8-[(1S)-1-phenylethoxy]quinazolin-4-ol (1.63 g) were added cesium carbonate (4.25 g) and PyBOP (2 g) at room temperature in this order, and the mixture was stirred at room temperature for 1 hour. tert-Butyl (3S)-3-(methylamino)pyrrolidine-1-carboxylate (1.58 g) was added at room temperature, and the mixture was stirred at 60°C for 2 hours. After the reaction mixture was allowed to cool to room temperature, ethyl acetate was added, and the mixture was filtered through celite (registered trademark) pad. The filtrate was concentrated under reduced pressure, and the resulting residue was purified by silica gel column chromatography (basic silica gel, hexane/ethyl acetate), thus obtaining tert-butyl (3S)-3-[{(7M)-6-cyclopropyl-7-[6-fluoro-5-methyl-1-(oxan-2-yl)-1H-indazol-4-yl]-2-[(2S)-2-methoxypropoxy]-8-[(1S)-1-phenylethoxy] quinazolin-4-yl } (methyl)amino]pyrrolidine-1 - carboxylate (1.9 g) as an oil.

### Production Example 63

Under nitrogen atmosphere, to a THF (4 mL) solution of (7M)-6-cyclopropyl-8-[(4-ethynylphenyl)methoxy]-7-[6-fluoro-5-methyl-1-(oxan-2-yl)-1H-indazol-4-yl]-2-{[(2S)-2-methoxypropoxy]quinazolin-4-ol (200 mg) were added PyBOP (335 mg) and cesium carbonate (205 mg) at room temperature, and the mixture was stirred at room temperature for 1 hour. A THF (1 mL) solution of tert-butyl (3R)-3-(methylamino)pyrrolidine-1-carboxylate (300 mg) and cesium carbonate (500 mg) were added at room temperature, and the mixture was stirred at 60°C for 2 hours. After the reaction mixture was allowed to cool to room temperature, ethyl acetate was added, and the mixture was filtered through celite (registered trademark) pad. The filtrate was concentrated under reduced pressure, and the resulting residue was purified by silica gel column chromatography (basic silica gel, hexane/ethyl acetate), thus obtaining tert-butyl (3R)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (313 mg) as an oil containing impurities.

### Production Example 73

Under nitrogen atmosphere, to a THF (15 mL) solution of (7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-ol (1.05 g) were added cesium carbonate (2.68 g) and PyBOP (1.20 g) at room temperature in this order, and the mixture was stirred at room temperature for 1 hour. tert-Butyl (3S)-3-(methylamino)pyrrolidine-1-carboxylate (0.94) was added at room temperature, and the mixture was stirred at 60°C overnight. Ethyl acetate was added to the reaction solution, and the insoluble materials were filtered through celite (registered trademark) pad. The filtrate was concentrated under reduced pressure, and the resulting residue was purified by silica gel column chromatography (basic silica gel, hexane/ethyl acetate), thus obtaining tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (1.62 g) as an oil.

### Production Example 74

Under nitrogen atmosphere, to a THF (4 mL) solution of (7M)-6-cyclopropyl-8-[(4-ethynylphenyl)methoxy]-7-[6-fluoro-5-methyl-1-(oxan-2-yl)-1H-indazol-4-yl]-2-{ [(2R)-oxolan-2-yl]methoxy}quinazolin-4-ol (106 mg) were added cesium carbonate (265 mg) and PyBOP (120 mg) at room temperature in this order, and the mixture was stirred at room temperature for 1 hour. tert-Butyl (3S)-3-(methylamino)pyrrolidine-1-carboxylate (62 µL) was added at room temperature, and the mixture was stirred at room temperature overnight. Another tert-butyl (3S)-3-(methylamino)pyrrolidine-1-carboxylate (100 µL) was added, and the mixture was stirred at 80°C for 2 hours. Ethyl acetate was added to the reaction solution, and the insoluble materials were separated by filtration through celite (registered trademark) pad. The filtrate was concentrated under reduced pressure, and the resulting residue was purified by silica gel column chromatography (basic silica gel, hexane/ethyl acetate), thus obtaining tert-butyl (3S)-3-{[(7M)-6-cyclopropyl-8-[(4-ethynylphenyl)methoxy]-7-[6-fluoro-5-methyl-1-(oxan-2-yl)-1H-indazol-4-yl]-2-{ [(2R)-oxolan-2-yl]methoxy}quinazolin-4-yl](methyl)amino}pyrrolidine-1-carboxylate (211 mg) as an oil.

### Production Example 75

Under nitrogen atmosphere, to a THF (4 mL) solution of (7M)-6-cyclopropyl-8-[(4-ethynylphenyl)methoxy]-7-[6-fluoro-5-methyl-1-(oxan-2-yl)-1H-indazol-4-yl]-2-{ [(2S)-oxolan-2-yl]methoxy}quinazolin-4-ol (164 mg) were added cesium carbonate (410 mg) and PyBOP (185 mg) at room temperature in this order, and the mixture was stirred at room temperature for 1 hour. tert-Butyl (3S)-3-(methylamino)pyrrolidine-1-carboxylate (96 µL) was added, and the mixture was stirred at room temperature overnight. Another tert-butyl (3S)-3-(methylamino)pyrrolidine-1-carboxylate (100 µL) was added, and the mixture was stirred at 80°C for 2 hours. Ethyl acetate was added to the reaction solution, and the insoluble materials were separated by filtration through celite (registered trademark) pad. The filtrate was concentrated under reduced pressure. The resulting residue was purified by silica gel column chromatography (basic silica gel, hexane/ethyl acetate), thus obtaining tert-butyl (3S)-3-{[(7M)-6-cyclopropyl-8-[(4-ethynylphenyl)methoxy]-7-[6-fluoro-5-methyl-1-(oxan-2-yl)-1H-indazol-4-yl]-2-{[(2S)-oxolan-2-yl]methoxy}quinazolin-4-yl](methyl)amino}pyrrolidine-1-carboxylate (199 mg) as an oil.

### Production Example 79

Under nitrogen atmosphere, to a THF (2 mL) solution of (7M)-6-cyclopropyl-8-[(4-ethynylphenyl)methoxy]-7-[6-fluoro-5-methyl-1-(oxan-2-yl)-1H-indazol-4-yl]-2-{ [(2S)-2-methoxypropoxy]quinazolin-4-ol (100 mg) were added PyBOP (165 mg) and cesium carbonate (255 mg) at room temperature in this order, and the mixture was stirred at room temperature for 1 hour. Cesium carbonate (105 mg) and tert-butyl (3S)-3-aminopyrrolidine-1-carboxylate (130 µL) were added at room temperature, and the mixture was stirred at room temperature overnight. Ethyl acetate was added to the reaction solution, and the insoluble materials were filtered through celite (registered trademark) pad. The filtrate was concentrated, and the resulting residue was purified by silica gel column chromatography (basic silica gel, hexane/ethyl acetate), thus obtaining tert-butyl (3S)-3-({(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}amino)pyrrolidine-1-carboxylate (143 mg) as an oil.

### Production Example 83

Under nitrogen atmosphere, to a solution in tBuOH (3 mL), THF (3 mL) and water (3 mL) of tert-butyl (3R)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (311 mg) and (4R)-1-[(2S)-2-azido-3-methylbutanoyl] -4-hydroxy-N- {(lR)-2-hydroxy-1 -[4-(4-methyl-1,3-thiazol-5-yl)phenyl] ethyl }-L-prolinamide (170 mg) were added sodium ascorbate (110 mg) and anhydrous copper(II) sulfate (30 mg) at room temperature, and the mixture was stirred at room temperature for 2 hours. An aqueous solution (13 mL) of disodium ethylenediaminetetraacetic acid (650 mg) was added, and the mixture was stirred at room temperature for 30 minutes. Then, the mixture was extracted with ethyl acetate. The organic layer was washed with water and saturated aqueous sodium chloride solution, was dried over anhydrous sodium sulfate and was filtered. The filtrate was concentrated under reduced pressure. The residue was purified by silica gel column chromatography (basic silica gel, CHCl₃/MeOH), thus obtaining tert-butyl (3R)-3-[{(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropoxy}quinazolin-4-yl}(methyl)amino}pyrrolidine-1-carboxylate (312 mg) as a solid.

### Production Example 84

To a mixture of tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(ethyl)amino]pyrrolidine-1-carboxylate (133 mg), (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-prolinamide (80 mg), tBuOH (1.2 mL), THF (1.2 mL) and water (1.2 mL) were added sodium ascorbate (66 mg) and anhydrous copper(II) sulfate (27 mg) at room temperature, and the mixture was stirred at room temperature for 3 hours. 2% aqueous disodium ethylenediamine tetraacetate solution and saturated aqueous sodium chloride solution were added, and the mixture was extracted with CH₂Cl₂ twice. The combined organic layer was dried over anhydrous magnesium sulfate. The insoluble materials were removed by filtration, and the filtrate was concentrated under reduced pressure. The residue was purified by silica gel column chromatography (basic silica gel, CHCl₃/MeOH), thus obtaining tert-butyl (3S)-3-[{(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl} -1H-1,2,3-triazol-4-yl)phenyl] methoxy} -2-[(2S)-2-methoxypropoxy}quinazolin-4-yl}(ethyl)amino}pyrrolidine-1-carboxylate (178 mg) as a solid.

### Production Example 92

To a solution in tBuOH (1 mL), THF (1 mL) and water (1 mL) of tert-butyl (3S)-3-[{(7M)-6-cyclopropyl-8-[(4-ethynyl-3-fluorophenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (115 mg) and (4R)-1-[(2S)-2-azido-3-methylbutanoyl]-4-hydroxy-N-{(1R)-2-hydroxy-1- [4-(4-methyl-1,3-thiazol-5-yl)phenyl] ethyl }-L-prolinamide (70 mg) were added sodium ascorbate (41 mg) and anhydrous copper(II) sulfate (11 mg) at room temperature, and the mixture was stirred at room temperature for 1 hour. An aqueous solution (5 mL) of disodium ethylenediaminetetraacetic acid (230 mg) was added, and the mixture was stirred at room temperature for 30 minutes and was extracted with ethyl acetate. The organic layer was washed with water and saturated aqueous sodium chloride solution, was dried over anhydrous sodium sulfate and was filtered. The filtrate was concentrated under reduced pressure. The residue was purified by silica gel column chromatography (basic silica gel, CHCl₃/MeOH), thus obtaining tert-butyl (3S)-3-[{(7M)-6-cyclopropyl-8-{[3-fluoro-4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl] -3-methyl-1-oxobutan-2-yl} -1H-1,2,3-triazol-4-yl)phenyl]methoxy}-7-[6-fluoro-5-methyl-1-(oxan-2-yl)-1H-indazol-4-yl]-2-[(2S)-2-methoxypropoxy}quinazolin-4-yl}(methyl)amino}pyrrolidine-1-carboxylate (171 mg) as a solid.

### Production Example 114

To a solution in tBuOH/THF/water (1:1:1 mixture liquid, 2.0 mL) of (4R)-1-[(2S)-2-azido-3-methylbutanoyl]-N-{(1R)-1-[4-(1-ethyl-1H-pyrazol-5-yl)phenyl]-2-hydroxyethyl}-4-hydroxy-L-prolinamide (80 mg) and tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (125 mg) were added anhydrous copper(II) sulfate (10 mg) and sodium ascorbate (30 mg) at room temperature, and the mixture was stirred at room temperature for 1 hour. Ethylenediaminetetraacetic acid disodium salt (175 mg) was added to the reaction mixture at room temperature, and the mixture was stirred at the same temperature for 30 minutes. Water was added to the reaction mixture, the mixture was stirred, and the resulting insoluble materials were filtered, thus obtaining tert-butyl (3S)-3-[{(7M)-6-cyclopropyl-8-{[4-(1-{(2S)-1-[(2S,4R)-2-({(1R)-1-[4-(1-ethyl-1H-pyrazol-5-yl)phenyl]-2-hydroxyethyl}carbamoyl)-4-hydroxypyrrolidin-1-yl]-3-methyl-1-oxobutan-2-yl}-1H-1,2,3-triazol-4-yl)phenyl]methoxy}-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (225 mg) as a solid.

### Production Example 115

Under an argon atmosphere, to a solution in tBuOH (1 mL), THF (1 mL) and water (1 mL) of (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-prolinamide (120 mg) and tert-butyl (3S)-3-{[(7M)-6-cyclopropyl-8-[(4-ethynylphenyl)methoxy]-7-[6-fluoro-5-methyl-1-(oxan-2-yl)-1H-indazol-4-yl]-2-{[(2S)-oxolan-2-yl]methoxy}quinazolin-4-yl](methyl)amino}pyrrolidine-1-carboxylate (199 mg) were added anhydrous copper(II) sulfate (18 mg), sodium ascorbate (80 mg) at room temperature, and the mixture was stirred at room temperature for 1 hour. Ethylenediaminetetraacetic acid disodium salt (300 mg) was added to the reaction mixture at room temperature, and the mixture was stirred at the same temperature for 1 hour. Water and ethyl acetate were added, and the mixture was extracted with ethyl acetate. The combined organic layer was dried over anhydrous sodium sulfate, was filtered and was concentrated under reduced pressure. The residue was purified by silica gel column chromatography (basic silica gel, CHCl₃/MeOH), thus obtaining tert-butyl (3S)-3-{ [(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl] methoxy} -2- { [(2S)-oxolan-2-yl] methoxy} quinazolin-4-yl](methyl)amino}pyrrolidine-1-carboxylate (236 mg) as a foam-like solid.

### Production Example 116

Under an argon atmosphere, to a solution in tBuOH/THF/water (1:1:1 mixture liquid, 2.0 mL) of (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-prolinamide (75 mg) and tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (230 mg) were added anhydrous copper(II) sulfate (10 mg) and sodium ascorbate (30 mg) at room temperature, and the mixture was stirred at room temperature for 1 hour. Ethylenediaminetetraacetic acid disodium salt (175 mg) was added to the reaction mixture at room temperature, and the mixture was stirred at the same temperature for 1 hour. Water and CHCl₃/MeOH (9/1) were added to the reaction mixture. The organic layer and the aqueous layer were separated, and the aqueous layer was extracted with CHCl₃/MeOH (9/1). The combined organic layer was dried over anhydrous sodium sulfate and was filtered. The filtrate was concentrated under reduced pressure. The resulting residue was purified by silica gel column chromatography (basic silica gel, CHCl₃/MeOH), thus obtaining tert-butyl (3S)-3-[{(7M)-6-cyclopropyl-7-[6-fluoro-5-methyl-1-(oxan-2-yl)-1H-indazol-4-yl]-8-{ [4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl] -3-methyl-1-oxobutan-2-yl} -1H-1,2,3-triazol-4-yl)phenyl]methoxy}-2-[(2S)-2-methoxypropoxy}quinazolin-4-yl}(methyl)amino}pyrrolidine-1-carboxylate (197 mg) as a foam-like solid.

### Production Example 117

Under an argon atmosphere, to a solution in tBuOH (1 mL), THF (1 mL) and water (1 mL) of (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-prolinamide (75 mg) and tert-butyl (3S)-3-{[(7M)-6-cyclopropyl-8-[(4-ethynylphenyl)methoxy]-7-[6-fluoro-5-methyl-1-(oxan-2-yl)-1H-indazol-4-yl]-2-{[(2R)-oxolan-2-yl]methoxy}quinazolin-4-yl](methyl)amino}pyrrolidine-1-carboxylate (123 mg) were added anhydrous copper(II) sulfate (11 mg), sodium ascorbate (50 mg) at room temperature, and the mixture was stirred at room temperature for 1 hour. Ethylenediaminetetraacetic acid disodium salt (200 mg) was added to the reaction mixture at room temperature, and the mixture was stirred at the same temperature for 1 hour. Water and ethyl acetate were added, and the mixture was extracted with ethyl acetate. The combined organic layer was dried over anhydrous sodium sulfate, was filtered and was concentrated under reduced pressure. The residue was purified by silica gel column chromatography (basic silica gel, CHCl₃/MeOH), thus obtaining tert-butyl (3S)-3-{ [(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl] -3-methyl-1-oxobutan-2-yl} -1H-1,2,3-triazol-4-yl)phenyl]methoxy}-2-{ [(2R)-oxolan-2-yl]methoxy}quinazolin-4-yl](methyl)amino}pyrrolidine-1-carboxylate (156 mg) as a foam-like solid.

### Production Example 124

To tert-butyl (3S)-3-({(7M)-6-cyclopropyl-8-[(4-ethynylphenyl)methoxy]-7-(6-fluoro-5-methyl-1H-indazol-4-yl)-2-[(oxan-4-yl)oxy] quinazolin-4-yl} amino)pyrrolidine-1-carboxylate (75 mg) were added tBuOH (1 mL), THF (1 mL) and water (1 mL), and (4R)-1-[(2S)-2-azido-3-methylbutanoyl] -4-hydroxy-N- {(1R)-2-hydroxy-1- [4-(4-methyl-1,3-thiazol-5-yl)phenyl]ethyl}-L-prolinamide (50 mg), copper(I) iodide (10 mg) and sodium ascorbate (30 mg) were added with stirring at room temperature, and the mixture was stirred at 50°C for 3 hours. The reaction solution was allowed to cool, 2% aqueous disodium ethylenediamine tetraacetate solution and ethyl acetate were added, and the mixture was stirred vigorously for 30 minutes. The mixture was extracted with ethyl acetate twice. The combined organic layer was washed with saturated aqueous sodium chloride solution, was dried over anhydrous magnesium sulfate and was concentrated under reduced pressure. The residue was dissolved in THF (1 mL) and water (0.10 mL), (tris(2-carboxyethyl)phosphine hydrochloride (10 mg) was added, and the mixture was stirred at room temperature overnight. The solvent was removed, and the resulting residue was purified by silica gel column chromatography (basic silica gel, CHCl₃/MeOH), thus obtaining tert-butyl (3S)-3-({(7M)-6-cyclopropyl-7-(6-fluoro-5-methyl-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl] -3-methyl-1-oxobutan-2-yl} -1H-1,2,3-triazol-4-yl)phenyl] methoxy} -2-[(oxan-4-yl)oxyl]quinazolin-4-yl}amino)pyrrolidine-1-carboxylate (77 mg) as a solid.

### Production Example 125

To a DMAc (80 mL) solution of tert-butyl [(1R)-1-(4-bromophenyl)-2-hydroxyethyl]carbamate (5.01 g) were added 4-methyl-1,3-thiazole (2.88 mL) and potassium acetate (3.11 g) at room temperature, and the mixture was degassed and substituted with argon three times each. Then, palladium acetate (356 mg) was added at room temperature, and the mixture was stirred at 100 °C for 16 hours. After the mixture was cooled to room temperature, ethyl acetate and water were added to the reaction mixture, and the insoluble materials were removed by filtration through celite (registered trademark) pad. Water was added to the filtrate, and the aqueous layer was extracted with ethyl acetate three times. The combined organic layer was washed with saturated aqueous sodium chloride solution, was dried over anhydrous sodium sulfate and was filtered. The filtrate was concentrated, and then the residue was purified by silica gel column chromatography (basic silica gel, hexane/ethyl acetate), thus obtaining tert-butyl {(1R)-2-hydroxy-1-[4-(4-methyl-1,3-thiazol-5-yl)phenyl]ethyl}carbamate (4.66 g) as a solid.

### Production Example 126

To a solution in CH₂Cl₂ (50 mL) and MeOH (40 mL) of tert-butyl {(1R)-2-hydroxy-1-[4-(4-methyl-1,3-thiazol-5-yl)phenyl]ethyl}carbamate (4.41 g) was added hydrogen chloride (4M DOX solution, 20 mL) portionwise under ice-bath cooling, and the mixture was stirred at room temperature for 6 hours. Diethyl ether was added to the reaction mixture, and the solid was filtered, was washed with diethyl ether and was dried under reduced pressure, thus obtaining (2R)-2-amino-2-[4-(4-methyl-1,3-thiazol-5-yl)phenyl]ethan-1-ol n hydrochloride (2.12 g) as a solid. The filtrate was concentrated under reduced pressure and was dried by heating under reduced pressure, thus obtaining (2R)-2-amino-2-[4-(4-methyl-1,3-thiazol-5-yl)phenyl]ethan-1-ol n hydrochloride (2.01 g) as a solid.

### Production Example 131

To a solution in CH₂Cl₂ (25 mL) and MeOH (25 mL) of tert-butyl {(1R)-1-[4-(1-ethyl-1H-pyrazol-5-yl)phenyl]-2-hydroxyethyl}carbamate (3.34 g), hydrogen chloride (4M DOX solution, 25.6 mL) was added at -20 to -10°C, and the mixture was stirred at room temperature for 5 hours. The reaction mixture was concentrated under reduced pressure, thus obtaining (2R)-2-amino-2-[4-(1-ethyl-1H-pyrazol-5-yl)phenyl]ethan-1-ol n hydrochloride (3.06 g) as a solid.

### Production Example 133

Under argon flow, a mixture of (2R)-2-amino-2-[4-(4-methyl-1,3-thiazol-5-yl)phenyl]ethan-1-ol n hydrochloride (2.12 g), (4R)-1-(tert-butoxycarbonyl)-4-hydroxy-L-proline (1.76 g) and DMF (22 mL) was cooled with ice bath, DIPEA (4.7 mL) was added, and then HATU (3.02 g) was added portionwise so that the internal temperature was maintained at 5°C or lower. The reaction mixture was stirred for 1 hour under ice-bath cooling and for 1 hour at room temperature. The reaction mixture was cooled with ice bath, water (120 mL) and saturated aqueous sodium chloride solution (50 ml) were added, and then the mixture was extracted with ethyl acetate three times. The aqueous layer was further extracted with ethyl acetate/iPrOH (9/1) three times. The combined organic layer was washed with saturated aqueous sodium chloride solution and was dried over anhydrous magnesium sulfate. The solution was concentrated under reduced pressure, and the residue was purified by silica gel column chromatography (CHCl₃/MeOH), thus obtaining tert-butyl (2S,4R)-4-hydroxy-2-({(1R)-2-hydroxy-1-[4-(4-methyl-1,3-thiazol-5-yl)phenyl]ethyl}carbamoyl)pyrrolidine-1-carboxylate (3.09 g) as an oil.

### Production Example 134

To a mixture of (2R)-2-amino-2-[4-(1-ethyl-1H-pyrazol-5-yl)phenyl]ethan-1-ol n hydrochloride (3.43 g), (4R)-1-(tert-butoxycarbonyl)-4-hydroxy-L-proline (2.81 g) and DMF (40 mL) was added DIPEA (7.8 mL) under ice-bath cooling, and then HATU (4.5 g) was added portionwise under ice-bath cooling. The mixture was stirred for 1 hour under ice-bath cooling and for 1 hour at room temperature. Under ice-bath cooling, water, saturated aqueous sodium chloride solution and ethyl acetate were added, and the aqueous layer was separated. The aqueous layer was extracted with ethyl acetate and then was extracted with ethyl acetate/iPrOH (9/1). The organic layer was washed with saturated aqueous sodium chloride solution and was dried over anhydrous magnesium sulfate. The insoluble materials were removed by filtration, and the filtrate was concentrated under reduced pressure. The residue was purified by silica gel column chromatography (CHCl₃/MeOH), thus obtaining tert-butyl (2S,4R)-2-({(1R)-1-[4-(1-ethyl- 1H-pyrazol-5-yl)phenyl] -2-hydroxyethyl}carbamoyl)-4-hydroxypyrrolidine-1-carboxylate (5.01 g) as an oil.

### Production Example 135

To a solution in CH₂Cl₂ (18 mL) and MeOH (18 mL) of tert-butyl (2S,4R)-4-hydroxy-2-({(1R)-2-hydroxy-1-[4-(4-methyl-1,3-thiazol-5-yl)phenyl]ethyl}carbamoyl)pyrrolidine-1-carboxylate (3.09 g) was added hydrogen chloride (4M DOX solution, 17 mL) under ice-bath cooling, and the mixture was stirred for 1 hour under ice-bath cooling and for 4 hour at room temperature. The mixture was concentrated under reduced pressure and was dried, thus obtaining (4R)-4-hydroxy-N-{(1R)-2-hydroxy-1-[4-(4-methyl-1,3-thiazol-5-yl)phenyl]ethyl}-L-prolinamide n hydrochloride (2.92 g) as a solid.

### Production Example 136

To a solution in CH₂Cl₂ (35 mL) and MeOH (30 mL) of tert-butyl (2S,4R)-2-({(1R)-1-[4-(1-ethyl-1H-pyrazol-5-yl)phenyl]-2-hydroxyethyl}carbamoyl)-4-hydroxypyrrolidine-1-carboxylate (5.01 g) was added hydrogen chloride (4M DOX solution, 28 mL) under cooling at -20 to -10°C, and the mixture was stirred at room temperature for 5 hours. The reaction mixture was concentrated under reduced pressure, thus obtaining (4R)-N-{(1R)-1-[4-(1-ethyl-1H-pyrazol-5-yl)phenyl]-2-hydroxyethyl]-4-hydroxy-L-prolinamide n hydrochloride (4.71 g) as a solid.

### Production Example 137

To a mixture of (4R)-4-hydroxy-N-{(1R)-2-hydroxy-1-[4-(4-methyl-1,3-thiazol-5-yl)phenyl]ethyl}-L-prolinamide n hydrochloride (3.81 g), N-(tert-butoxycarbonyl)-L-valine (2.16 g) and DMF (45 mL) was added DIPEA (6.2 mL), and then HATU (3.61 g) was added portionwise under ice-bath cooling. The mixture was stirred for 1 hour under ice-bath cooling and for 1 hour at room temperature. Under ice-bath cooling, water, saturated aqueous sodium chloride solution and ethyl acetate were added, and the aqueous layer was separated. The aqueous layer was extracted with ethyl acetate and then was extracted with ethyl acetate/iPrOH (9/1). The combined organic layer was washed with saturated aqueous sodium chloride solution and was dried over anhydrous sodium sulfate. The insoluble materials were removed by filtration, and the filtrate was concentrated under reduced pressure. The residue was purified by silica gel column chromatography (CHCl₃/MeOH), thus obtaining N-(tert-butoxycarbonyl)-L-valyl-(4R)-4-hydroxy-N-{(1R)-2-hydroxy-1-[4-(4-methyl-1,3-thiazol-5-yl)phenyl]ethyl}-L-prolinamide (4.43 g) as a solid.

### Production Example 138

To a DMF (55 mL) solution of (4R)-N-{(1R)-1-[4-(1-ethyl-1H-pyrazol-5-yl) phenyl]-2-hydroxyethyl}-4-hydroxy-L-prolineamide n hydrochloride (4.7 g) and N-(tert-butoxycarbonyl)-L-valine (2.7 g) was added DIPEA (7.7 mL), and HATU (4.5 g) was added portionwise under ice-bath cooling. The mixture was stirred under ice-bath cooling for 1 hour and at room temperature for 1 hour, and then water/brine (1/1) and ethyl acetate were added under ice-bath cooling to divide the mixture into layers. The aqueous layer was extracted twice with ethyl acetate and was extracted twice with ethyl acetate/iPrOH (9/1). The combined organic layer was washed with saturated aqueous sodium chloride solution and was then dried over anhydrous sodium sulfate. The insoluble materials were removed by filtration, and the filtrate was concentrated under reduced pressure. The resulting residue was purified by silica gel column chromatography (CHCl₃/MeOH), thus obtaining N-(tert-butoxycarbonyl)-L-valyl-(4R)-N-{(1R)-1-[4-(1-ethyl-1H-pyrazol-5-yl)phenyl]-2-hydroxyethyl}-4-hydroxy-L-prolinamide (5.01 g) as a solid.

### Production Example 139

To a solution in CH₂Cl₂ (35 mL) and MeOH (35 mL) of N-(tert-butoxycarbonyl)-L-valyl-(4R)-4-hydroxy-N-{(1R)-2-hydroxy-1-[4-(4-methyl-1,3-thiazol-5-yl)phenyl]ethyl}-L-prolinamide (4.43 g) was added hydrogen chloride (4M DOX solution, 20 mL) under cooling at -20 to -15°C, and the mixture was stirred at room temperature for 6 hours. The reaction mixture was concentrated under reduced pressure, thus obtaining L-valyl-(4R)-4-hydroxy-N-{(1R)-2-hydroxy-1-[4-(4-methyl-1,3-thiazol-5-yl)phenyl]ethyl}-L-prolinamide n hydrochloride (4.21 g) as a solid.

### Production Example 140

To a solution in CH₂Cl₂ (30 mL) and MeOH (30 mL) of N-(tert-butoxycarbonyl)-L-valyl-(4R)-N-{(1R)-1-[4-(1-ethyl-1H-pyrazol-5-yl)phenyl]-2-hydroxyethyl}-4-hydroxy-L-prolinamide (4.92 g) was slowly added hydrogen chloride (4M DOX solution, 22 mL) under cooling at -20 to -15°C, and the mixture was stirred at room temperature for 5 hours. The mixture was concentrated under reduced pressure, thus obtaining L-valyl-(4R)-N-{(1R)-1-[4-(1-ethyl- 1H-pyrazol-5-yl)phenyl] -2-hydroxyethyl} -4-hydroxy-L-prolinamide n hydrochloride (5.18 g) as a solid.

### Production Example 141

To a mixture of L-valyl-(4R)-4-hydroxy-N-{(1R)-2-hydroxy-1-[4-(4-methyl-1,3-thiazol-5-yl)phenyl]ethyl}-L-prolinamide n hydrochloride (1.71 g), TEA (3.2 mL), THF (20 mL) and MeCN (20 mL) was added a MeCN (5 mL) solution of 2-azido-1,3-dimethylimidazolinium hexafluorophosphate (1.06 g) dropwise slowly over 10 minutes or more under ice-bath cooling, and the mixture was stirred under ice-bath cooling for 5 hours. Water, saturated aqueous sodium chloride solution and ethyl acetate were added, and the aqueous layer and the organic layer were separated. The aqueous layer was extracted with ethyl acetate, and the combined organic layer was dried over anhydrous sodium sulfate. The insoluble materials were removed by filtration, and the filtrate was concentrated under reduced pressure. The residue was purified by silica gel column chromatography (CHCl₃/MeOH), thus obtaining (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-prolinamide (1.07 g) as a solid.

### Production Example 142

Under an argon atmosphere, a MeCN (10 mL) solution of 2-azido-1,3-dimethylimidazolinium hexafluorophosphate (2.7 g) was added to a mixture of L-valyl-(4R)-N-{ (1R)-1-[4-(1-ethyl-1H-pyrazol-5-yl)phenyl] -2-hydroxyethyl} -4-hydroxy-L-prolinamide n hydrochloride (4.27 g), DIPEA (8.54 mL), THF (55 mL) and MeCN (55 mL) portionwise over 20 minutes or more under cooling in an ice/aqueous sodium chloride solution bath (internal temperature of 3°C or lower). The reaction mixture was stirred for 2 hours under cooling in an ice/aqueous sodium chloride solution bath. Water, saturated aqueous sodium chloride solution and ethyl acetate were added, and the aqueous layer and the organic layer were separated. The aqueous layer was extracted with ethyl acetate, and the combined organic layer was dried over sodium sulfate. After separating the drying agent by filtration, the filtrate was concentrated under reduced pressure. The residue was purified by silica gel column chromatography (CHCl₃/MeOH), thus obtaining (4R)-1-[(2S)-2-azido-3-methylbutanoyl]-N-{(1R)-1-[4-(1-ethyl-1H-pyrazol-5-yl)phenyl] -2-hydroxyethyl} -4-hydroxy-L-prolinamide (2.81 g) as a solid.

### Production Example 143

Under an argon atmosphere, to a mixture of tert-butyl (3S)-3-({(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}amino)pyrrolidine-1-carboxylate (220 mg) and DMF (2 mL) was added sodium hydride (55% mineral oil dispersion, 26 mg) under cooling in an ice/MeOH bath, and the mixture was stirred at it was for 30 minutes. Subsequently, ethyl iodide (0.08 mL) was added, and then the mixture was stirred at room temperature for 1.5 hours. Ice water and saturated aqueous ammonium chloride solution were added under ice-bath cooling, and the mixture was extracted twice with ethyl acetate. The combined organic layer was washed with saturated aqueous sodium chloride solution and was dried over anhydrous magnesium sulfate. The drying agent was removed by filtration, and the filtrate was concentrated under reduced pressure. The resulting residue was purified by silica gel column chromatography (hexane/ethyl acetate), thus obtaining tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(ethyl)amino]pyrrolidine-1-carboxylate (137 mg) as a solid.

### Production Example 144

Under an argon atmosphere, to a mixture of tert-butyl N-[(1R)-1-(4-bromophenyl)-2-hydroxyethyl]carbamate (4.43 g), 1-ethyl-5-(4,4,5,5-tetramethyl-1,3,2-dioxaborolan-2-yl)-1H-pyrazole (4.67 g), potassium carbonate (3.87 g), DOX (80 mL) and water (8 mL) was added PdCl₂(dppf)·CH₂Cl₂ (1.14 g), and the mixture was stirred at 100°C for 16 hours. After the mixture was allowed to cool to room temperature, ethyl acetate was added, and the mixture was filtered through celite (registered trademark) pad and was concentrated under reduced pressure. The residue was purified by silica gel column chromatography (basic silica gel, hexane/ethyl acetate), thus obtaining tert-butyl {(1R)-1-[4-(1-ethyl-1H-pyrazol-5-yl)phenyl]-2-hydroxyethyl}carbamate (3.74 g) as a solid.

### Production Example 146

Under nitrogen atmosphere, to a 1,2-dichloroethane (10 mL) solution of trimethyltin(IV) hydroxide (575 mg) was added (4R)-1-[(2S)-2-(4-{4-[({(7M)-4-{[(3S)-1-(tert-butoxycarbonyl)pyrrolidin-3-yl](methyl)amino}-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]phenyl}-1H-1,2,3-triazol-1-yl)-3-methylbutanoyl]-4-hydroxy-L-proline methyl ester (695 mg) at room temperature, and the mixture was stirred at 80°C for 65 hours. After the mixture was allowed to cool to room temperature, ice-cooled 1M hydrochloric acid was added, and the mixture was extracted with CHCl₃/MeOH (9/1) twice. The combined organic layer was washed with 1M hydrochloric acid, was dried over anhydrous sodium sulfate and was filtered. The filtrate was concentrated under reduced pressure, and The residue was purified by silica gel column chromatography (CHCl₃/MeOH), thus obtaining (4R)-1-[(2S)-2-(4-{4-[({(7M)-4-{[(3S)-1-(tert-butoxycarbonyl)pyrrolidin-3-yl](methyl)amino}-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]phenyl}-1H-1,2,3-triazol-1-yl)-3-methylbutanoyl]-4-hydroxy-L-proline (663 mg) as a solid.

### Production Example 149

To a mixture of (2R)-2-amino-2-(4-fluorophenyl)ethan-1-ol (18 mg), (4R)-1-[(2S)-2-(4-{4-[({(7M)-4-{[(3S)-1-(tert-butoxycarbonyl)pyrrolidin-3-yl](methyl)amino}-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]phenyl}-1H-1,2,3-triazol-1-yl)-3-methylbutanoyl]-4-hydroxy-L-proline (90 mg) and DMF (0.25 mL) were added DIPEA (80 µL) and HATU (40 mg) under ice-bath cooling. The mixture was warmed to room temperature and was stirred overnight. Water (5 mL) and saturated aqueous sodium hydrogen carbonate solution (1 mL) were added, and the mixture was stirred for 1 hour. The resulting solid was filtered, thus obtaining tert-butyl (3S)-3-[{(7M)-6-cyclopropyl-7-[6-fluoro-5-methyl-1-(oxan-2-yl)-1H-indazol-4-yl]-8-{[4-(1-{(2S)-1-[(2S,4R)-2-{[(1R)-1-(4-fluorophenyl)-2-hydroxyethyl] carbamoyl } -4-hydroxypyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}(methyl)amino]pyrrolidine-1-carboxylatete (87.5 mg) as a colorless solid.

### Production Example 163

THF (40 mL) was added to (7M)-4-tert-butoxy-6-cyclopropyl-7-[6-fluoro-5-methyl--2-(triphenylmethyl)-2H-indazol-4-yl]-2-[(2S)-2-methoxypropoxy]-8-[(1S)-1-phenylethoxy]quinazoline (2.89 g), 3,4-dihydro-2H-pyran (3.1 mL) and 4-methylbenzene-1-sulfonic acid monohydrate (330 mg) were added at room temperature, and then, the mixture was stirred at 50°C for 6 hours. The reaction mixture was allowed to cool to room temperature and was then concentrated under reduced pressure. Saturated aqueous sodium hydrogen carbonate solution was added, and the mixture was extracted with ethyl acetate twice. The organic layer was dried over anhydrous magnesium sulfate, was filtered and was concentrated. The resulting residue was purified by silica gel column chromatography (hexane/ethyl acetate), thus obtaining (7M)-6-cyclopropyl-7-[6-fluoro-5-methyl-1-(oxan-2-yl)-1H-indazol-4-yl]-2-[(2S)-2-methoxypropoxy]-8-[(1S)-1-phenylethoxy]quinazolin-4-ol (1.63 g) as a foam-like solid.

### Production Example 171

Under an argon gas atmosphere, to a mixture of tert-butyl (3S)-3-(methylamino)pyrrolidine-1-carboxylate (1.52 g), THF (15 mL), DOX (30 mL) and DIPEA (5 mL) was added 7-bromo-2,4-dichloro-8-fluoro-6-iodoquinazoline (3 g) with stirring under cooling in an ice/MeOH bath, and the mixture was stirred at the same temperature for 1.5 hours. Saturated aqueous ammonium chloride solution and ice water were poured into the reaction container, and the mixture was stirred as it was for 1.5 hours. Then. the insoluble materials were filtered while washing with water and was dried under reduced pressure at 40°C for 5 hours, thus obtaining tert-butyl (3S)-3-[(7-bromo-2-chloro-8-fluoro-6-iodoquinazolin-4-yl)(methyl)amino]pyrrolidine-1-carboxylate (3.82 g) as a solid.

### Production Example 172

To a mixture of tert-butyl (3S)-3-[{6-cyclopropyl-2-(ethylsulfanyl)-7-[6-fluoro-5-methyl-2-(triphenylmethyl)-2H-indazol-4-yl]-8-[(1S)-1-phenylethoxy]quinazolin-4-yl}(methyl)amino]pyrrolidine-1-carboxylate (about 2.5:1 diastereomeric mixture derived from axial chirality, 25.5 g) and CH₂Cl₂ (350 mL) was added m-chloroperbenzoic acid (about 30% water content, 14.5 g) with stirring under ice-bath cooling, and under an argon atmosphere, the mixture was stirred at room temperature for 2.5 hours. Ice water/saturated aqueous sodium thiosulfate solution/saturated aqueous sodium hydrogen carbonate solution was poured into the reaction solution, and the mixture was stirred for about 20 minutes at room temperature and was then extracted with CHCl₃. The organic layer was dried over anhydrous magnesium sulfate, and the solvent was concentrated under reduced pressure. The residue was purified by silica gel column chromatography (hexane/ethyl acetate), thus obtaining tert-butyl (3S)-3-[{6-cyclopropyl-2-(ethanesulfonyl)-7-[6-fluoro-5-methyl-2-(triphenylmethyl)-2H-indazol-4-yl]-8-[(1S)-1-phenylethoxy]quinazolin-4-yl}(methyl)amino]pyrrolidine-1-carboxylate (about 4.3:1 diastereomeric mixture derived from axial chirality, 5.37 g) as a solid and tert-butyl (3S)-3-[{6-cyclopropyl-2-(ethanesulfonyl)-7-(6-fluoro-5-methyl-1H-indazol-4-yl)-8-[(1S)-1-phenylethoxy]quinazolin-4-yl}(methyl)amino]pyrrolidine-1-carboxylate (about 2:1 diastereomeric mixture derived from axial chirality, 8.25 g) as a solid.

A mixture of the resulting tert-butyl (3S)-3-[{6-cyclopropyl-2-(ethanesulfonyl)-7-(6-fluoro-5-methyl-1H-indazol-4-yl)-8-[(1S)-1-phenylethoxy]quinazolin-4-yl}(methyl)amino]pyrrolidine-1-carboxylate (about 2:1 diastereomeric mixture derived from axial chirality, 7.9 g) and EtOH (80 mL) was stirred at room temperature for 3 days. The insoluble materials were filtered while washing with a small amount of EtOH and was dried at 40°C under reduced pressure, thus obtaining tert-butyl (3S)-3-[{(7M)-6-cyclopropyl-2-(ethanesulfonyl)-7-(6-fluoro-5-methyl-1H-indazol-4-yl)-8-[(1S)-1-phenylethoxy]quinazolin-4-yl}(methyl)amino]pyrrolidine-1-carboxylate (single diastereomer, 4.42 g) as a solid.

tert-Butyl (3S)-3-[{6-cyclopropyl-2-(ethanesulfonyl)-7-[6-fluoro-5-methyl-2-(triphenylmethyl)-2H-indazol-4-yl]-8-[(1S)-1-phenylethoxy]quinazolin-4-yl}(methyl)amino]pyrrolidine-1-carboxylate (about 4.3:1 diastereomeric mixture derived from axial chirality) obtained in the above reaction was similarly converted to tert-butyl (3S)-3-[{(7M)-6-cyclopropyl-2-(ethanesulfonyl)-7-(6-fluoro-5-methyl-1H-indazol-4-yl)-8-[(1S)-1-phenylethoxy]quinazolin-4-yl}(methyl)amino]pyrrolidine-1-carboxylate (single diastereomer) by the following operation.

To a mixture of tert-butyl (3S)-3-[{6-cyclopropyl-2-(ethanesulfonyl)-7-[6-fluoro-5-methyl-2-(triphenylmethyl)-2H-indazol-4-yl]-8-[(1S)-1-phenylethoxy]quinazolin-4-yl}(methyl)amino]pyrrolidine-1-carboxylate (about 4.3:1 diastereomeric mixture derived from axial chirality, 6.33 g) and THF (70 mL) was added 4-methylbenzene-1-sulfonic acid monohydrate (0.13 g), and the mixture was stirred under an argon atmosphere overnight. Another 4-methylbenzene-1-sulfonic acid monohydrate (0.15 g) was added, and the mixture was stirred at room temperature for 1 day. The reaction solvent was concentrated under reduced pressure, and the residue was purified by silica gel column chromatography (hexane/ethyl acetate), thus obtaining tert-butyl (3S)-3-[{6-cyclopropyl-2-(ethanesulfonyl)-7-(6-fluoro-5-methyl-1H-indazol-4-yl)-8-[(1S)-1-phenylethoxy]quinazolin-4-yl}(methyl)amino]pyrrolidine-1-carboxylate (about 5:1 diastereomeric mixture derived from axial chirality, 3.21 g) as a solid. The resulting unprotected form (about 5:1 diastereomeric mixture derived from axial chirality, 3.2 g) was dissolved in EtOH (30 mL), and the mixture was stirred at room temperature for 1 day. The resulting insoluble materials were filtered while washing with a small amount of EtOH and were dried under reduced pressure at 40°C overnight, thus obtaining tert-butyl (3S)-3-[{(7M)-6-cyclopropyl-2-(ethanesulfonyl)-7-(6-fluoro-5-methyl-1H-indazol-4-yl)-8-[(1S)-1-phenylethoxy]quinazolin-4-yl}(methyl)amino]pyrrolidine-1-carboxylate (single diastereomer, 2.23 g) as a solid.

### Production Example 173

To a mixture of 4-bromo-6-fluoro-1H-indazole (235 g), TEA (183 mL) and CH₂Cl₂ (1880 mL) was added 1,1',1"-(chloromethanetriyl)tribenzene (335 g) at room temperature, and the mixture was stirred at 25°C for 16 hours. The reaction mixture was poured into ice water (1.5 L), and the organic layer and the aqueous layer were separated. The aqueous layer was extracted with CH₂Cl₂ (400 mL) three times. The combined organic layer was dried over anhydrous sodium sulfate. Then, the insoluble materials were removed by filtration, and the filtrate was concentrated under reduced pressure. Petroleum ether (550 mL) was added to the resulting residue for trituration (0°C, 2 hours), and then 4-bromo-6-fluoro-2-(triphenylmethyl)-2H-indazole (508.98 g) was obtained as a solid by collecting by filtration and drying under reduced pressure.

### Production Example 174

To a mixture of 4-bromo-6-fluoro-2-(triphenylmethyl)-2H-indazole (100 g) and 2-methyltetrahydrofuran (1000 mL) was added lithium diisopropylamide (2 M THF solution, 214.28 mL) at -78°C under nitrogen atmosphere, and the mixture was stirred at -78°C for 2.5 hours. Methyl iodide (26.68 mL) was added at -78°C, and the mixture was stirred at 25°C for 2.5 hours. Water (2000 mL) was added to quench the reaction, and the mixture was extracted with ethyl acetate (800 mL) twice. The combined organic layer was dried over anhydrous sodium sulfate. Then, the insoluble materials were removed by filtration, and the filtrate was concentrated under reduced pressure. Ethyl acetate (50 mL)/petroleum ether (50 mL) were added to the resulting residue for trituration, and then 4-bromo-6-fluoro-5-methyl-2-(triphenylmethyl)-2H-indazole (81 g) was obtained as a solid by collecting by filtration and drying under reduced pressure.

### Production Example 175

To a mixture of 4-bromo-6-fluoro-5-methyl-2-(triphenylmethyl)-2H-indazole (100 g), 4,4,4',4',5,5,5',5'-octamethyl-2,2'-bi-1,3,2-dioxaborolane (61.42 g), triphenylphosphine (10.57 g), potassium acetate (59.34 g) and DOX (1000 mL) was added palladium acetate (4.52 g) under nitrogen atmosphere at room temperature. After the reaction mixture was degassed and filled with nitrogen gas three times each, the mixture was stirred at 100°C for 12 hours under nitrogen atmosphere. After cooling, water (1500 mL) was added, and the mixture was extracted with ethyl acetate (900 mL) three times. The combined organic layer was dried over anhydrous sodium sulfate, and then the insoluble materials were removed by filtration. Activated carbon (50 g) was added to the resulting solution, and the solution was stirred at 20°C for 1 hour and filtered while washing with ethyl acetate (50 ml) three times. The filtrate was concentrated. MeOH (200 mL) was added to the resulting residue for trituration, and 6-fluoro-5-methyl-4-(4,4,5,5-tetramethyl-1,3,2-dioxaborolan-2-yl)-2-(triphenylmethyl)-2H-indazole (110 g) was obtained as a solid by collecting by filtration and drying under reduced pressure.

### Production Example 176

Under nitrogen atmosphere, DIPEA (200 µL) was added to a CH₂Cl₂ (2 mL) solution of (4-ethynyl-3-fluorophenyl)methanol (110 mg) under ice-bath cooling, and then methanesulfonyl chloride (80 µL) was added. After the reaction mixture was stirred at room temperature overnight, water was added, and the mixture was extracted with CHCl₃. The organic layer was washed with water and saturated aqueous sodium chloride solution and was dried over anhydrous magnesium sulfate. The insoluble materials were removed by filtration, and the filtrate was concentrated under reduced pressure, thus obtaining 4-(chloromethyl)-1-ethynyl-2-fluorobenzene (122 mg) as an oil.

### Production Example 177

To a mixture of 2-amino-4-bromo-3-fluoro-5-iodobenzoic acid (10 g) and N-methyl-2-pyrrolidone (20 mL) was added trimethyl orthoacetate (10.5 mL), and the mixture was stirred at 110°C for 12 hours. After the mixture was cooled to room temperature, MeOH (20 ml) was added, and the resulting solid was filtered while washing with ice-cooled methanol (about 60 ml) and was dried under reduced pressure, thus obtaining 2-acetamido-4-bromo-3-fluoro-5-iodobenzoic acid methyl ester (6.67 g) as a solid.

### Production Example 178

Under an argon atmosphere, to a mixture of lithium bis(trimethylsilyl)amide (1.3 M THF solution, 100 mL) and THF (150 mL) was added 2-acetamido-4-bromo-3-fluoro-5-iodobenzoic acid methyl ester (18 g) portionwise under cooling in a water bath. The mixture was stirred at 40°C for 1 hour and was then cooled to room temperature, water was added, and the mixture was washed twice with ethyl acetate. Under ice-bath cooling, the aqueous layer was acidified with hydrochloric acid (1M, 140 ml) and ice water and was stirred for a while. The precipitated solid was filtered and was dried under reduced pressure. The resulting solid was filtered while washing with methanol and was dried under reduced pressure, thus obtaining 7-bromo-8-fluoro-4-hydroxy-6-iodoquinoline -2(1H)-one (13.6 g) as a solid.

### Production Example 179

Under an argon atmosphere, phosphoryl chloride (24 mL) was added to 7-bromo-8-fluoro-4-hydroxy-6-iodoquinoline -2(1H)-one (5 g), and under ice-bath cooling DIPEA (7 mL) was slowly added. The mixture was stirred at 110°C for 1 hour. The reaction solution was cooled to room temperature and was dried at room temperature under reduced pressure. Ice water was added to the residue, and the mixture was stirred for 30 minutes. The precipitated solid was filtered, was washed with water and was then dried under reduced pressure, thus obtaining 7-bromo-2,4-dichloro-8-fluoro-6-iodoquinoline (5.03 g).

### Production Example 180

Under an argon atmosphere, to a mixture of 7-bromo-2,4-dichloro-8-fluoro-6-iodoquinoline (6.4 g) and DMAc (70 mL) was added DABCO (1.8 g), and the mixture was stirred at 40°C for 2 hours. Ethanethiol (1.4 mL) was added at 60°C, and the mixture was stirred for 1 hour. After the mixture was cooled to room temperature, water was added, and the mixture was stirred for 5 minutes. The resulting solid was filtered and was dried under reduced pressure. The resulting solid was purified by silica gel column chromatography (hexane/ethyl acetate), thus obtaining 7-bromo-4-chloro-2-(ethylsulfanyl)-8-fluoro-6-iodoquinoline (5.76 g) as a solid.

### Production Example 181

Under an argon atmosphere, a mixture prepared by adding tBuOK (1.39 g) to a dehydrated THF (40 mL) solution of (1S)-1-phenylethan-1-ol (1.56 mL) and stirring at room temperature for 30 minutes was added dropwise to a mixture of 7-bromo-4-chloro-2-(ethylsulfanyl)-8-fluoro-6-iodoquinoline (5.5 g) and dehydrated THF (40 mL) under ice-bath cooling over 20 minutes, and the mixture was stirred at the same temperature for 10 minutes. Separately, under an argon atmosphere, a mixture prepared by adding tBuOK (415 mg) to a dehydrated THF (10 mL) solution of (1S)-1-phenylethan-1-ol (0.45 mL) and stirring at room temperature for 10 minutes was slowly added dropwise to the above reaction mixture under ice-bath cooling, and the mixture was stirred at the same temperature for 10 minutes. Saturated aqueous ammonium chloride solution, ice and ethyl acetate were added to divide the mixture into layers, and the aqueous layer was extracted with ethyl acetate twice. The combined organic layer was washed with saturated aqueous sodium chloride solution and was dried over anhydrous magnesium sulfate. The insoluble materials were removed by filtration, and the filtrate was concentrated under reduced pressure. The residue was purified by silica gel column chromatography (hexane/ethyl acetate), thus obtaining 7-bromo-4-chloro-2-(ethylsulfanyl)-6-iodo-8-[(1S)-1-phenylethoxy]quinoline (4.9 g) as an oil. Slightly impure fractions were concentrated, thus obtaining 7-bromo-4-chloro-2-(ethylsulfanyl)-6-iodo-8-[(1S)-1-phenylethoxy]quinoline (1.56 g) as an oil.

### Production Example 182

Under an argon atmosphere, a mixture prepared by adding tBuOK (1.59 g) to a DMAc (20 mL) solution of tert-butyl (3S)-3-hydroxypyrrolidine-1-carboxylate (2.7 g) and stirring at room temperature for 10 minutes was added dropwise to a mixture of 7-bromo-4-chloro-2-(ethylsulfanyl)-6-iodo-8-[(1S)-1-phenylethoxy]quinoline (6.46 g) and DMAc (40 mL) under ice-bath cooling, and the mixture was stirred at the same temperature for 30 minutes. Saturated aqueous ammonium chloride solution, ice and ethyl acetate were added, and the mixture was stirred to divide the mixture into layers. The aqueous layer was extracted with ethyl acetate twice. The combined organic layer was washed with saturated aqueous sodium chloride solution, was dried over anhydrous magnesium sulfate and was concentrated under reduced pressure. The residue was purified by silica gel column chromatography (hexane/ethyl acetate), thus obtaining tert-butyl (3S)-3-({7-bromo-2-(ethylsulfanyl)-6-iodo-8-[(1S)-1-phenylethoxy]quinolin-4-yl}oxy)pyrrolidine-1-carboxylate (4.6 g) as a solid.

### Production Example 183

To a mixture of tert-butyl N-[(1R)-1-(4-bromophenyl)-2-hydroxyethyl]carbamate (1 g), 2,2-dimethoxypropane (3.3 mL) and acetone (15 mL) was added a boron trifluoride-diethyl ether complex (26 µL), and the mixture was stirred at room temperature for 1 hour. TEA (66 µL) was added, and the mixture was stirred at room temperature for 10 minutes. The mixture was concentrated under reduced pressure, and the residue was purified by silica gel column chromatography (hexane/ethyl acetate), thus obtaining tert-butyl (4R)-4-(4-bromophenyl)-2,2-dimethyl-1,3-oxazolidine-3-carboxylate (1.09 g) as a solid.

### Production Example 184

To a DOX (1.69 mL) solution of tert-butyl (4R)-4-(4-bromophenyl)-2,2-dimethyl-1,3-oxazolidine-3-carboxylate (300 mg) and 1,3-oxazolidin-2-one (183 mg) were added copper(I) iodide (32 mg), racemic-(1R,2R)-cyclohexane-1,2-diamine (20 µL) and potassium carbonate (290 mg) at room temperature. Under microwave irradiation, the mixture was stirred for 2 hours at 140°C and for 1 hour at 150°C. Ethyl acetate and water were added, and the mixture was filtered through celite (registered trademark) pad. The filtrate was concentrated under reduced pressure, and the residue was purified by silica gel column chromatography (hexane/ethyl acetate), thus obtaining tert-butyl (4R)-2,2-dimethyl-4-[4-(2-oxo-1,3-oxazolidin-3-yl)phenyl]-1,3-oxazolidine-3-carboxylate (120 mg) as a solid.

### Production Example 185

In DMSO (10 mL), tert-butyl (4R)-4-(4-bromophenyl)-2,2-dimethyl-1,3-oxazolidine-3-carboxylate (858 mg), 2-methyl-1H-imidazole (500 mg), copper(I) iodide (95 mg), quinolin-8-ol (138 mg) and potassium carbonate (670 mg) were suspended, and under an argon atmosphere, under microwave irradiation, the suspension was reacted at 150°C for 3 hours. After cooling to room temperature, ethyl acetate and water were added, and the mixture was extracted with ethyl acetate twice. The combined organic layer was washed with saturated aqueous sodium chloride solution and was then dried over anhydrous magnesium sulfate. The insoluble materials were removed by filtration, and the filtrate was concentrated under reduced pressure. The resulting residue was purified by silica gel column chromatography (basic silica gel, hexane/ethyl acetate), thus obtaining tert-butyl (4R)-2,2-dimethyl-4-[4-(2-methyl-1H-imidazol-1-yl)phenyl]-1,3-oxazolidine-3-carboxylate (500 mg) as an oil.

### Production Example 187

Under an argon atmosphere, bis(tri-tert-butylphosphine)palladium(0) (18 mg) was added to a mixture of 4-methyl-1,3-oxazole-5-carboxylic acid (178 mg), tetra-n-butylammonium chloride (195 mg), tert-butyl (4R)-4-(4-bromophenyl)-2,2-dimethyl-1,3-oxazolidine-3-carboxylate (250 mg), cesium carbonate (344 mg) and DMF (2.5 mL), and under microwave irradiation, the mixture was stirred at 170°C for 30 minutes. The mixture was cooled to room temperature and was then diluted with ethyl acetate, and the insoluble materials were removed by filtration through celite (registered trademark) pad. The filtrate was washed with water and saturated aqueous sodium chloride solution and was dried over anhydrous magnesium sulfate. The insoluble materials were removed by filtration, and the filtrate was concentrated under reduced pressure. The resulting residue was purified by silica gel column chromatography (CHCl₃/MeOH), thus obtaining tert-butyl (4R)-2,2-dimethyl-4-[4-(4-methyl-1,3-oxazol-5-yl)phenyl]-1,3-oxazolidine-3-carboxylate (215 mg) as a solid.

### Production Example 188

tert-Butyl (3S)-3-{ [(7M)-6-cyclopropyl-8-[(4-ethynylphenyl)methoxy]-7-[6-fluoro-5-methyl-1-(oxan-2-yl)-1H-indazol-4-yl]-2-{ [(2S,3S)-3-hydroxybutan-2-yl]oxy}quinazolin-4-yl](methyl)amino]pyrrolidine-1-carboxylate (60 mg), dehydrated THF (2 mL) and methyl iodide (35 µL) were added, and under an argon gas atmosphere, sodium hydride (55% mineral oil dispersion, 10 mg) was added with stirring under cooling in an ice/MeOH bath under an argon atmosphere, and the mixture was stirred at room temperature overnight. Ice/saturated aqueous ammonium chloride solution were added to the reaction container, and the mixture was extracted with ethyl acetate twice. The combined organic layer was washed with saturated aqueous sodium chloride solution and was dried over anhydrous magnesium sulfate. The solvent was removed under reduced pressure, and the resulting residue was purified by silica gel column chromatography (hexane/ethyl acetate), thus obtaining tert-butyl (3S)-3-{[(7M)-6-cyclopropyl-8-[(4-ethynylphenyl)methoxy]-7-[6-fluoro-5-methyl-1-(oxan-2-yl)-1H-indazol-4-yl]-2-{ [(2S,3S)-3-methoxybutan-2-yl]oxy}quinazolin-4-yl](methyl)amino}pyrrolidine-1-carboxylate (50 mg) as a solid.

### Production Example 192

Under an argon atmosphere, cesium carbonate (2.3 g) was added to a mixture of tetrahydro-2H-pyran-4-ol (272 µL), 7-bromo-2,4-dichloro-8-fluoro-6-iodoquinoline (1 g), DABCO (280 mg) and DMAc (10 mL), and the mixture was stirred at 60°C for 6 hours and at 80°C for 3 hours. After the mixture was allowed to cool to room temperature, ethyl acetate and water were added, and the mixture was stirred at room temperature for 30 minutes. The precipitated solid was filtered and was dried under reduced pressure. The resulting solid was purified by silica gel column chromatography (hexane/ethyl acetate), thus obtaining 7-bromo-4-chloro-8-fluoro-6-iodo-2-[(oxan-4-yl)oxy]quinoline (692 mg) as a solid.

### Production Example 203

Under an argon atmosphere, to a mixture of 7-bromo-2,4-dichloro-8-fluoro-6-iodoquinoline (10 g), DABCO (3.3 g), cesium carbonate (23.3 g) and DMAc(100 mL) was added (2S)-2-methoxypropan-1-ol (3 mL), the mixture was stirred at room temperature for 1.5 hours and then at 60°C for 12 hours. After the mixture was allowed to cool to room temperature, water and ethyl acetate were added. The mixture was stirred for a while, and then, two layers were separated. The aqueous layer was extracted with ethyl acetate. The combined organic layer was washed with saturated aqueous sodium chloride solution and was dried over anhydrous magnesium sulfate. The insoluble materials were removed by filtration, and the filtrate was concentrated. The residue was purified by silica gel column chromatography (hexane/ethyl acetate), thus obtaining 7-bromo-4-chloro-8-fluoro-6-iodo-2-[(2S)-2-methoxypropoxy]quinoline (5.97 g) as a solid.

### Production Example 204

Under an argon atmosphere, to a THF (50 mL) solution of 7-bromo-4-chloro-8-fluoro-6-iodo-2-[(2S)-2-methoxypropoxy]quinoline (6.47 g) and (1S)-1-phenylethan-1-ol (3.34 mL) was added tBuOK (3.06 g) in a cooling bath (about -10°C), and the mixture was stirred at the same temperature for 30 minutes. Saturated aqueous ammonium chloride solution was added under ice-bath cooling, and the mixture was extracted twice with ethyl acetate. The combined organic layer was washed with saturated aqueous sodium chloride solution and was dried over anhydrous magnesium sulfate. The insoluble materials were removed by filtration, and the filtrate was concentrated under reduced pressure. The residue was purified by silica gel column chromatography (hexane/ethyl acetate), thus obtaining 7-bromo-4-chloro-6-iodo-2-[(2S)-2-methoxypropoxy]-8-[(1S)-1-phenylethoxy]quinoline (4.67 g) as an oil.

### Production Example 205

To a DMAc (5 mL) solution of 7-bromo-4-chloro-6-iodo-2-[(2S)-2-methoxypropoxy]-8-[(1S)-1-phenylethoxy]quinoline (1.6 g) were added tert-butyl (1S,4S)-2,5-diazabicyclo[2.2.1]heptane-2-carboxylate (832 mg) and potassium carbonate (768 mg) at room temperature, and the mixture was stirred at 120°C overnight. Another tert-butyl (1S,4S)-2,5-diazabicyclo[2.2.1]heptane-2-carboxylate (278 mg) and potassium carbonate (384 mg) were added at 120°C, and the mixture was stirred at the same temperature overnight. The mixture was cooled to room temperature, saturated aqueous ammonium chloride solution was added, and the mixture was extracted twice with ethyl acetate. The combined organic layer was washed with saturated aqueous sodium chloride solution and was dried over anhydrous magnesium sulfate. The solution was concentrated under reduced pressure, and the residue was purified by silica gel column chromatography (hexane/ethyl acetate), thus obtaining tert-butyl (1S, 4S)-5-{7-bromo-6-iodo-2-[(2S)-2-methoxypropoxy]-8-[(1S)-1-phenylethoxy]quinolin-4-yl}-2,5-diazabicyclo[2.2.1]heptane-2-carboxylate (1.02 g) as a foam-like solid.

### Production Example 206

A mixture of 7-bromo-4-chloro-6-iodo-2-[(2S)-2-methoxypropoxy]-8-[(1S)-1-phenylethoxy]quinoline (377 mg), tert-butyl (3S)-3-aminopyrrolidine-1-carboxylate (250 mg), potassium carbonate (130 mg) and DMAc (5 mL) was stirred at 130°C for 7 days under an argon atmosphere. The mixture was cooled to room temperature, water was added, and the mixture was extracted twice with ethyl acetate. The combined organic layer was washed with saturated aqueous sodium chloride solution, was dried over anhydrous magnesium sulfate and was concentrated under reduced pressure. The residue was purified by silica gel column chromatography (hexane/ethyl acetate), thus obtaining tert-butyl (3S)-3-({7-bromo-6-iodo-2-[(2S)-2-methoxypropoxy]-8-[(1S)-1-phenylethoxy]quinolin-4-yl}amino)pyrrolidine-1-carboxylate (259 mg) as a foam-like solid.

### Production Example 207

tert-Butyl (3S)-3-({7-bromo-6-iodo-2-[(2S)-2-methoxypropoxy]-8-[(1S)-1-phenylethoxy]quinolin-4-yl}amino)pyrrolidine-1-carboxylate (285 mg) was dissolved in DMF (3 mL), sodium hydride (60% mineral oil dispersion, 19 mg) was added under ice-bath cooling, and the mixture was stirred at room temperature for 1 hour. Methyl iodide (28 µL) was added at room temperature, and the mixture was stirred at the same temperature for 1 hour. Ice and saturated aqueous ammonium chloride solution were added under ice-bath cooling, and the mixture was extracted twice with ethyl acetate. The combined organic layer was washed with saturated aqueous sodium chloride solution, was dried over anhydrous magnesium sulfate and was concentrated under reduced pressure. The residue was purified by silica gel column chromatography (hexane/ethyl acetate), thus obtaining tert-butyl (3S)-3[{7-bromo-6-iodo-2- [(2S)-2-methoxypropoxy]-8-[(1S)-1-phenylethoxy]quinolin-4-yl}(methyl)amino]pyrrolidine-1-carboxylate (238 mg) as a foam-like solid.

### Production Example 210

Under an argon atmosphere, a mixture of tert-butyl (1S,4S)-5-{7-bromo-6-cyclopropyl-2-[(2S)-2-methoxypropoxy]-8-[(1S)-1-phenylethoxy]quinolin-4-yl}-2,5-diazabicyclo[2.2.1]heptane-2-carboxylate (0.75 g), 6-fluoro-5-methyl-4-(4,4,5,5-tetramethyl-1,3,2-dioxaborolan-2-yl)-2-(triphenylmethyl)-2H-indazole (900 mg), dicyclohexyl(2',6'-diisopropoxy-1,1'-biphenyl]-2-yl)phosphine (138 mg), palladium(II) acetate(31 mg), anhydrous barium hydroxide (0.61 g), DOX (30 mL) and water (6 mL) was stirred at 50°C for 30 minutes. 6-fluoro-5-methyl-4-(4,4,5,5-tetramethyl-1,3,2-dioxaborolan-2-yl)-2-(triphenylmethyl)-2H-indazole (600 mg) was added at the same temperature, and the mixture was further stirred at 50°C for 1 minutes. After the mixture was cooled to room temperature, ethyl acetate and water were added, and the insoluble materials were removed by filtration through celite (registered trademark) pad. The two layers of filtrate were separated, and the aqueous layer was extracted with ethyl acetate. The combined organic layer was washed with saturated aqueous sodium chloride solution and was dried over anhydrous magnesium sulfate. The insoluble materials were removed by filtration, and then the filtrate was concentrated under reduced pressure. The residue was purified by silica gel column chromatography (hexane/ethyl acetate), thus obtaining tert-butyl (1S,4S)-5-{6-cyclopropyl-7-[6-fluoro-5-methyl-2-(triphenylmethyl)-2H-indazol-4-yl]-2-[(2S)-2-methoxypropoxy]-8-[(1S)-1-phenylethoxy]quinolin-4-yl}-2,5-diazabicyclo[2.2.1]heptane-2-carboxylate (about 4:1 diastereomeric mixture derived from axial chirality, 866 mg) as a solid. MeOH (5 mL) was added to the resulting solid (866 mg). The mixture was heated to 60°C to dissolve the solid and was stirred at room temperature for 3 days under an argon atmosphere. The precipitated solid was filtered while washing with a small amount of MeOH to give tert-butyl (1S, 4S)-5-{6-cyclopropyl-7-[6-fluoro-5-methyl-2-(triphenylmethyl)-2H-indazol-4-yl]-2-[(2S)-2-methoxypropoxy]-8-[(1S)-1-phenylethoxy]quinolin-4-yl}-2,5-diazabicyclo[2.2.1]heptane-2-carboxylate (almost single diastereomer with unknown axial chirality configuration, 200 mg) as a solid.

### Production Example 218

To a mixture of methyl 3-cyclopropyl-3-oxopropanoate (17 mL) and trimethyl orthoformate (20 mL) was added concentrated sulfuric acid (500 µL) under ice-bath cooling, and under an argon atmosphere, the mixture was stirred overnight at room temperature. The reaction solution was concentrated and dried at room temperature under reduced pressure for 4 hours. 5-aminopyrimidine-2,4(1H,3H)-dione (11.4 g) and DMAc (300 mL) were added to the residue at room temperature, and under an argon atmosphere, the mixture was stirred at 110°C overnight. The reaction solution was concentrated under reduced pressure, and water was added to the residue. The resulting solid was filtered and was dried at 50°C for 1 hour under reduced pressure, thus obtaining methyl 3-cyclopropyl-3-[(2,4-dioxo-1,2,3,4-tetrahydropyrimidin-5-yl)amino]prop-2-enoate (22.3 g) as a solid.

### Production Example 219

Methyl 3-cyclopropyl-3-[(2,4-dioxo-1,2,3,4-tetrahydropyrimidin-5-yl)amino]prop-2-enoate (870 mg) was dissolved in NMP (17 mL) at room temperature and stirred at 220°C for 5 hours under an argon atmosphere. The reaction solution was concentrated under reduced pressure, and water was added to the residue. The resulting solid was filtered and was dried at 50°C for 1 hour under reduced pressure, thus obtaining 6-cyclopropylpyrido[3,2-d]pyrimidine-2,4,8-triol (230 mg) as a solid.

### Production Example 220

To a DMF (90 mL) solution of 6-cyclopropylpyrido[3,2-d]pyrimidine-2,4,8-triol (5.71 g) was added N-bromosuccinimide (9.3 g) under ice-bath cooling, and the mixture was stirred at room temperature overnight. Water was added under ice-bath cooling, the resulting solid was filtered, and was dried at 50°C for 3 hours under reduced pressure, thus obtaining 7-bromo-6-cyclopropylpyrido[3,2-d]pyrimidine-2,4,8-triol (2.12 g) as a solid.

### Production Example 221

To a mixture of 7-bromo-6-cyclopropylpyrido[3,2-d]pyrimidine-2,4,8-triol (2.11 g) and N,N-diethylaniline (3.4 mL) was added phosphorus oxychloride (38.2 g) at room temperature, and under an argon atmosphere, the mixture was stirred at 100°C overnight. The reaction solution was concentrated under reduced pressure, MeCN (35 mL) and DIPEA (6 mL) were added to the residue under ice-bath cooling, and the mixture was stirred for 1 minute. Then, tert-butyl(1S,4S)-2,5-diazabicyclo[2.2.1]heptane-2-carboxylate (1.96 g) was added under ice-bath cooling, and the mixture was stirred at room temperature for 1 hour. The reaction solution was concentrated under reduced pressure, and the resulting residue was purified by silica gel column chromatography (hexane/ethyl acetate), thus obtaining tert-butyl (1S,4S)-5-(7-bromo-2.8-dichloro-6-cyclopropylpyrido[3,2-d]pyrimidin-4-yl)-2,5-diazabicyclo[2.2.1]heptane-2-carboxylate (1.29 g) as a solid.

### Production Example 222

tert-Butyl (1S,4S)-5-(7-bromo-2,8-dichloro-6-cyclopropylpyrido[3,2-d]pyrimidin-4-yl)-2,5-diazabicyclo[2.2.1]heptane-2-carboxylate (2.4 g), DMF (20 mL), THF (20 mL) and tetrahydro-2H-pyran-4-ol (2.2 mL) were mixed at room temperature, and cesium carbonate (7.6 g) and DABCO (100 mg) were added under ice-bath cooling. Under an argon atmosphere, the mixture was stirred at 50°C overnight, and then, the reaction was quenched with saturated aqueous ammonium chloride solution under ice-bath cooling. Water and ethyl acetate were added, and the organic layer and the aqueous layer were separated. The aqueous layer was extracted with ethyl acetate three times. The combined organic layer was dried over anhydrous sodium sulfate, was filtered and was concentrated under reduced pressure. The resulting residue was purified by silica gel column chromatography (hexane/ethyl acetate), thus obtaining tert-butyl (1S,4S)-5-{7-bromo-8-chloro-6-cyclopropyl-2-[(oxan-4-yl)oxy]pyrido[3,2-d]pyrimidin-4-yl}-2,5-diazabicyclo[2.2.1]heptane-2-carboxylate (1.23 g) as a solid.

### Production Example 225

To a THF (10 mL) solution of tert-butyl (1S,4S)-5-{6-cyclopropyl-7-[6-fluoro-5-methyl-2-(triphenylmethyl)-2H-indazol-4-yl]-2-[(oxan-4-yl)oxy]-8-[(1S)-1-phenylethoxy]pyrido[3,2-d]pyrimidin-4-yl}-2,5-diazabicyclo[2.2.1]heptane-2-carboxylate (470 mg) were added 4-methylbenzene-1-sulfonic acid monohydrate (60 mg) and 3,4-dihydro-2H-pyran (1.1 mL) under ice-bath cooling, and under an argon atmosphere, the mixture was stirred at room temperature overnight. TEA (0.4 mL) was added under ice-bath cooling, and then the reaction solution was concentrated under reduced pressure. The residue was purified by silica gel column chromatography (basic silica gel, hexane/ethyl acetate), thus obtaining tert-butyl (1S,4S)-5-{6-cyclopropyl-7-[6-fluoro-5-methyl-1-(oxan-2-yl)-1H-indazol-4-yl]-2-[(oxan-4-yl)oxy]-8-[(1S)-1-phenylethoxy]pyrido[3,2-d]pyrimidin-4-yl}-2,5-diazabicyclo[2.2.1]heptane-2-carboxylate (291 mg) as a solid.

In the same manner as in the production methods of the Production Examples described above, the compounds shown in Tables presented later were produced. In addition, the production method, the structure and the physiochemical data of the compound of each Production Example are shown in Tables presented later.

### Example 2

Phosphoric acid (85%, 500 µL) was added to a mixture of tert-butyl 3-({(7M)-6-cyclopropyl-7-[6-fluoro-5-methyl-1-(oxan-2-yl)-1H-indazol-4-yl]-8-{ [4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl] -3-methyl-1-oxobutan-2-yl} -1H-1,2,3-triazol-4-yl)phenyl]methoxy}-2-[(oxan-4-yl)oxy]quinazolin-4-yl}(methyl)oxy)azetidine-1-carboxylate (355 mg) and CH₂Cl₂ (10 mL), and the mixture was stirred at room temperature overnight. Saturated aqueous sodium hydrogen carbonate solution was added to the mixture, and then, water and CHCl₃/MeOH (5/1) were added. The mixture was stirred at room temperature for 30 minutes and was extracted with CHCl₃/MeOH (5/1). The combined organic layer was dried over anhydrous sodium sulfate, was filtered and was concentrated under reduced pressure, thus obtaining (4R)-1-[(2S)-2-(4-{4-[({(7M)-4-[(azetidin-3-yl)oxy]-6-cyclopropyl-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N- { (1R)-2-hydroxy-1-[4-(4-methyl-1,3-thiazol-5-yl)phenyl]ethyl}-L-prolinamide (355 mg) as a solid.

### Example 9

Under nitrogen atmosphere, to a CH₂Cl₂ (2 mL) solution of tert-butyl (3S)-3-{[(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2--{[(2R)-oxolan-2-yl]methoxy}quinazolin-4-yl}(methyl)amino]pyrrolidine-1-carboxylate (156 mg) was added trifluoroacetic acid (0.5 mL) under ice-bath cooling, and the mixture was stirred at room temperature for 2 hours. The reaction mixture was concentrated under reduced pressure, and MeCN, saturated aqueous sodium hydrogen carbonate solution and water were added to the residue. The mixture was stirred at room temperature for 20 minutes. The reaction mixture was purified by ODS column chromatography (MeCN/0.1% aqueous formic acid solution), and fractions containing target substance were concentrated. The residue was dissolved in CHCl₃/iPrOH (9/1), saturated aqueous sodium hydrogen carbonate solution was added, and the mixture was extracted twice with CHCl₃/MeOH (5/1). The combined organic layer is dried over anhydrous sodium sulfate and was concentrated under reduced pressure, thus obtaining (4R)-1-[(2S)-2-{4-[4-({[(7M) 6-cyclopropyl-7-(6-fluoro-5-methyl-1H-indazol-4-yl)-4-{ methyl[(3S)-pyrrolidin-3-yl] amino }-2- { [(2R)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide (47 mg) as a foam-like solid.

### Example 10

Under nitrogen atmosphere, to a CH₂Cl₂ (2 mL) solution of tert-butyl (3S)-3-{ [(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl] methoxy} -2-- { [(2S)-oxolan-2-yl] methoxy} quinazolin-4-yl}(methyl)amino]pyrrolidine-1-carboxylate (236 mg) was added trifluoroacetic acid (0.5 mL) under ice-bath cooling, and the mixture was stirred at room temperature for 2 hours. The reaction mixture was concentrated under reduced pressure, and MeCN, saturated aqueous sodium hydrogen carbonate solution and water were added to the residue. The mixture was stirred at room temperature for 20 minutes. The reaction mixture was purified by ODS column chromatography (MeCN/0.1% aqueous formic acid solution), and fractions containing target substance were concentrated. The residue was dissolved in CHCl₃/iPrOH (9/1), saturated aqueous sodium hydrogen carbonate solution was added, and the mixture was extracted twice with CHCl₃/MeOH (5/1). The combined organic layer is dried over anhydrous sodium sulfate and was concentrated under reduced pressure, thus obtaining (4R)-1-[(2S)-2-{4-[4-({[(7M) 6-cyclopropyl-7-(6-fluoro-5-methyl-1H-indazol-4-yl)-4-{ methyl[(3S)-pyrrolidin-3-yl] amino }-2- { [(2S)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide (103 mg) as a foam-like solid.

### Example 11

Under nitrogen atmosphere, to a CH₂Cl₂ (2 mL) solution of tert-butyl (3S)-3-[{(7M)-6-cyclopropyl-7-[6-fluoro-5-methyl-1-(oxan-2-yl)-1H-indazol-4-yl]-8-{ [4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl]-3-methyl-1-oxobutan-2-yl} -1H-1,2,3-triazol-4-yl)phenyl]methoxy}-2-[(2S)-2-methoxypropoxy}quinazolin-4-yl}(methyl)amino]pyrrolidine-1-carboxylate (197 mg) was added trifluoroacetic acid (0.5 mL) under ice-bath cooling, and the mixture was stirred at room temperature for 2 hours. The reaction mixture was concentrated under reduced pressure, and MeCN, saturated aqueous sodium hydrogen carbonate solution and water were added to the residue. The mixture was stirred at room temperature for 20 minutes. The reaction mixture was purified by ODS column chromatography (MeCN/0.1% aqueous formic acid solution), and fractions containing target substance were concentrated. The residue was dissolved in CHCl₃/iPrOH (9/1), saturated aqueous sodium hydrogen carbonate solution was added, and the mixture was extracted twice with CHCl₃/MeOH (5/1). The combined organic layer is dried over anhydrous sodium sulfate and was concentrated under reduced pressure, thus obtaining (4R)-1-[(2S)-2-{4-[4-({ [(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino} quinazolin-8-yl]oxy}methyl)phenyl]-1H-1,2,3-triazol-1-yl}-3-methylbutanoyl]-4-hydroxy-N-{ (1R)-2-hydroxy-1-[4-(4-methyl-1,3-thiazol-5-yl)phenyl] ethyl }-L-prolinamide (65 mg) as a foam-like solid.

### Example 13

TFA (1 mL) was added to a CH₂Cl₂ (1 mL) solution of tert-butyl (3S)-3-[{(7M)-6-cyclopropyl-8-{[4-(1-{(2S)-1-[(2S,4R)-2-({(1R)-1-[4-(1-ethyl-1H-pyrazol-5-yl)phenyl]-2-hydroxyethyl}carbamoyl)-4-hydroxypyrrolidin-1-yl]-3-methyl-1-oxobutan-2-yl}-1H-1,2,3-triazol-4-yl)phenyl]methoxy}-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylatet (224 mg) at room temperature. The mixture was stirred at room temperature for 2 hours. The reaction solvent was concentrated under reduced pressure, and MeCN, saturated aqueous sodium hydrogen carbonate solution and water were added to the residue. The mixture was stirred at room temperature for 20 minutes.

The resulting suspension was purified by ODS column chromatography (MeCN/0.1% aqueous formic acid solution), and desired fractions were concentrated under reduced pressure. The residue was dissolved in CHCl₃/iPrOH (9/1), saturated aqueous sodium hydrogen carbonate solution was added, and the mixture was extracted twice with CHCl₃/MeOH (5/1). The combined organic layer is dried over anhydrous sodium sulfate and was concentrated under reduced pressure, thus obtaining (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino} quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-5-thiazol-5-yl)phenyl]-2-hydroxyethyl}-4-hydroxy-L-prolinamide (109 mg) as a foam-like solid.

### Example 44

Under nitrogen atmosphere, to a CH₂Cl₂ (2 mL) solution of tert-butyl (3S)-3-[{(7M)-6-cyclopropyl-8-[6-fluoro-5-methyl-1-(oxan-2-yl)-1H-indazol-4-yl]-8-{[3-fluoro-4-(1-{(2S)-1-[(2S,4R)-4-hydroxy-2--({(1R)-2-hydroxy-1-[4-(4-methyl-1,3-thiazol-5-yl)phenyl] ethyl }carbamoyl)pyrrolidin-1 -yl] -3-methyl-1 -oxobutan-2-yl} -1H-1,2,3-triazol-4-yl)phenyl]methoxy}-7-[6-fluoro-5-methyl-1-(oxan-2-yl)-1H-indazol-4-yl]-2-[(2S)-2-methoxypropoxy}quinazolin-4-yl}(methyl)amino]pyrrolidine-1-carboxylate (169 mg) was added trifluoroacetic acid (1 mL) under ice-bath cooling, and the mixture was stirred at room temperature for 2 hours. Saturated aqueous sodium hydrogen carbonate solution was added to the reaction mixture, and the mixture was extracted twice with CHCl₃/MeOH (9/1). The combined organic layer was dried over anhydrous sodium sulfate and was concentrated under reduced pressure. The resulting residue was purified by ODS column chromatography (MeCN/0.1% aqueous formic acid solution). Saturated aqueous sodium hydrogen carbonate solution was added to fractions containing the target compound, and the mixture was extracted twice with CHCl₃/MeOH (9/1). The combined organic layer is dried over anhydrous sodium sulfate and was concentrated under reduced pressure, thus obtaining (4R)-1-[(2S)-2-{4-[4-({ [(7M)-6-cyclopropyl-7-(6-fluoro-5-methyl-1H-indazol-4-yl)-2-[(2S)-2-methoxypropoxy] -4- {methyl[(3S)-pyrrolidin-3-yl] amino} quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide (92 mg) as a solid.

### Example 50

To mixture of tert-butyl (3S)-3-[{(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropoxy}quinazolin-4-yl}(ethyl)amino]pyrrolidine-1-carboxylate (175 mg) and of CH₂Cl₂ (3 mL) was added trifluoroacetic acid (1 mL) under cooling in an ice/MeOH bath, and under an argon atmosphere, the mixture was stirred at room temperature for 2 hours. The resulting reaction mixture was concentrated under reduced pressure, and ice water, saturated aqueous sodium hydrogen carbonate solution and CHCl₃/MeOH (10/1) were added to the residue. The mixture was stirred for 20 minutes and was then divided into layers. The aqueous layer was again extracted with CHCl₃/MeOH (10/1), and the combined organic layer was dried over anhydrous magnesium sulfate. The solution was concentrated under reduced pressure, and the resulting residue was purified by silica gel column chromatography (basic silica gel, CHCl₃/MeOH), thus obtaining (4R)-1-[(2S)-2-(4-{4-[({(7M)-6-cyclopropyl-4-{ethyl[(3S)-pyrrolidin-3-yl]amino}-7-(6-fluoro-5-methyl-1H-indazol-4-yl)-2-[(2S)-2-methoxypropoxy]quinazolin-8-yl}oxy)methyl)phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl] ethyl }-L-prolinamide (101 mg) as a solid.

### Example 53

Under nitrogen atmosphere, to a CH₂Cl₂ (3 mL) solution of tert-butyl (3R)-3-[{(7M)-6-cyclopropyl-7-[6-fluoro-5-methyl-1-(oxan-2-yl)-1H-indazol-4-yl]-8-{ [4-(1-{(2S)-1-[(2S,4R)-4-hydroxy-2-({(1R)-2-hydroxy-1-[4-(4-methyl-1,3-thiazol-5-yl)phenyl] ethyl }carbamoyl)pyrrolidin-1-yl] -3-methyl-1-oxobutan-2-yl} -1H-1,2,3-triazol-4-yl)phenyl]methoxy}-2-[(2S)-2-methoxypropoxy}quinazolin-4-yl}(methyl)amino]pyrrolidine-1-carboxylate (310 mg) was added trifluoroacetic acid (1.5 mL) under ice-bath cooling, and then, the mixture was stirred at room temperature for 2 hours. Saturated aqueous sodium hydrogen carbonate solution was added to the mixture, and the mixture was extracted twice with CHCl₃/MeOH (9/1). The combined organic layer was dried over anhydrous sodium sulfate, and the solution was concentrated under reduced pressure. The resulting residue was purified by ODS column chromatography (MeCN/0.1% aqueous formic acid solution). Fractions containing the target compound were combined, saturated aqueous sodium hydrogen carbonate solution was added, and the mixture was extracted twice with CHCl₃/MeOH (9/1). The combined organic layer was dried over anhydrous sodium sulfate and was concentrated under reduced pressure. The residue was dissolved in EtOH, and then the mixture was concentrated under reduced pressure, thus obtaining (4R)-1-[(2S)-2-{4-[4-({ [(7M)-6-cyclopropyl-7-(6-fluoro-5-methyl-1H-indazol-4-yl)-2-[(2S)-2-methoxypropoxy]-4-{methyl[(3R)-pyrrolidin-3-yl]amino} quinazolin-8-yl]oxy}methyl)phenyl]-1H-1,2,3-triazol-1-yl}-3-methylbutanoyl]-4-hydroxy-N-{ (1R)-2-hydroxy-1-[4-(4-methyl-1,3-thiazol-5-yl)phenyl]ethyl}-L-prolinamide (117 mg) as a solid.

### Example 57

To an iPrOH (5 mL) solution of tert-butyl (1S,4S)-5-{6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(oxan-4-yl)oxy]pyrido[3,2-d]pyrimidin-4-yl}-2,5-diazabicyclo[2.2.1]heptane-2-carboxylate (axially chiral mixture, 120 mg) was added methanesulfonic acid (0.3 mL) under ice-bath cooling, and the mixture was stirred at 50°C for 3 hours. TEA (2 mL) was added under ice-bath cooling, and then the reaction solution was concentrated under reduced pressure. The residue was purified by ODS column chromatography (0.1% formic acid in water/0.1% formic acid in MeCN), fractions on the lower polar side of the two peaks having the same molecular weight as with the target substance were collected, and CHCl₃/iPrOH (3/1) and saturated aqueous sodium hydrogen carbonate solution was added to the fractions at room temperature. The organic layer and the aqueous layer were separated, and the aqueous layer was extracted three times with CHCl₃/iPrOH (3/1). The combined organic layer was dried over anhydrous sodium sulfate, was filtered and was concentrated under reduced pressure. The residue was dissolved in a small amount of ethyl acetate/iPrOH (10/1), and hexane was added. The precipitated solid was filtered, was washed with water and was then dried under reduced pressure at 50°C overnight, thus obtaining (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]pyrido[3,2-d]pyrimidin-8-yl}oxy)methyl]phenyl}-1H-1,2,3-triazol-1-yl)-3-methylbutanoyl] -4-hydroxy-N- {(lR)-2-hydroxy-1 -[4-(4-methyl-1,3-thiazol-5-yl)phenyl]ethyl}-L-prolinamide (single axial chirality, 40.4 mg) as a solid.

In the same manner as in the production methods of the Examples described above, the Example compounds shown in Tables presented later were produced. In addition, the production method and the physiochemical data of the compound of each Example are shown in Tables presented later.

In the tables presented below, the following abbreviations are sometimes used.

PEx: Production Example No., Ex: Example No., PSyn: Production Example No. produced in the same method, Syn: Example No. produced in the same method (for example, Syn: 9 represents that it was produced by the same method as for Example 9), Str: chemical structural formula (a compound with "*" in the chemical structural formula represents that the compound is single structure with regard to the axial chirality or central chirality). n HCl: n hydrochloride (the compound with Production Example No. shows that the compound is monohydrochloride to trihydrochloride), DAT: physiochemical data, ESI+: m/z value in mass spectrometry (ionization method ESI, [M+H]⁺ unless otherwise specified), ESI-: m/z value in mass spectrometry (ionization method ESI, [M-H]⁻ unless otherwise specified), NMR: δ value (ppm) of peak in ¹H-NMR (500 MHz) in DMSO-d₆ at 27°C, NMR (100°C): δ value (ppm) of peak in ¹H-NMR (500 MHz) in DMSO-d₆ at 100°C, s: singlet (spectrum), d: doublet (spectrum), dd: double doublet (spectrum), ddd: double double doublet (spectrum), t: triplet (spectrum), dt: double triplet (spectrum), q: quartet (spectrum), m: multiplet (spectrum), br: broad (spectrum) (example: br s).

**[Table 5-1]**

| PEx | Str |
|---|---|
| 1 | 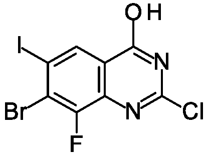 |
| 2 | 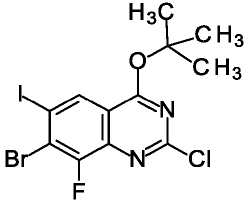 |
| 3 | 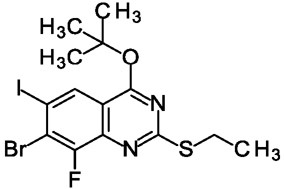 |
| 4 | 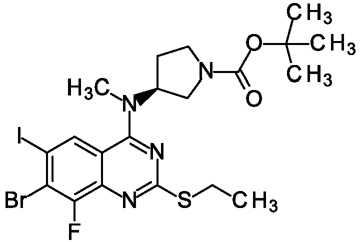 |
| 5 | 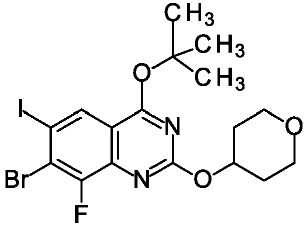 |

**[Table 5-2]**

| PEx | Str |
|---|---|
| 6 | 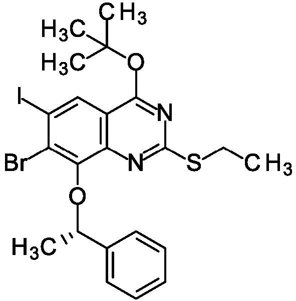 |
| 7 | 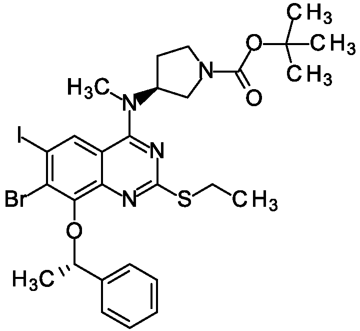 |
| 8 | 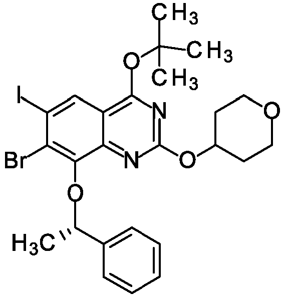 |
| 9 | 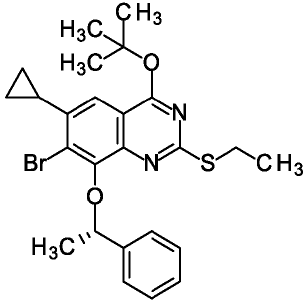 |

**[Table 5-3]**

| PEx | Str |
|---|---|
| 10 | 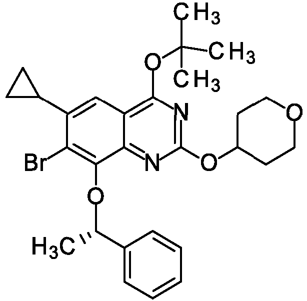 |
| 11 | 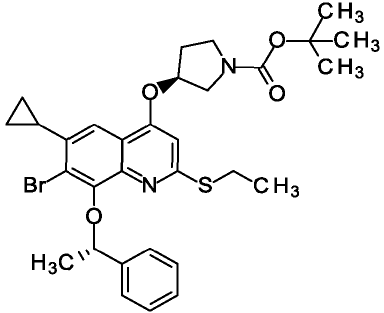 |
| 12 | 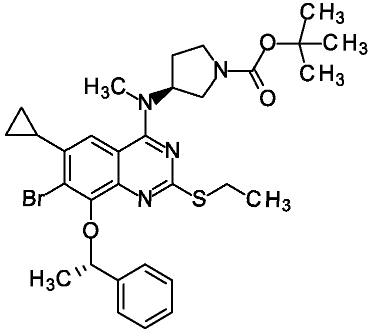 |

**[Table 5-4]**

| PEx | Str |
|---|---|
| 13 | 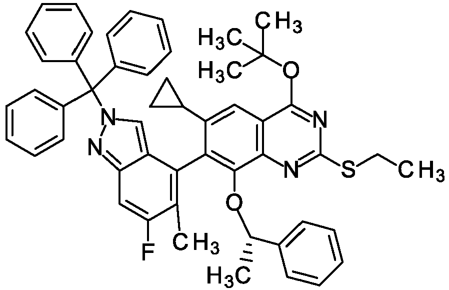 |
| 14 | 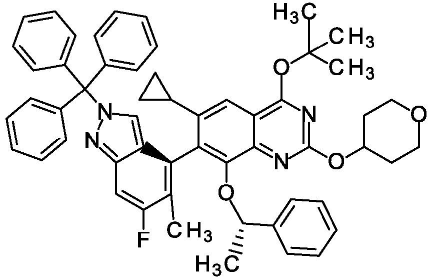 |
| 15 | 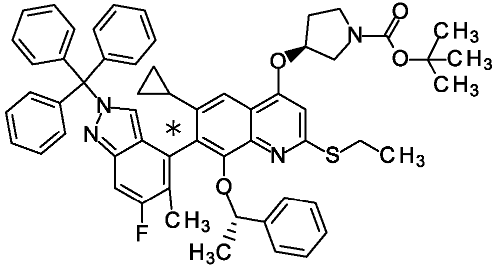 |
| 16 | 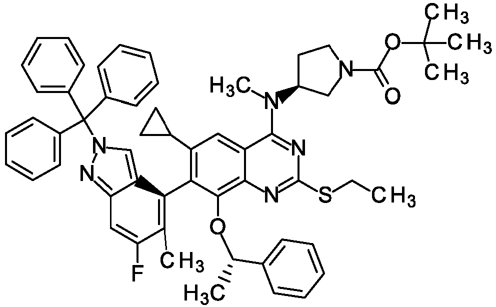 |

**[Table 5-5]**

| PEx | Str |
|---|---|
| 17 | 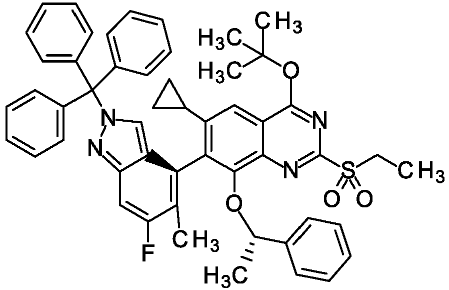 |
| 18 | 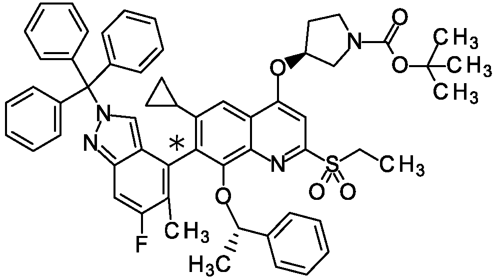 |
| 19 | 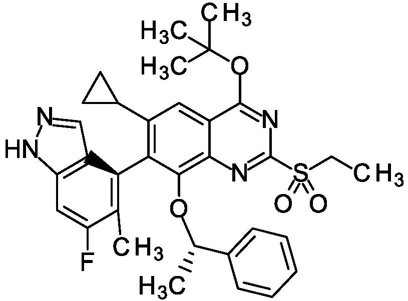 |
| 20 | 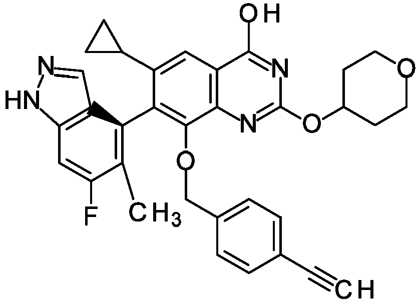 |

**[Table 5-6]**

| PEx | Str |
|---|---|
| 21 | 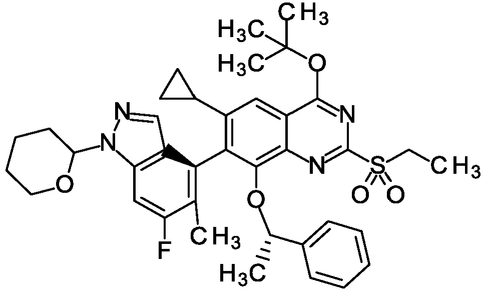 |
| 22 | 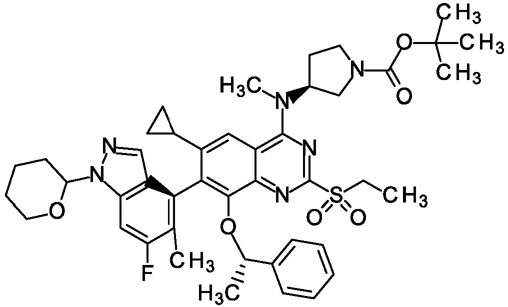 |
| 23 | 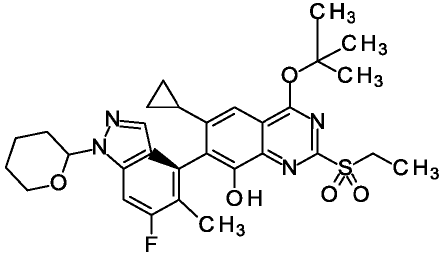 |
| 24 | 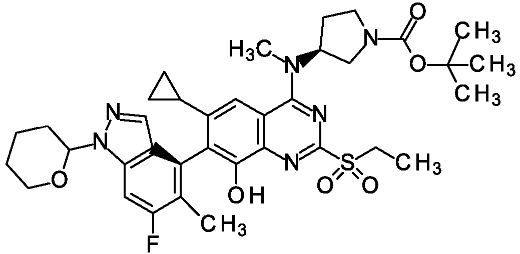 |

**[Table 5-7]**

| PEx | Str |
|---|---|
| 25 | 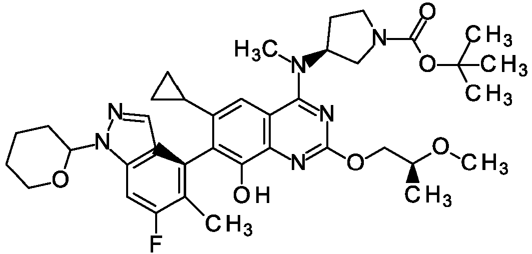 |
| 26 | 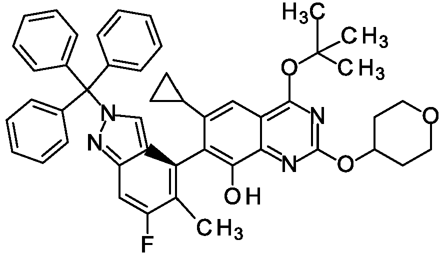 |
| 27 | 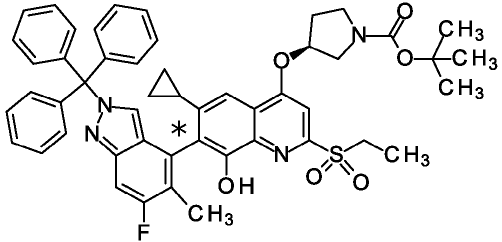 |
| 28 | 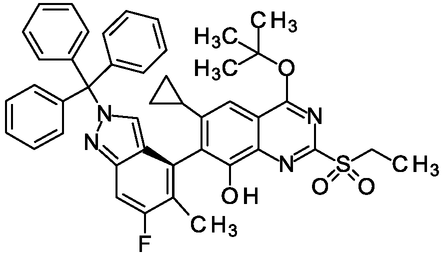 |

**[Table 5-8]**

| PEx | Str |
|---|---|
| 29 | 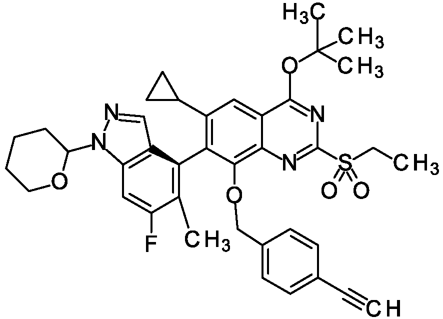 |
| 30 | 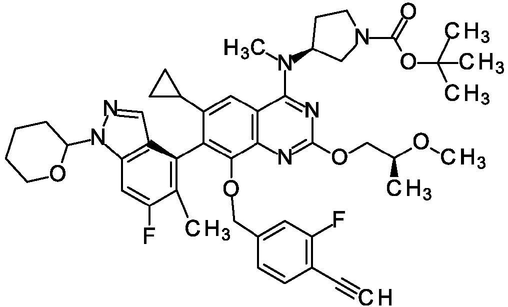 |
| 31 | 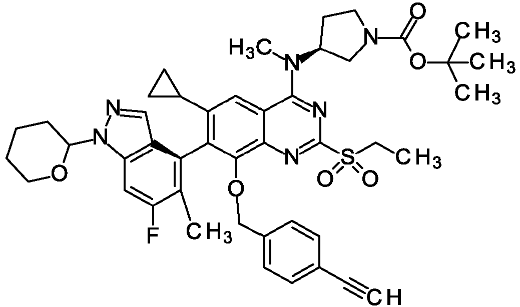 |

**[Table 5-9]**

| PEx | Str |
|---|---|
| 32 | 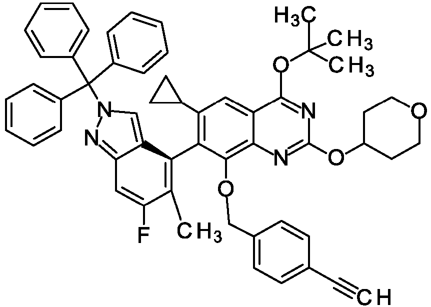 |
| 33 | 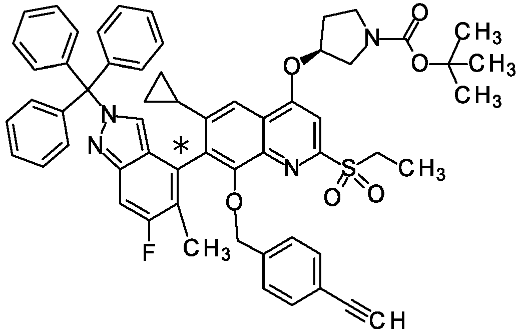 |
| 34 | 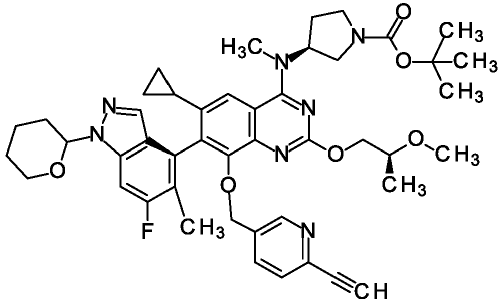 |

**[Table 5-10]**

| PEx | Str |
|---|---|
| 35 | 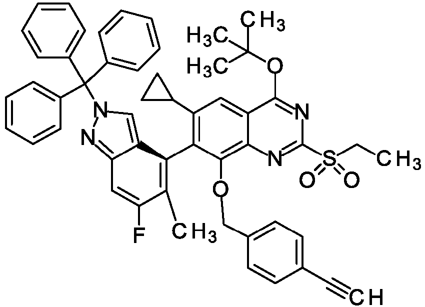 |
| 36 | 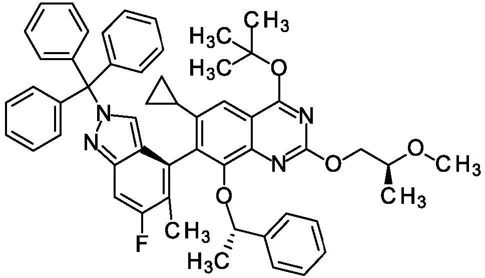 |
| 37 | 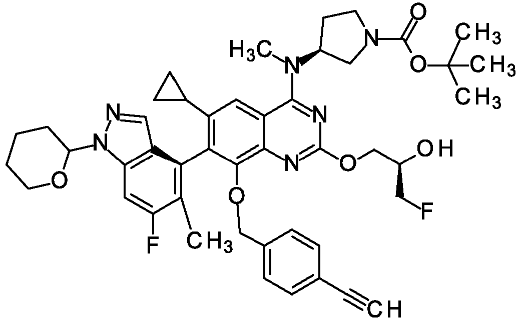 |

**[Table 5-11]**

| PEx | Str |
|---|---|
| 38 | 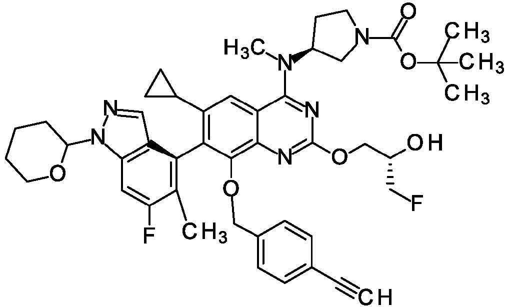 |
| 39 | 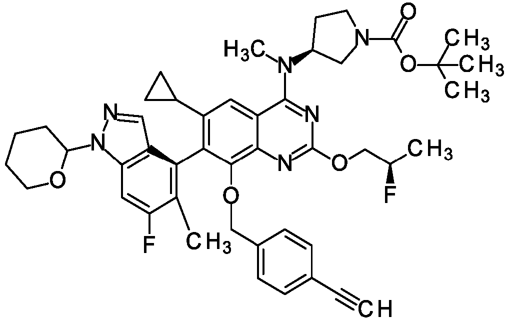 |
| 40 | 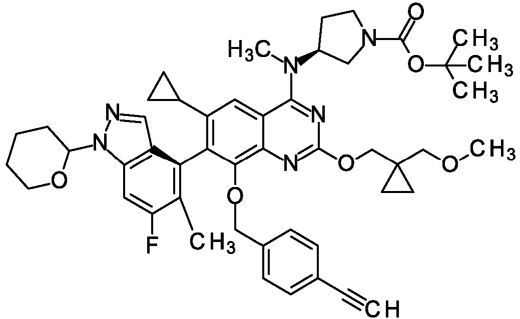 |

**[Table 5-12]**

| PEx | Str |
|---|---|
| 41 | 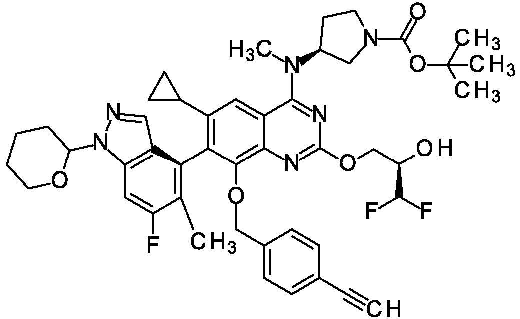 |
| 42 | 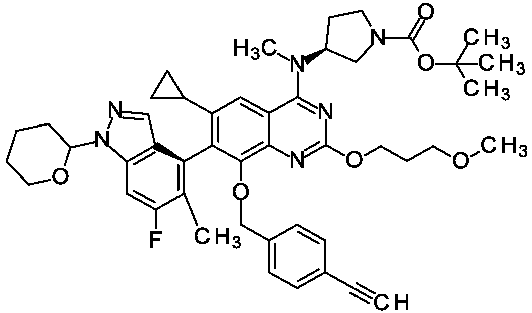 |
| 43 | 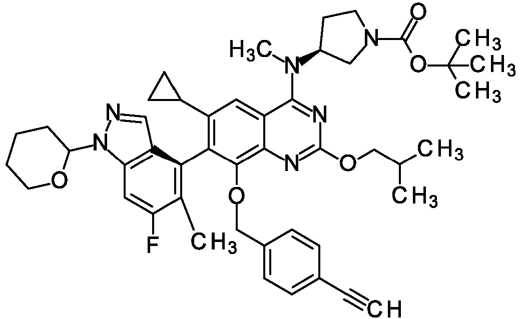 |

**[Table 5-13]**

| PEx | Str |
|---|---|
| 44 | 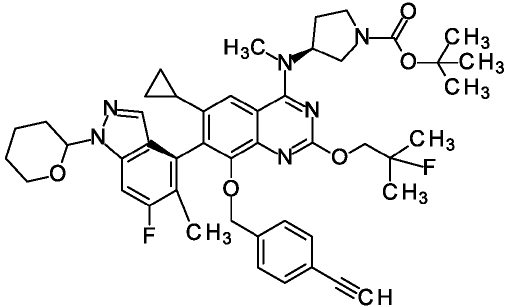 |
| 45 | 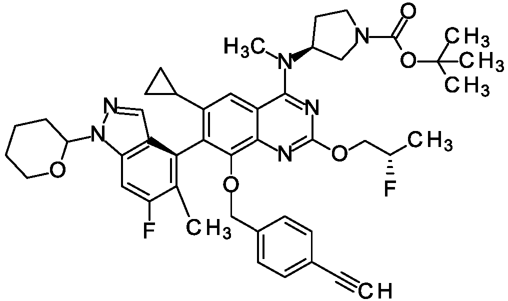 |
| 46 | 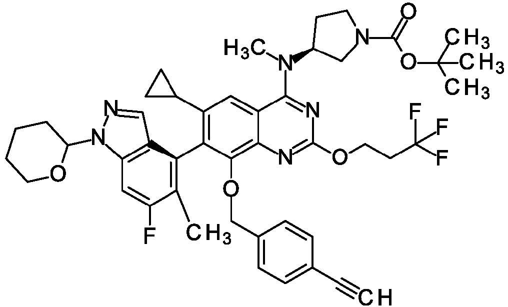 |

**[Table 5-14]**

| PEx | Str |
|---|---|
| 47 | 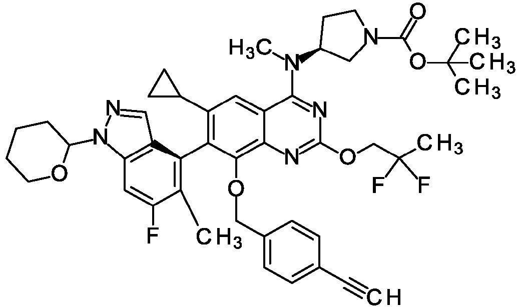 |
| 48 | 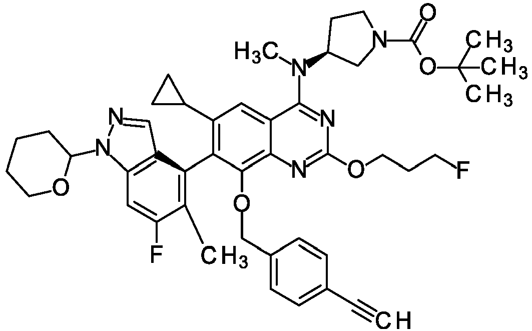 |
| 49 | 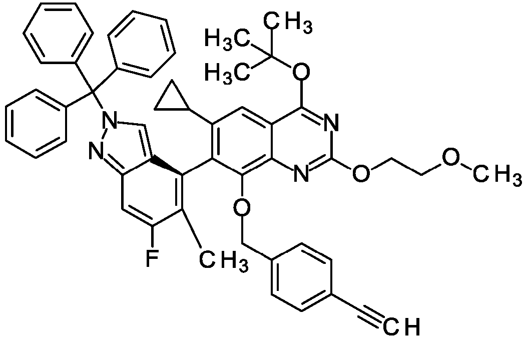 |

**[Table 5-15]**

| PEx | Str |
|---|---|
| 50 | 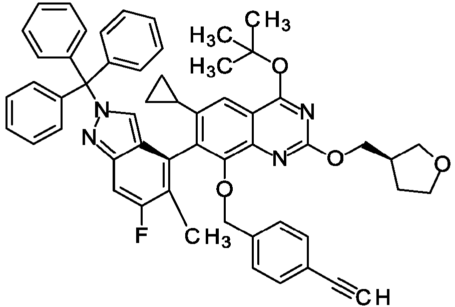 |
| 51 | 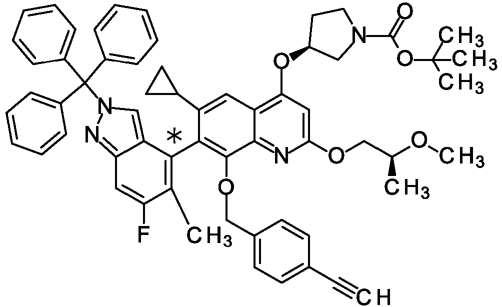 |
| 52 | 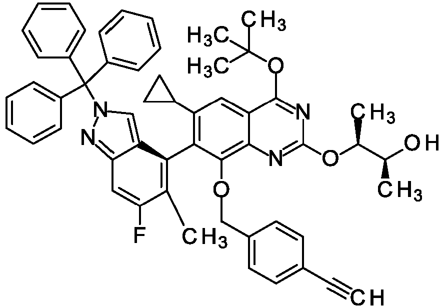 |

**[Table 5-16]**

| PEx | Str |
|---|---|
| 53 | 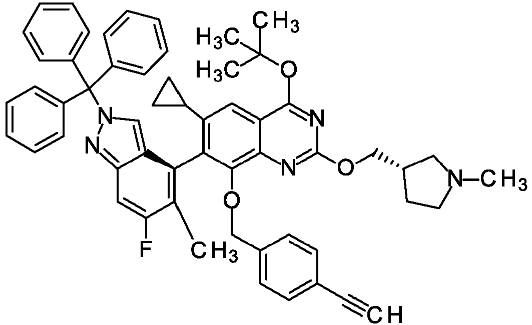 |
| 54 | 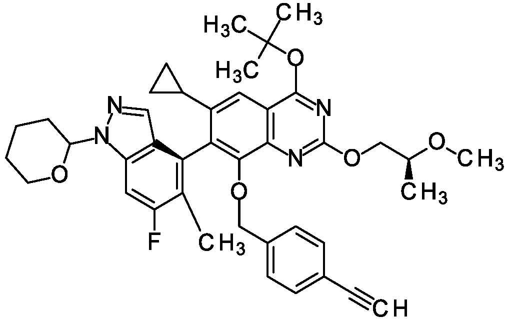 |
| 55 | 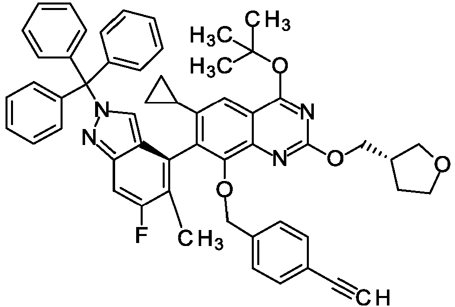 |

**[Table 5-17]**

| PEx | Str |
|---|---|
| 56 | 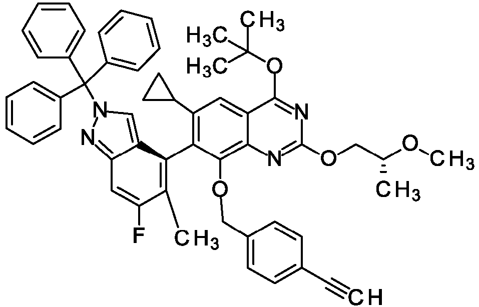 |
| 57 | 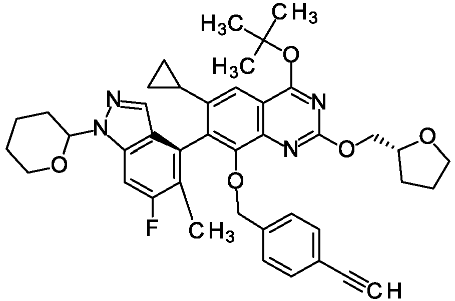 |
| 58 | 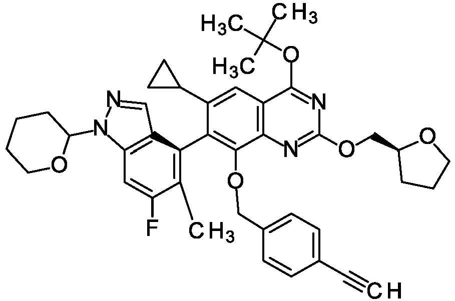 |
| 59 | 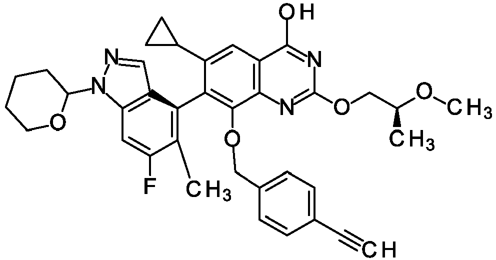 |

**[Table 5-18]**

| PEx | Str |
|---|---|
| 60 | 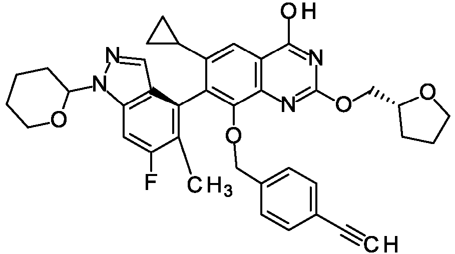 |
| 61 | 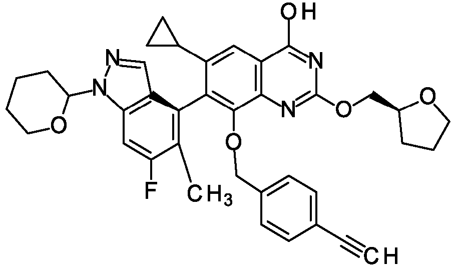 |
| 62 | 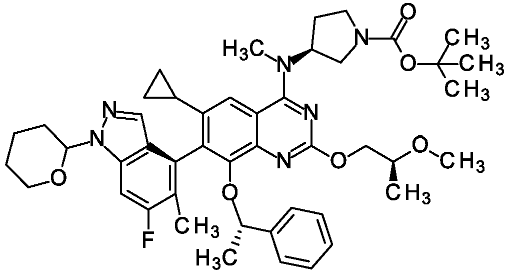 |
| 63 | 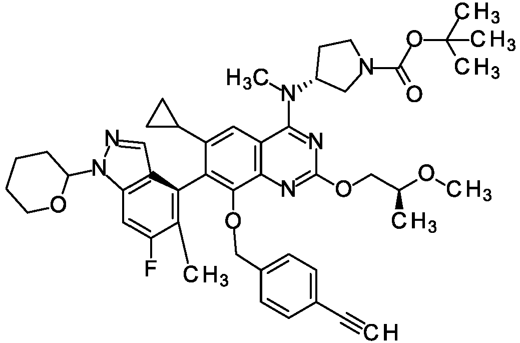 |

**[Table 5-19]**

| PEx | Str |
|---|---|
| 64 | 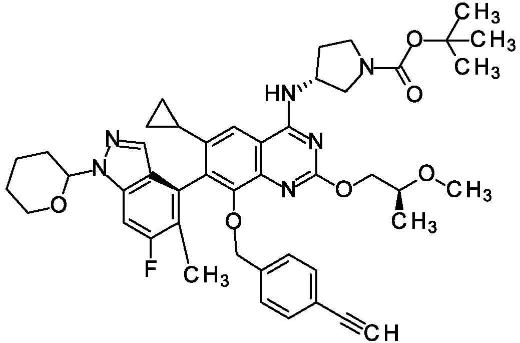 |
| 65 | 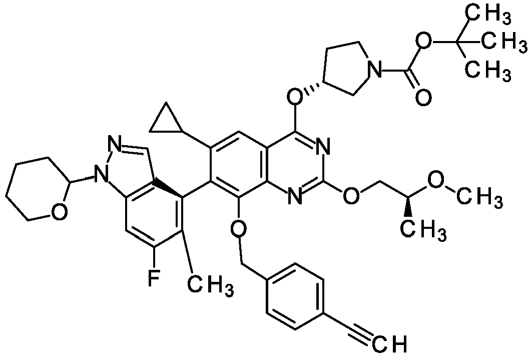 |
| 66 | 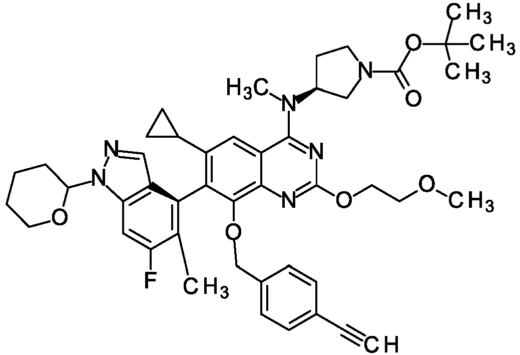 |

**[Table 5-20]**

| PEx | Str |
|---|---|
| 67 | 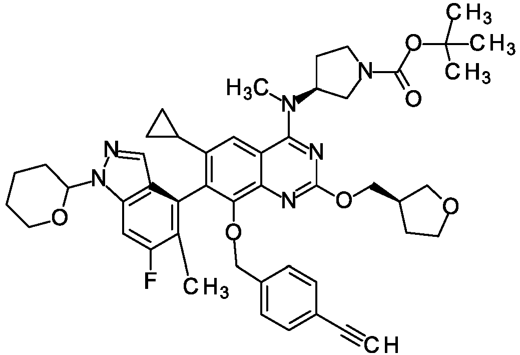 |
| 68 | 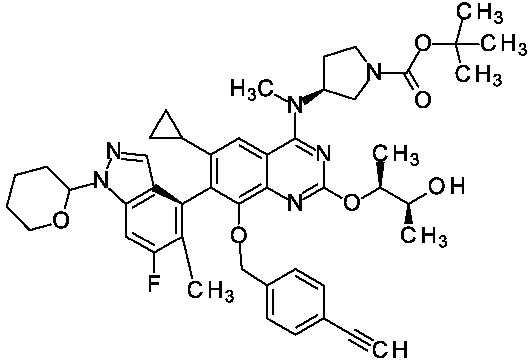 |
| 69 | 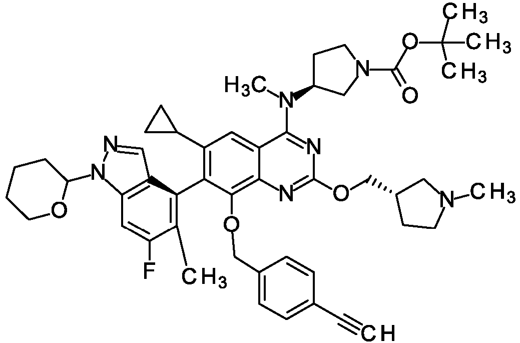 |

**[Table 5-21]**

| PEx | Str |
|---|---|
| 70 | 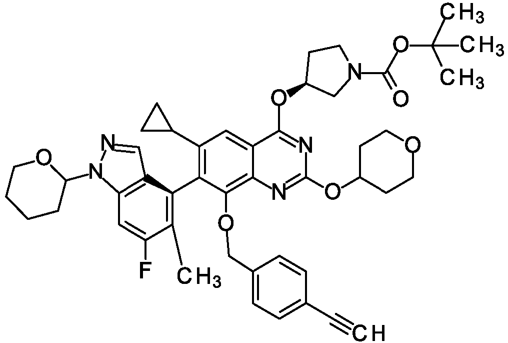 |
| 71 | 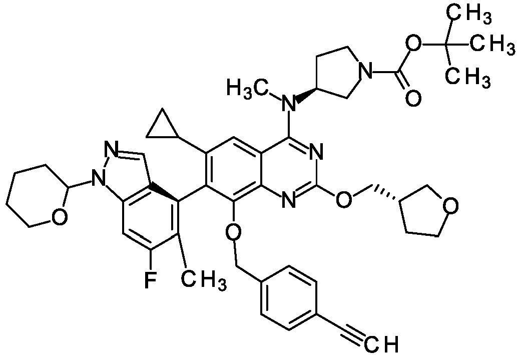 |
| 72 | 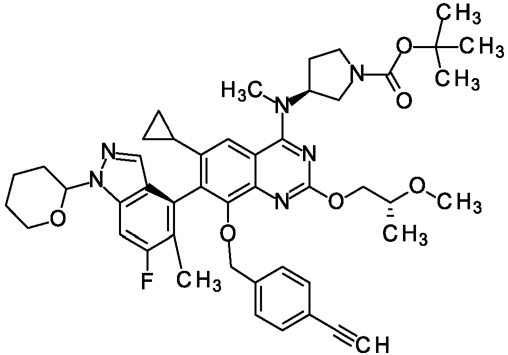 |

**[Table 5-22]**

| PEx | Str |
|---|---|
| 73 | 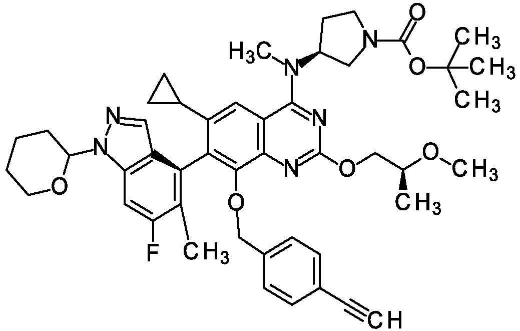 |
| 74 | 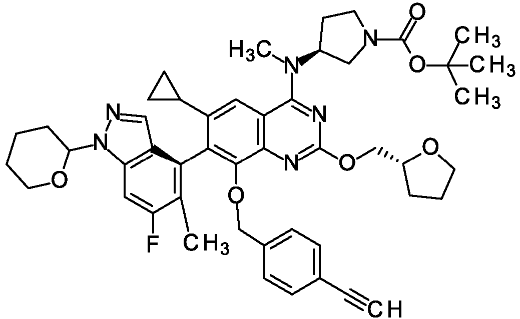 |
| 75 | 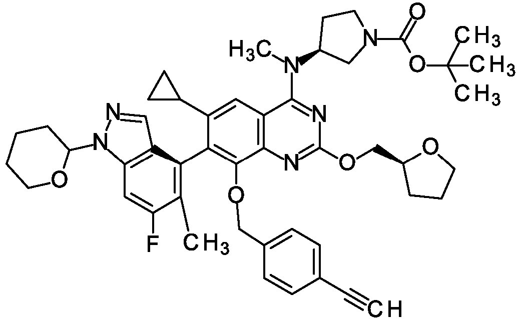 |

**[Table 5-23]**

| PEx | Str |
|---|---|
| 76 | 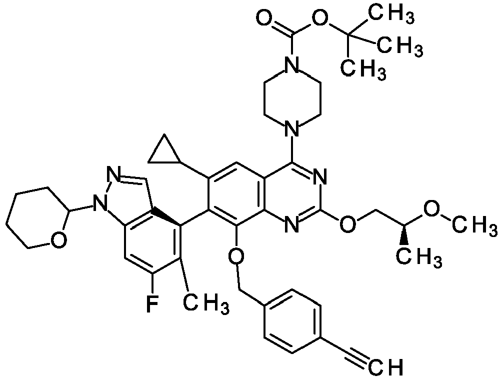 |
| 77 | 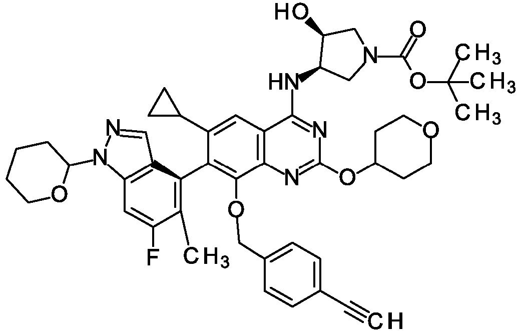 |
| 78 | 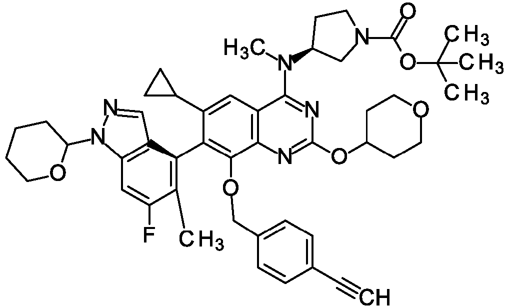 |

**[Table 5-24]**

| PEx | Str |
|---|---|
| 79 | 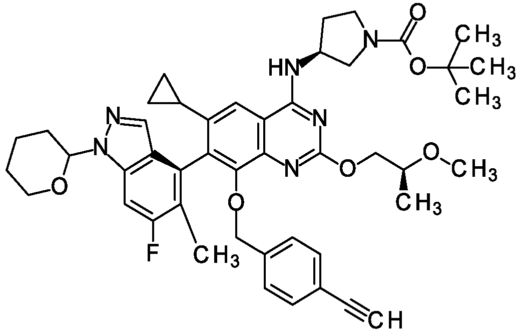 |
| 80 | 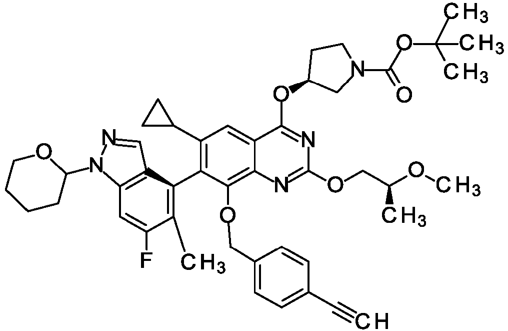 |
| 81 | 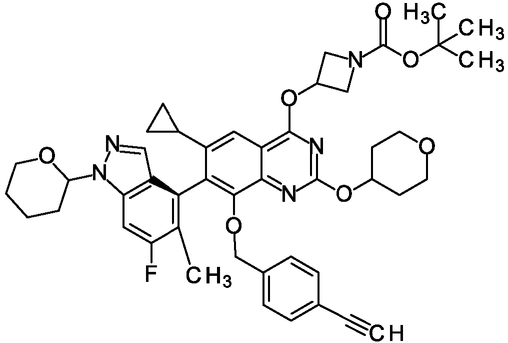 |

**[Table 5-25]**

| PEx | Str |
|---|---|
| 82 | 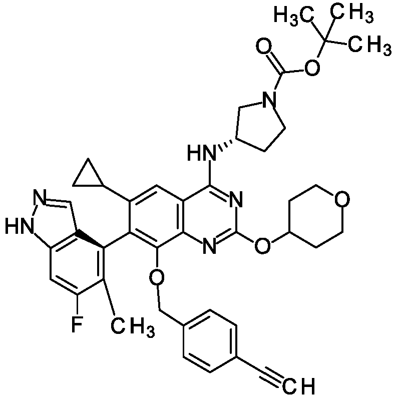 |
| 83 | 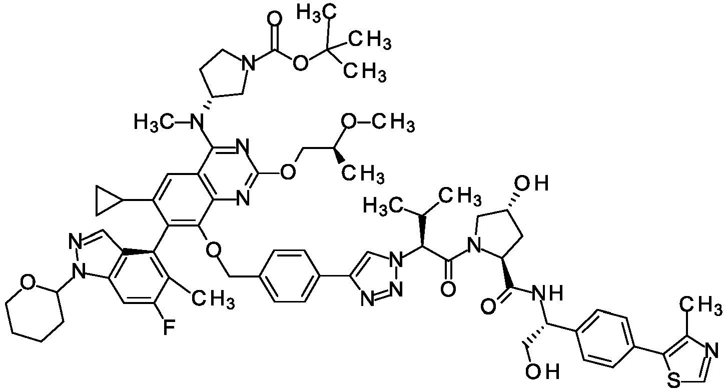 |
| 84 | 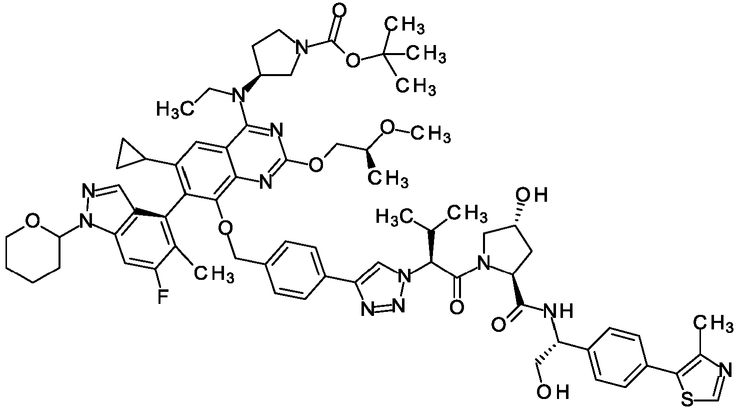 |

**[Table 5-26]**

| PEx | Str |
|---|---|
| 85 | 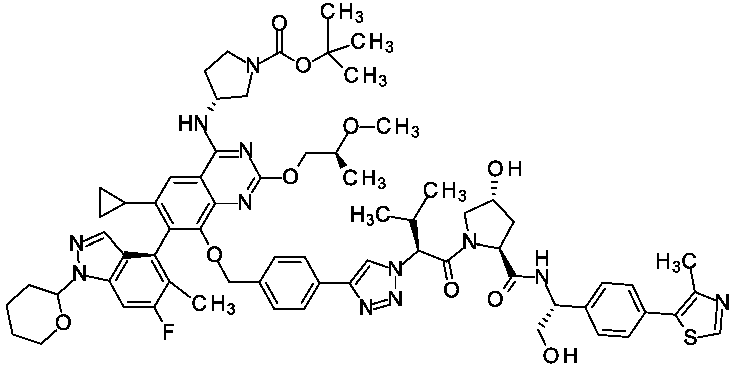 |
| 86 | 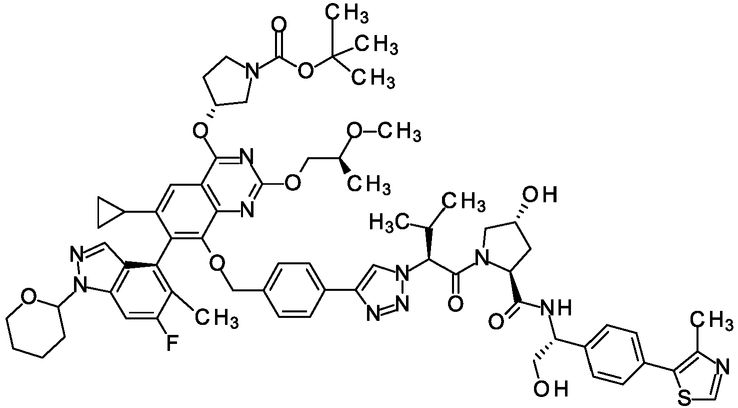 |
| 87 | 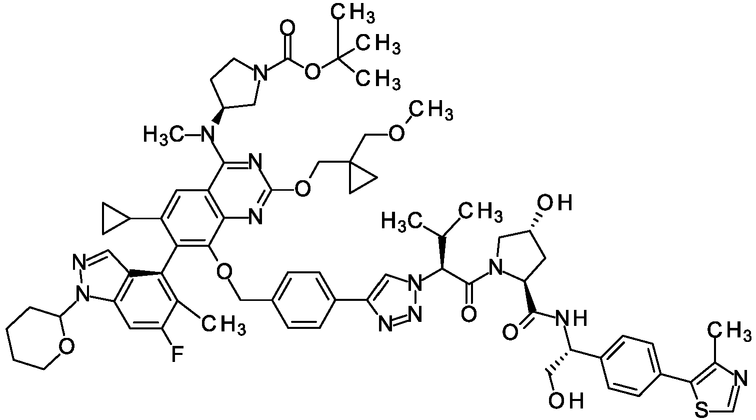 |

**[Table 5-27]**

| PEx | Str |
|---|---|
| 88 | 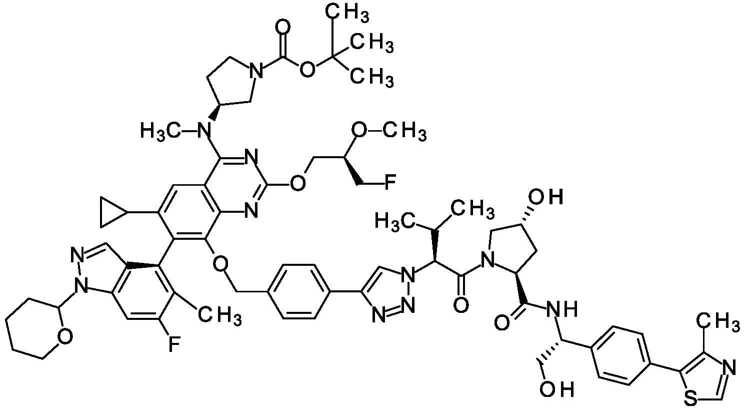 |
| 89 | 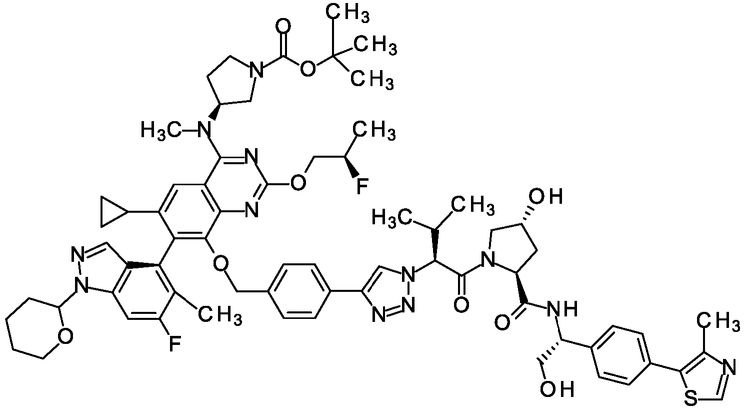 |
| 90 | 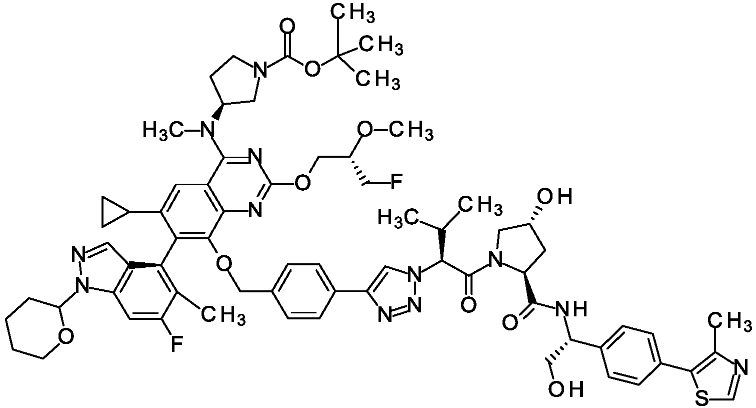 |

**[Table 5-28]**

| PEx | Str |
|---|---|
| 91 | 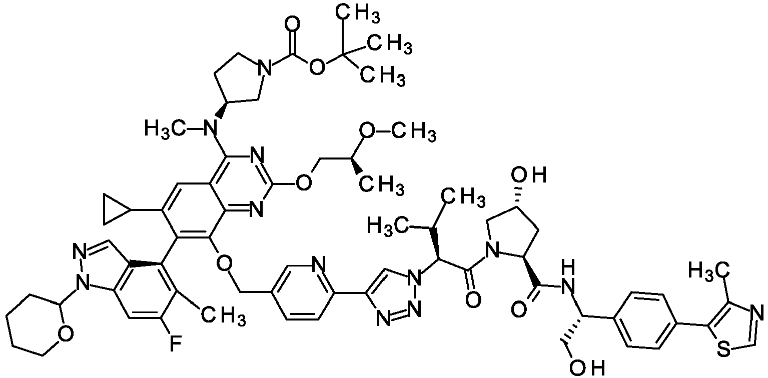 |
| 92 | 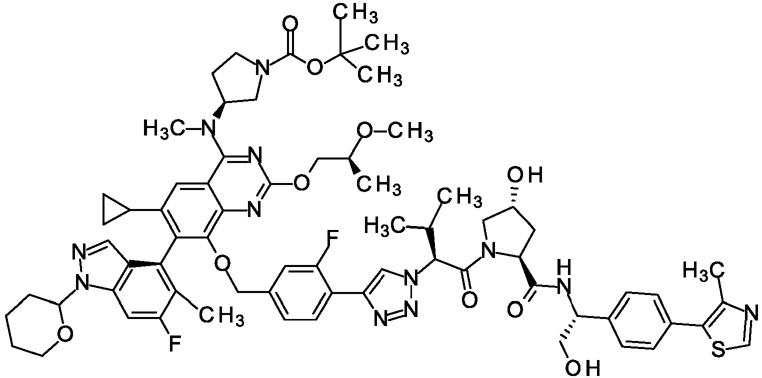 |
| 93 | 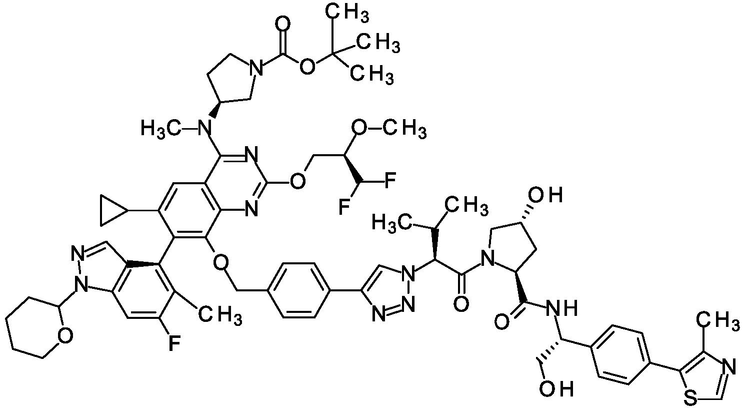 |

**[Table 5-29]**

| PEx | Str |
|---|---|
| 94 | 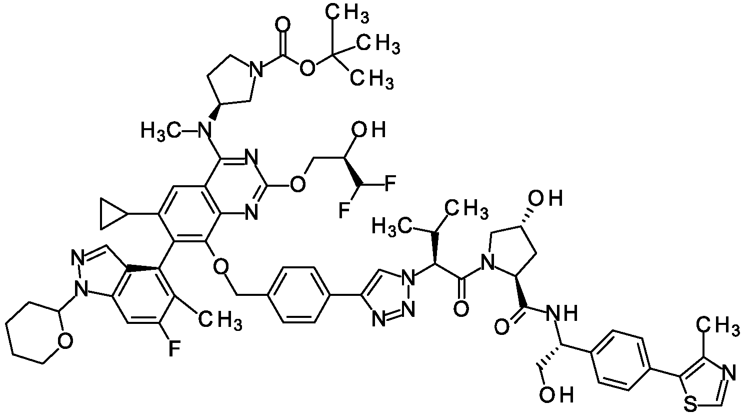 |
| 95 | 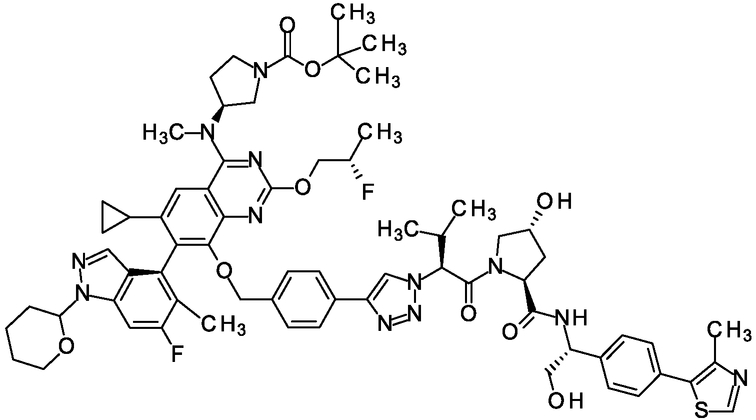 |
| 96 | 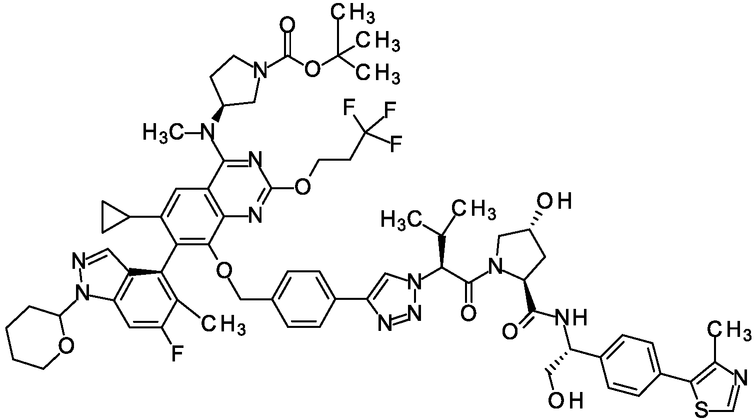 |

**[Table 5-30]**

| PEx | Str |
|---|---|
| 97 | 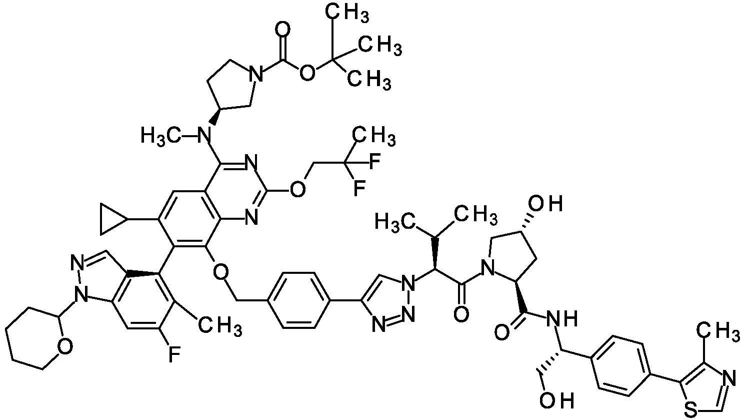 |
| 98 | 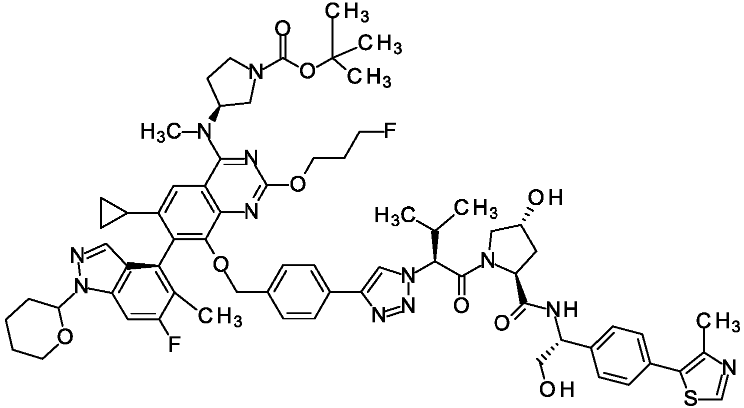 |
| 99 | 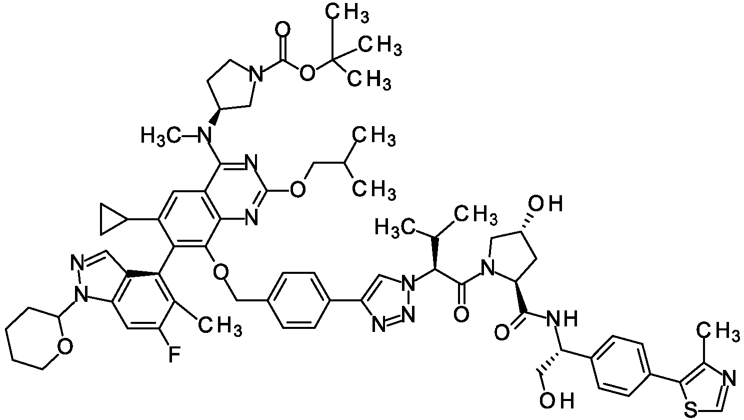 |

**[Table 5-31]**

| PEx | Str |
|---|---|
| 100 | 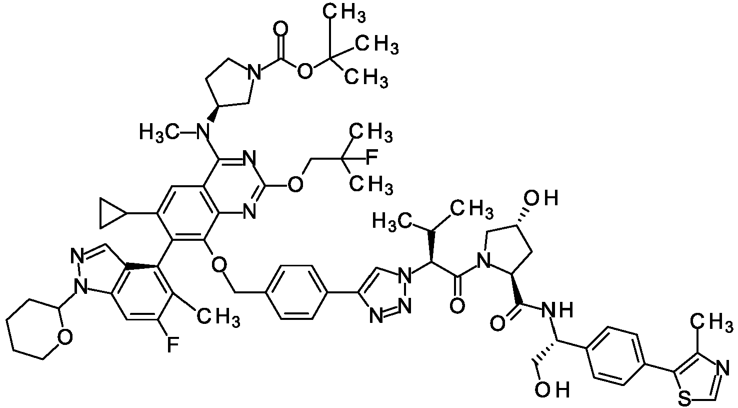 |
| 101 | 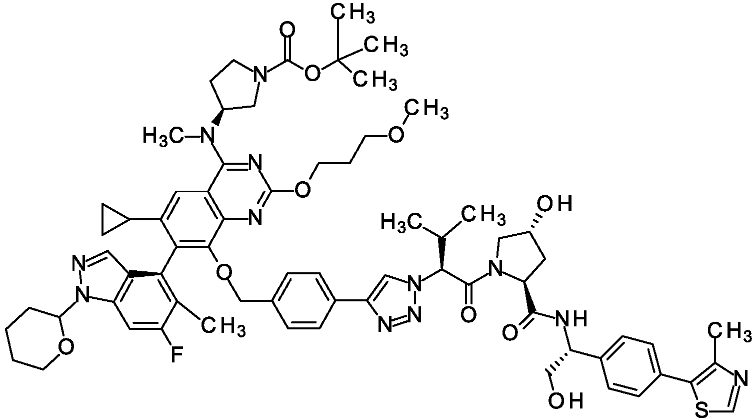 |
| 102 | 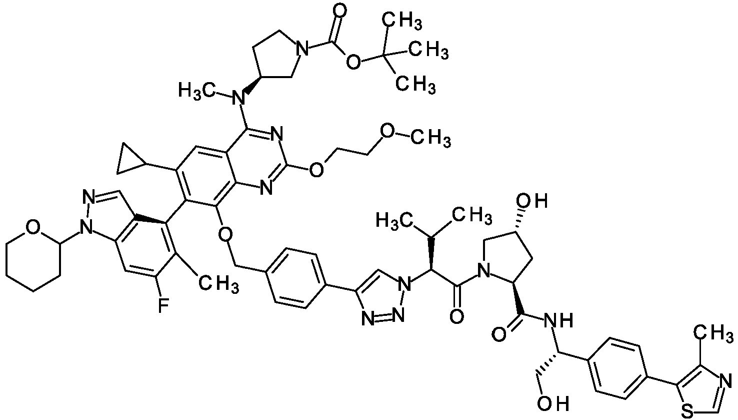 |

**[Table 5-32]**

| PEx | Str |
|---|---|
| 103 | 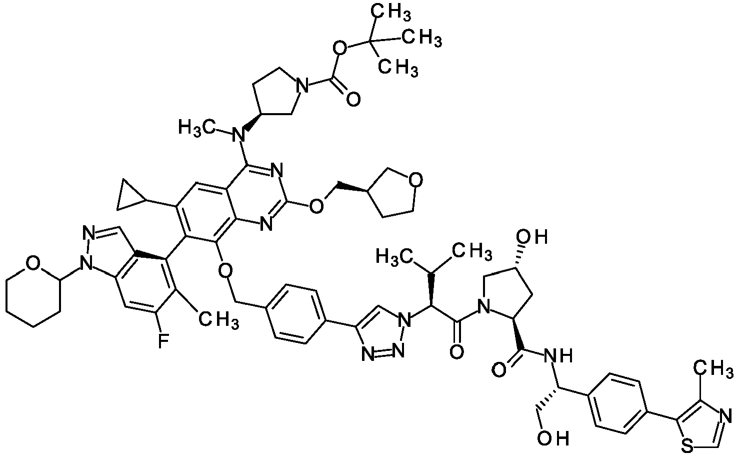 |
| 104 | 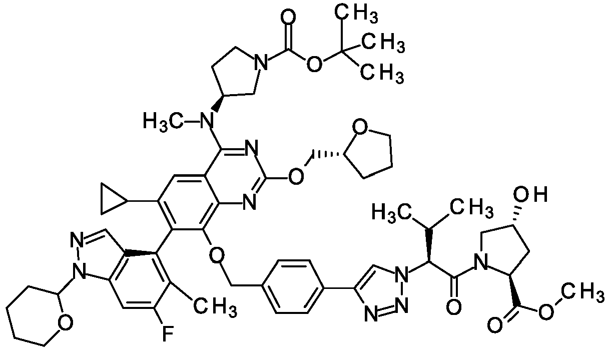 |
| 105 | 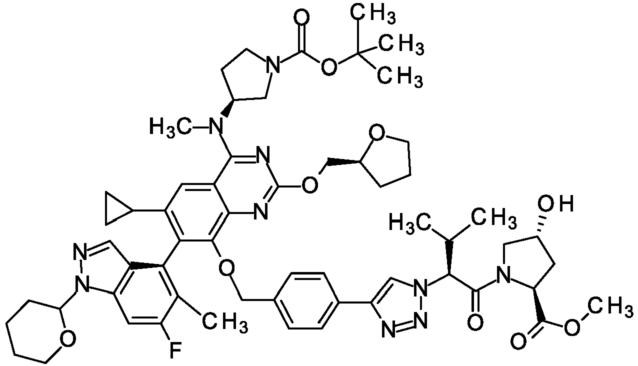 |

**[Table 5-33]**

| PEx | Str |
|---|---|
| 106 | 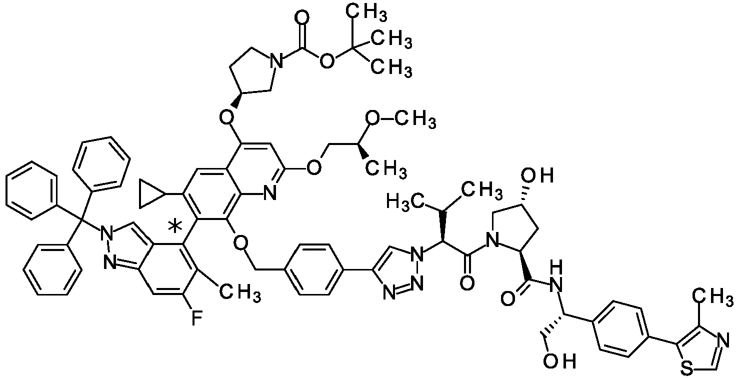 |
| 107 | 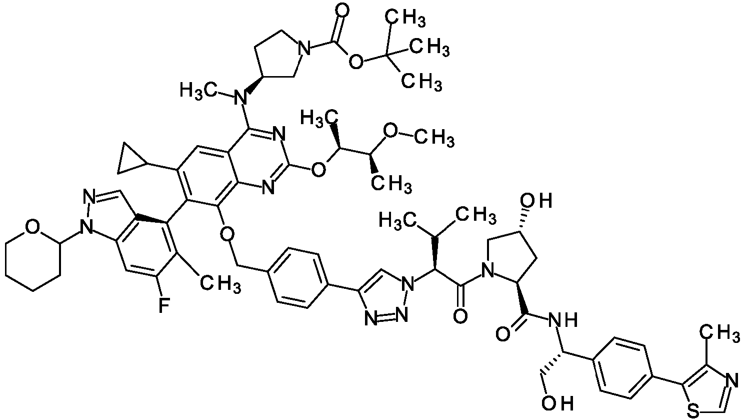 |
| 108 | 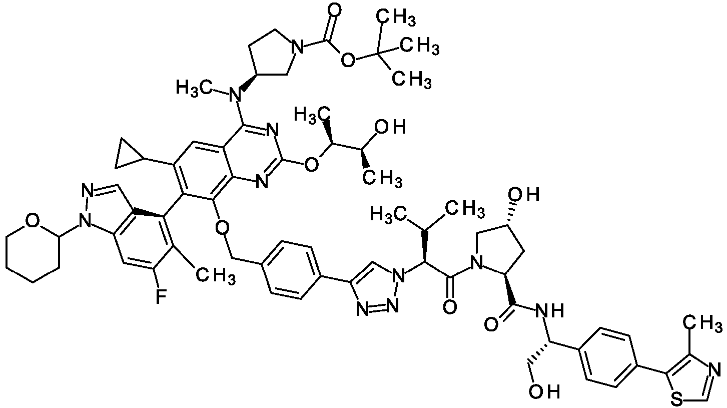 |

**[Table 5-34]**

| PEx | Str |
|---|---|
| 109 | 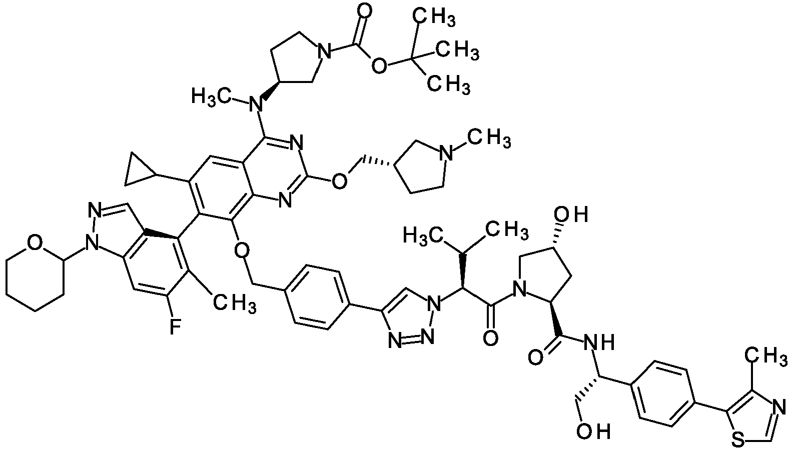 |
| 110 | 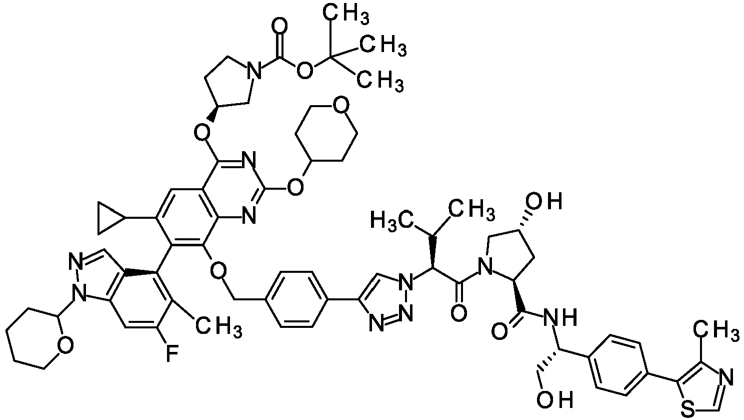 |
| 111 | 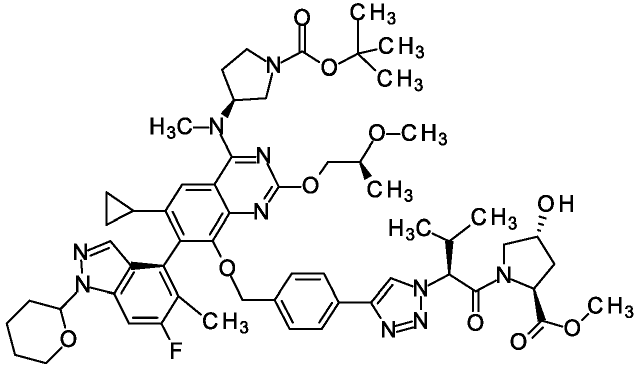 |

**[Table 5-35]**

| PEx | Str |
|---|---|
| 112 | 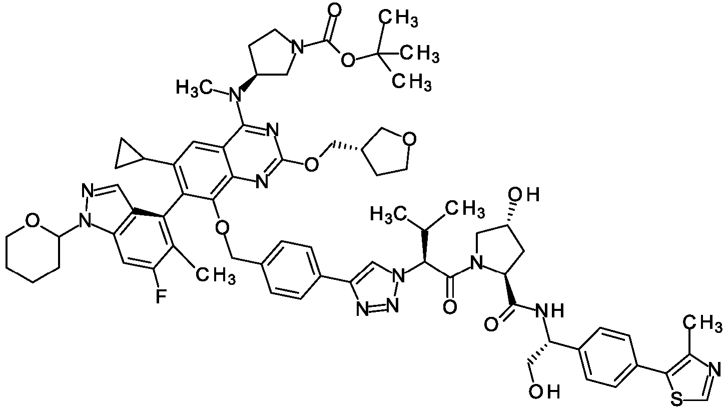 |
| 113 | 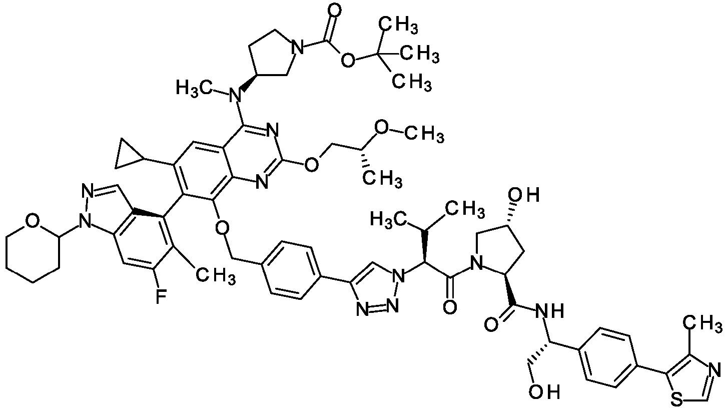 |
| 114 | 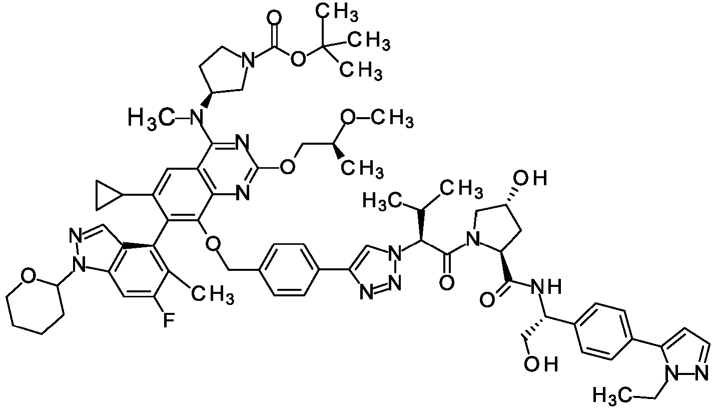 |

**[Table 5-36]**

| PEx | Str |
|---|---|
| 115 | 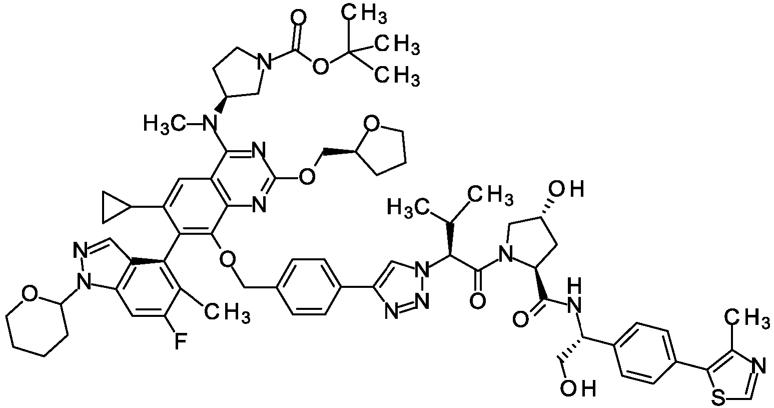 |
| 116 | 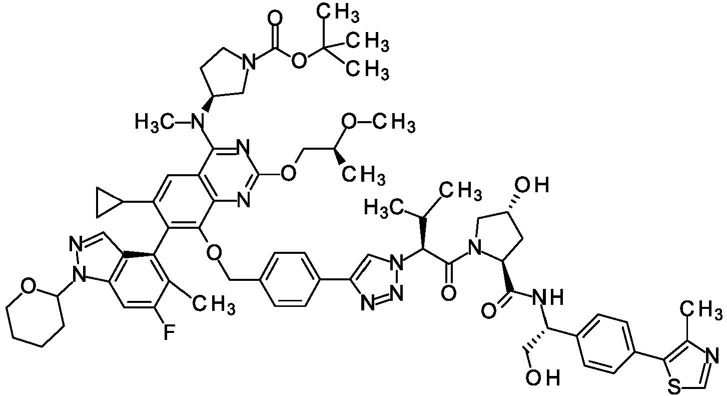 |
| 117 | 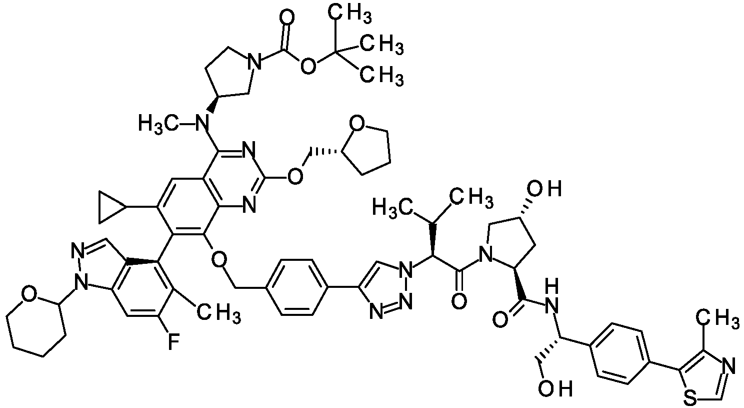 |

**[Table 5-37]**

| PEx | Str |
|---|---|
| 118 | 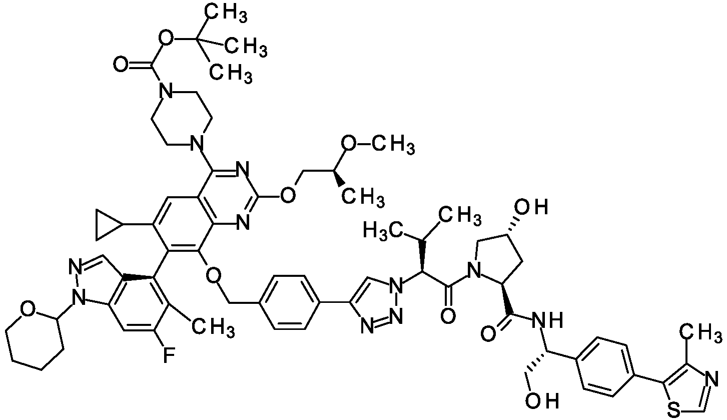 |
| 119 | 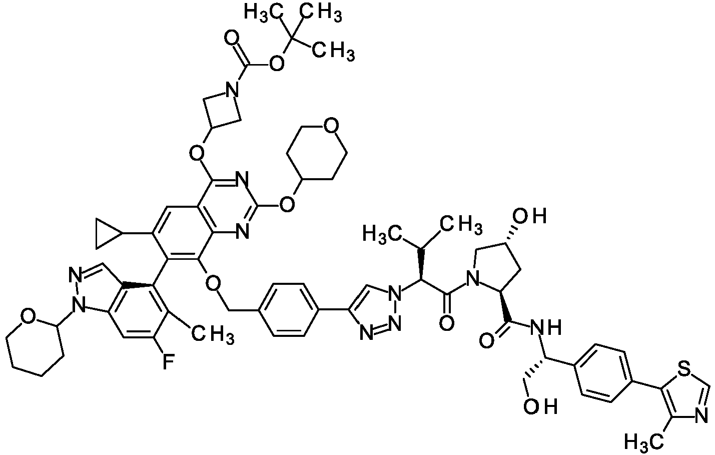 |
| 120 | 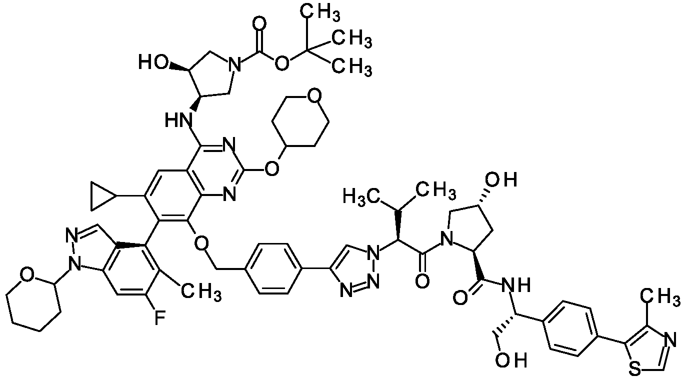 |

**[Table 5-38]**

| PEx | Str |
|---|---|
| 121 | 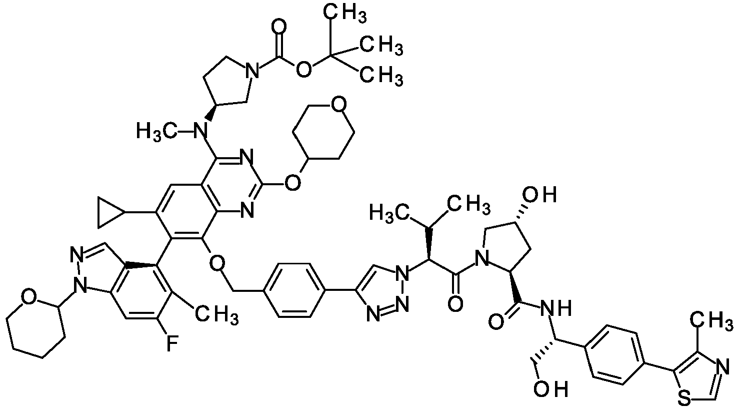 |
| 122 | 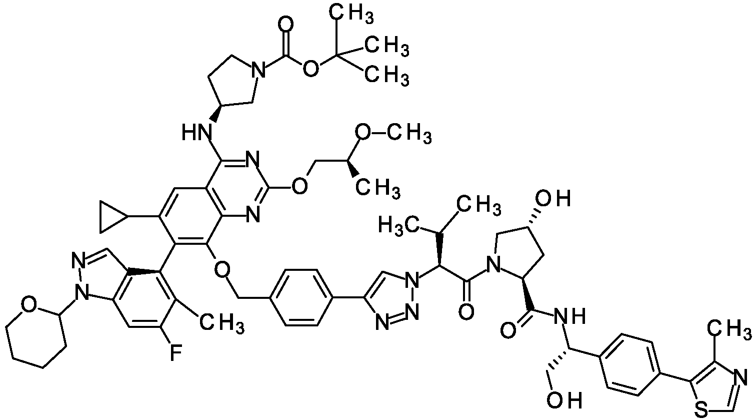 |
| 123 | 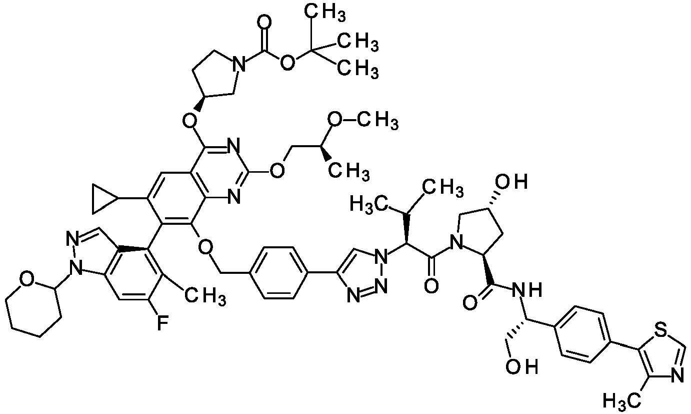 |

**[Table 5-39]**

| PEx | Str |
|---|---|
| 124 | 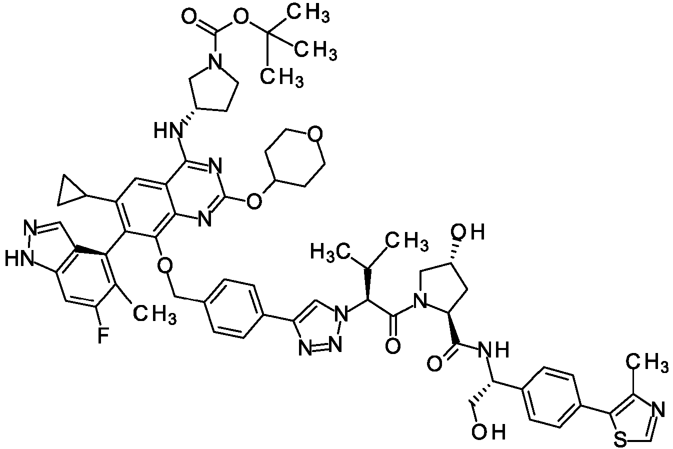 |
| 125 | 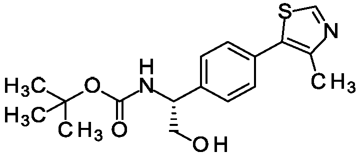 |
| 126 | 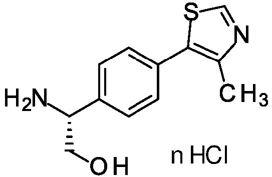 |
| 127 | 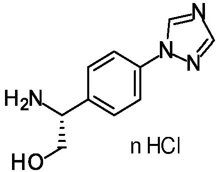 |
| 128 | 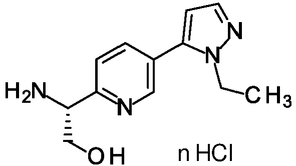 |

**[Table 5-40]**

| PEx | Str |
|---|---|
| 129 | 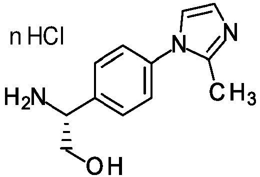 |
| 130 | 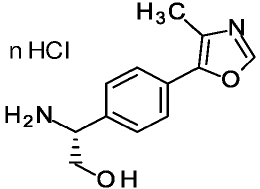 |
| 131 | 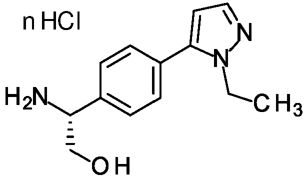 |
| 132 | 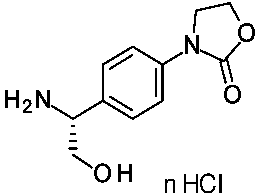 |
| 133 | 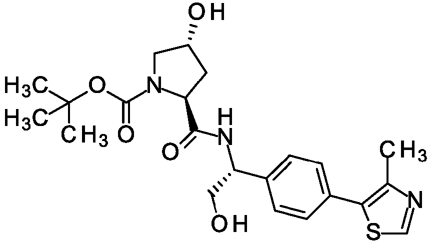 |

**[Table 5-41]**

| PEx | Str |
|---|---|
| 134 | 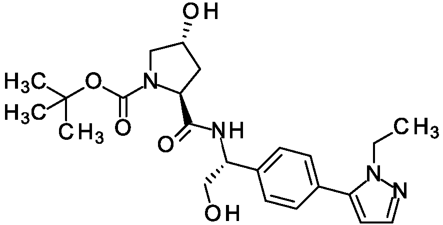 |
| 135 | 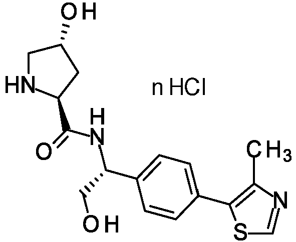 |
| 136 | 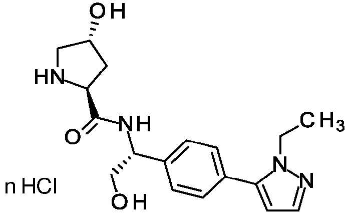 |
| 137 | 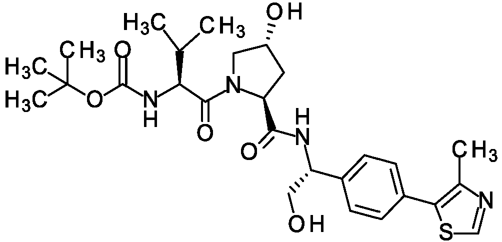 |
| 138 | 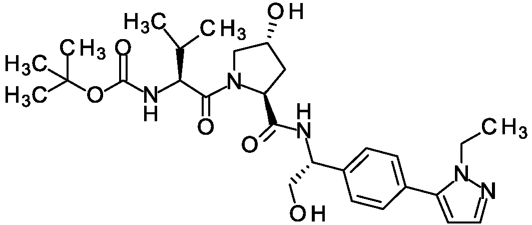 |

**[Table 5-42]**

| PEx | Str |
|---|---|
| 139 | 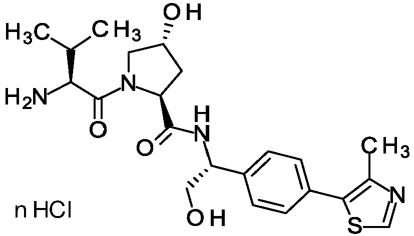 |
| 140 | 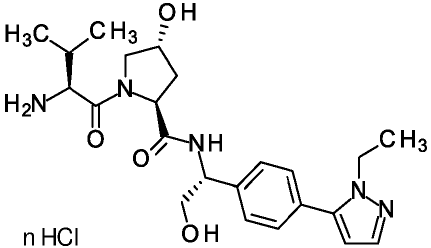 |
| 141 | 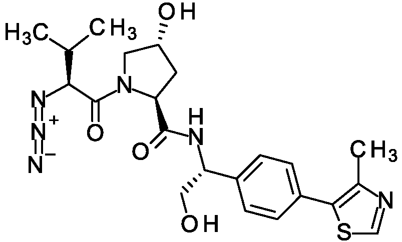 |
| 142 | 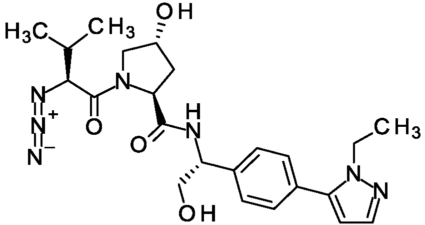 |

**[Table 5-43]**

| PEx | Str |
|---|---|
| 143 | 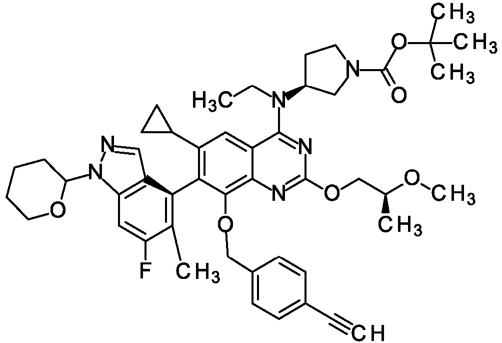 |
| 144 | 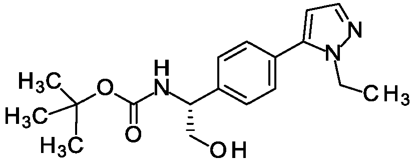 |
| 145 | 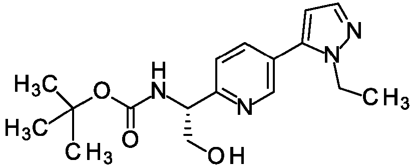 |
| 146 | 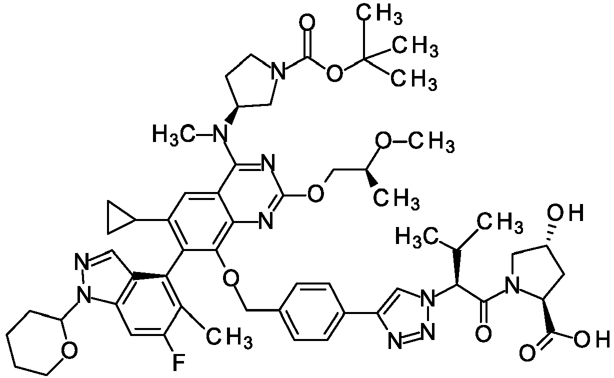 |

**[Table 5-44]**

| PEx | Str |
|---|---|
| 147 | 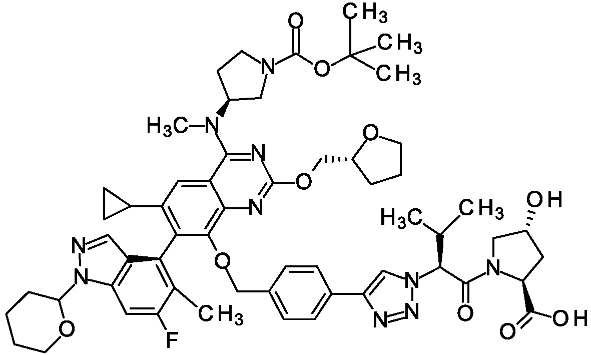 |
| 148 | 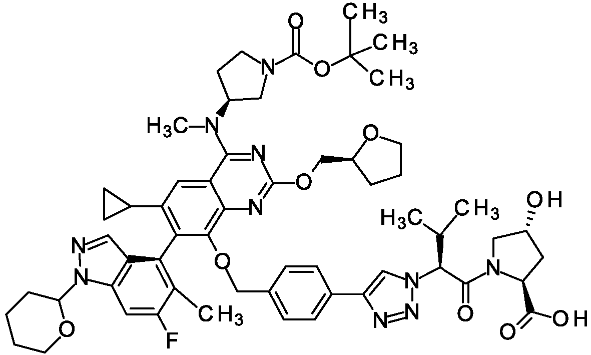 |
| 149 | 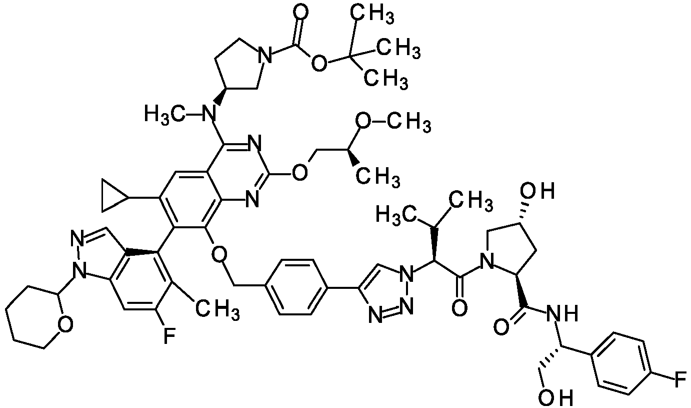 |

**[Table 5-45]**

| PEx | Str |
|---|---|
| 150 | 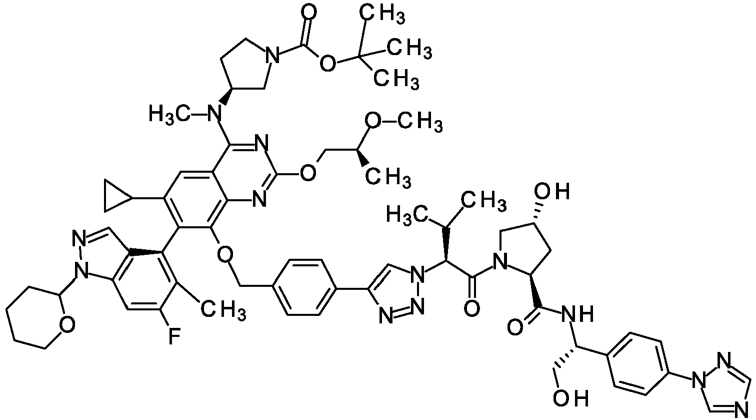 |
| 151 | 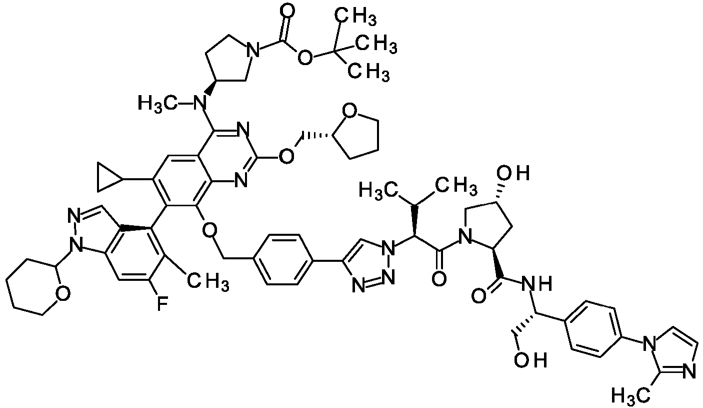 |
| 152 | 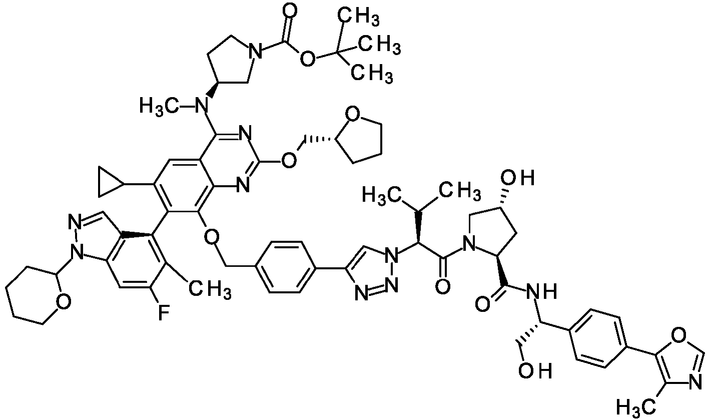 |

**[Table 5-46]**

| PEx | Str |
|---|---|
| 153 | 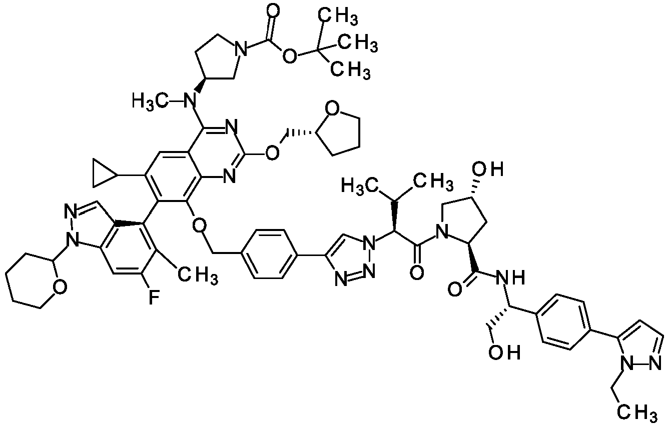 |
| 154 | 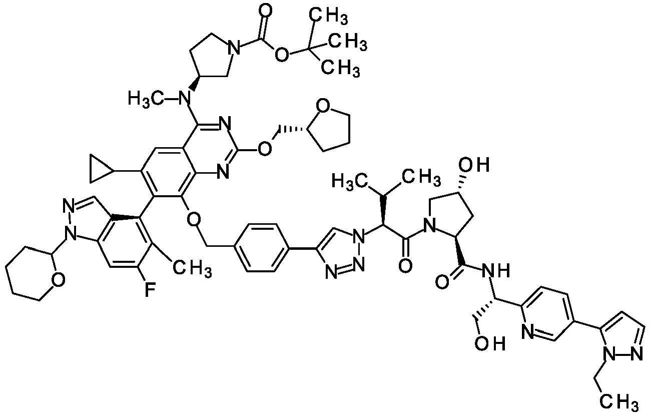 |
| 155 | 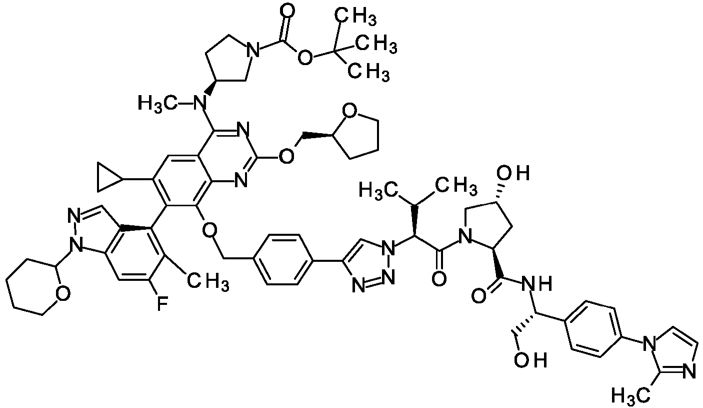 |

**[Table 5-47]**

| PEx | Str |
|---|---|
| 156 | 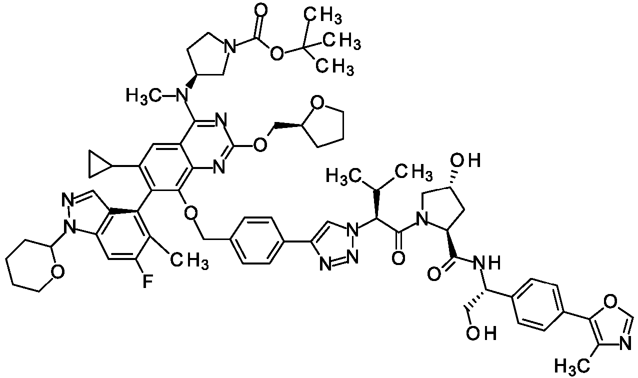 |
| 157 | 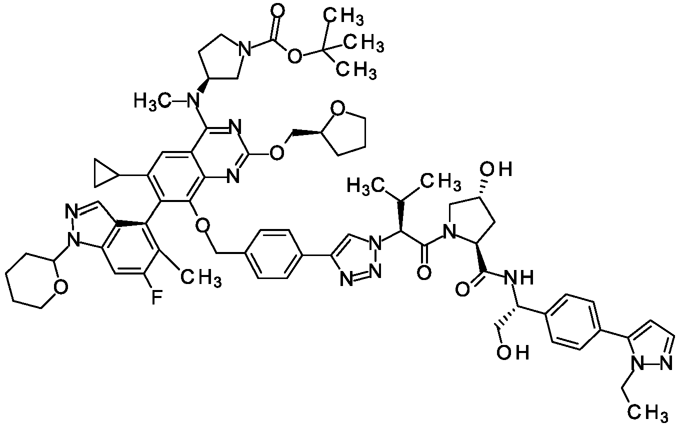 |
| 158 | 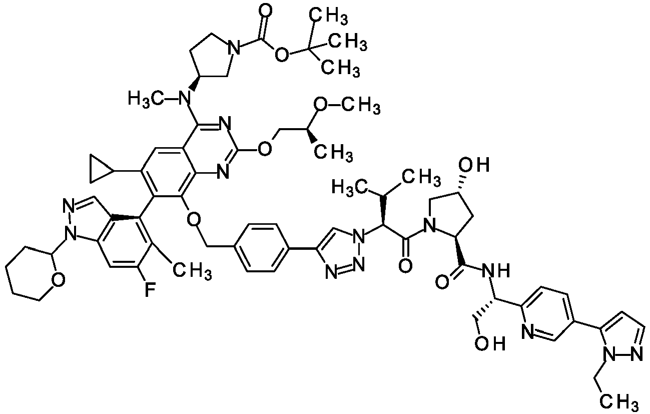 |

**[Table 5-48]**

| PEx | Str |
|---|---|
| 159 | 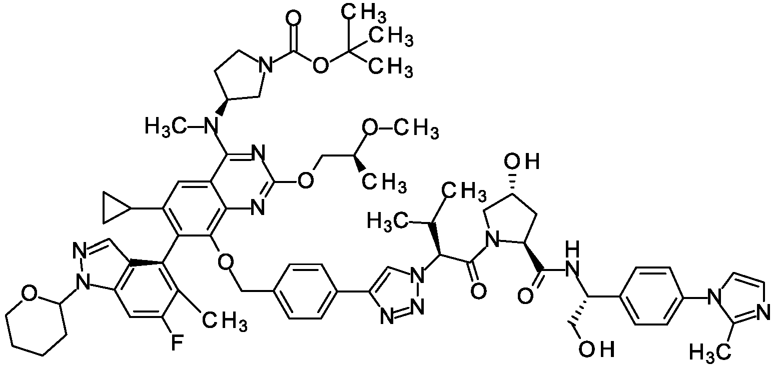 |
| 160 | 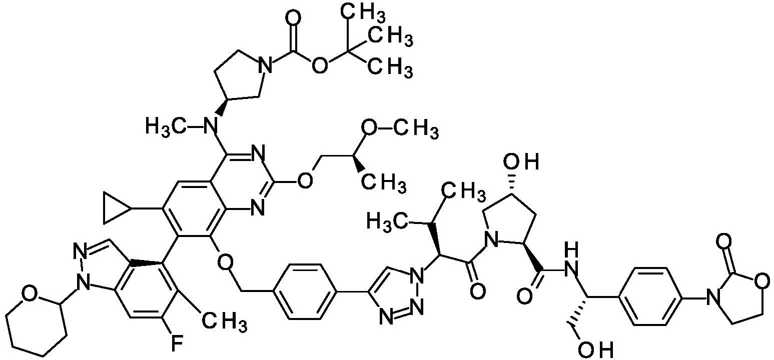 |
| 161 | 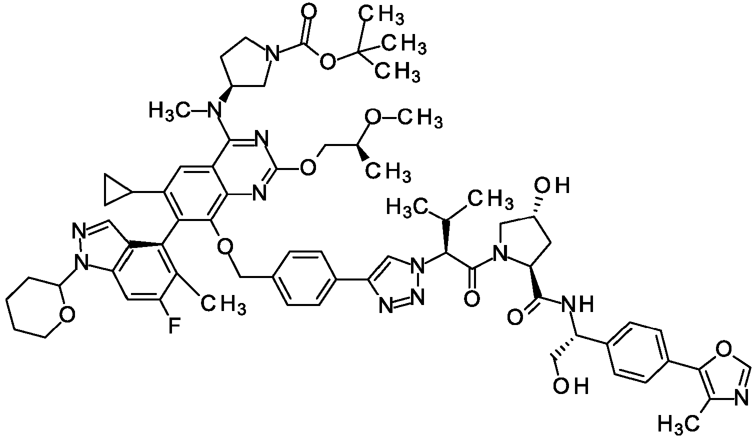 |

**[Table 5-49]**

| PEx | Str |
|---|---|
| 162 | 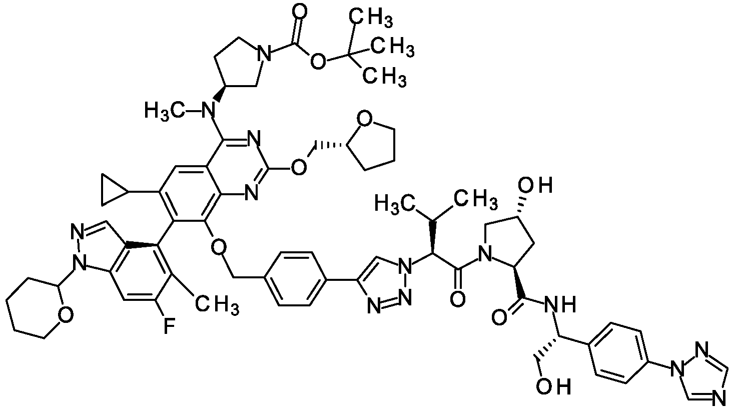 |
| 163 | 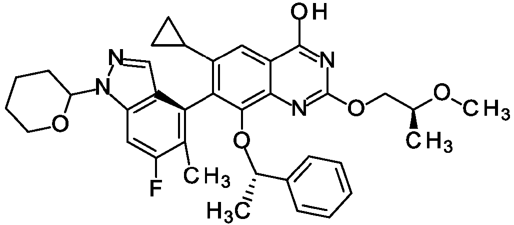 |
| 164 | 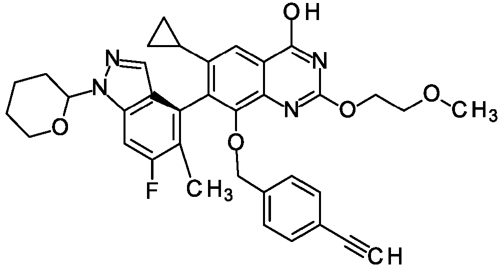 |
| 165 | 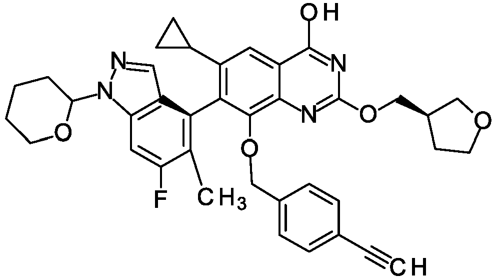 |

**[Table 5-50]**

| PEx | Str |
|---|---|
| 166 | 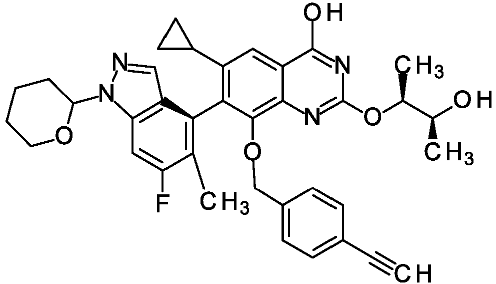 |
| 167 | 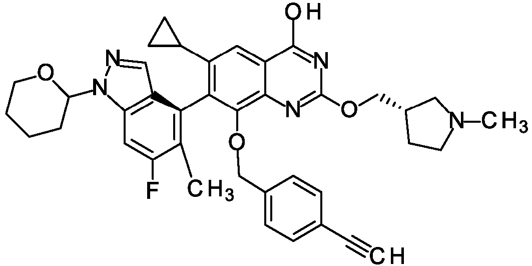 |
| 168 | 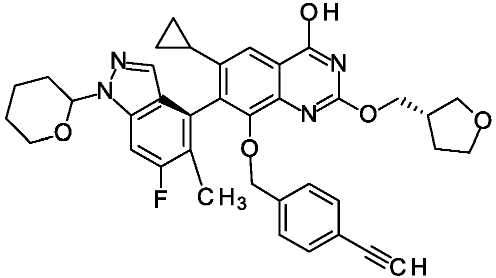 |
| 169 | 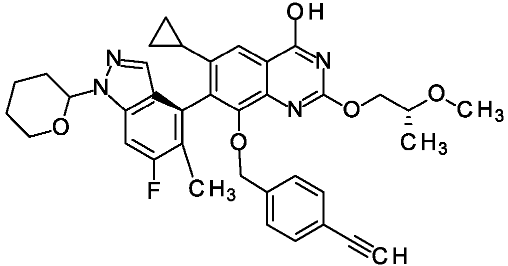 |

**[Table 5-51]**

| PEx | Str |
|---|---|
| 170 | 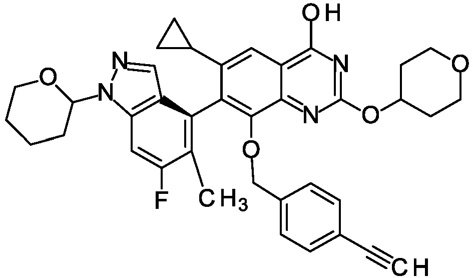 |
| 171 | 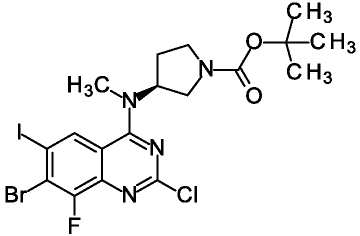 |
| 172 | 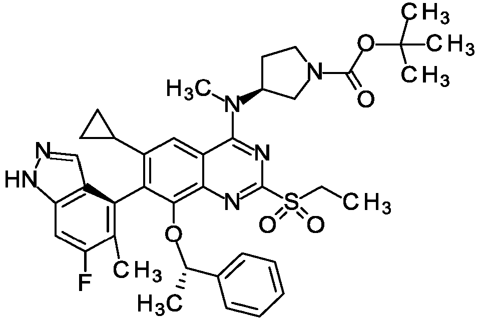 |
| 173 | 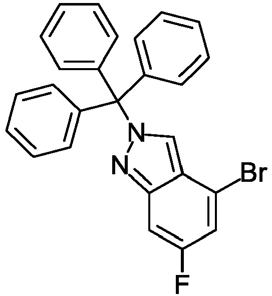 |

**[Table 5-52]**

| PEx | Str |
|---|---|
| 174 | 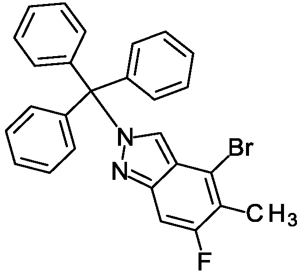 |
| 175 | 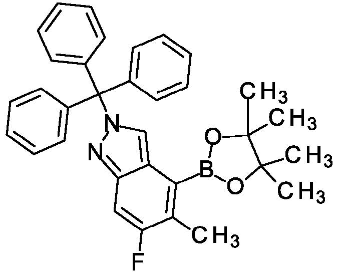 |
| 176 | 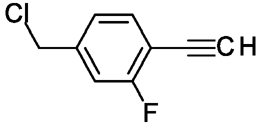 |
| 177 | 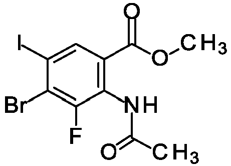 |
| 178 | 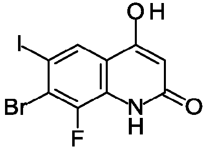 |

**[Table 5-53]**

| PEx | Str |
|---|---|
| 179 | 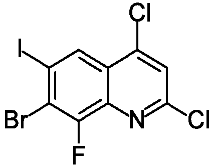 |
| 180 | 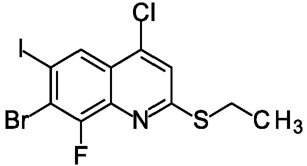 |
| 181 | 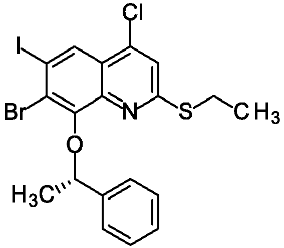 |
| 182 | 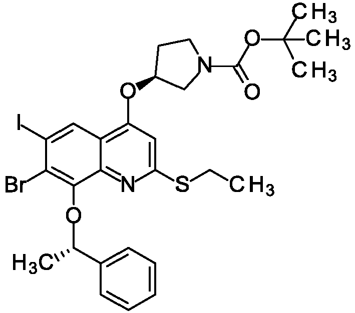 |
| 183 | 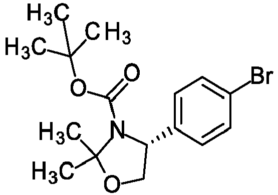 |

**[Table 5-54]**

| PEx | Str |
|---|---|
| 184 | 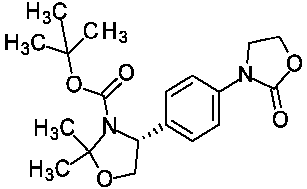 |
| 185 | 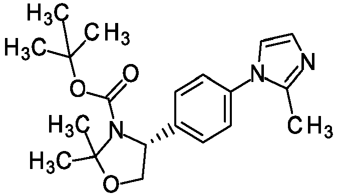 |
| 186 | 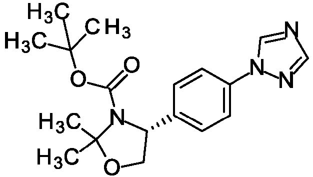 |
| 187 | 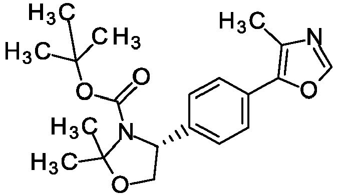 |
| 188 | 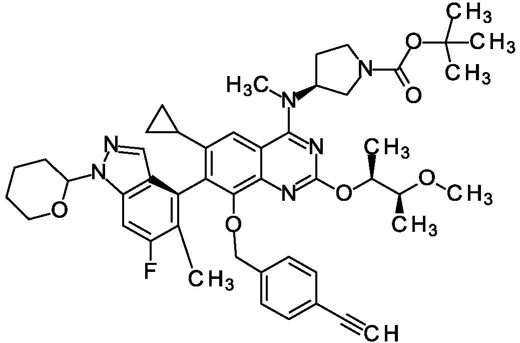 |

**[Table 5-55]**

| PEx | Str |
|---|---|
| 189 | 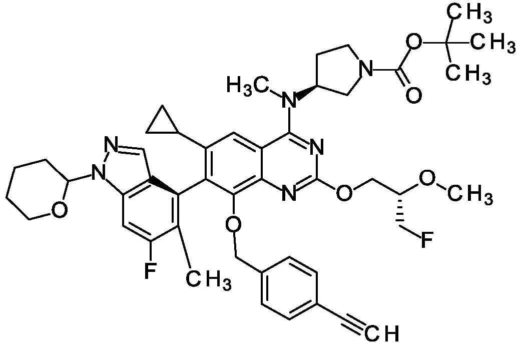 |
| 190 | 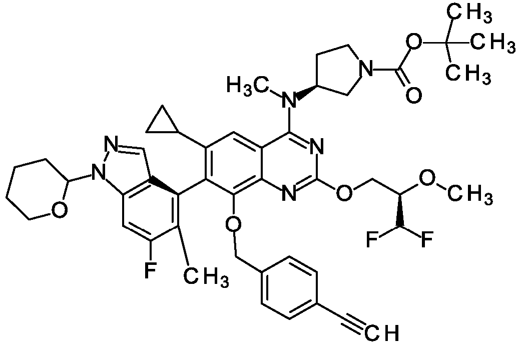 |
| 191 | 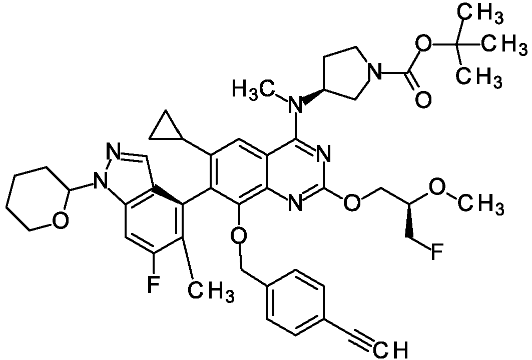 |

**[Table 5-56]**

| PEx | Str |
|---|---|
| 192 | 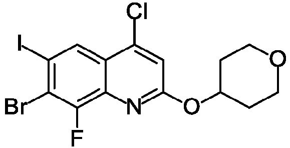 |
| 193 | 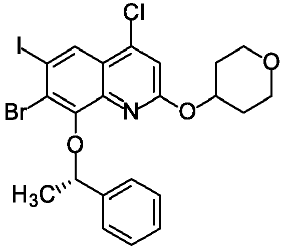 |
| 194 | 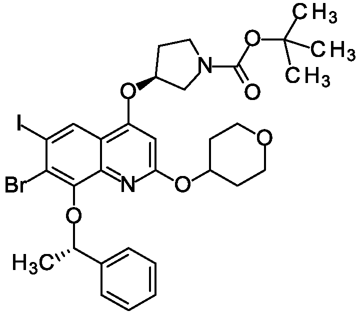 |
| 195 | 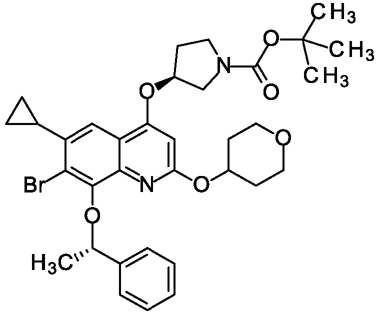 |

**[Table 5-57]**

| PEx | Str |
|---|---|
| 196 | 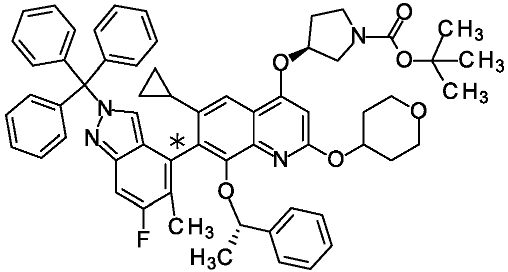 |
| 197 | 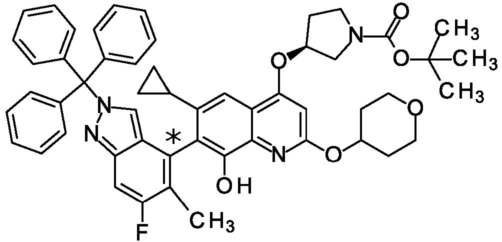 |
| 198 | 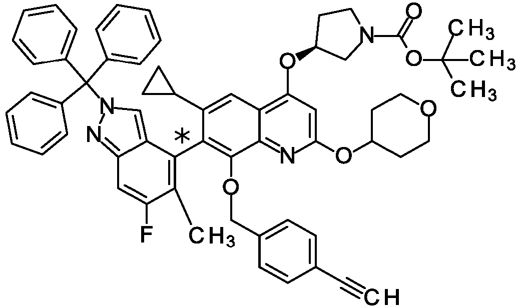 |
| 199 | 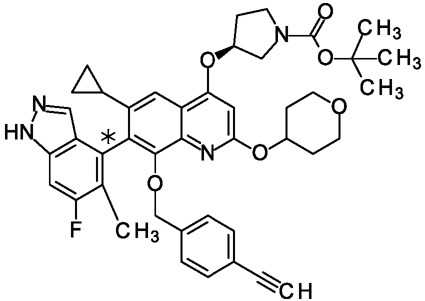 |

**[Table 5-58]**

| PEx | Str |
|---|---|
| 200 | 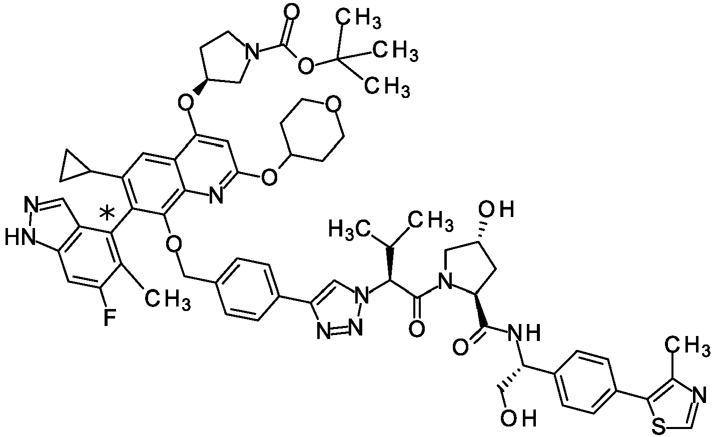 |
| 201 | 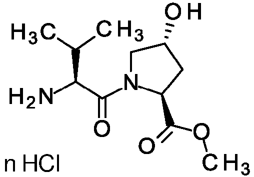 |
| 202 | 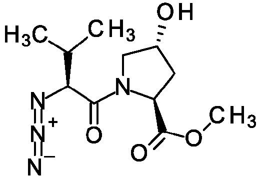 |

**[Table 5-59]**

| PEx | Str |
|---|---|
| 203 | 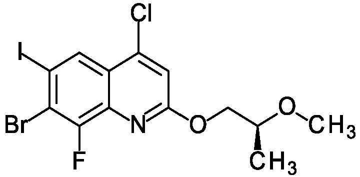 |
| 204 | 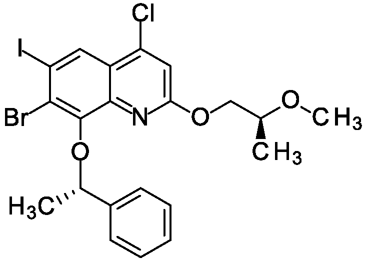 |
| 205 | 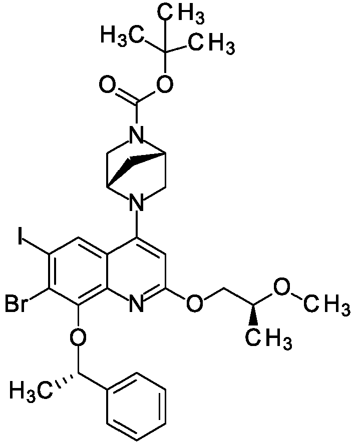 |
| 206 | 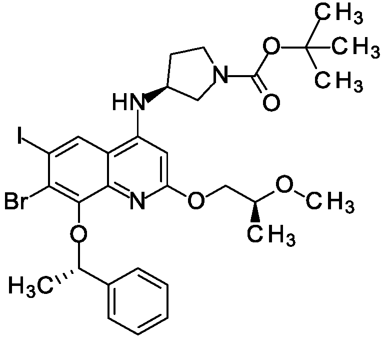 |

**[Table 5-60]**

| PEx | Str |
|---|---|
| 207 | 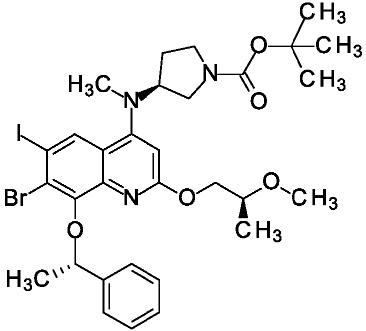 |
| 208 | 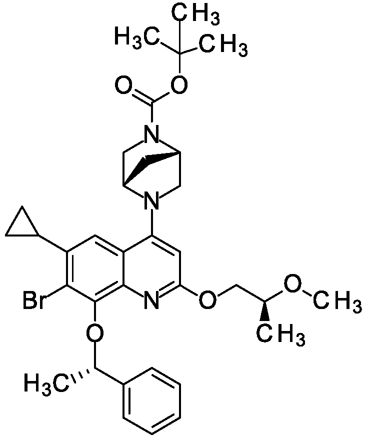 |
| 209 | 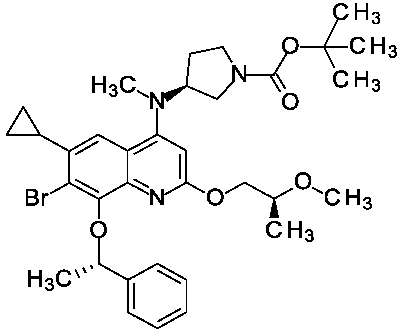 |

**[Table 5-61]**

| PEx | Str |
|---|---|
| 210 | 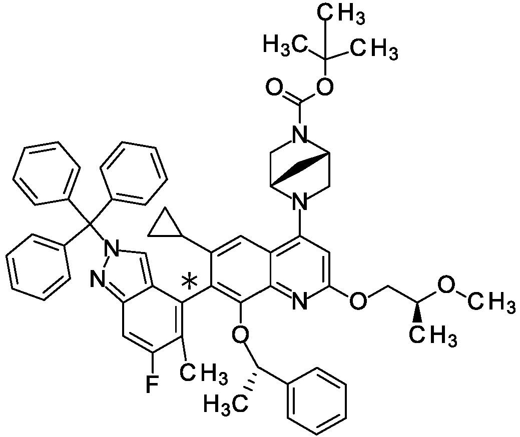 |
| 211 | 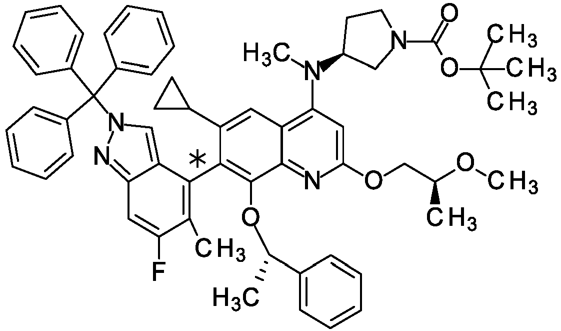 |
| 212 | 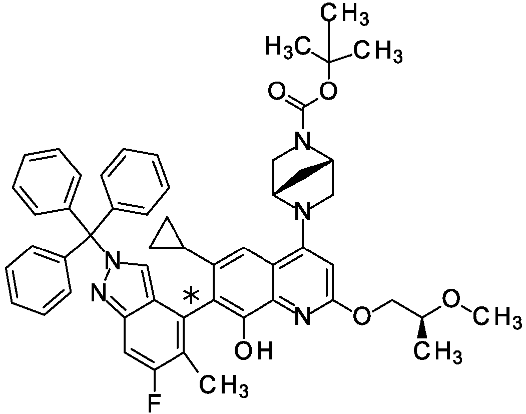 |

**[Table 5-62]**

| PEx | Str |
|---|---|
| 213 | 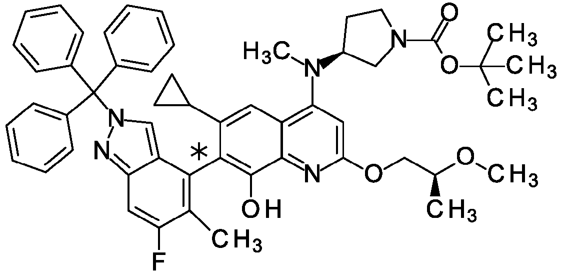 |
| 214 | 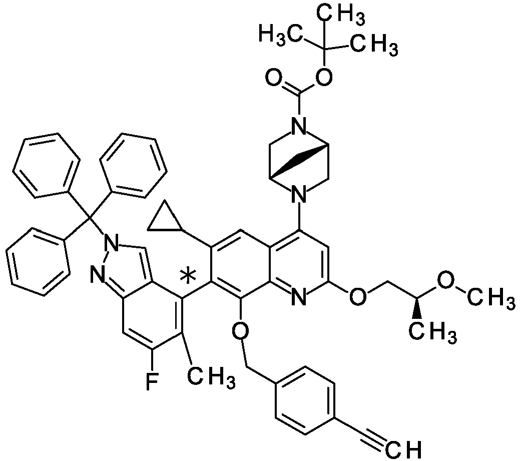 |
| 215 | 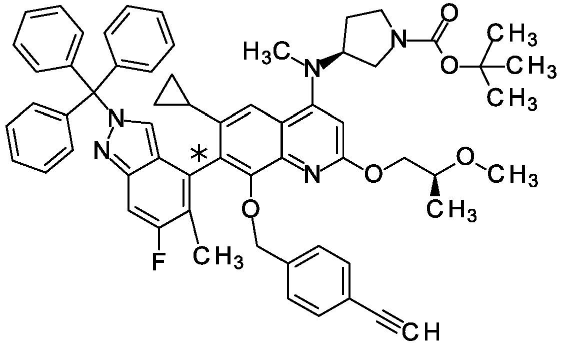 |

**[Table 5-63]**

| PEx | Str |
|---|---|
| 216 | 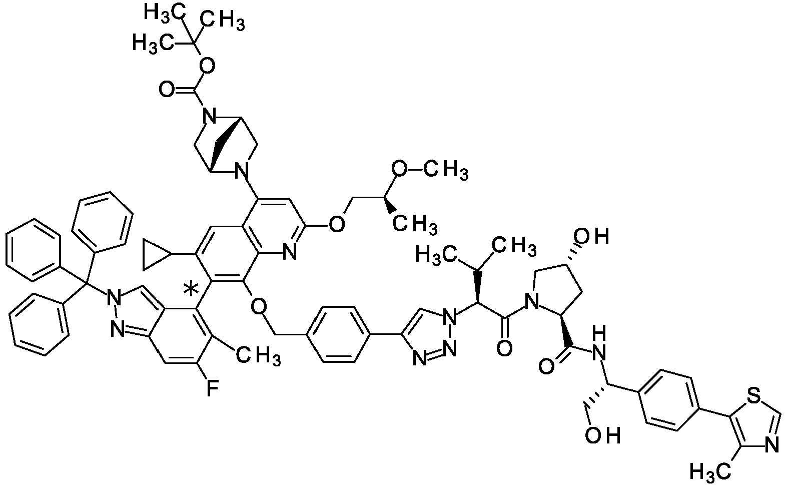 |
| 217 | 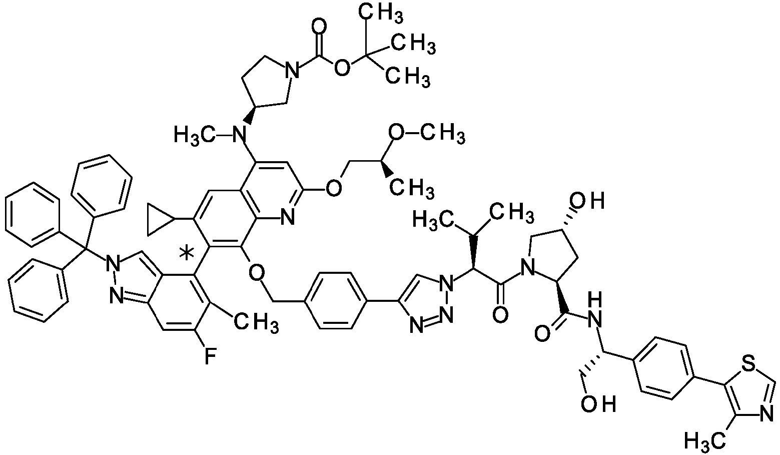 |

**[Table 5-64]**

| PEx | Str |
|---|---|
| 218 | 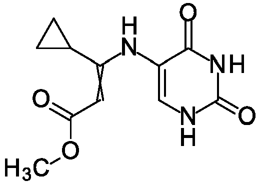 |
| 219 | 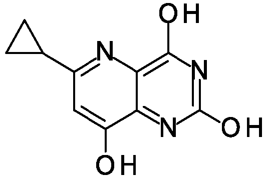 |
| 220 | 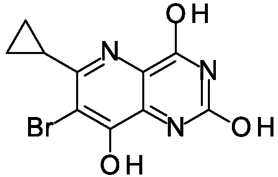 |
| 221 | 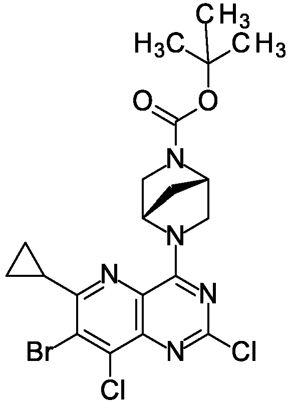 |

**[Table 5-65]**

| PEx | Str |
|---|---|
| 222 | 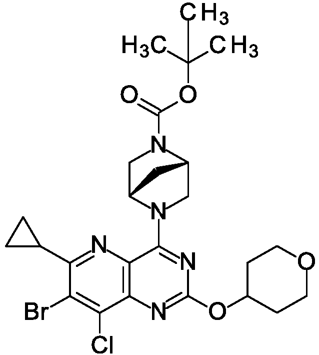 |
| 223 | 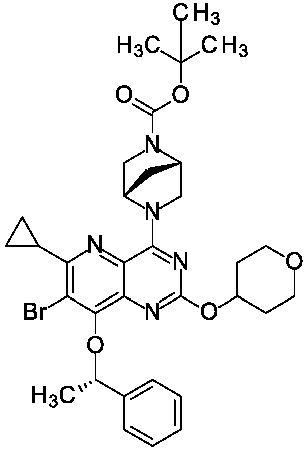 |
| 224 | 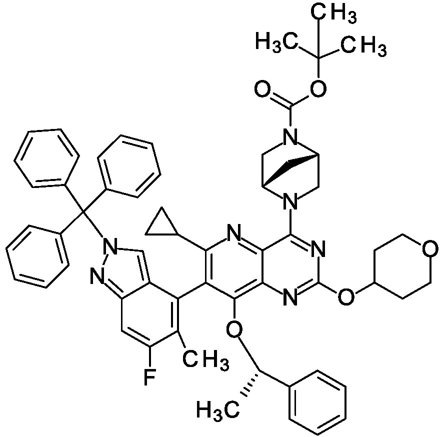 |

**[Table 5-66]**

| PEx | Str |
|---|---|
| 225 | 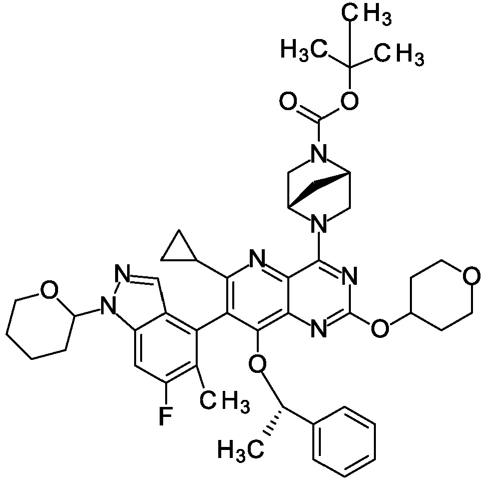 |
| 226 | 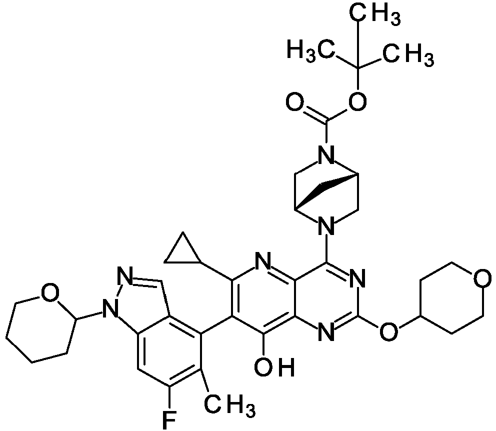 |

**[Table 5-67]**

| PEx | Str |
|---|---|
| 227 | 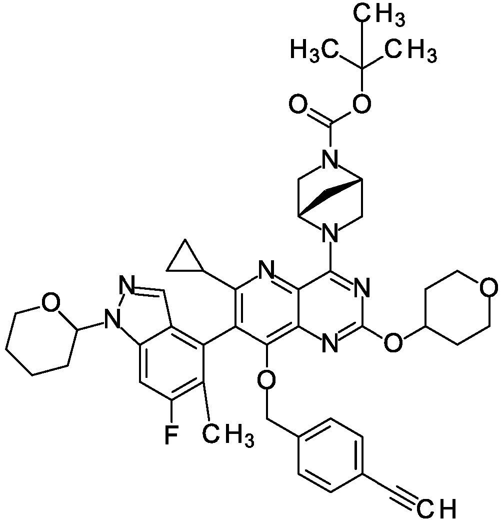 |
| 228 | 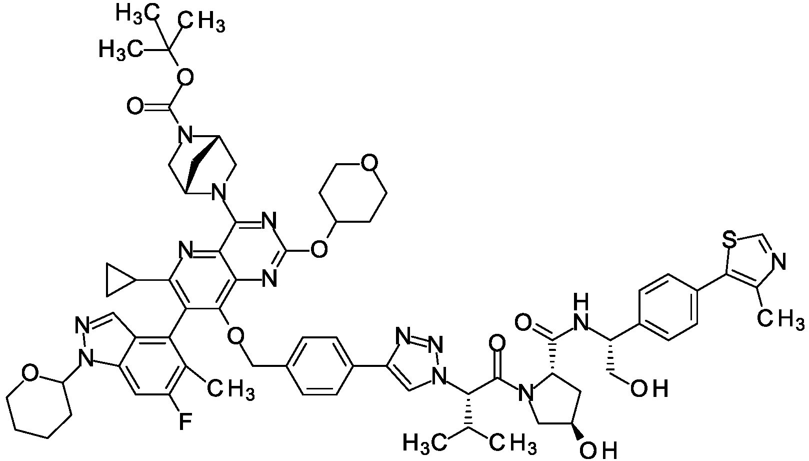 |

**[Table 6-1]**

| PEx | PSyn | DAT |
|---|---|---|
| 1 | 1 | ESI+: 402.9, 404.9, 406.9 |
| 2 | 2 | ESI+: 480.9, 482.8 [M+Na]+ |
| 3 | 3 | ESI+: 508.9 [M+Na]+ |
| 4 | 3 | ESI+: 613.3 |
| 5 | 5 | ESI+: 527.0 |
| 6 | 6 | ESI+: 587.2 |
| 7 | 6 | ESI+: 715.4 |
| 8 | 6 | ESI+: 629.1 |
| 9 | 9 | ESI+: 503.4 |
| 10 | 9 | ESI+: 541.3, 543.2 |
| 11 | 9 | ESI+: 613.2 |
| 12 | 9 | ESI+: 629.5 |
| 13 | 13 | ESI+: 813.4 |
| 14 | 14 | ESI+: 853.7 |
| | | NMR: 0.29-0.36 (1H, m), 0.54-0.62 (1H, m), 0.64-0.77 (2H, m), 0.87 (3H, d), 1.36-1.43 (1H, m), 1.63-1.72 (2H, m), 1.69 (9H, s), 1.77 (3H, s), 1.99-2.08 (2H, m), 3.27-3.35 (2H, m), 3.80-3.92 (2H, m), 5.06-5.15 (1H, m), 6.03 (1H, q), 6.84-6.88 (2H, m), 7.01-7.06 (6H, m), 7.17-7.23 (4H, m), 7.27-7.32 (9H, m), 7.36 (1H, d), 7.48 (1H, d) |
| 15 | 15 | ESI+: 925.3 |
| | | NMR: 0.29-0.37 (1H, m), 0.54-0.64 (1H, m), 0.64-0.77 (2H, m), 0.78-0.87 (3H, m), 1.22-1.46 (13H, m), 1.83 (3H, br s), 2.16-2.28 (2H, m), 3.07-3.26 (2H, m), 3.41-3,50 (2H, m), 3.55-3.62 (2H, m), 5.28 (1H, br s), 6.21-6.29 (1H, m), 6.82-6.89 (2H, m), 6.91 (1H, s), 7.00-7.08 (6H, m), 7.16-7.24 (4H, m), 7.25-7.33 (9H, m), 7.33-7.37 (1H, m), 7.48 (1H, d) |

**[Table 6-2]**

| PEx | PSyn | DAT |
|---|---|---|
| 16 | 13 | ESI+: 939.8 |
| 17 | 17 | ESI+: 867.7 [M+Na]+ |
| | | NMR: 0.43-0.50 (1H, m), 0.66-0.75 (1H, m), 0.77-0.89 (2H, m), 0.94 (3H, d), 1.25 (3H, t), 1.46-1.54 (1H, m), 1.75 (9H, s), 1.80 (3H, d), 3.47-3.54 (2H, m), 5.94 (1H, q), 6.90-6.94 (2H, m), 7.01-7.09 (6H, m), 7.16-7.24 (3H, m), 7.28-7.35 (9H, m), 7.35 (1H, s), 7.44 (1H, d), 7.52 (1H, d) |
| 18 | 17 | ESI+: 979.6 [M+Na]+ |
| 19 | 19 | ESI+: 603.6 |
| 20 | 20 | ESI+: 565.3 |
| 21 | 21 | ESI+: 687.7 |
| 22 | 21 | ESI+: 813.5 |
| 23 | 23 | ESI+: 605.4 [M+Na]+ |
| 24 | 23 | ESI+: 709.5 |
| 25 | 25 | ESI+: 705.5 |
| 26 | 23 | ESI+: 749.6 |
| 27 | 23 | ESI+: 875.4 [M+Na]+ |
| 28 | 23 | ESI+: 763.5 [M+Na]+ |
| 29 | 29 | ESI+: 697.3 |
| 30 | 30 | ESI+: 837.8 |
| 31 | 29 | ESI+: 823.6 |
| 32 | 29 | ESI+: 863.7 |
| 33 | 29 | ESI+: 989.6 [M+Na]+ |

**[Table 6-3]**

| PEx | PSyn | DAT |
|---|---|---|
| 34 | 29 | ESI+: 820.7 |
| 35 | 29 | ESI+: 877.6 [M+Na]+ |
| 36 | 36 | ESI+: 841.6 |
| 37 | 36 | ESI+: 823.7 |
| 38 | 36 | ESI+: 823.7 |
| 39 | 36 | ESI+: 807.7 |
| 40 | 36 | ESI+: 845.7 |
| 41 | 36 | ESI+: 841.7 |
| 42 | 36 | ESI+: 819.8 |
| 43 | 36 | ESI+: 803.6 |
| 44 | 36 | ESI+: 821.7 |
| 45 | 36 | ESI+: 807.8 |
| 46 | 36 | ESI+: 843.7 |
| 47 | 36 | ESI+: 825.7 |
| 48 | 36 | ESI+: 807.8 |
| 49 | 36 | ESI+: 837.7 |
| 50 | 36 | ESI+: 863.6 |
| 51 | 36 | ESI+: 963.7 |
| 52 | 36 | ESI+: 851.8 |
| 53 | 36 | ESI+: 876.8 |
| 54 | 54 | ESI+: 693.6 |
| 55 | 36 | ESI+: 863.4 |
| 56 | 36 | ESI+: 851.5 |
| 57 | 57 | ESI+: 705.5 |
| 58 | 58 | ESI+: 705.5 |
| 59 | 59 | ESI+: 637.4 |
| 60 | 60 | ESI+: 649.5 |
| 61 | 61 | ESI+: 649.5 |
| 62 | 62 | ESI+: 809.8 |
| 63 | 63 | ESI+: 819.8 |
| 64 | 62 | ESI+: 805.8 |
| 65 | 62 | ESI+: 806.6 |
| 66 | 62 | ESI+: 805.6 |
| 67 | 62 | ESI+: 831.7 |
| 68 | 62 | ESI+: 819.7 |
| 69 | 62 | ESI+: 844.8 |

**[Table 6-4]**

| PEx | PSyn | DAT |
|---|---|---|
| 70 | 62 | ESI+: 818.7 |
| 71 | 62 | ESI+: 831.7 |
| 72 | 62 | ESI+: 819.7 |
| 73 | 73 | ESI+: 819.7 |
| 74 | 74 | ESI+: 831.8 |
| 75 | 75 | ESI+: 831.8 |
| 76 | 62 | ESI+: 805.5 |
| 77 | 62 | ESI+: 833.4 |
| 78 | 62 | ESI+: 831.7 |
| 79 | 79 | ESI+: 805.5 |
| 80 | 62 | ESI+: 806.3 |
| 81 | 62 | ESI+: 804.4 |
| 82 | 62 | ESI+: 733.4 |
| 83 | 83 | ESI+: 1313.8 [M+Na]+ |
| 84 | 84 | ESI+: 1305.6 |
| 85 | 83 | ESI+: 1277.8 |
| 86 | 83 | ESI+: 1279.7 |
| 87 | 83 | ESI+: 1317.7 |
| 88 | 83 | ESI-: 1307.7 |
| 89 | 83 | ESI+: 1279.8 |
| 90 | 83 | ESI+: 1310.2 |
| 91 | 83 | ESI+: 1314.7 [M+Na]+ |
| 92 | 92 | ESI+: 1332.5 [M+Na]+ |
| 93 | 83 | ESI+: 1327.8 |
| 94 | 83 | ESI+: 1314.7 |
| 95 | 83 | ESI+: 1279.7 |
| 96 | 83 | ESI+: 1315.8 |
| 97 | 83 | ESI+: 1298.4 |
| 98 | 83 | ESI+: 1280.8 |
| 99 | 83 | ESI+: 1276.5 |
| 100 | 83 | ESI+: 1293.7 |
| 101 | 83 | ESI+: 1313.7 [M+Na]+ |
| 102 | 83 | ESI+: 1279.3 |
| 103 | 83 | ESI+: 1304.6 |
| 104 | 83 | ESI+: 1101.8 |
| 105 | 83 | ESI+: 1101.8 |

**[Table 6-5]**

| PEx | PSyn | DAT |
|---|---|---|
| 106 | 83 | ESI+: 1457.6 [M+Na]+ |
| 107 | 83 | ESI+: 1328.1 [M+Na]+ |
| 108 | 83 | ESI+: 1292.9 |
| 109 | 83 | ESI+: 1318.0 |
| 110 | 83 | ESI+: 1313.0 [M+Na]+ |
| 111 | 83 | ESI+: 1089.8 |
| 112 | 83 | ESI+: 1304.3 |
| 113 | 83 | ESI+: 1314.8 [M+Na]+ |
| 114 | 114 | ESI+: 1312.0 [M+Na]+ |
| 115 | 115 | ESI+: 1303.3 |
| 116 | 116 | ESI+: 1291.9 |
| 117 | 117 | ESI+: 1304.2 |
| 118 | 83 | ESI+: 1277.8 |
| 119 | 83 | ESI+: 1277.2 |
| 120 | 83 | ESI+: 1306.5 |
| 121 | 83 | ESI+: 1326.0 [M+Na]+ |
| 122 | 83 | ESI+: 1277.4 |
| 123 | 83 | ESI+: 1278.3 |
| 124 | 124 | ESI+: 1205.4 |
| 125 | 125 | ESI+: 335.2 |
| 126 | 126 | ESI+: 235.2 |
| 127 | 126 | ESI+: 205.3 |
| 128 | 126 | ESI+: 233.3 |
| 129 | 126 | ESI+: 218.2 |
| 130 | 126 | ESI+: 219.1 |
| 131 | 131 | ESI+: 232.2 |
| 132 | 126 | ESI+: 245.1 [M+Na]+ |
| 133 | 133 | ESI+: 448.3 |
| 134 | 134 | ESI+: 445.3 |
| 135 | 135 | ESI+: 348.2 |
| 136 | 136 | ESI+: 345.2 |
| 137 | 137 | ESI+: 547.4 |
| 138 | 138 | ESI+: 544.3 |
| 139 | 139 | ESI+: 447.3 |
| 140 | 140 | ESI+: 444.3 |
| 141 | 141 | ESI+: 473.3 |

**[Table 6-6]**

| PEx | PSyn | DAT |
|---|---|---|
| 142 | 142 | ESI+: 470.3 |
| 143 | 143 | ESI+: 833.6 |
| 144 | 144 | ESI+: 332.2 |
| 145 | 144 | ESI+: 333.4 |
| 146 | 146 | ESI+: 1076.1 |
| 147 | 146 | ESI+: 1087.8 |
| 148 | 146 | ESI+: 1087.8 |
| 149 | 149 | ESI+: 1213.5 |
| 150 | 149 | ESI+: 1262.7 |
| 151 | 149 | ESI+: 1286.8 |
| 152 | 149 | ESI+: 1287.7 |
| 153 | 149 | ESI+: 1301.6 |
| 154 | 149 | ESI+: 1323.7 [M+Na]+ |
| 155 | 149 | ESI+: 1286.9 |
| 156 | 149 | ESI+: 1288.6 |
| 157 | 149 | ESI+: 1300.9 |
| 158 | 149 | ESI+: 1290.2 |
| 159 | 149 | ESI+: 1297.0 [M+Na]+ |
| 160 | 149 | ESI+: 1280.3 |
| 161 | 149 | ESI+: 1276.2 |
| 162 | 149 | ESI+: 1273.7 |
| 163 | 163 | ESI+: 627.5 |
| 164 | 163 | ESI+: 623.5 |
| 165 | 163 | ESI+: 649.6 |
| 166 | 163 | ESI+: 637.3 |
| 167 | 163 | ESI+: 662.5 |
| 168 | 163 | ESI+: 649.3 |
| 169 | 163 | ESI+: 637.5 |
| 170 | 163 | ESI+: 649.4 |
| 171 | 171 | ESI+: 585.2, 587.1 |

**[Table 6-7]**

| PEx | PSyn | DAT |
|---|---|---|
| 172 | 172 | ESI+: 729.5 NMR: 0.59-0.68 (1H, m), 0.69-0.82 (3H, m), 1.03-1.08 (3H, m), 1.27 (3H, m), 1.34-1.42 (1H, m), 1.44 (9H, s), 1.70 (3H, d), 2.23-2.35 (2H, m), 3.32-3.43 (5H, m), 3.47-3.59 (3H, m), 3.73-3.89 (1H, m), 5.07-5.15 (1H, m), 5.83 (1H, q), 6.78-6.83 (2H, m), 7.10-7.16 (2H, m), 7.16-7.21 (1H, m), 7.39 (1H, d), 7.41 (1H, s), 7.54 (1H, s), 13.09 (1H, br s) |
| 173 | 173 | NMR: 7.08-7.15 (6H, m), 7.36-7.44 (10H, m), 7.49-7.54 (1H, m), 7.89 (1H, d) |
| 174 | 174 | NMR: 2.34 (3H, d), 7.07-7.13 (6H, m), 7.36-7.43 (9H, m), 7.51 (1H, d), 7.79 (1H, d) |
| 175 | 175 | NMR: 1.21 (12H, s), 2.44 (3H, d), 7.04-7.11 (6H, m), 7.34-7.44 (9H, m), 7.49 (1H, d), 8.09 (1H, d) |
| 176 | 176 | EI: 168.0 [M]+ NMR: 4.54 (1H, s), 4.79 (2H, s), 7.31 (1H, dd), 7.41 (1H, dd), 7.57 (1H, t) |
| 177 | 177 | ESI+: 417.9 |
| 178 | 178 | ESI+: 386.0 |
| 179 | 179 | ESI+: 419.8, 421.9, 423.9 |
| 180 | 180 | ESI+: 445.9, 447.9 |
| 181 | 181 | ESI+: 548.0, 550.0 |
| 182 | 182 | ESI+: 699.2, 701.2 |
| 183 | 183 | ESI+: 378.2 [M+Na]+ |
| 184 | 184 | ESI+: 385.2 [M+Na]+ |
| 185 | 185 | ESI+: 358.3 |
| 186 | 185 | ESI+: 345.3 |
| 187 | 187 | ESI+: 359.2 |
| 188 | 188 | ESI+: 833.8 |
| 189 | 188 | ESI+: 837.8 |
| 190 | 188 | ESI+: 855.7 |

**[Table 6-8]**

| PEx | PSyn | DAT |
|---|---|---|
| 191 | 188 | ESI+: 837.7 |
| 192 | 192 | ESI+: 485.9, 487.9, 489.8 |
| 193 | 181 | ESI+: 588.0 |
| 194 | 182 | ESI+: 741.1 |
| 195 | 9 | ESI+: 655.2 |
| 196 | 13 | ESI+: 965.3 |
| 197 | 23 | ESI+: 861.4 |
| 198 | 29 | ESI+: 975.4 |
| 199 | 19 | ESI+: 733.4 |
| 200 | 124 | ESI+: 1205.8 |
| 201 | 139 | ESI+: 245.2 |
| 202 | 141 | ESI+: 271.2 |

**[Table 6-9]**

| PEx | PSyn | DAT |
|---|---|---|
| 203 | 203 | ESI+: 474.0 |
| 204 | 204 | ESI+: 598.1, 600.2 [M+Na]+ |
| 205 | 205 | ESI+: 740.3 |
| 206 | 206 | ESI+: 728.2 |
| 207 | 207 | ESI+: 740.2 |
| 208 | 9 | ESI+: 654.4 |
| 209 | 9 | ESI+: 656.6 |
| 210 | 210 | ESI+: 964.7 |
| 211 | 210 | ESI+: 966.8 |
| 212 | 23 | ESI+: 860.6 |
| 213 | 23 | ESI+: 862.7 |
| 214 | 29 | ESI+: 974.7 |
| 215 | 29 | ESI+: 976.8 |
| 216 | 117 | ESI+: 1446.7 |
| 217 | 117 | ESI+: 1448.7 |

**[Table 6-10]**

| PEx | PSyn | DAT |
|---|---|---|
| 218 | 218 | ESI+: 252.1 |
| 219 | 219 | ESI+: 220.1 |
| 220 | 220 | ESI+: 298.0 |
| 221 | 221 | ESI+: 514.2, 516.2 |
| 222 | 222 | ESI+: 580.3, 582.2 |
| 223 | 6 | ESI+: 668.5 |
| 224 | 13 | ESI+: 978.6 |
| 225 | 225 | ESI+: 820.6 |
| 226 | 23 | ESI+: 716.5 |
| 227 | 29 | ESI+: 830.6 |
| 228 | 117 | ESI+: 1324.8 [M+Na]+ |

**[Table 7-1]**

| Ex | Str |
|---|---|
| 1 | 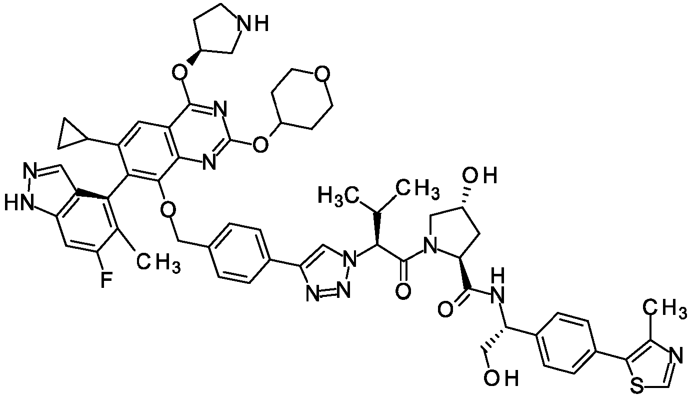 |
| 2 | 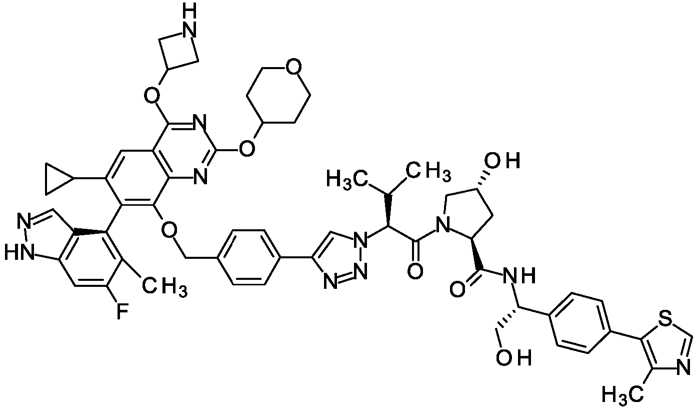 |
| 3 | 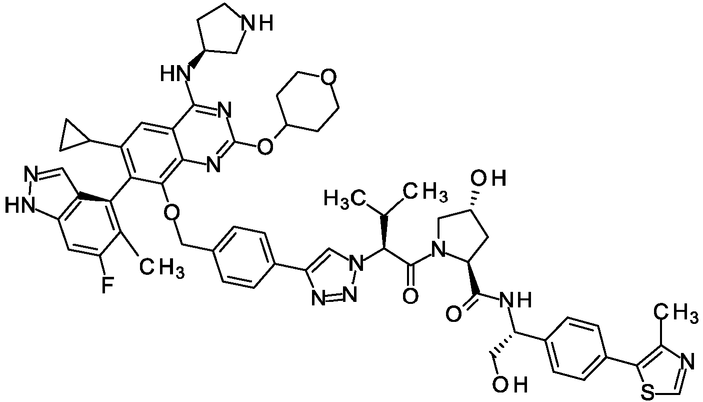 |

**[Table 7-2]**

| Ex | Str |
|---|---|
| 4 | 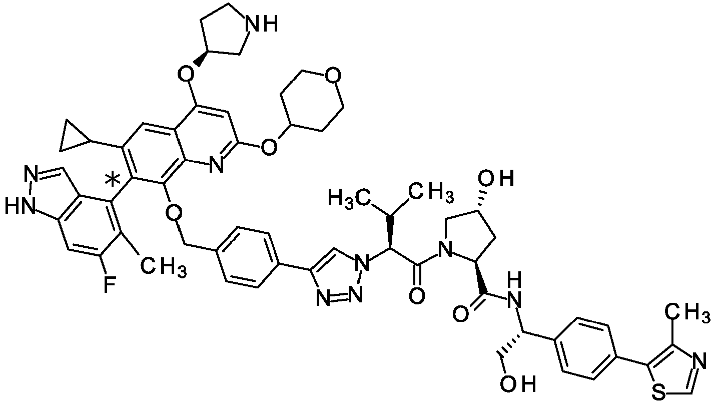 |
| 5 | 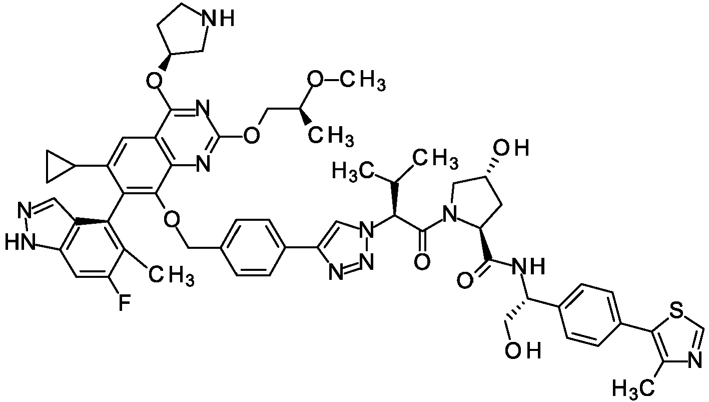 |
| 6 | 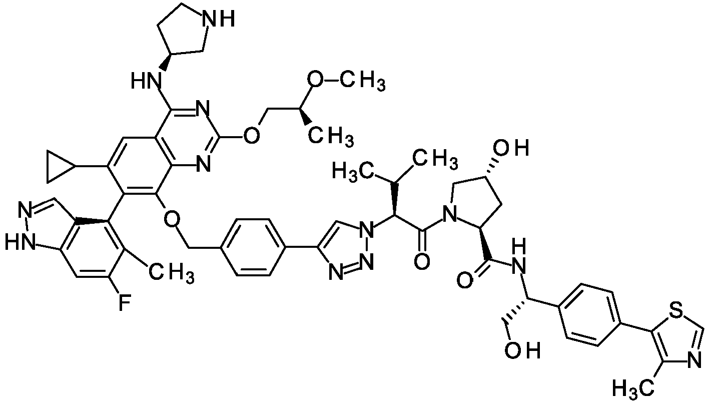 |

**[Table 7-3]**

| Ex | Str |
|---|---|
| 7 | 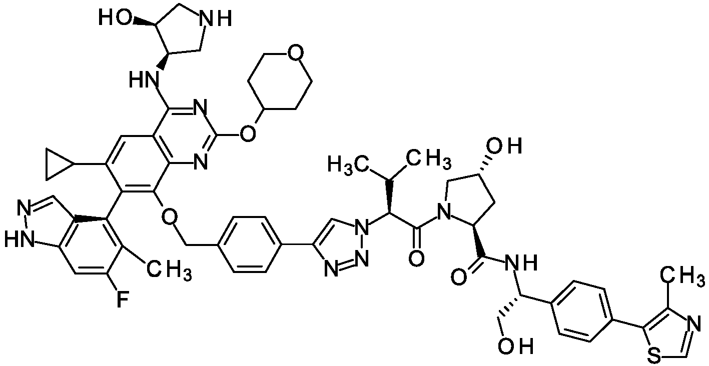 |
| 8 | 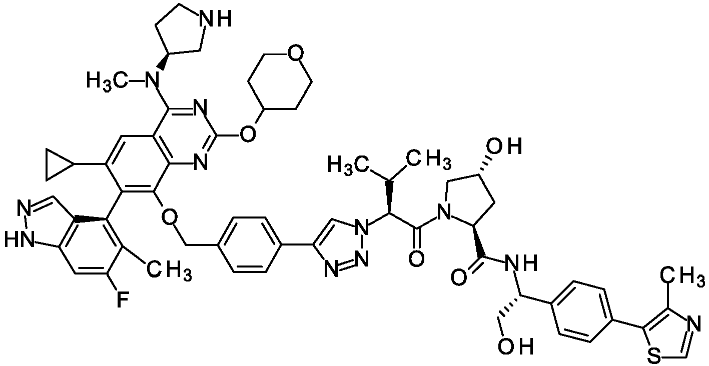 |
| 9 | 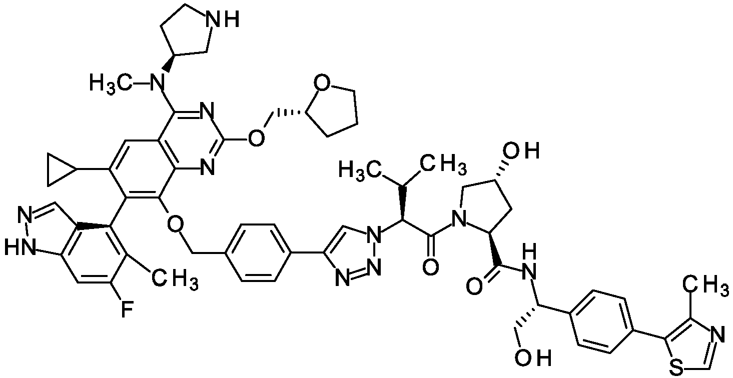 |

**[Table 7-4]**

| Ex | Str |
|---|---|
| 10 | 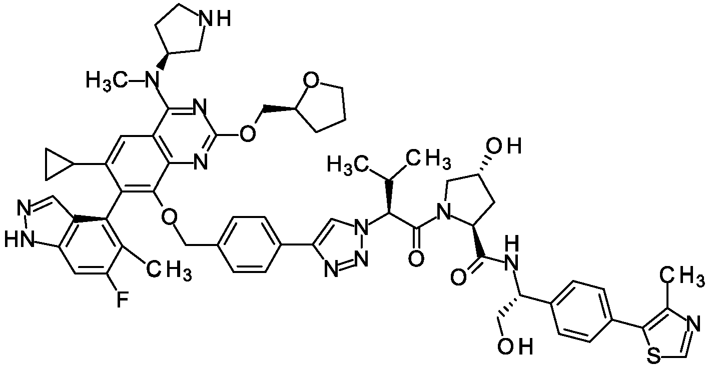 |
| 11 | 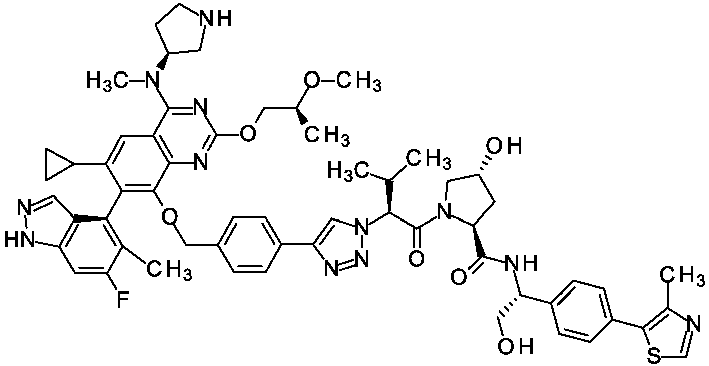 |
| 12 | 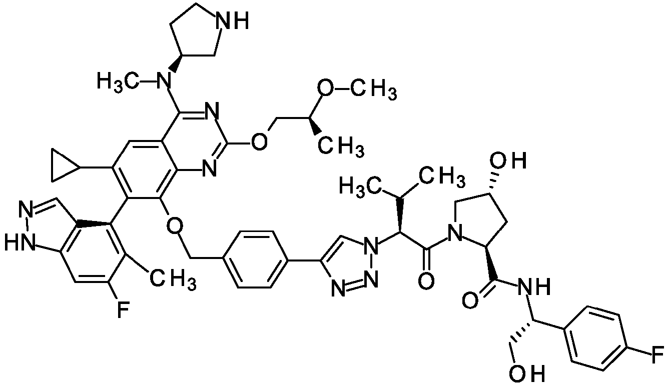 |

**[Table 7-5]**

| Ex | Str |
|---|---|
| 13 | 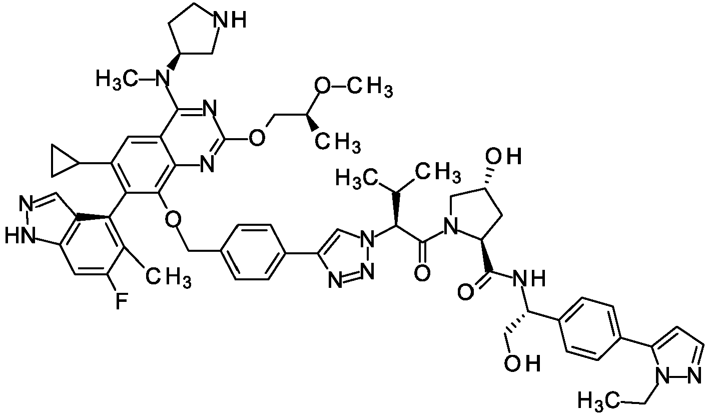 |
| 14 | 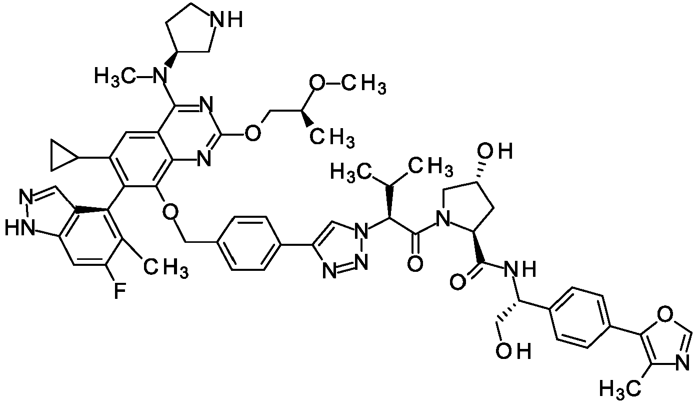 |
| 15 | 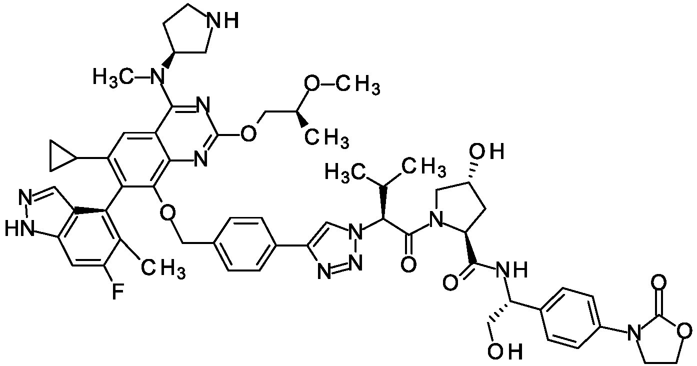 |

**[Table 7-6]**

| Ex | Str |
|---|---|
| 16 | 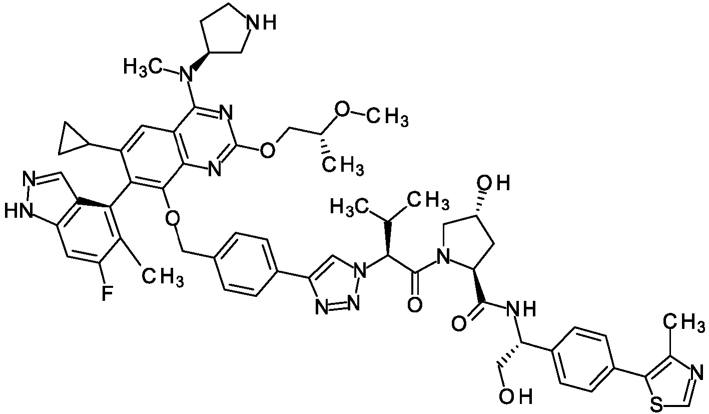 |
| 17 | 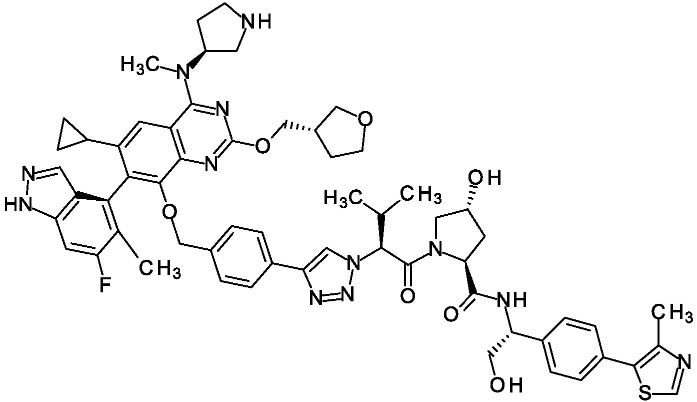 |
| 18 | 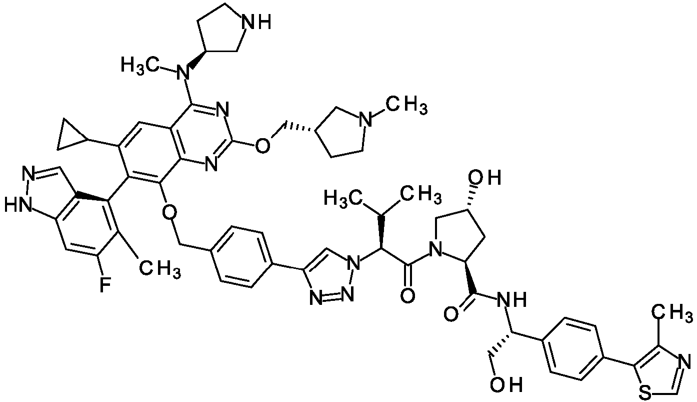 |

**[Table 7-7]**

| Ex | Str |
|---|---|
| 19 | 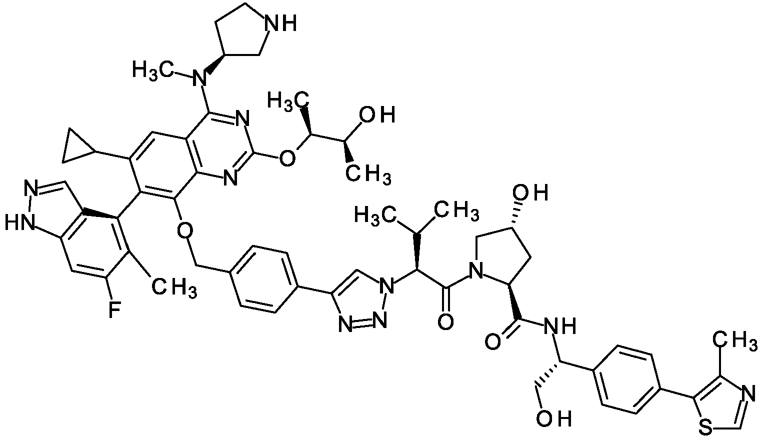 |
| 20 | 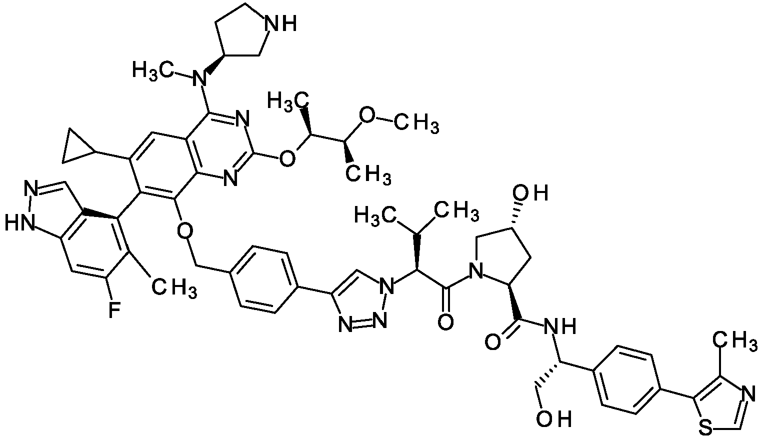 |
| 21 | 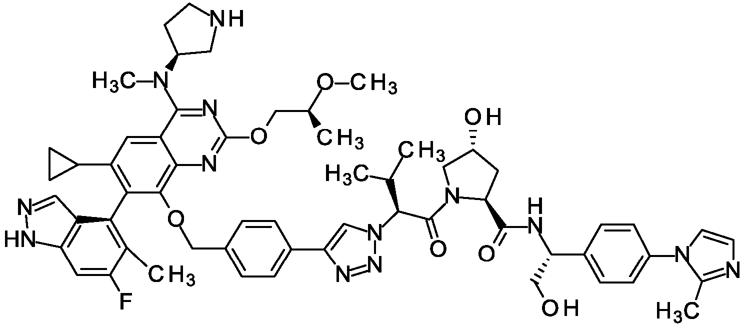 |

**[Table 7-8]**

| Ex | Str |
|---|---|
| 22 | 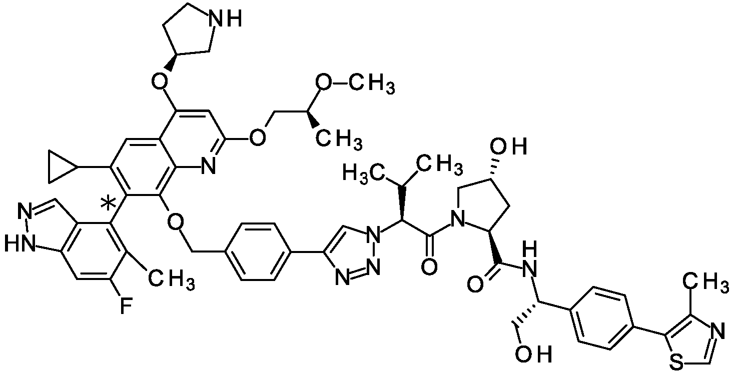 |
| 23 | 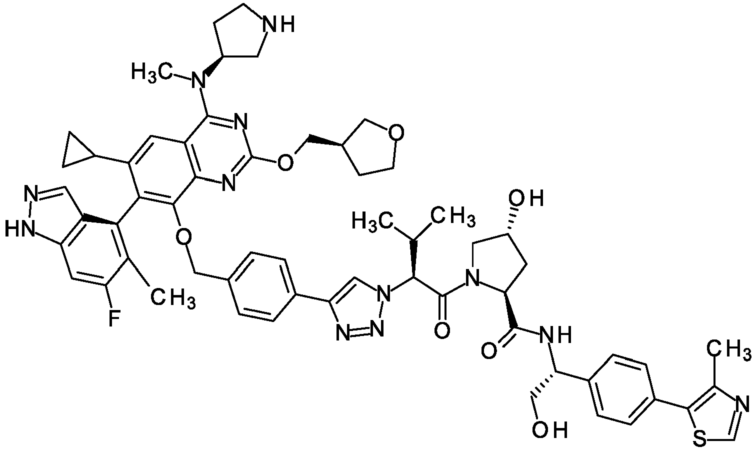 |
| 24 | 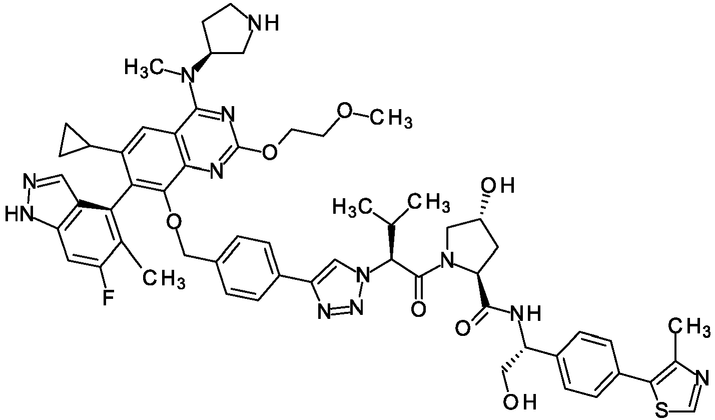 |

**[Table 7-9]**

| Ex | Str |
|---|---|
| 25 | 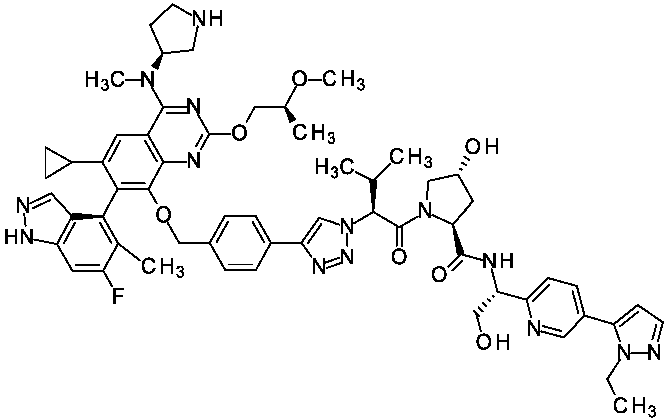 |
| 26 | 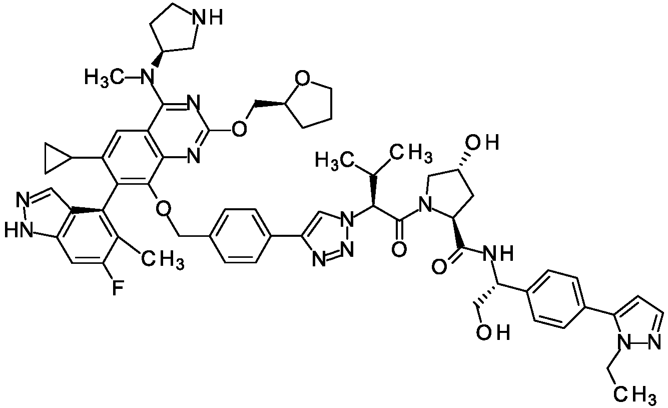 |
| 27 | 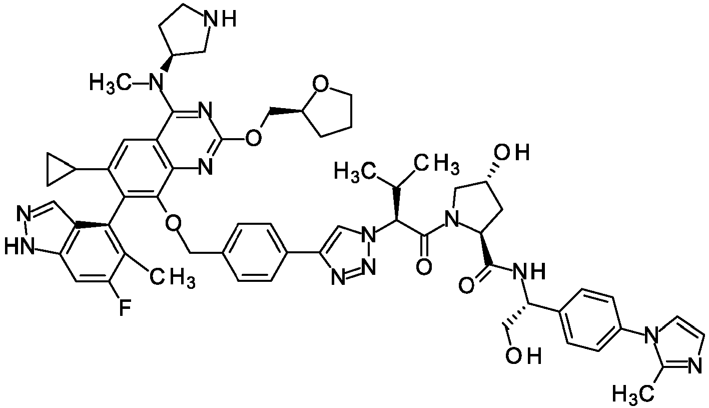 |

**[Table 7-10]**

| Ex | Str |
|---|---|
| 28 | 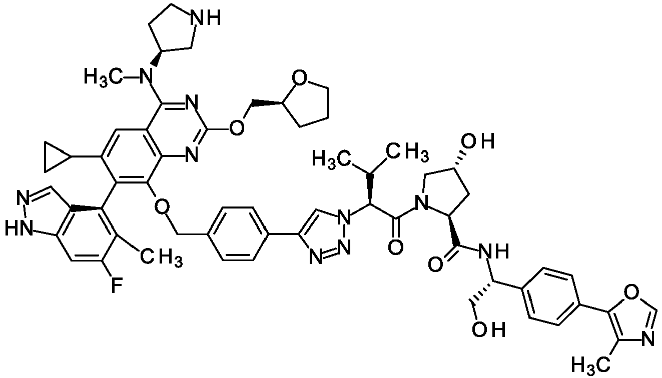 |
| 29 | 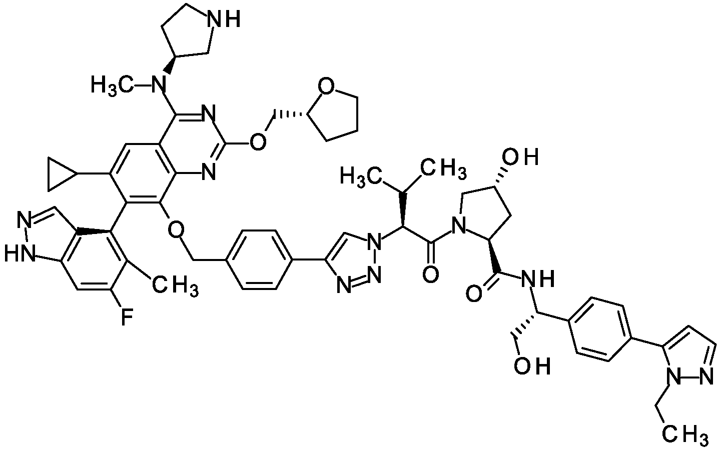 |
| 30 | 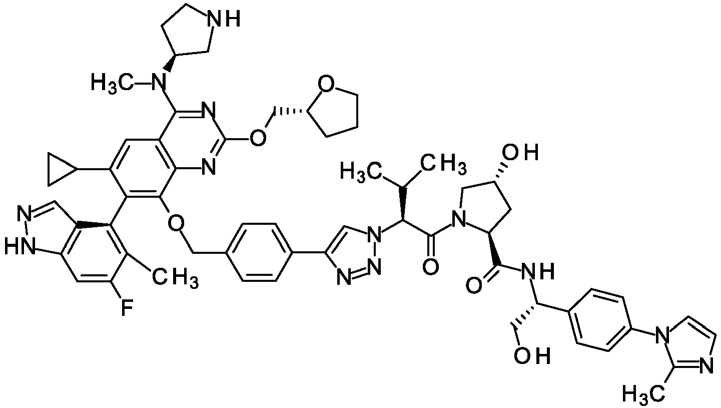 |

**[Table 7-11]**

| Ex | Str |
|---|---|
| 31 | 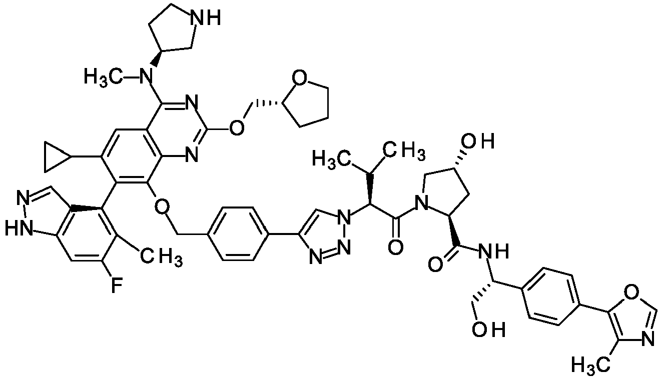 |
| 32 | 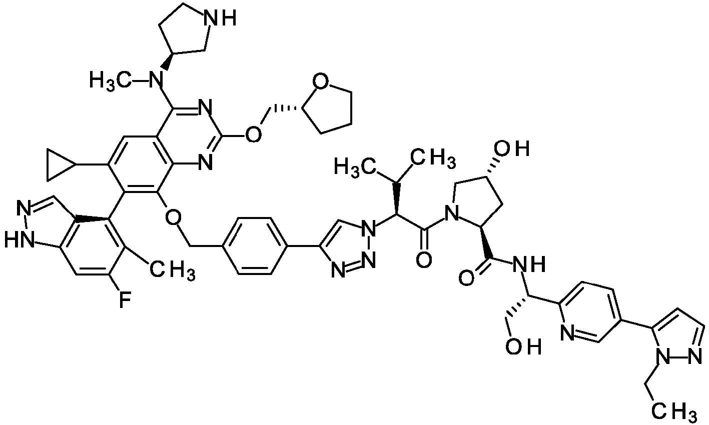 |
| 33 | 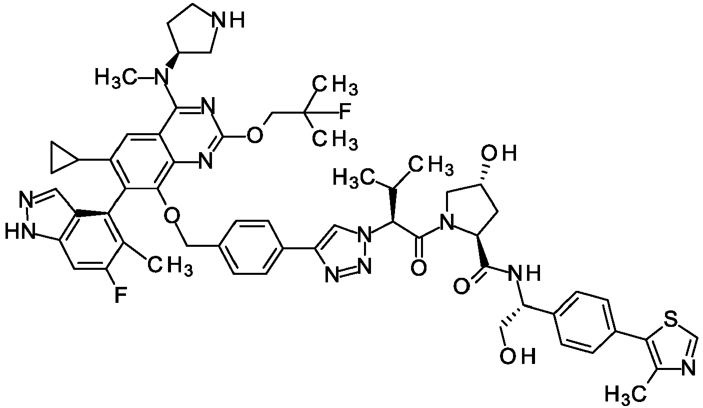 |

**[Table 7-12]**

| Ex | Str |
|---|---|
| 34 | 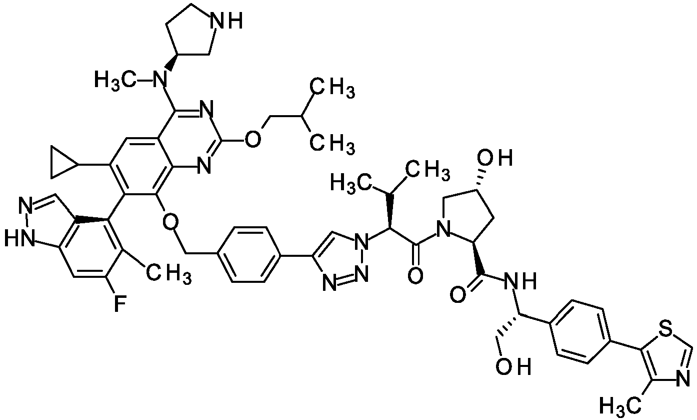 |
| 35 | 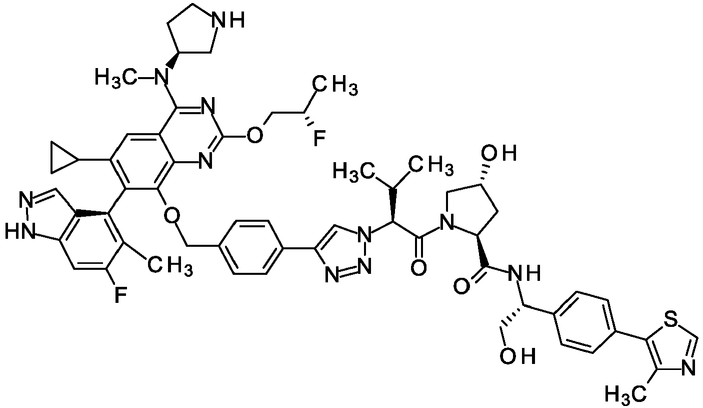 |
| 36 | 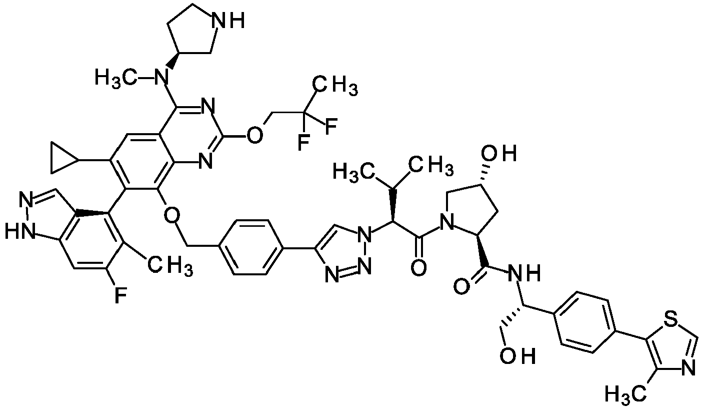 |

**[Table 7-13]**

| Ex | Str |
|---|---|
| 37 | 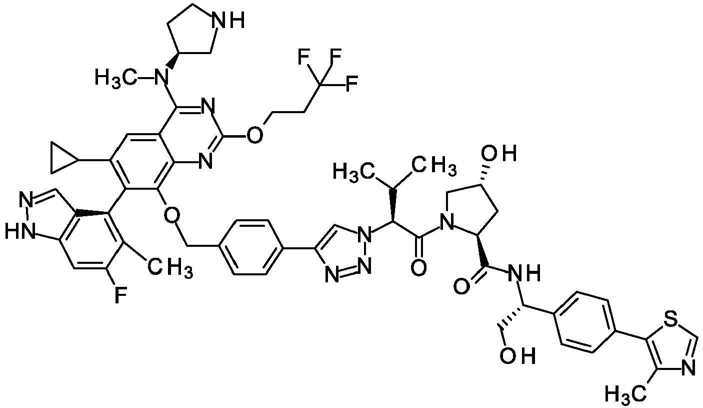 |
| 38 | 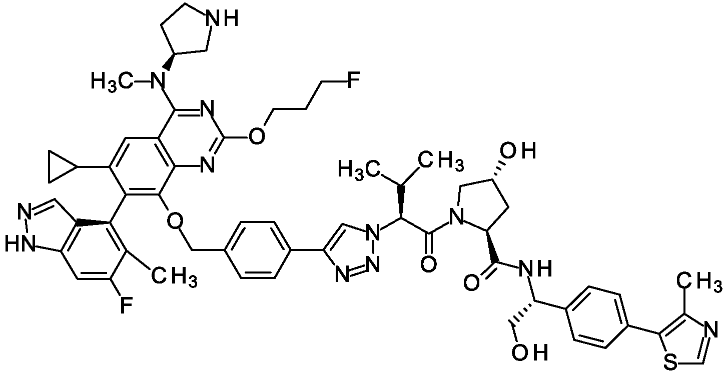 |
| 39 | 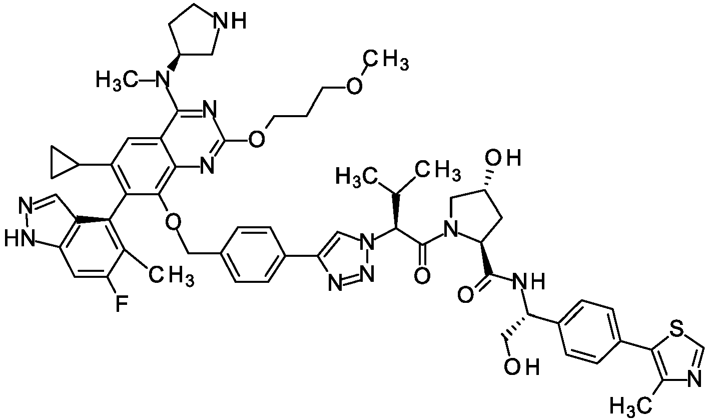 |

**[Table 7-14]**

| Ex | Str |
|---|---|
| 40 | 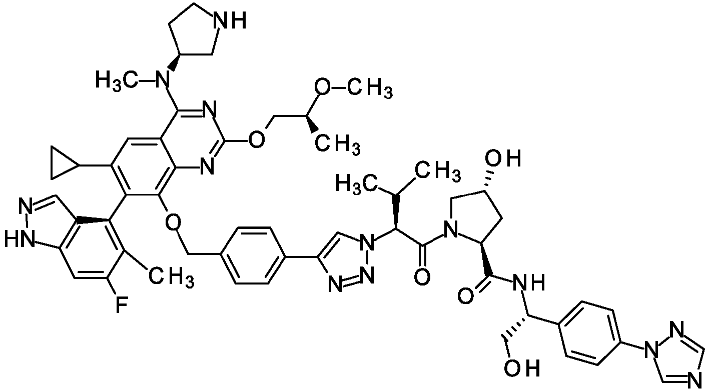 |
| 41 | 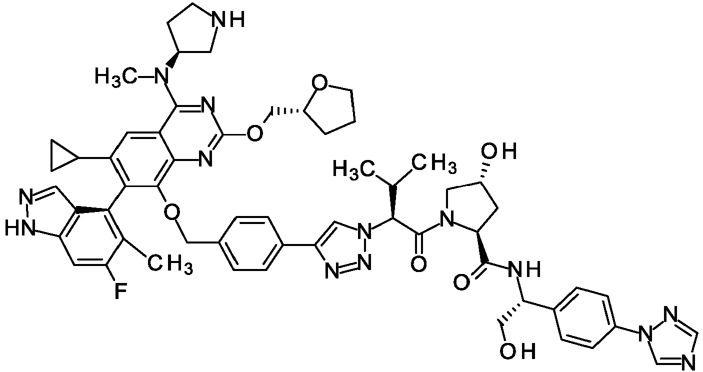 |
| 42 | 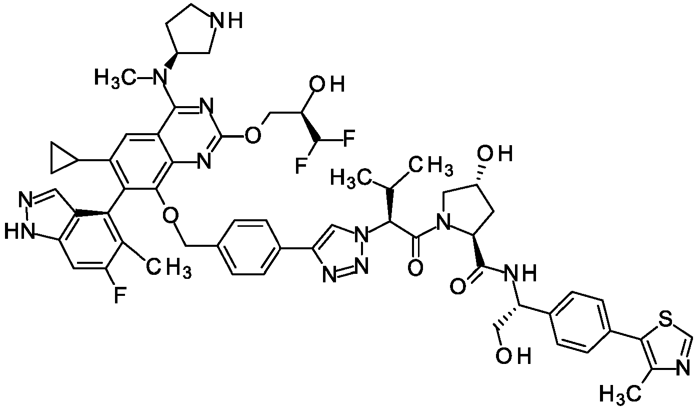 |

**[Table 7-15]**

| Ex | Str |
|---|---|
| 43 | 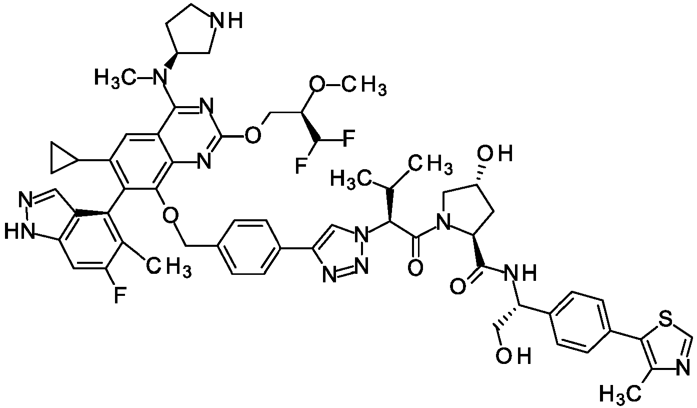 |
| 44 | 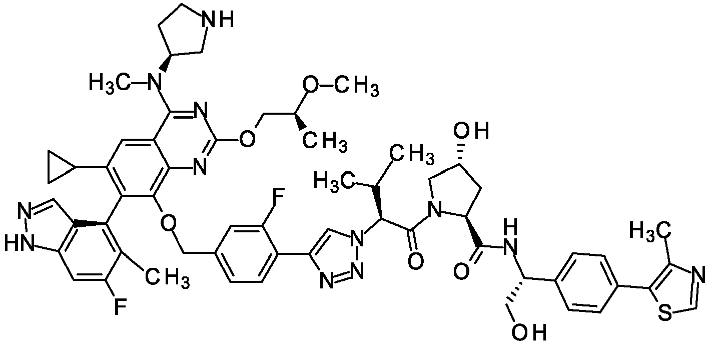 |
| 45 | 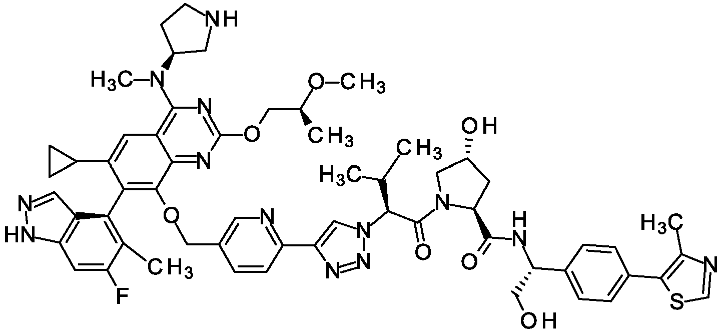 |

**[Table 7-16]**

| Ex | Str |
|---|---|
| 46 | 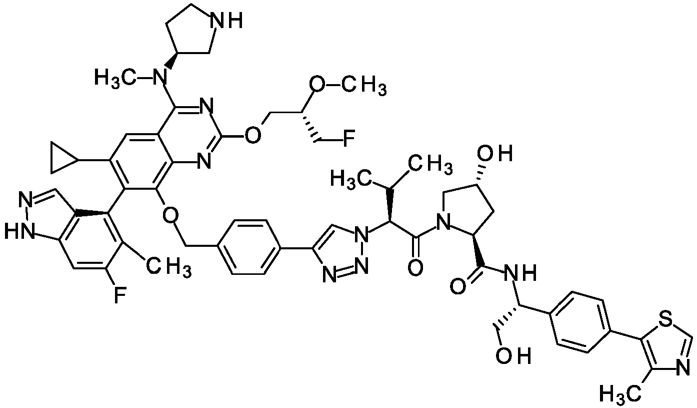 |
| 47 | 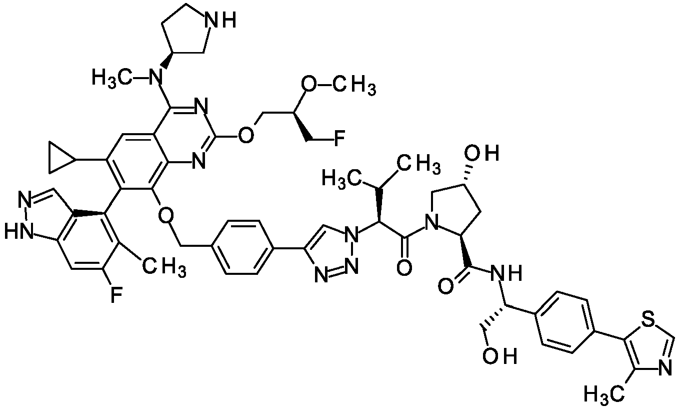 |
| 48 | 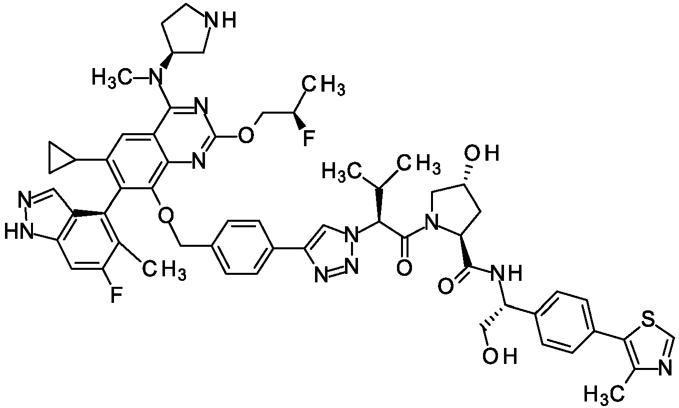 |

**[Table 7-17]**

| Ex | Str |
|---|---|
| 49 | 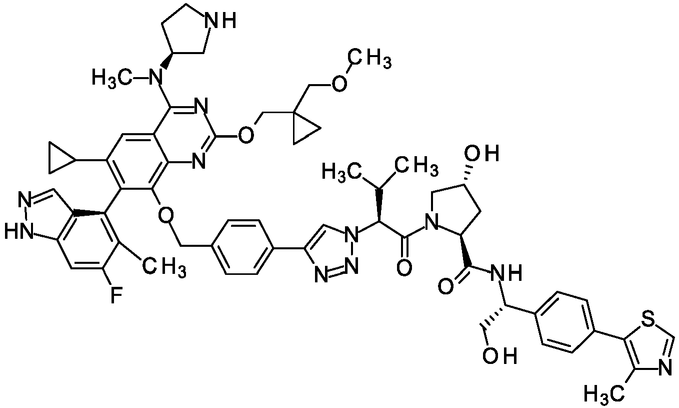 |
| 50 | 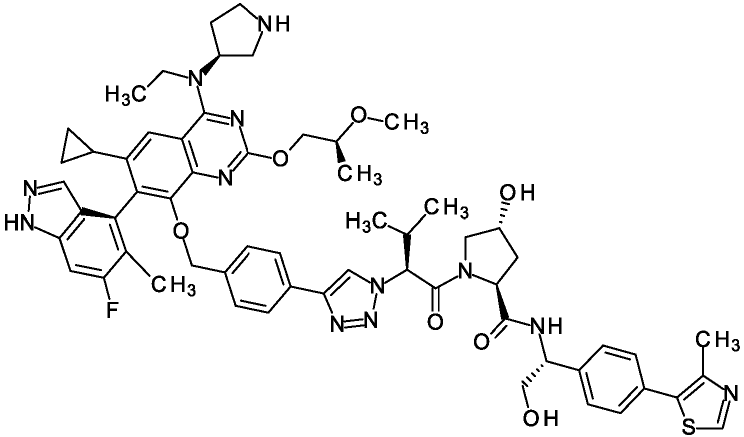 |
| 51 | 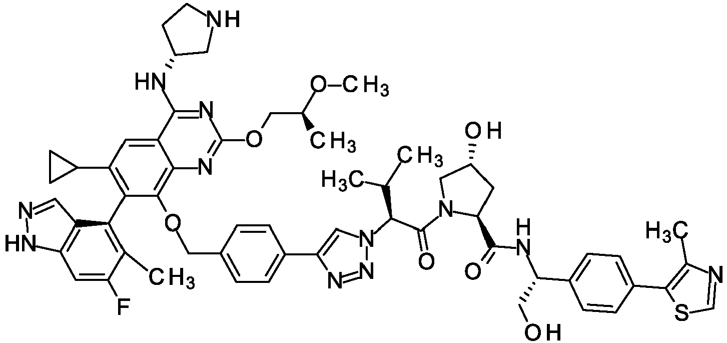 |

**[Table 7-18]**

| Ex | Str |
|---|---|
| 52 | 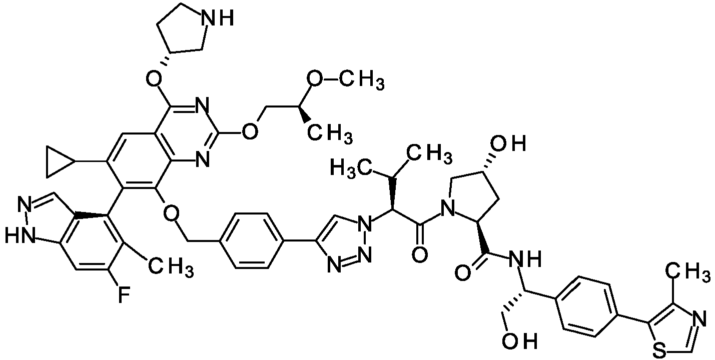 |
| 53 | 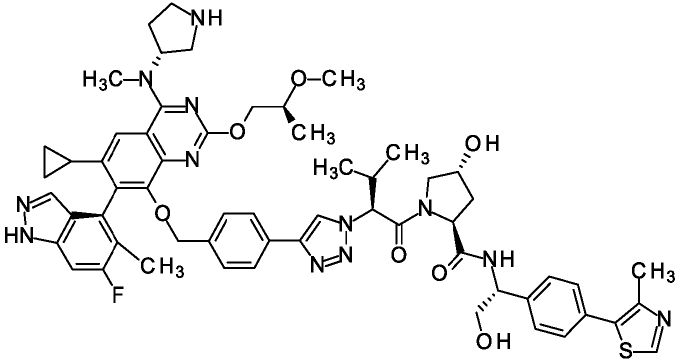 |
| 54 | 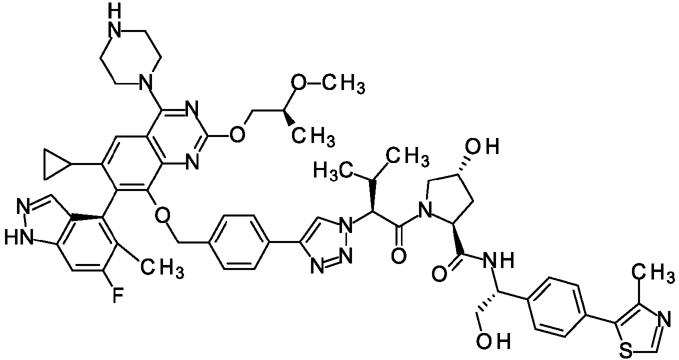 |

**[Table 7-19]**

| Ex | Str |
|---|---|
| 55 | 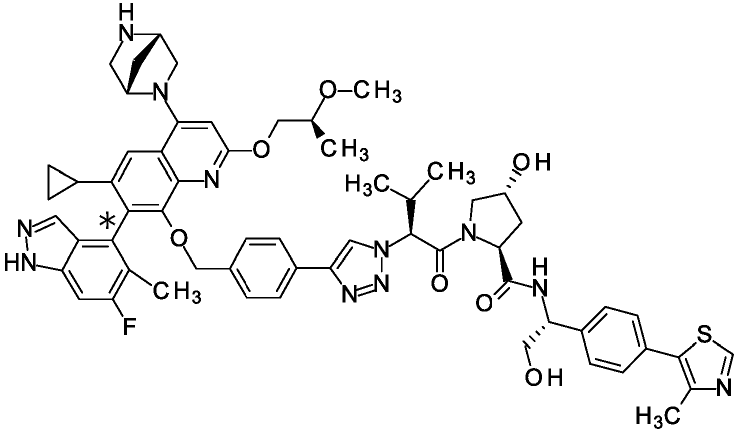 |
| 56 | 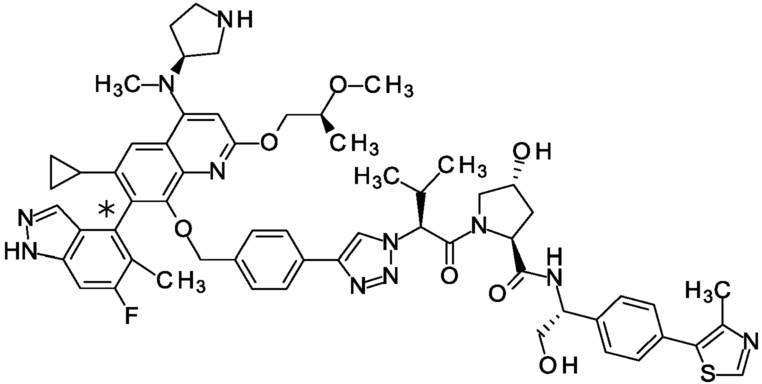 |

**[Table 7-20]**

| Ex | Str |
|---|---|
| 57 | 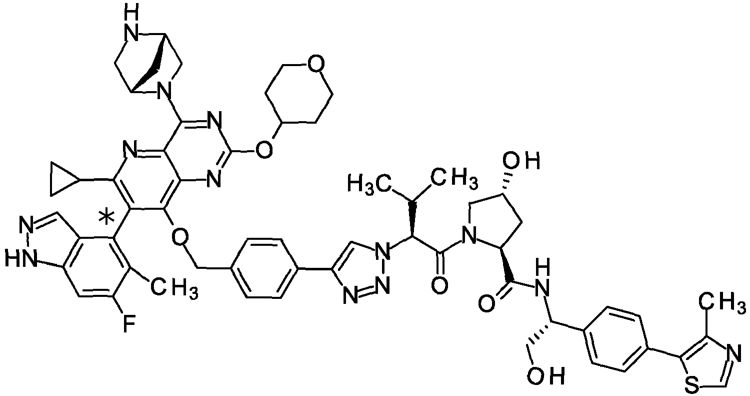 |

**[Table 8-1]**

| Ex | Syn | DAT |
|---|---|---|
| 1 | 9 | ESI+: 1128.7 [M+Na]+ |
| 2 | 2 | ESI+: 1114.8 [M+Na]+ |
| 3 | 9 | ESI+: 1105.4 |
| 4 | 9 | ESI+: 1105.4 |
| 5 | 9 | ESI+: 1095.6 |
| 6 | 9 | ESI+: 1093.8 |
| 7 | 9 | ESI+: 1121.7 |
| 8 | 9 | ESI+: 1119.2 |
| 9 | 9 | ESI+: 1119.7 |
| | | NMR (100°C): 0.51-0.70 (4H, m), 0.77 (3H, br d), 1.07 (3H, d), 1.36-1.45 (1H, m), 1.59-1.68 (1H, m), 1.75-1.89 (2H, m), 1.89-2.00 (3H, m), 2.01 (3H, d), 2.01-2.09 (1H, m), 2.09-2.18 (1H, m), 2.20-2.35 (1H, m), 2.45 (3H, s), 2.48-2.60 (1H, m), 2.86 (1H, dt), 2.97 (1H, dd), 3.03 (1H, ddd), 3.18 (1H, dd), 3.25 (3H, s), 3.60 (1H, br d), 3.63-3.73 (3H, m), 3.75-3.81 (1H, m), 3.84 (1H, br dd), 4.16-4.23 (1H, m), 4.29-4.39 (3H, m), 4.41-4.48 (1H, m), 4.52 (1H, br t), 4.80 (1H, br d), 4.86-4.95 (3H, m), 5.23 (1H, d), 5.28 (1H, br d), 6.86(2H, d), 7.30 (1H, d), 7.38-7.44 (5H, m), 7.46 (1H, d), 7.60 (2H, br d), 8.00 (1H, br d), 8.42 (1H, s), 8.88 (1H, s), 12.74 (1H, br s) |
| 10 | 10 | ESI+: 1119.8 |
| 11 | 11 | ESI+: 1107.6 |
| | | NMR (100°C): 0.50-0.57 (1H, m), 0.57-0.70 (3H, m), 0.77 (3H, d), 1.06 (3H, d), 1.15 (3H, d), 1.37-1.45 (1H, m), 1.89-2.00 (2H, m), 2.01 (3H, d), 2.01-2.09 (1H, m), 2.09-2.17 (1H, m), 2.20-2.34 (1H, m), 2.45 (3H, s), 2.50-2.60 (1H, m), 2.83-2.90 (1H, m), 2.97 (1H, dd), 3.03 (1H, ddd), 3.18 (1H, dd), 3.25 (3H, s), 3.30 (3H, s), 3.56-3.64 (1H, m), 3.64-3.75 (3H, m), 3.83 (1H, dd), 4.29 (1H, dd), 4.33-4.39 (2H, m), 4.41-4.47 (1H, m), 4.52 (1H, br t), 4.77-4.83 (1H, m), 4.86-4.97 (3H, m), 5.25 (1H, d), 5.28 (1H, d), 6.84 (2H, d), 7.30 (1H, d), 7.38-7.44 (5H, m), 7.46 (1H, d), 7.60 (2H, d), 8.00 (1H, br d), 8.42 (1H, s), 8.88 (1H, s), 12.75 (1H, br s) |

**[Table 8-2]**

| Ex | Syn | DAT |
|---|---|---|
| 12 | 9 | ESI+: 1028.8 |
| 13 | 13 | ESI+: 1105.0 |
| | | NMR(100°C): 0.51-0.71 (4H, m), 0.77 (3H, d), 1.07 (3H, d), 1.16 (3H, d), 1.30 (3H, t), 1.38-1.45 (1H, m), 1.87-1.97 (1H, m), 1.98-2.15 (2H, m), 2.01 (3H, d), 2.22-2.31 (1H, m), 2.50-2.60 (1H, m), 3.04-3.11 (1H, m), 3.15 (1H, dd), 3.22-3.32 (2H, m), 3.28 (3H, s), 3.30 (3H, s), 3.42 (1H, dd), 3.60 (1H, br d), 3.66-3.76 (3H, m), 3.84(1H, br dd), 4.11(2H, q), 4.29-4.39 (3H, m), 4.46 (1H, br s), 4.52 (1H,br t), 4.81 (1H, br s), 4.88-4.94 (2H, m), 4.94-5.01 (1H, m), 5.25 (1H, d), 5.29 (1H, br d), 6.26(1H, d), 6.85(2H, d), 7.31 (1H, d), 7.35-7.39 (2H, m), 7.40-7.46 (5H, m), 7.60 (2H, br d), 8.01 (1H, br d), 8.42 (1H, s), 12.76 (1H, br s) |
| 14 | 9 | ESI+: 1091.9 |
| 15 | 9 | ESI+: 1095.4 |
| 16 | 9 | ESI+: 1107.2 |
| 17 | 9 | ESI+: 1119.4 |
| 18 | 9 | ESI+: 1132.8 |
| 19 | 9 | ESI+: 1107.9 |
| 20 | 9 | ESI+: 1121.8 |
| 21 | 9 | ESI+: 1090.9 |
| 22 | 9 | ESI+: 1093.8 |
| 23 | 9 | ESI+: 1119.7 |
| 24 | 9 | ESI+: 1093.8 |
| 25 | 9 | ESI+: 1105.7 |
| 26 | 9 | ESI-: 1114.6 |
| 27 | 9 | ESI+: 1102.9 |
| 28 | 9 | ESI+: 1103.9 |
| 29 | 9 | ESI+: 1116.9 |
| 30 | 9 | ESI+: 1102.8 |
| 31 | 9 | ESI+: 1104.0 |
| 32 | 9 | ESI+: 1117.7 |

**[Table 8-3]**

| Ex | Syn | DAT |
|---|---|---|
| 33 | 9 | ESI+: 1110.5 |
| 34 | 9 | ESI+: 1091.7 |
| 35 | 9 | ESI+: 1095.8 |
| 36 | 9 | ESI+: 1113.8 |
| 37 | 9 | ESI+: 1131.6 |
| 38 | 9 | ESI+: 1095.7 |
| 39 | 9 | ESI+: 1108.7 |
| 40 | 9 | ESI+: 1077.7 |
| 41 | 9 | ESI+: 1089.7 |
| 42 | 9 | ESI+: 1129.6 |
| 43 | 9 | ESI+: 1143.8 |
| 44 | 44 | ESI+: 1125.6 |
| | | NMR (100°C): 0.51-0.70 (4H, m), 0.77 (3H, d), 1.07 (3H, d), 1.14 (3H, d), 1.37-1.45 (1H, m), 1.89-1.99 (2H, m), 2.00-2.09 (1H, m), 2.01 (3H, d), 2.09-2.17 (1H, m), 2.22-2.35 (1H, m), 2.45 (3H, s), 2.48-2.58 (1H, m), 2.83-2.99 (2H, m), 3.00-3.06 (1H, ddd), 3.18 (1H, dd), 3.25 (3H, s), 3.30 (3H, s), 3.60 (1H, br d), 3.65-3.75 (3H, m), 3.83 (1H, dd), 4.25-4.39 (3H, m), 4.44-4.50 (1H, m), 4.53 (1H, t), 4.74-4.84 (1H, m), 4.87-5.00 (3H, m), 5.27 (1H, d), 5.35 (1H, d), 6.61 (1H, d), 6.76 (1H, d), 7.30 (1H, d), 7.38-7.44 (5H, m), 7.45 (1H, d), 7.83 (1H, t), 8.02 (1H, br d), 8.26 (1H, br d), 8.88 (1H, s), 12.75 (1H, br s) |
| 45 | 9 | ESI+: 1108.7 |
| 46 | 9 | ESI+: 1125.7 |
| 47 | 9 | ESI+: 1125.8 |
| 48 | 9 | ESI+: 1096.5 |
| 49 | 9 | ESI+: 1134.8 |

**[Table 8-4]**

| Ex | Syn | DAT |
|---|---|---|
| 50 | 50 | ESI+: 1121.7 |
| | | NMR (100°C): 0.53-0.64 (3H, m), 0.64-0.72 (1H, m), 0.77 (3H, br d), 1.07 (3H, d), 1.15 (3H, d), 1.26 (3H, t), 1.38-1.46 (1H, m), 1.87-2.01 (2H, m), 2.01 (3H, d), 2.01-2.10 (1H, m), 2.12 (1H, ddd), 2.21-2.33 (1H, m), 2.45 (3H, s), 2.50-2.60 (1H, m), 2.81-2.30 (2H, m), 3.06 (1H, ddd), 3.14 (1H, dd), 3.31 (3H, s), 3.56-3.64 (1H, m), 3.64-3.78 (4H, m), 3.84 (1H, br dd), 4.30 (1H, dd), 4.33-4.39 (2H, m), 4.41-4.48 (1H, m), 4.52 (1H, br t), 4.75 (1H, dt), 4.78-4.83 (1H, m), 4.86-4.96 (3H, m), 5.25 (1H, d), 5.28 (1H, d), 6.85 (2H, d), 7.31 (1H, d), 7.36-7.44 (5H, m), 7.48 (1H, d), 7.60 (2H, br d), 8.00 (1H, br d), 8.42 (1H, s), 8.88 (1H, s), 12.75 (1H, br s) |
| 51 | 9 | ESI+: 1093.5 |
| 52 | 9 | ESI+: 1094.5 |
| 53 | 53 | ESI+: 1107.8 |
| | | NMR (100°C): 0.51-0.69 (4H, m), 0.77 (3H, d), 1.07 (3H, d), 1.15 (3H, d), 1.38-1.44 (1H, m), 1.89-2.00 (2H, m), 2.01 (3H, d), 2.01-2.09 (1H, m), 2.10-2.18 (1H, m), 2.22-2.33 (1H, m), 2.45 (3H, s), 2.50-2.60 (1H, m), 2.83-3.07 (3H, m), 3.18 (1H, dd), 3.25 (3H, s), 3.30 (3H, s), 3.60 (1H, br d), 3.65-3.75 (3H, m), 3.84 (1H, br dd), 4.27-4.39 (3H, m), 4.45 (1H, br s), 4.52 (1H, t), 4.73-4.84 (1H, m), 4.86-4.97 (3H, m), 5.25 (1H, d), 5.28 (1H, br d), 6.85 (2H, d), 7.31 (1H, d), 7.38-7.45 (5H, m), 7.46 (1H, d), 7.60 (2H, br d), 8.00 (1H, br d), 8.42 (1H, s), 8.88 (1H, s), 12.75 (1H, br s) |
| 54 | 9 | ESI+: 1093.3 |

**[Table 8-5]**

| Ex | Syn | DAT |
|---|---|---|
| 55 | 9 | ESI+: 1104.4 |
| 56 | 9 | ESI+: 1107.9 |

**[Table 8-6]**

| Ex | Syn | DAT |
|---|---|---|
| 57 | 57 | ESI+: 1118.5 |

As examples of specific compounds of the formula (I) and the formula (IA) included in the present invention, compounds having any of the following structures are shown. These compounds can be produced also by the typical production methods shown above, the production methods of the Production Examples and the Examples, a combination of the production methods or a method that is obvious to a person skilled in the art.

These compounds are expected to be excellent in the degradation-inducing action on a G12D mutant KRAS protein and useful as a G12D mutant KRAS inhibitor and can be used as an active ingredient of a pharmaceutical composition, for example, a pharmaceutical composition for treating pancreatic cancer.

**[Table 9-1]**

| No. | Str |
|---|---|
| AA1 | 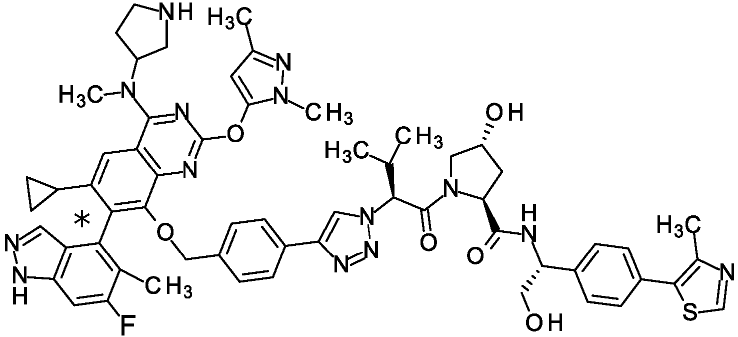 |
| AA2 | 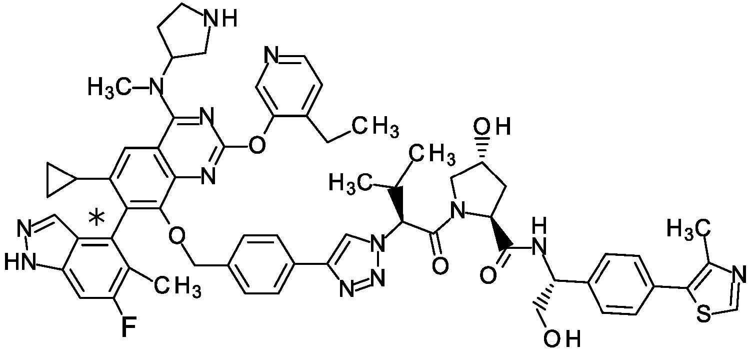 |
| AA3 | 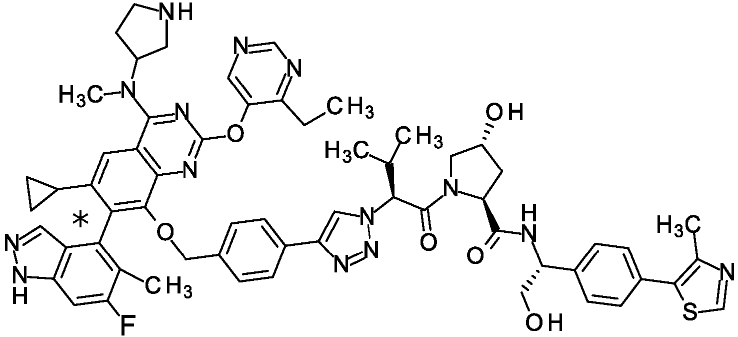 |

**[Table 9-2]**

| No. | Str |
|---|---|
| AA4 | 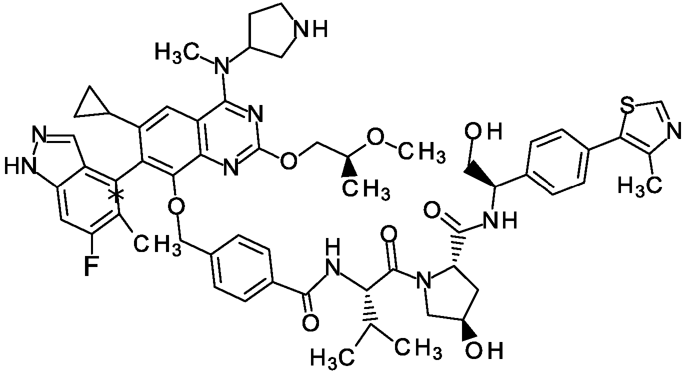 |
| AA5 | 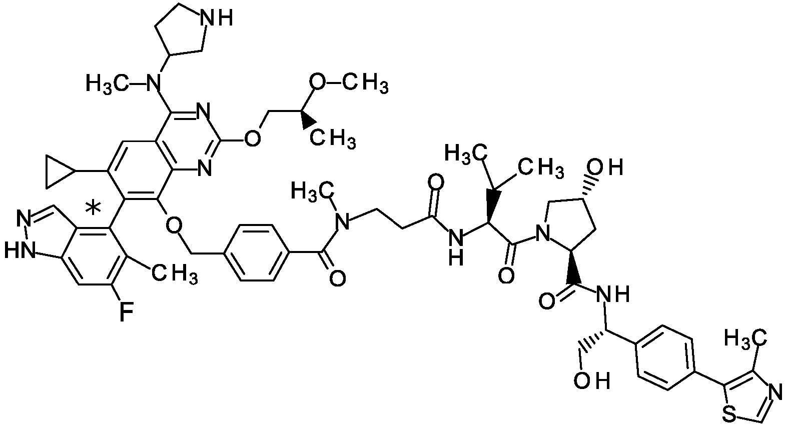 |
| AA6 | 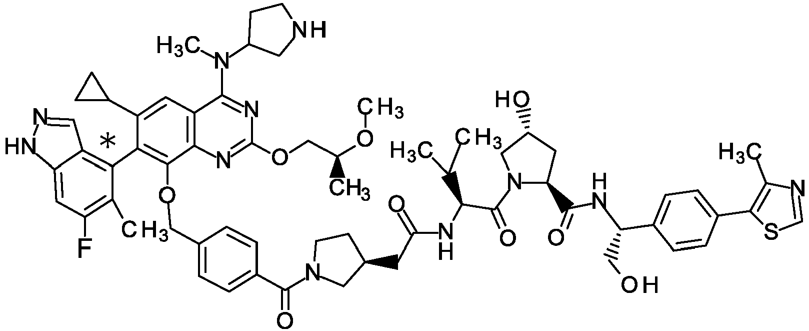 |

**[Table 9-3]**

| No. | Str |
|---|---|
| AA7 | 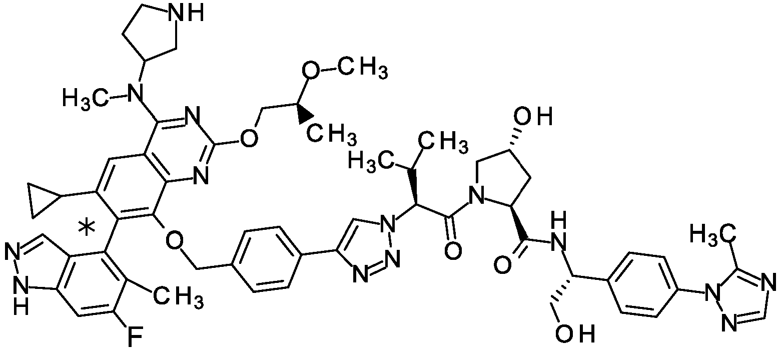 |
| AA8 | 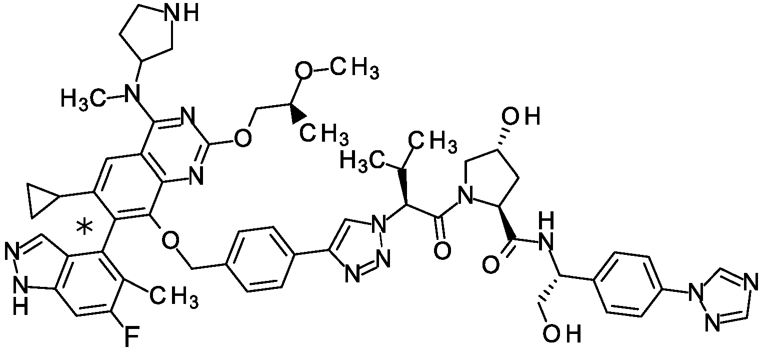 |
| AA9 | 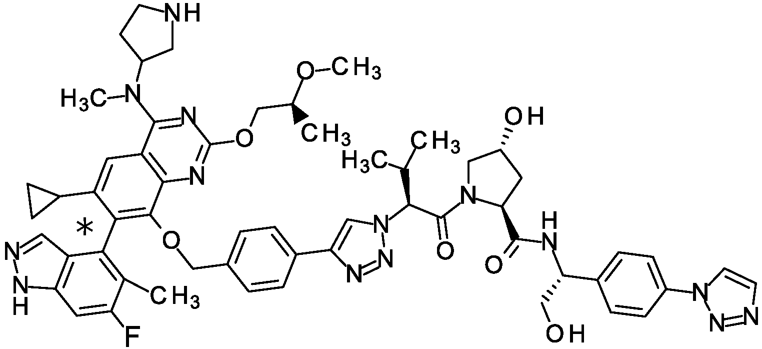 |

**[Table 9-4]**

| No. | Str |
|---|---|
| AA10 | 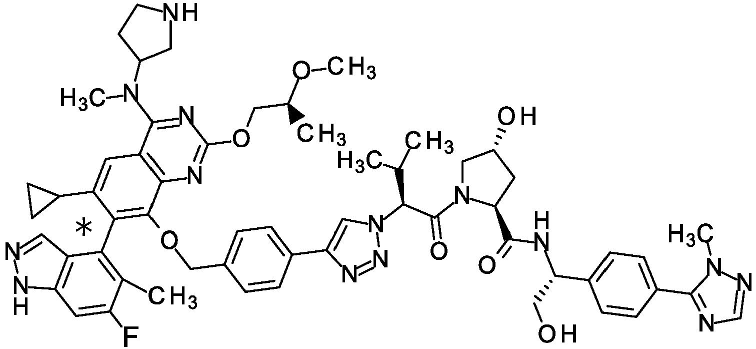 |
| AA11 | 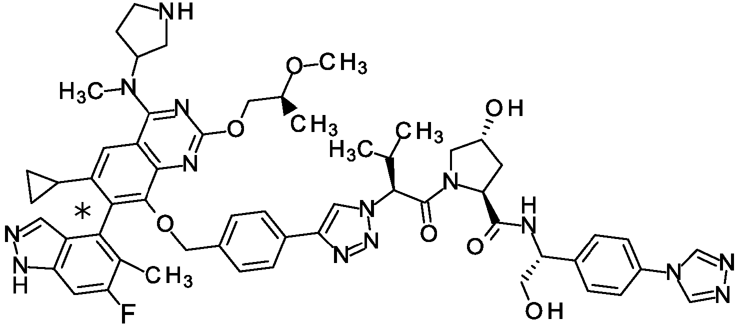 |
| AA12 | 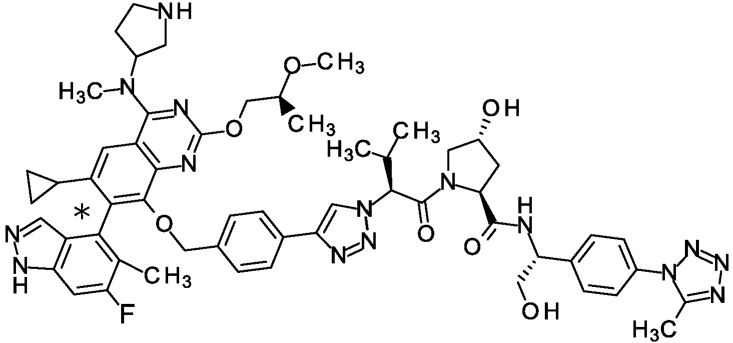 |

**[Table 9-5]**

| No. | Str |
|---|---|
| AA13 | 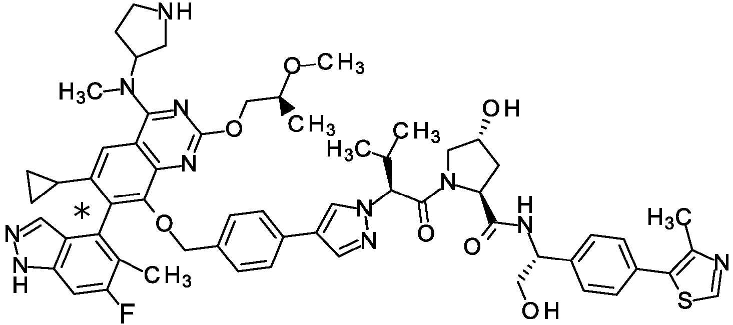 |
| AA14 | 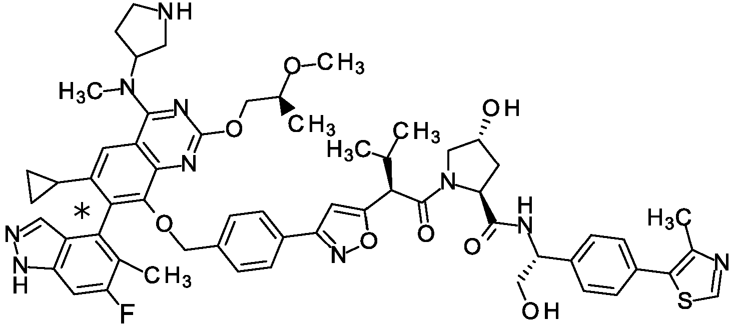 |
| AA15 | 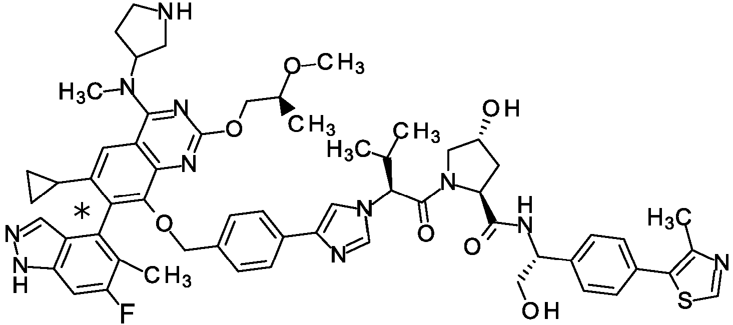 |

**[Table 9-6]**

| No. | Str |
|---|---|
| AA16 | 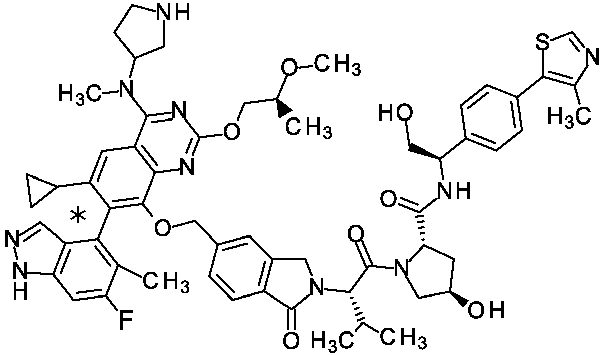 |
| AA17 | 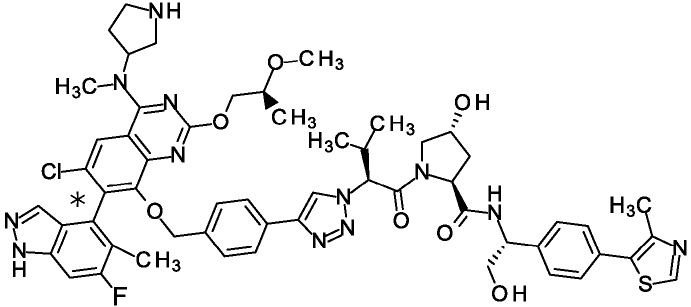 |
| AA18 | 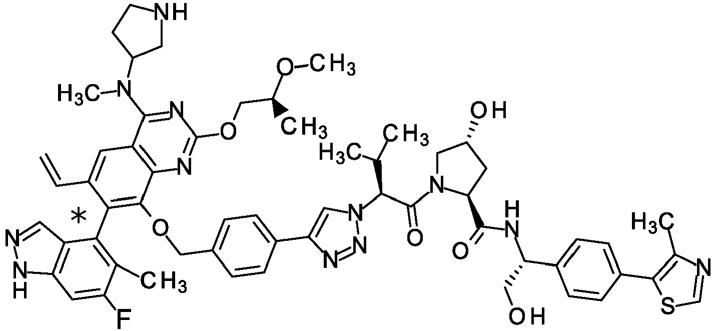 |

**[Table 9-7]**

| No. | Str |
|---|---|
| AA19 | 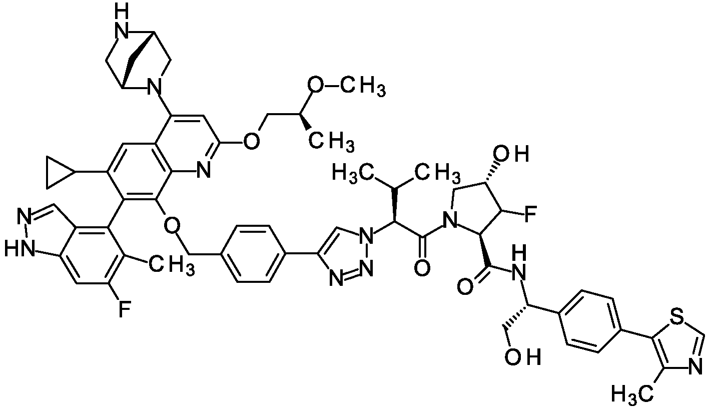 |
| AA20 | 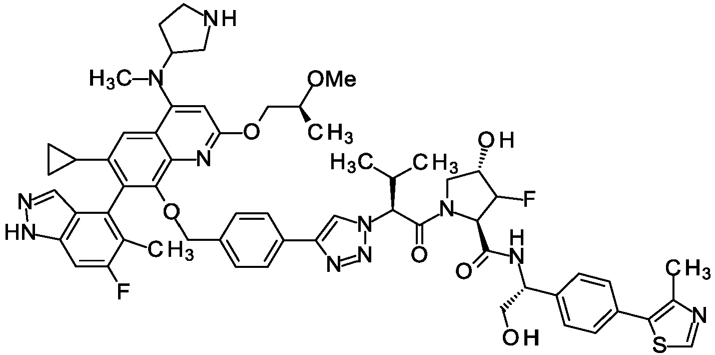 |
| AA21 | 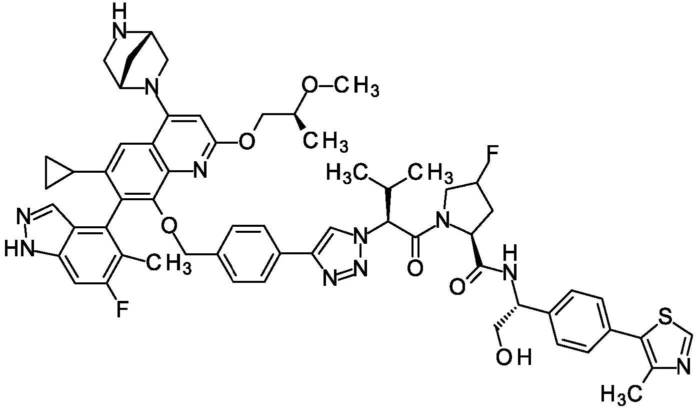 |

**[Table 9-8]**

| No. | Str |
|---|---|
| AA22 | 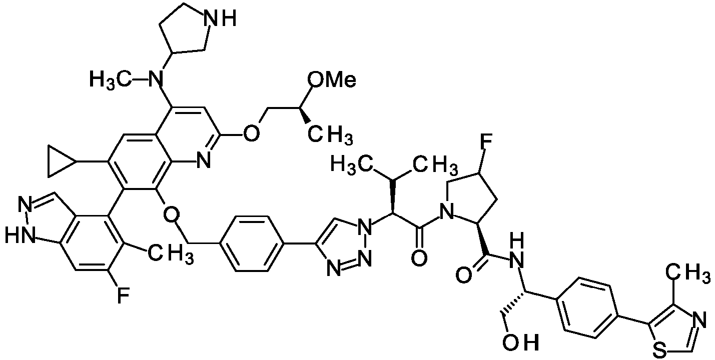 |
| AA23 | 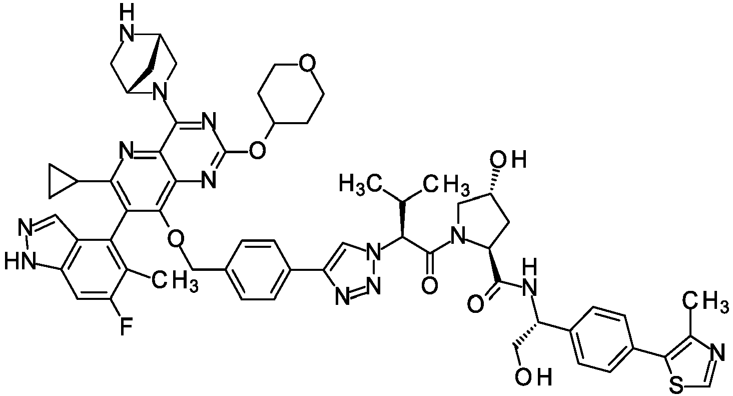 |
| AA24 | 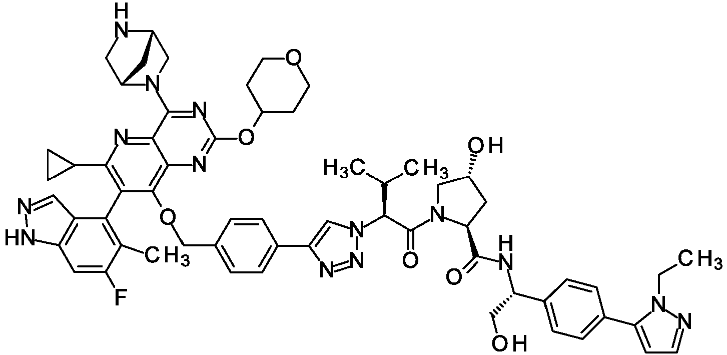 |

**[Table 9-9]**

| No. | Str |
|---|---|
| AA25 | 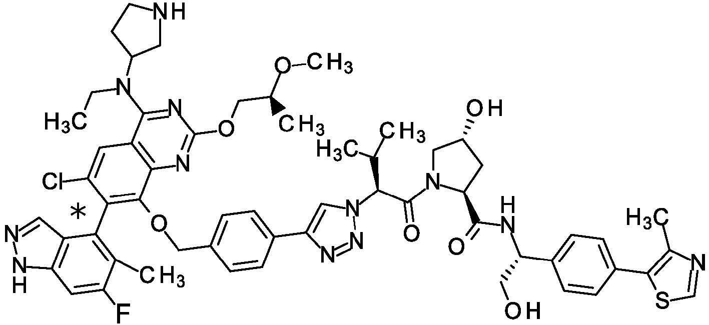 |
| AA26 | 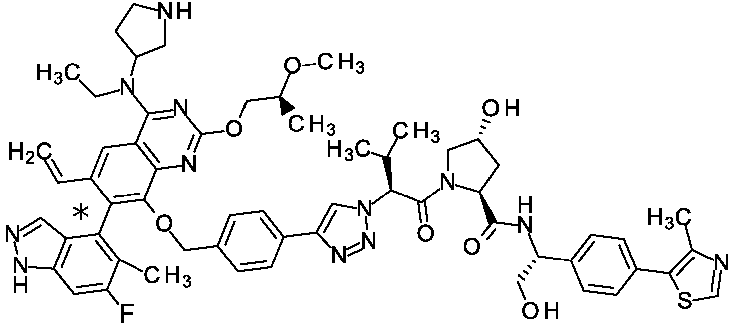 |
| AA27 | 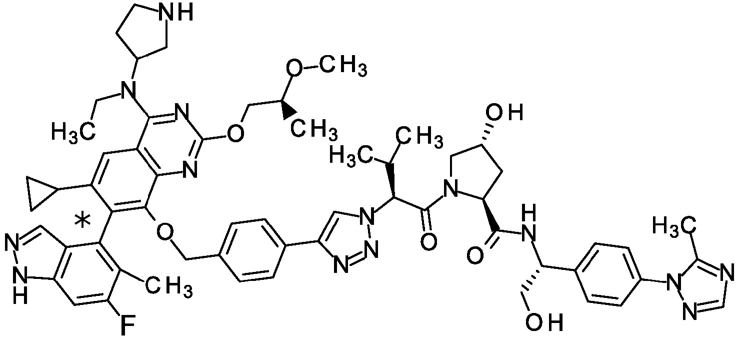 |

**[Table 9-10]**

| No. | Str |
|---|---|
| AA28 | 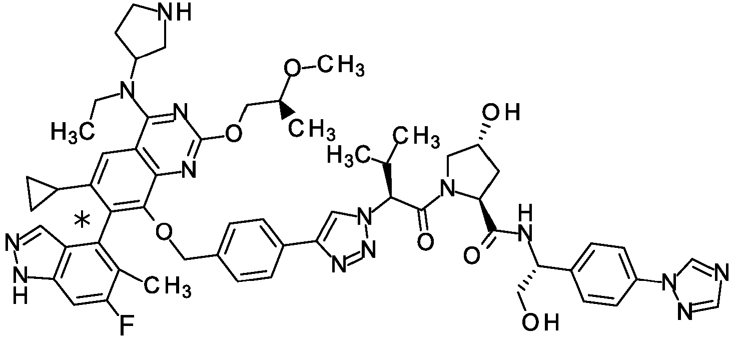 |
| AA29 | 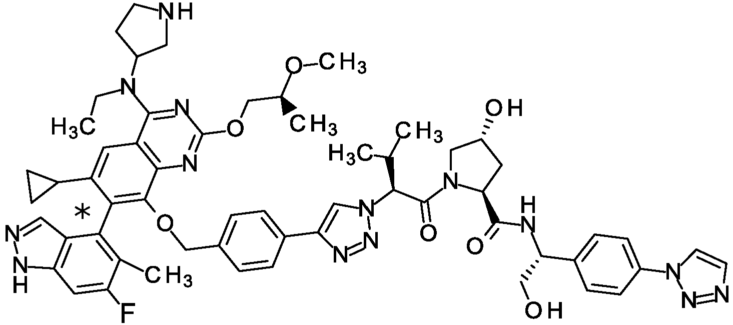 |
| AA30 | 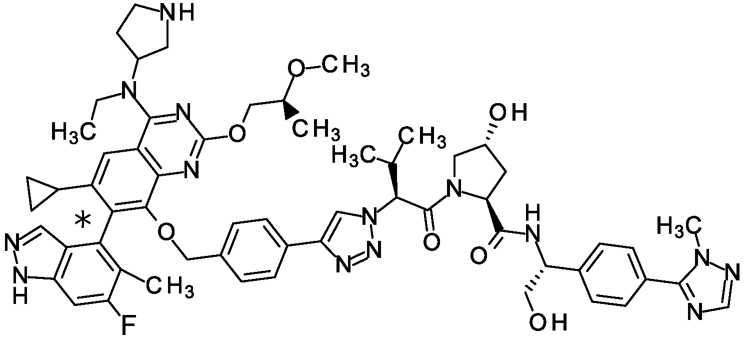 |

**[Table 9-11]**

| No. | Str |
|---|---|
| AA31 | 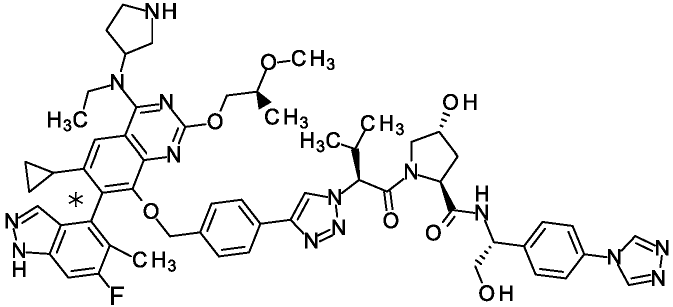 |
| AA32 | 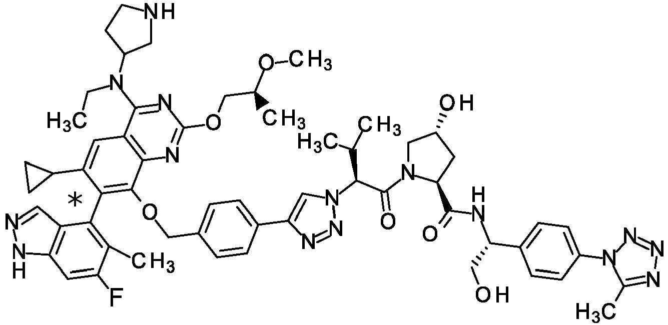 |
| AA33 | 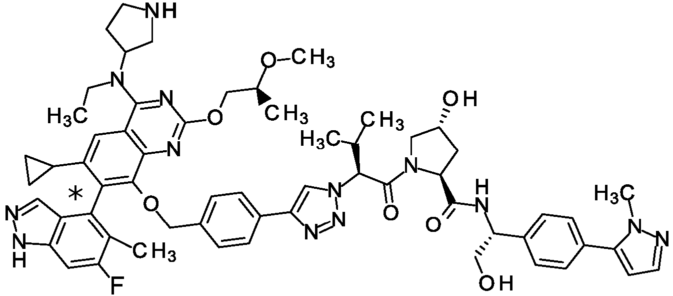 |
| AA34 | 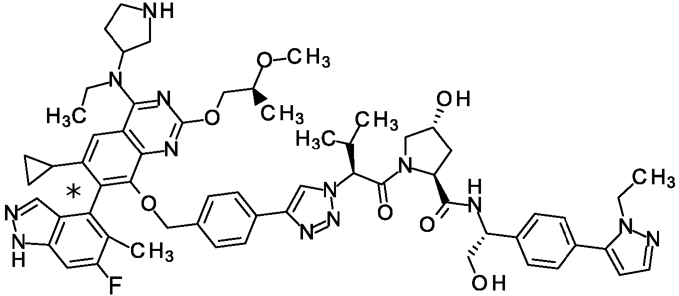 |

### INDUSTRIAL APPLICABILITY

The compound or a salt thereof of the present invention is excellent in the degradation-inducing action on a G12D mutant KRAS protein, is useful as a G12D mutant KRAS inhibitor and can be used as an active ingredient of a pharmaceutical composition, for example, a pharmaceutical composition for treating pancreatic cancer.

## Claims

1. A compound of the formula (IA) or a salt thereof, 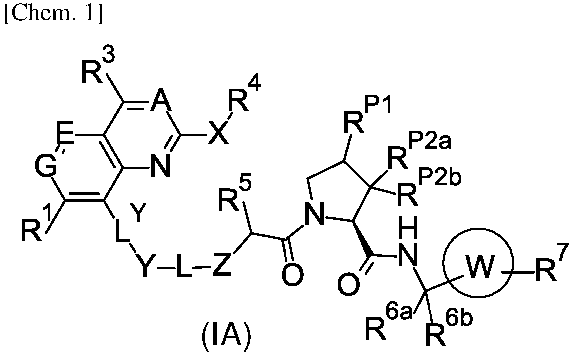 (wherein in the formula,
A is CR^{A} or N,
R^{A} is H or C₁₋₃ alkyl,
E is CH or N,
G is CR² or N, and
R¹ is naphthyl optionally substituted with OH or a group selected from the group consisting of the formula (II), the formula (III) and the formula (IV) below, 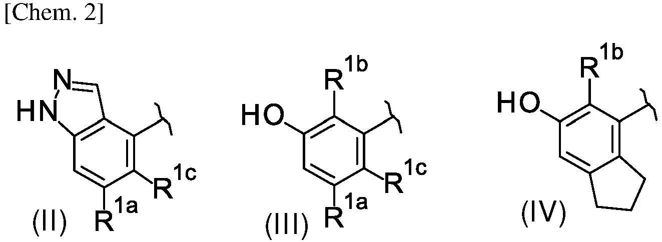
R^{1a} and R^{1b}, which are the same as or different from each other, are H, methyl, F or Cl,
R^{1c} is F, Cl, methyl or ethyl,
R² is H, halogen, optionally substituted C₁₋₃ alkyl, cyclopropyl or vinyl,
R³ is -P-Q or V,
P is -CH₂-, -O- or -N(R^{P})-,
R^{P} is H or optionally substituted C₁₋₃ alkyl, and
Q is the formula (V) or the formula (VI) below, 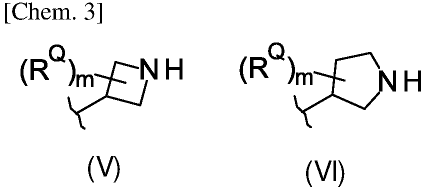
V is the formula (VII) below, 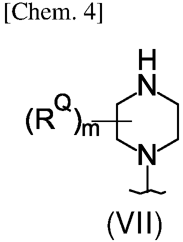
each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
(i) when A is CR^{A} or (ii) when A is N, and E or G is N,
V may be a 7-membered or 8-membered saturated or unsaturated bridged heterocyclic group in addition to the formula (VII),
m is 0 to 2,
R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms,
R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen,
R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen,
R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms,
L^{Y} is -O-(optionally substituted C₁₋₃ alkylene)-, -S-(optionally substituted C₁₋₃ alkylene)-, -SO₂-(optionally substituted C₁₋₃ alkylene)-, -NR^{Y}-(optionally substituted C₁₋₃ alkylene)-, -(optionally substituted C₁₋₃ alkylene)-O-, -(optionally substituted C₁₋₃ alkylene)-S-, -(optionally substituted C₁₋₃ alkylene)-SO₂- or -(optionally substituted C₁₋₃ alkylene)-NR^{Y}-,
R^{Y} is H or C₁₋₃ alkyl,
R^{P1} is OH or F,
R^{P2a} is H or F,
R^{P2b} is H,
W is optionally substituted phenylene or 6-membered optionally substituted heteroarenediyl containing one to three nitrogen atoms,
X is a bond, -CH₂-, -O-, -S- or -NR^{4x}-,
R^{4x} is H or C₁₋₃ alkyl,
Y is phenylene or pyridinediyl, wherein the phenylene may be substituted with F,
L is -(L¹-L²-L³-L⁴)-,
L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
R^{L1} is H or C₁₋₃ alkyl, and
Z is NH or 5-membered heteroarenediyl containing one to four hetero atoms selected from the group consisting of oxygen, sulfur and nitrogen,
or Y-L-Z is the formula (VIII) below.)

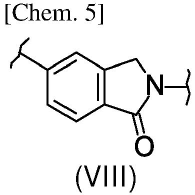

2. A compound of the formula (I) or a salt thereof, 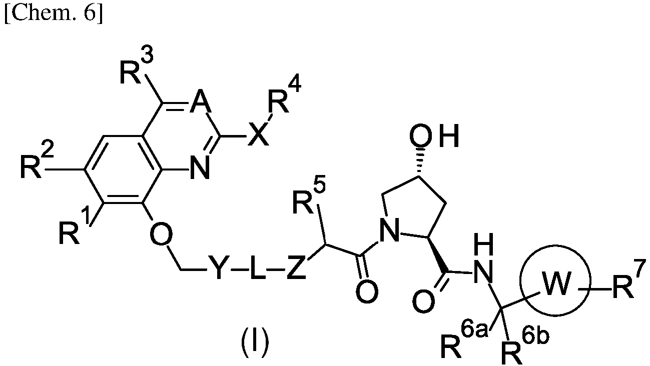 (wherein in the formula,
A is CR^{A} or N,
R^{A} is H or C₁₋₃ alkyl, and
R¹ is naphthyl optionally substituted with OH or a group selected from the group consisting of the formula (II), the formula (III) and the formula (IV) below, 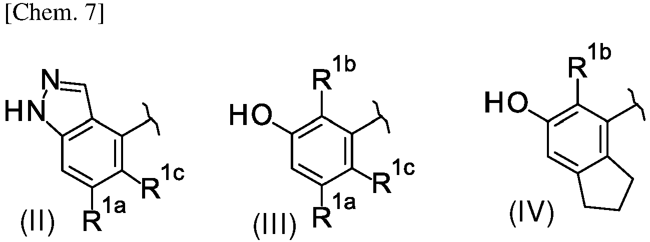
R^{1a} and R^{1b}, which are the same as or different from each other, are H, methyl, For Cl,
R^{1c} is F, Cl, methyl or ethyl,
R² is H, halogen, optionally substituted C₁₋₃ alkyl, cyclopropyl or vinyl,
R³ is -P-Q or V,
P is -CH₂-, -O- or -N(R^{P})-,
R^{P} is H or optionally substituted C₁₋₃ alkyl, and
Q is the formula (V) or the formula (VI) below, 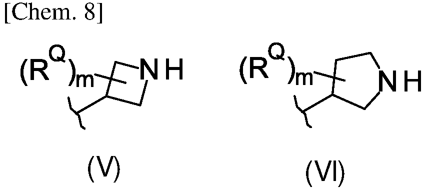
V is the formula (VII) below, 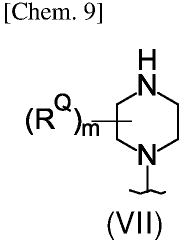
each R^{Q}, which is the same as or different from each other, are OH or C₁₋₃ alkyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII),
m is 0 to 2,
R⁴ is optionally substituted C₁₋₆ alkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered optionally substituted heteroaryl containing one to three nitrogen atoms,
R⁵ is optionally substituted C₁₋₆ alkyl, optionally substituted C₃₋₆ cycloalkyl or a 4-membered to 6-membered optionally substituted saturated heterocyclic group containing one hetero atom selected from the group consisting of oxygen, sulfur and nitrogen,
R^{6a} and R^{6b}, which are the same as or different from each other, are H or optionally substituted C₁₋₆ alkyl, or R^{6a} and R^{6b} may form together with the carbon to which they are attached, optionally substituted C₃₋₆ cycloalkyl or a 4-membered to 6-membered optionally substituted saturated heterocyclic group containing one hetero atom selected from the group consisting of oxygen, sulfur and nitrogen,
R⁷ is H, halogen, C₁₋₃ alkyl, -SO₂CH₃, C₃₋₆ cycloalkyl, a 4-membered to 6-membered optionally substituted saturated heterocyclic group containing one or two hetero atoms selected from the group consisting of oxygen, sulfur and nitrogen, 5-membered optionally substituted heteroaryl containing one to four hetero atoms selected from the group consisting of oxygen, sulfur and nitrogen or 6-membered heteroaryl containing one to three nitrogen atoms,
W is optionally substituted phenylene or 6-membered optionally substituted heteroarenediyl containing one to three nitrogen atoms,
X is a bond, -CH₂-, -O-, -S- or -NR^{4x}-,
R^{4x} is H or C₁₋₃ alkyl,
Y is phenylene or pyridinediyl, wherein the phenylene may be substituted with F,
L is -(L¹-L²-L³-L⁴)-,
L¹, L², L³ and L⁴, which are the same as or different from each other, are a group selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
R^{L1} is H or C₁₋₃ alkyl, and
Z is NH or 5-membered heteroarenediyl containing one to four hetero atoms selected from the group consisting of oxygen, sulfur and nitrogen,
or Y-L-Z is the formula (VIII) below.)

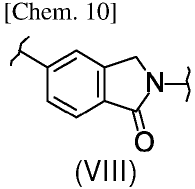

3. The compound or a salt thereof according to claim 2,
Y is phenylene or pyridinediyl, wherein the phenylene may be substituted with F,
L is a bond, C₁₋₃ alkylene, C=O or a group selected from the group consisting of the formula (XX), the formula (XXI), the formula (XXII), the formula (XXIII), the formula (XXIV) and the formula (XXV) below, 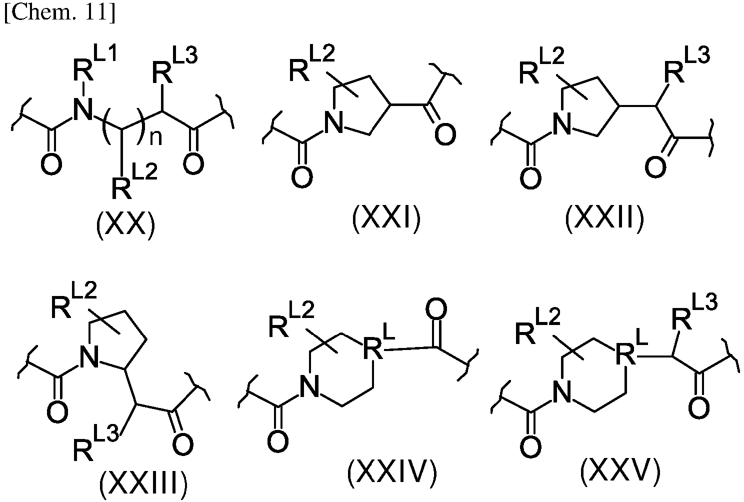
R^{L1} is H or C₁₋₃ alkyl,
R^{L2} and R^{L3}, which are the same as or different from each other, are H, F, OH, OCH₃ or optionally substituted C₁₋₃ alkyl,
R^{L} is CH or N,
n is an integer of one or two, and
Z is NH or a group selected from the group consisting of the formula (XXVI), the formula (XXVII), the formula (XXVIII) and the formula (XXIX) below, 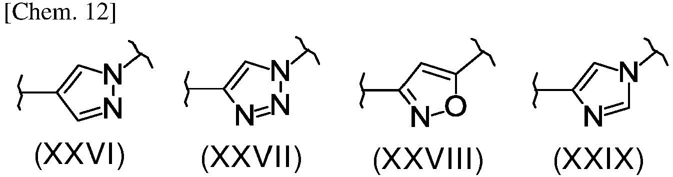
or Y-L-Z is the formula (VIII) below.

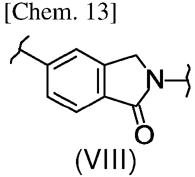

4. The compound or a salt thereof according to claim 3, wherein R¹ is the formula (IIa) or the formula (IIIa) below, 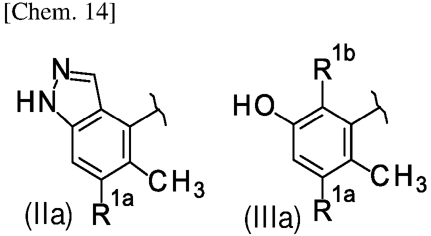
R^{1a} and R^{1b}, which are the same as or different from each other, are H, methyl, For Cl,
R³ is as follows,
(i) when A is CR^{A}, R³ is -P-Q, and
P is -O-, or
(ii) when A is N, R³ is -P-Q or V, P is -O- or -N(R^{P})-, and
R^{P} is H or C₁₋₃ alkyl,
in either case (i) or (ii), Q is the formula (V) or the formula (VI) below, 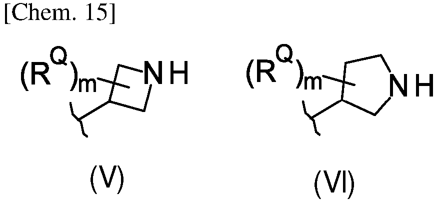
V is the formula (VII) below, 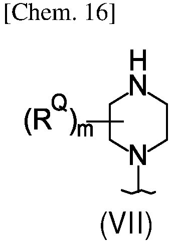
each R^{Q}, which is the same as or different from each other, are OH or methyl,
wherein R^{Q'} is attached only to a carbon atom that is an atom forming a ring selected from the group consisting of an azetidine ring represented by the formula (V), a pyrrolidine ring represented by the formula (VI) and a piperazine ring represented by the formula (VII), and
m is 0 to 2,
R⁴ is optionally substituted C₁₋₆ alkyl, a 4-membered to 6-membered optionally substituted saturated heterocyclic group containing one or two hetero atoms selected from the group consisting of oxygen, sulfur and nitrogen, optionally substituted pyrazolyl, optionally substituted pyridyl or optionally substituted pyrimidinyl,
R⁵ is methyl, ethyl, isopropyl, tert-butyl or C₃₋₆ cycloalkyl,
R^{6a} and R^{6b}, which are the same as or different from each other, are H or C₁₋₃ alkyl, wherein the C₁₋₃ alkyl may be substituted with a group selected from the group consisting of F, OH, OCH₃ and N(CH₃)₂, or R^{6a} and R^{6b} may form C₃₋₆ cycloalkyl together with the carbon to which they are attached,
R⁷ is H, halogen, C₁₋₃ alkyl, -SO₂CH₃, C₃₋₆ cycloalkyl or a group selected from the group consisting of the formula (IX), the formula (X), the formula (XI), the formula (XII), the formula (XIII), the formula (XIV), the formula (XV), the formula (XVI), the formula (XVII) and the formula (XVIII) below, 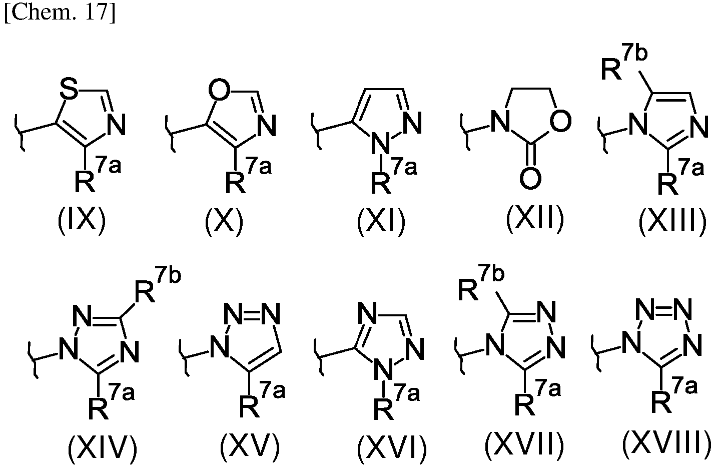
R^{7a} and R^{7b}, which are the same as or different from each other, are H or C₁₋₃ alkyl optionally substituted with OH, and
W is the following formula (XIX), 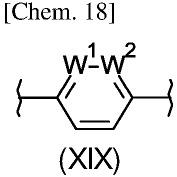
W¹ and W² are as follows,
(i) W¹ is CH, and W² is C-SO₂CH₃, or
(ii) W¹ and W², which are the same as or different from each other, are CH, CF, CCl, CCH₃ or N,
wherein when W¹ is CH and W² is C-SO₂CH₃, R⁷ is H,
X is - O or - NR^{4x}-,
R^{4x} is H or C₁₋₃ alkyl,
Y is phenylene or pyridinediyl, wherein the phenylene may be substituted with F,
L is a bond, C=O or a group selected from the group consisting of the formula (XX) and the formula (XXII) below, 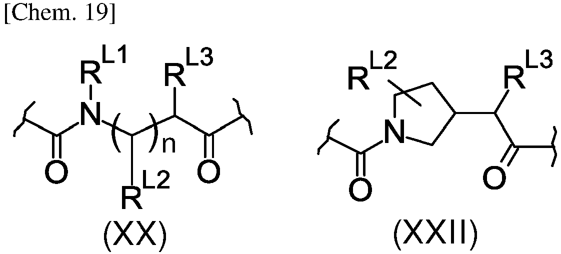
R^{L1} is H or C₁₋₃ alkyl,
R^{L2} and R^{L3}, which are the same as or different from each other, are H, F, OH, OCH₃ or optionally substituted C₁₋₃ alkyl,
n is an integer of one or two, and
Z is NH or a group selected from the group consisting of the formula (XXVI), the formula (XXVII), the formula (XXVIII) and the formula (XXIX) below, 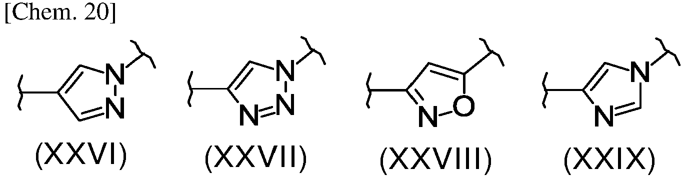
or Y-L-Z is the formula (VIII) below,

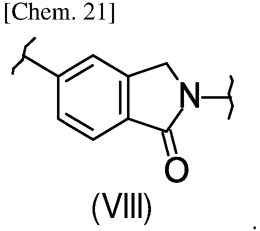

5. The compound or a salt thereof according to claim 4, wherein A is CH or N,
R² is halogen, C₁₋₃ alkyl, cyclopropyl or vinyl, wherein the C₁₋₃ alkyl may be substituted with a group selected from the group consisting of OH and OCH₃,
R³ is as follows,
(i) when A is CH, R³ is -P-Q, and
P is -O-, or
(ii) when A is N, R³ is -P-Q or V,
P is -O- or -N(R^{P})-,
R^{P} is H or C₁₋₃ alkyl,
in either case (i) or (ii), Q is the formula (V) or the formula (VI) below, 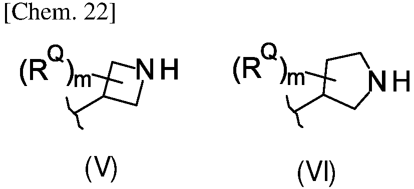
V is the formula (VII) below, 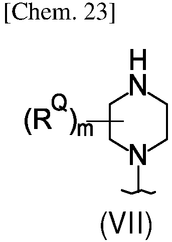
each R^{Q}, which is the same as or different from each other, are OH or methyl, wherein R^{Q} is attached only to a carbon atom that is an atom forming a ring selected from the group consisting of an azetidine ring represented by the formula (V), a pyrrolidine ring represented by the formula (VI) and a piperazine ring represented by the formula (VII), and
m is 0 to 2,
R⁴ is C₁₋₆ alkyl optionally substituted with a group selected from the group consisting of F, OH, OCH₃, CF₃, CHF₂, optionally substituted cyclopropyl, optionally substituted pyrrolidinyl and optionally substituted tetrahydrofuranyl or tetrahydropyranyl, and
R⁷ is halogen or a group selected from the group consisting of the formula (IX), the formula (X), the formula (XI), the formula (XII), the formula (XIII) and the formula (XIV) below, 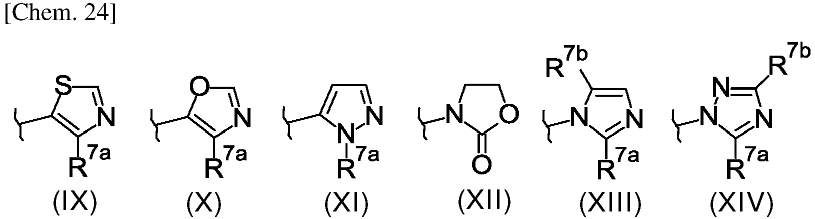
R^{7a} and R^{7b}, which are the same as or different from each other, are H or C₁₋₃ alkyl optionally substituted with OH,
The compound or a salt thereof in which W is the following formula (XIX), 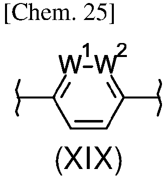
W¹ and W², which are the same as or different from each other, are CH or N,
X is -O- or -NH-,
Y is phenylene or pyridinediyl, wherein the phenylene may be substituted with F,
L is a bond, and
Z is the formula (XXVII) below,

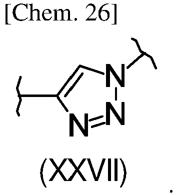

6. The compound or a salt thereof according to claim 4,
wherein A is N,
R³ is -P-Q,
P is -O- or -N(R^{P})-,
R^{P} is H or C₁₋₃ alkyl, and
Q is the formula (V) or the formula (VI) below, 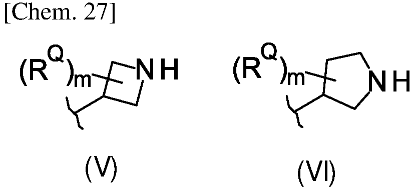
each R^{Q}, which is the same as or different from each other, are OH or methyl,
wherein R^{Q} is attached only to a carbon atom that is an atom forming a ring selected from the group consisting of an azetidine ring represented by the formula (V) and a pyrrolidine ring represented by the formula (VI),
m is 0 to 2, and
W is the following formula (XIX), 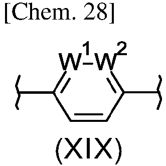
W¹ and W², which are the same as or different from each other, are CH, CF, CCl, CCH₃ or N,
Y is phenylene or pyridinediyl, wherein the phenylene may be substituted with F,
L is a bond, C=O or a group selected from the group consisting of the formula (XX) and the formula (XXII) below, 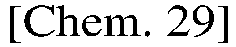 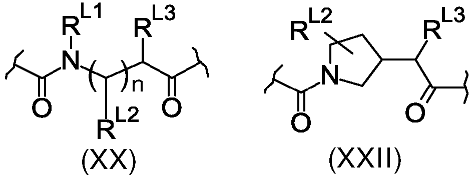
R^{L1} is H or C₁₋₃ alkyl,
R^{L2} and R^{L3}, which are the same as or different from each other, are H, F, OH, OCH₃ or optionally substituted C₁₋₃ alkyl,
n is an integer of one or two, and
Z is NH or a group selected from the group consisting of the formula (XXVI), the formula (XXVII), the formula (XXVIII) and the formula (XXIX) below,

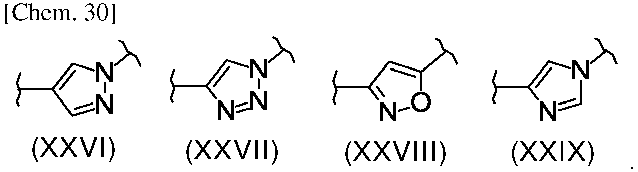

7. The compound or a salt thereof according to claim 4,
wherein A is CH,
R³ is -P-Q,
P is -O-, and
Q is the formula (V) or the formula (VI) below, 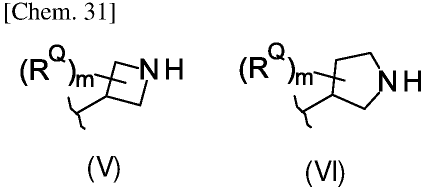
each R^{Q}, which is the same as or different from each other, are OH or methyl,
wherein R^{Q} is attached only to a carbon atom that is an atom forming a ring selected from the group consisting of an azetidine ring represented by the formula (V) and a pyrrolidine ring represented by the formula (VI),
m is 0 to 2, and
W is the following formula (XIX), 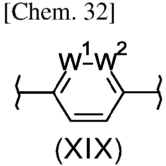
W¹ and W², which are the same as or different from each other, are CH, CF, CCl, CCH₃ or N,
Y is phenylene or pyridinediyl, wherein the phenylene may be substituted with F, and
L is a bond, C=O or a group selected from the group consisting of the formula (XX) and the formula (XXII) below, 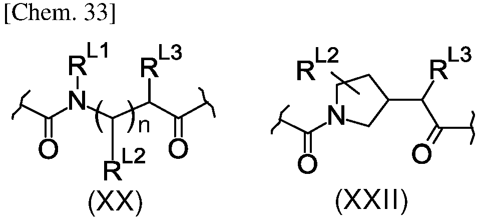
R^{L1} is H or C₁₋₃ alkyl,
R^{L2} and R^{L3}, which are the same as or different from each other, are H, F, OH, OCH₃ or
optionally substituted C₁₋₃ alkyl,
n is an integer of one or two, and
Z is NH or a group selected from the group consisting of the formula (XXVI), the formula (XXVII), the formula (XXVIII) and the formula (XXIX) below,

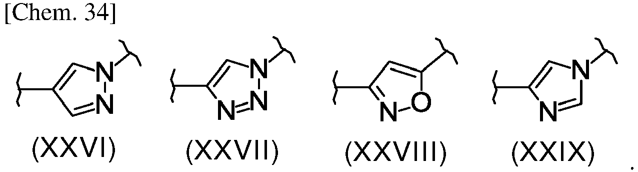

8. The compound or a salt thereof according to claim 4,
wherein A is N,
R³ is V, and
V is the formula (VII) below, 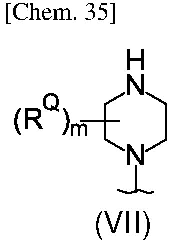
each R^{Q}, which is the same as or different from each other, are OH or methyl,
wherein R^{Q} is attached only to a carbon atom that is a ring-forming atom of a piperazine ring represented by the formula (VII),
m is 0 to 2, and
W is the following formula (XIX), 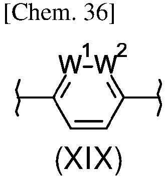
W¹ and W², which are the same as or different from each other, are CH, CF, CCl, CCH₃ or N,
Y is phenylene or pyridinediyl, wherein the phenylene may be substituted with F, and
L is a bond, C=O or a group selected from the group consisting of the formula (XX) and the formula (XXII) below, 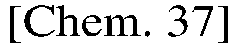 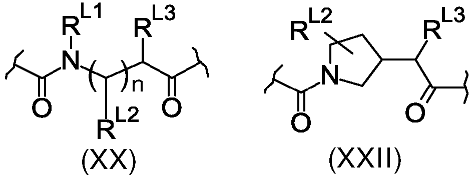
R^{L1} is H or C₁₋₃ alkyl,
R^{L2} and R^{L3}, which are the same as or different from each other, are H, F, OH, OCH₃ or optionally substituted C₁₋₃ alkyl,
n is an integer of one or two, and
Z is NH or a group selected from the group consisting of the formula (XXVI), the formula (XXVII), the formula (XXVIII) and the formula (XXIX) below,

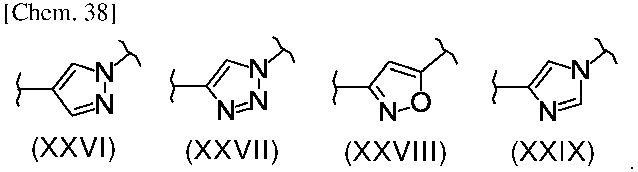

9. The compound or a salt thereof according to claim 5, wherein A is N, and
R¹ is the formula (IIb) below, 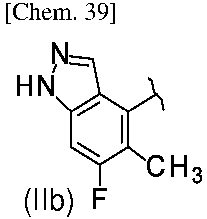
R³ is -P-Q,
P is -O- or -N(R^{P})-,
R^{P} is H or C₁₋₃ alkyl, and
Q is the formula (V) or the formula (VI) below, 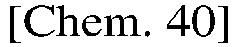 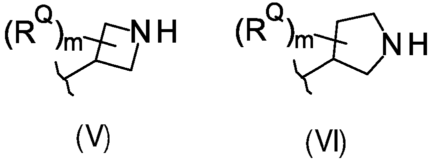
each R^{Q}, which is the same as or different from each other, are OH or methyl,
wherein R^{Q} is attached only to a carbon atom that is an atom forming a ring selected from the group consisting of an azetidine ring represented by the formula (V) and a pyrrolidine ring represented by the formula (VI),
m is 0 or 1, and
X is -O-.

10. The compound or a salt thereof according to claim 9,
wherein R² is cyclopropyl,
R³ is -P-Q,
P is -N(R^{P})-,
R^{P} is H or C₁₋₃ alkyl, and
Q is the formula (V) or the formula (VI) below, 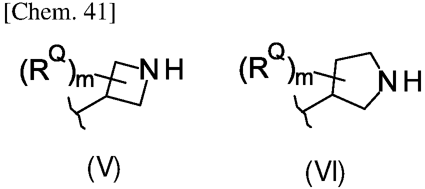
each R^{Q}, which is the same as or different from each other, are OH or methyl,
wherein R^{Q} is attached only to a carbon atom that is an atom forming a ring selected from the group consisting of an azetidine ring represented by the formula (V) and a pyrrolidine ring represented by the formula (VI),
m is 0 or 1,
R⁴ is C₁₋₆ alkyl optionally substituted with a group selected from the group consisting of OCH₃ and a tetrahydrofuranyl group or tetrahydropyranyl,
R⁵ is isopropyl,
R^{6a} is H, and R^{6b} is C₁₋₃ alkyl optionally substituted with OH, and
R⁷ is the formula (IX), the formula (X), the formula (XI) or the formula (XII) below, 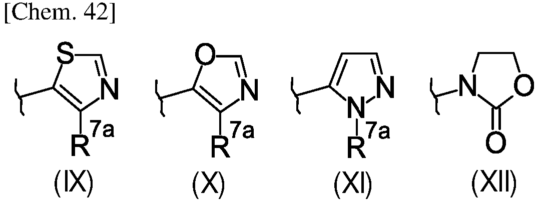
R^{7a} is C₁₋₃ alkyl optionally substituted with OH, and
W is the following formula (XIX), 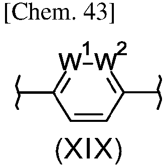
W¹ and W² are each CH, and
Y is phenylene optionally substituted with F.

11. The compound or a salt thereof according to claim 1 or 2,
wherein compounds of the formula (I) and the formula (IA) are selected from the group consisting of
(4R)-1-[(2S)-2-{4-[4-({[(7M)-6-cyclopropyl-7-(6-fluoro-5-methyl-1H-indazol-4-yl)-4-{ methyl[(3S)-pyrrolidin-3-yl] amino }-2-{ [(2R)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({[(7M)-6-cyclopropyl-7-(6-fluoro-5-methyl-1H-indazol-4-yl)-4-{ methyl[(3S)-pyrrolidin-3-yl] amino }-2-{ [(2S)-oxolan-2-yl] methoxy} quinazolin-8-yl}oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-t4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide,
(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol- 5- yl)phenyl] - 2-hydroxyethyl} -4-hydroxy- L- prolinamide,
(4R)-1-[(2S)-2-{4-[4-({[(7M)-6-cyclopropyl-7-(6-fluoro-5-methyl-1H-indazol-4-yl)-2-[(2S)-2-methoxypropoxy] -4- { methyl[(3S)-pyrrolidin-3-yl] amino } quinazolin-8-yl]oxy}methyl}methyl)-2-fluorophenyl]-1H-1,2,3-triazol-1-yl}-3-methylbutanoyl]-4-hydroxy-N-{ (1R)-2-hydroxy-1-[4-(4-methyl-1,3-thiazol-5-yl)phenyl]ethyl}-L-prolinamide,
(4R)-1-[(2S)-2-(4-{4-[({(7M)-6-cyclopropyl-4-{ethyl[(3S)-pyrrolidin-3-yl]amino}-7-(6-fluoro-5-methyl-1H-indazol-4-yl)-2-[(2S)-2-methoxypropoxy]quinazolin-8-yl}oxy)methyl)phenyl}-1H-1,2,3-triazol-1-yl)-3-methylbutanoyl] -4-hydroxy-N- { (1R)-2-hydroxy-1-[4-(4-methyl-1,3-thiazol-5-yl)phenyl]ethyl}-L-prolinamide, and
(4R)-1-[(2S)-2-{4-[4-({[(7M)-6-cyclopropyl-7-(6-fluoro-5-methyl-1H-indazol-4-yl)-2-[(2S)-2-methoxypropoxy] -4- { methyl[(3R)-pyrrolidin-3-yl] amino } quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide.

12. A pharmaceutical composition comprising the compound or a salt thereof according to claim 1 or 2 and one or more pharmaceutically acceptable excipients.

13. The pharmaceutical composition according to claim 12, which is a pharmaceutical composition for treating pancreatic cancer.

14. Use of the compound or a salt thereof according to claim 1 or 2 for the manufacture of a pharmaceutical composition for treating pancreatic cancer.

15. The compound or a salt thereof according to claim 1 or 2 for use in treatment of pancreatic cancer.

16. Use of the compound or a salt thereof according to claim 1 or 2 for treatment of pancreatic cancer.

17. A method for treating pancreatic cancer, the method comprising administering an effective amount of the compound or a salt thereof according to claim 1 or 2 to a subject.
